(19) Europäisches Patentamt
European Patent Office
Office européen des brevets

(11) EP 4 063 433 B1

(12) **EUROPEAN PATENT SPECIFICATION**

(45) Date of publication and mention of the grant of the patent:
**04.09.2024 Bulletin 2024/36**

(21) Application number: **20888940.2**

(22) Date of filing: **18.03.2020**

(51) International Patent Classification (IPC):
***C08B 37/08*** $^{(2006.01)}$ ***C08F 8/14*** $^{(2006.01)}$
***C08F 8/34*** $^{(2006.01)}$ ***C08H 1/06*** $^{(2006.01)}$
***C08L 89/06*** $^{(2006.01)}$ ***C08B 37/00*** $^{(2006.01)}$
***C08L 51/02*** $^{(2006.01)}$ ***C08L 5/08*** $^{(2006.01)}$
***C08F 289/00*** $^{(2006.01)}$ ***C08F 265/06*** $^{(2006.01)}$
***C08F 261/04*** $^{(2006.01)}$ ***C08J 3/075*** $^{(2006.01)}$
***C08G 65/334*** $^{(2006.01)}$ ***C08F 251/00*** $^{(2006.01)}$
***A61L 31/14*** $^{(2006.01)}$ ***A61L 27/52*** $^{(2006.01)}$

(52) Cooperative Patent Classification (CPC):
(C-Sets available)
**C08G 65/3344; A61L 27/52; A61L 31/145; C08B 37/003; C08B 37/0069; C08B 37/0072; C08F 8/14; C08F 8/34; C08F 251/00; C08F 261/04; C08F 265/06; C08F 289/00; C08H 1/06; C08J 3/075; C08L 5/08;** (Cont.)

(86) International application number:
**PCT/CN2020/079823**

(87) International publication number:
**WO 2021/098099 (27.05.2021 Gazette 2021/21)**

(54) **HYDROGEL OF MERCAPTO-MODIFIED MACROMOLECULAR COMPOUND, AND PREPARATION METHOD THEREFOR AND USE THEREOF**

HYDROGEL AUS MERCAPTO-MODIFIZIERTER MAKROMOLEKULARER VERBINDUNG, HERSTELLUNGSVERFAHREN DAFÜR UND VERWENDUNG DAVON

HYDROGEL DE COMPOSÉ MACROMOLÉCULAIRE MODIFIÉ PAR MERCAPTO, PROCÉDÉ DE PRÉPARATION ASSOCIÉ ET UTILISATION CORRESPONDANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2019 CN 201911130069**

(43) Date of publication of application:
**28.09.2022 Bulletin 2022/39**

(73) Proprietor: **BLAFAR BIOTECHNOLOGY (HANGZHOU) LTD**
**Hangzhou, Zhejiang 310018 (CN)**

(72) Inventor: **WANG, Wenxin**
**A94W1k6 Dublin (IE)**

(74) Representative: **Huang, Liwei**
**Cäcilienstraße 12**
**40597 Düsseldorf (DE)**

(56) References cited:
**CN-A- 101 338 036** **CN-A- 108 676 178**
**US-A1- 2016 009 872** **US-A1- 2017 355 799**

- **HAHN S K ET AL: "Injectable hyaluronic acid microhydrogels for controlled release formulation of erythropoietin", JOURNAL OF BIOMEDICAL MATERIALS RESEARCH PART A, JOHN WILEY & SONS, US, vol. 80A, no. 4, 15 March 2007 (2007-03-15), pages 916 - 924, XP002719831, ISSN: 1549-3296, [retrieved on 20061027], DOI: 10.1002/JBM.A.30997**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SIMONE STICHLER ET AL.: "Thiol-ene Clickable Poly(glycidol) Hydrogels for Biofabrication", ANNALS OF BIOMEDICAL ENGINEERING, vol. 45, no. 1,, 13 May 2016 (2016-05-13), XP036125863, ISSN: 0090-6964, DOI: 10.1007/s10439-016-1633-3**

(52) Cooperative Patent Classification (CPC): (Cont.)
**C08L 51/02; C08L 89/06;** C08J 2305/08; C08J 2351/02; C08J 2389/06; C08J 2405/08; C08J 2489/06

C-Sets
**C08F 8/14, C08F 120/20;**
**C08F 8/14, C08F 216/06;**
**C08F 8/34, C08F 8/14, C08F 120/20;**
**C08F 8/34, C08F 8/14, C08F 216/06**

## Description

The present application claims priority to Chinese Patent Application No. 201911130069.5 filed to China National Intellectual Property Administration on Nov. 18, 2019 and entitled "HYDROGEL OF MERCAPTO-MODIFIED MACROMOLECULAR COMPOUND, AND PREPARATION METHOD THEREFOR AND USE THEREOF".

TECHNICAL FIELD

The present disclosure relates to the field of biomaterials, and particularly relates to a hydrogel of sulfhydryl-modified polymer compound and a preparation method therefor and use thereof.

BACKGROUND

Biomedical materials, also called biomaterials for short, are novel high-tech materials for diagnosing, treating, repairing or replacing diseased tissues and organs in organisms or enhancing the functions thereof. One of the key techniques of tissue engineering is to use biomaterials to prepare cell scaffolds which have good biocompatibility and can be degraded and absorbed by the body. Gel state is an intermediate state between solid and liquid, and a hydrogel refers to a hydrophilic cross-linked three-dimensional polymer network which can swell in water and can retain a large amount of water without dissolving, and the water content of the hydrogel can reach 90% or more. Hydrogel is an ideal biomaterial that, by itself or through simple modification, can have desirable physical and chemical properties that are similar to those of the natural extracellular matrix, and meanwhile, exhibit good permeability to oxygen, nutrients, cell metabolites, and water-soluble metal ions. The hydrophilic polymer for preparing the hydrogel is classified into a natural polymer and a synthetic polymer according to the source. The natural polymer includes collagen, gelatin, fibrin, polysaccharide and the like, and the synthetic polymer includes synthetic polypeptide, polyethylene glycol (PEG) and its derivatives, polymethylmethacrylate (PMMA) and its derivatives, poly(lactic-co-glycolic acid) (PLGA) and its derivatives, and the like. Injectable in situ cross-linked hydrogel has also received increasing attention in recent years. The injectable in situ cross-linked hydrogel is characterized in that it is in a flowable liquid state prior to injection while it can form a gel that fully conforms to the shape of a target site when injected into the target site. The injectable property not only makes the operation process simple and convenient, but also can avoid the pain of the patient caused by the implantation operation and greatly reduce the traumatic property of operation.

Among the natural polymers, hyaluronic acid (HA) has drawn the attention of researchers due to its excellent properties. Natural hyaluronic acid is a natural heteropolysaccharide composed of alternating units of D-glucuronic acid and N-acetylglucosamine. Followed by decades of research, hyaluronic acid was found to be widely present in connective tissues of humans and other vertebrates, for example, in tissues and organs such as the intercellular space, tissues of the deployable joints, umbilical cord, skin, cartilage, vascular wall, synovial fluid and cockscomb. Hyaluronic acid is a linear polymer polysaccharide with disaccharide repeating units in its structure. D-glucuronic acid in the repeating units is liked with N-acetylglucosamine through β -1,3 glycosidic bonds, and thousands of disaccharide repeating units are liked through β-1,4 glycosidic bonds to form a fully straight-chain and linear structure. Hyaluronic acid is generally present in the form of a sodium salt in the physiological state of the human body. The sodium hyaluronate and the gel thereof are widely applied in the fields such as orthopedics, gynecology and plastic surgery, and can also be applied in ophthalmic surgery as a carrier for ophthalmic preparations or directly as an ophthalmic preparation, that is, the sodium hyaluronate products also have important applications in ophthalmic surgery. Sodium hyaluronate is also an important constituent of the synovial fluid and cartilage. After its content in joints is increased, it can enhance the viscosity and lubricating function of the synovial fluid, and play a role in protecting the cartilage, promoting the joint healing and regeneration, relieving pain, increasing the joint range of motion and the like. It has been reported in the literature that the hyaluronic acid and the sodium salt thereof are safe and effective ideal substances in preventing and reducing adhesions caused by the gynecological and obstetric surgery as shown in numerous animal studies and clinical applications. The aqueous solution of sodium hyaluronate is a non-Newtonian fluid and has good viscoelasticity and rheology. In general, the low concentration of hyaluronic acid solution mainly exhibits viscosity, and the high concentration of hyaluronic acid solution mainly exhibits elasticity, so that the concentration of hyaluronic acid solution can be adjusted as needed in practice.

The natural hyaluronic acid or the sodium salt thereof has clear disadvantages, in addition to its wide range of applications and various clear advantages. Firstly, the natural hyaluronic acid or the sodium salt thereof has a short half-life in vivo, with the degradation time in organisms generally being not more than 7 days. The main reason for the short half-life is that the natural hyaluronic acid or the sodium salt thereof has a small average molecular weight and good fluidity, is easily dispersed in tissues and then absorbed and metabolized, and such fact is directly indicated by low viscosity in a solution state. Secondly, the natural hyaluronic acid or the sodium salt thereof has disadvantages of poor stability and easy degradation. Thirdly, the natural hyaluronic acid or the sodium salt thereof has a disadvantage of being excessively hydrophilic.

Other natural polymer compounds also have similar problems of hyaluronic acid. Preparing a hydrogel of a natural polymer compound can solve the problems of low mechanical strength and the like to some extent. In the existing research, in order to obtain hydrogel of natural polymer compound with ideal physical and mechanical properties and biodegradation rate, chemical cross-linking is widely applied in the process of preparing hydrogel. Functional groups with high chemical activity, such as carboxyl, hydroxyl and amino, are often applied in chemical cross-linking reaction, and commonly used chemical cross-linking agents generally contain bifunctional groups, such as diamine, dihydrazine, dialdehyde and diol. However, these cross-linking agents are usually cytotoxic and, if left over, will affect the biocompatibility of the hydrogel material. It is necessary to develop a novel chemically cross-linked polymer hydrogel to avoid cytotoxicity caused by the addition of additional chemicals in the cross-linking reaction. Meanwhile, modem medicine requires that the biomaterials can have certain plasticity and controllability in use to realize a minimally invasive treatment effect. Taking hyaluronic acid as an example, in the prior art, hydrogel prepared with hyaluronic acid as a main starting material has the following obvious disadvantages or technical prejudices: firstly, because there's cross-linking application of micromolecules containing epoxy groups in the cross-linking reaction of hyaluronic acid, and the toxic epoxy small molecule cross-linking agent is left over in the cross-linked hyaluronic acid, adverse reaction or toxic action is inevitably generated after such a cross-linked hyaluronic acid is prepared into hydrogel, and the application of the hydrogel of hyaluronic acid is restricted; secondly, the hydrogel prepared with the cross-linked hyaluronic acid obtained by subjecting the chemically modified hyaluronic acid to cross-linking reaction is high in price, and it has definite but limited improvement in viscosity, water retention, the shaping effect and the like compared with the hydrogel prepared by cross-linking of a hyaluronic acid that is not structurally modified or reconstructed; thirdly, in the prior art, the cross-linking reaction for generating the cross-linked hyaluronic acid from hyaluronic acid requires certain reaction conditions or relatively demanding reaction conditions, in situ cross-linking cannot be realized in a physiological state, and the product can be obtained only by pre-crosslinking and pre-filling, which greatly affects the application range of the product and the compliance in corresponding group of people with treatment or cosmetology needs.

In recent years, sulfhydryl-modified polymer compound has attracted attention from researchers because of its characteristics such as being easily cross-linked to form hydrogel and oxidation resistance. The sulfhydryl modification process of existing biocompatible polymers generally refers to a chemical modification process for introducing free sulfhydryls. In general, free sulfhydryls can be introduced into side chain groups of polysaccharides, proteins and synthetic macromolecules, such as carboxyl, amino and hydroxyl, through appropriate chemical reactions. Still taking hyaluronic acid as an example, in the prior art, a sulfhydrylated hyaluronic acid obtained by introducing free sulfhydryl groups into hyaluronic acid through a chemical reaction has characteristics which are summarized as having certain improvement in physical and chemical properties, biocompatibility or the like compared with natural hyaluronic acid but still being unable to overcome the following disadvantages. 1. the rate of self-crosslinking or cross-linking with other substances is relatively slow, and addition of small molecule oxidants is usually required to accelerate the cross-linking reaction; 2. the hydrogel obtained by cross-linking of the new compound, the sulfhydryl-modified hyaluronic acid, is not substantially preferable or does not have enough distinguishing technical characteristics compared with the existing commercially available products or other products in terms of such key indexes as physical and chemical properties and biocompatibility, which are mainly reflected in viscosity, metabolism persistence and shaping effect; 3. the sulfhydrylated hyaluronic acid prepared using any synthesis method in the prior art has the disadvantage of high toxicity or overhigh cost. These reasons above are the root of affecting the industrial production and wider application of the existing synthetic preparation technology of the sulfhydrylated hyaluronic acid. In addition, in the prior art, the hydrogel prepared by subjecting the sulfhydrylated hyaluronic acid to cross-linking reaction has disadvantages or technical prejudices, including: 1. in the prior art, the hydrogel prepared with the cross-linked hyaluronic acid obtained by subjecting the chemically modified hyaluronic acid to cross-linking reaction is high in price compared with the hydrogel prepared by cross-linking of a natural hyaluronic acid; 2. in the prior art, the hydrogel prepared with the cross-linked hyaluronic acid obtained by subjecting the chemically modified hyaluronic acid to cross-linking reaction has definite but limited improvement in viscosity, water retention, the shaping effect and the like compared with the hydrogel prepared by cross-linking of a natural hyaluronic acid; 3. in the prior art, chemical modification of hyaluronic acid is somewhat uncontrollable, which will affect the quality of the cross-linked hyaluronic acid, and thus the quality of corresponding hydrogel fluctuates in a large range, the consistency of the treatment effect or the cosmetic and plastic effect of hydrogel products in different batches cannot be realized, and a greater role of hydrogel in the application field is also affected.

Some prior arts can be seen in: 1)US 20170355799A1; 2) "Injectable hyaluronic acid microhydrogels for controlled release formulatioin of erythropoietin", XP-002719831, Journal of Biomadical Materials Research Part A, pp. 916-924, 27 Oct. 2006.

## SUMMARY

The present invention is set out in the appended claims.

An object of the present disclosure is to provide a hydrogel with a novel structure generated by gelation of a

sulfhydryl-modified polymer compound with a novel structure and at least one of the following substances: an acryloylated polymer compound and a small molecule cross-linking agent containing acryloyl group. Specifically, in the present disclosure, a sulfhydryl-modified polymer compound with a novel structure is in combination with an acryloylated polymer compound and/or a small molecule cross-linking agent containing acryloyl group to form a hydrogel, and the sulfhydryl-modified polymer compound can be cross-linked with the acryloylated polymer compound and/or the small molecule cross-linking agent containing acryloyl group under physiological conditions to form the hydrogel; in addition, the formed hydrogel is remarkably superior to those in the prior art in terms of the physical properties and chemical properties related to the shaping effect, the metabolism resistance and the degradation resistance, and particularly, its metabolism resistance and degradation resistance are significantly superior to those of the hydrogels in the prior art; furthermore, due to the rapid sulfhydryl-ethenyl cross-linking reaction, a hydrogel system formed by the two compounds can be rapidly gelled in situ after being injected into a body. Based on this, the hydrogel of the present disclosure is more suitable for use in the fields of biopharmaceuticals, medical cosmetology, cosmetics and the like.

A second object of the present disclosure is to provide a method for preparing the aforementioned hydrogel, which has the following advantages: highly toxic epoxy small molecule cross-linking agents and catalysts do not need to be added in the cross-linking reaction, thus fundamentally avoiding the presence of possible residue of toxic substance in the purification process; catalysis conditions such as illumination and heating are not required; the degree of the cross-linking reaction is controllable; and the cost of the cross-linking reaction is moderate and is superior to that in the prior art.

In a first aspect, the present disclosure provides a hydrogel, which has a completely new chemical structure and is prepared by gelation of a system comprising a sulfhydryl-modified polymer compound;

the sulfhydryl-modified polymer compound is at least one of the following series of compounds:

a series of sulfhydryl-modified polymer compounds, wherein to be modified polymer compounds comprise at least one selected from -COOH, -NH2, -OH, an acrylate group of formula a, an acrylamide group of formula b and an acryloyl group of formula c in the structure,

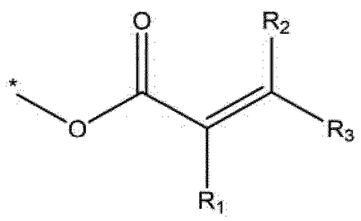
formula a

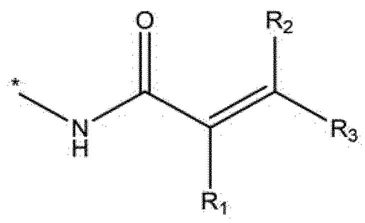
formula b

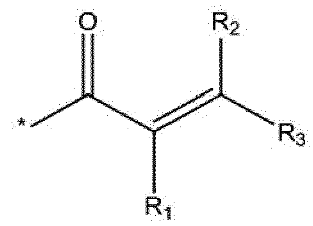
formula c

wherein part or all of the -COOH and/or the -NH2 and/or the -OH and/or the acrylate group and/or the acrylamide group and/or the acryloyl group are modified to form a side chain with the following terminal group:

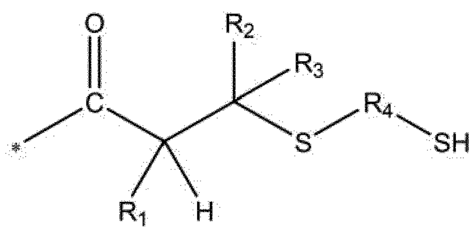

wherein in the above group, * represents a linking site; R1 is selected from hydrogen, halogen, an aliphatic group, an aromatic group and the like; R2 and R3 are the same or different and independently from each other are selected from hydrogen, halogen, an aliphatic group, an aromatic group and the like; R4 is a polysulfhydryl compound fragment;

the system further comprises at least one of the following substances:

C1. an acryloylated polymer compound, and
C2. a small molecule cross-linking agent containing an acryloyl group.

In a second aspect, the present disclosure provides a preparation method for the aforementioned hydrogel, which comprises the following step:
gelling a system comprising the following substances:

(i) the sulfhydryl-modified polymer compound, and
(ii) at least one from the substance C1 and the substance C2,

thus obtaining the hydrogel.
In a third aspect, the present disclosure provides use of the aforementioned hydrogel in the fields of biomedicine, medical cosmetic plastic surgery, cosmetics and the like.

Beneficial Effects of Present Disclosure

The present disclosure provides a hydrogel with an innovative structure, which is obtained by subjecting a sulfhydryl-modified polymer compound with an innovative structure, as a starting material, to a cross-linking reaction. Compared with hydrogels in the prior art (such as the hydrogels in the prior art obtained by subjecting hyaluronic acid or modified hyaluronic acid, as a starting material, to a cross-linking reaction), the hydrogel has unexpected technical advantages in the aspects of physical and chemical properties, shaping effect, metabolism resistance, degradation resistance and the like.
The hydrogel of the present disclosure has the following advantages: 1. in the process of modifying and reconstructing the structure of the polymer compound and in the subsequent process of cross-linking reaction, no toxic epoxy small molecule cross-linking agent is used, and the hydrogel product has the advantage of higher safety. 2. compared with the hydrogels in the prior art (such as the existing cross-linked hyaluronic acid hydrogels), the hydrogel product of the present disclosure has the technical advantages of better viscosity, water retention, shaping effect and the like. 3. the hydrogel of the present disclosure can realize cross-linking reaction without adding any catalyst, and the reaction conditions are easier to realize and are superior to the cross-linking reaction conditions of polymer compounds in the prior art and the cross-linking conditions of modified polymer compounds in the prior art as well. 4. the in situ cross-linking under physiological condition in the true sense is realized for the first time, and the end point of the cross-linking reaction is controllable; the controllability of the end point is not only embodied in the in vitro cross-linking reaction but also in the in vivo cross-linking reaction in an animal or a human, and a large number of animal experiments have proved that the end point of the cross-linking reaction is single, stable and reproducible no matter the cross-linking reaction is in vivo or in vitro of animal. 5. experimental research shows that the series of hydrogels of the present disclosure are better in stability and are degradation resistant under room temperature and the conditions of accelerated stability test, and they have better metabolism resistance in animals and the like.
The present disclosure realizes in situ cross-linking under physiological conditions in the true sense; namely, the cross-linking reaction can be completed under the conditions of room temperature and normal pressure; or after the hydrogel is injected into tissues of an animal or a human, the cross-linking reaction can still be realized in the tissues, so that the degradation resistance and metabolism resistance of the hydrogel product are significantly improved, and thus the using effect of the hydrogel injection product is remarkably improved. Besides, due to the unique technology of the present disclosure, the controllability of the cross-linking degree of the hydrogel product injected into the animal or human can be realized before the in vitro cross-linking or mixing stage; namely, the cross-linking reaction with controllable cross-linking reaction end point can be realized after the hydrogel product is injected into the animal or human, and thus the safety and the therapeutic effect of the product are ensured.
The present disclosure also provides a method for preparing the hydrogel, wherein in the method, the reaction can be completed at room temperature and normal pressure, and the reaction conditions are mild and easy to be realized, which are the technical basis for realizing the in situ cross-linking of the hydrogel under physiological conditions.

BRIEF DESCRIPTION OF THE DRAWINGS

FIG. 1 shows the reaction equation of Preparation Example 5;
FIG. 2 shows the reaction equation of Preparation Example 6;

FIG. 3 shows the reaction equation of Preparation Example 7;
FIG. 4 shows the reaction equation of Preparation Example 8;
FIG. 5 shows the reaction equation of Preparation Example 15;
FIG. 6 shows the reaction equation of Preparation Example 16;
FIG. 7 shows the reaction equation of Preparation Example 17;
FIG. 8 shows the reaction equation of Preparation Example 18;
FIG. 9 shows the reaction equation of Preparation Example 19;
FIG. 10 shows the reaction equation of Preparation Example 20;
FIG. 11 shows the cell biocompatibility experiments with hydrogel samples;
FIG. 12 shows the in vivo shaping and supporting effect (height) of hydrogel samples;
FIG. 13 shows the in vivo shaping and supporting effect (basal area) of hydrogel samples;
FIG. 14 shows the in vivo degradation experiments with hydrogel samples;
FIG. 15 shows the structural formula of HA-A1 and the 1H-NMR spectrum thereof;
FIG. 16 shows the structural formula of HA-A2 and the 1H-NMR spectrum thereof;
FIG. 17 shows the structural formula of HA-MA1 and the 1H-NMR spectrum thereof;
FIG. 18 shows the structural formula of HA-MA2 and the 1H-NMR spectrum thereof;
FIG. 19 shows the structural formula of CHS-A and the 1H-NMR spectrum thereof;
FIG. 20 shows the structural formula of CHS-MA and the 1H-NMR spectrum thereof;
FIG. 21 shows the structural formula of Gelatin-A and the 1H-NMR spectrum thereof;
FIG. 22 shows the structural formula of Gelatin-MA and the 1H-NMR spectrum thereof;
FIG. 23 shows the structural formula of CTS-A and the 1H-NMR spectrum thereof;
FIG. 24 shows the structural formula of CTS-MA and the 1H-NMR spectrum thereof;
FIG. 25 shows the structural formula of HA-A1-SH1 and the 1H-NMR spectrum thereof;
FIG. 26 shows the structural formula of HA-A2-SH1 and the 1H-NMR spectrum thereof;
FIG. 27 shows the structural formula of HA-MA1-SH1 and the 1H-NMR spectrum thereof;
FIG. 28 shows the structural formula of HA-MA2-SH1 and the 1H-NMR spectrum thereof;
FIG. 29 shows the structural formula of CHS-A-SH1 and the 1H-NMR spectrum thereof;
FIG. 30 shows the structural formula of CHS-MA-SH1 and the 1H-NMR spectrum thereof;
FIG. 31 shows the structural formula of Gelatin-A-SH1 and the 1H-NMR spectrum thereof;
FIG. 32 shows the structural formula of Gelatin-MA-SH1 and the 1H-NMR spectrum thereof;
FIG. 33 shows the structural formula of CTS-A-SH1 and the 1H-NMR spectrum thereof;
FIG. 34 shows the structural formula of CTS-MA-SH1 and the 1H-NMR spectrum thereof;
FIG. 35 shows the structural formula of PHEMA-A and the 1H-NMR spectrum thereof;
FIG. 36 shows the structural formula of PHEMA-MA and the 1H-NMR spectrum thereof;
FIG. 37 shows the structural formula of PVA-A and the 1H-NMR spectrum thereof;
FIG. 38 shows the structural formula of PVA-MA and the 1H-NMR spectrum thereof;
FIG. 39 shows the structural formula of PHEMA-A-SH1 and the 1H-NMR spectrum thereof;
FIG. 40 shows the structural formula of PHEMA-MA-SH1 and the 1H-NMR spectrum thereof;
FIG. 41 shows the structural formula of PVA-A-SH1 and the 1H-NMR spectrum thereof;
FIG. 42 shows the structural formula of PVA-MA-SH1 and the 1H-NMR spectrum thereof;
FIG. 43 shows the structural formula of HB-PEG-SH1 and the 1H-NMR spectrum thereof;
FIG. 44 shows the structural formula of HA-A1-SH2 and the 1H-NMR spectrum thereof;
FIG. 45 shows the structural formula of HA-A1-SH3 and the 1H-NMR spectrum thereof;
FIG. 46 shows the structural formula of HA-A2-SH2 and the 1H-NMR spectrum thereof;
FIG. 47 shows the structural formula of HA-A2-SH3 and the 1H-NMR spectrum thereof;
FIG. 48 shows the structural formula of HA-A2-SH4 and the 1H-NMR spectrum thereof;
FIG. 49 shows the structural formula of HA-A2-SH5 and the 1H-NMR spectrum thereof;
FIG. 50 shows the structural formula of HA-A2-SH6 and the 1H-NMR spectrum thereof;
FIG. 51 shows the structural formula of HA-A2-SH7 and the 1H-NMR spectrum thereof;
FIG. 52 shows the structural formula of HA-A2-SH8 and the 1H-NMR spectrum thereof;
FIG. 53 shows the structural formula of HA-MA1-SH5 and the 1H-NMR spectrum thereof;
FIG. 54 shows the structural formula of HA-MA1-SH6 and the 1H-NMR spectrum thereof;
FIG. 55 shows the structural formula of HA-MA2-SH7 and the 1H-NMR spectrum thereof;
FIG. 56 shows the structural formula of HA-MA2-SH8 and the 1H-NMR spectrum thereof;
FIG. 57 shows the reaction equation of Preparation Example 25;
FIG. 58 shows the reaction equation of Preparation Example 26;
FIG. 59 shows the reaction equation of Preparation Example 27;
FIG. 60 shows the reaction equation of Preparation Example 28;

FIG. 61 shows the reaction equation of Preparation Example 29 (wherein i = 10-90%, j = 10-90%, i2 + i3 = i, j2 + j3 = j, h = j, i + j = 100%, and k1 = 1-1000);
FIG. 62 shows the reaction equation of Preparation Example 30;
FIG. 63 shows the reaction equation of Preparation Example 31;
FIG. 64 shows the reaction equation of Preparation Example 32;
FIG. 65 shows the reaction equation of Preparation Example 33;
FIG. 66 shows the reaction equation of Preparation Example 34;
FIG. 67 shows the reaction equation of Preparation Example 35;
FIG. 68 shows the reaction equation of Preparation Example 36;
FIG. 69 shows the reaction equation of Preparation Example 37;
FIG. 70 shows the reaction equation of Preparation Example 38;
FIG. 71 shows the reaction equation of Preparation Example 39;
FIG. 72 shows the reaction equation of Preparation Example 40;
FIG. 73 shows the reaction equation of Preparation Example 41;
FIG. 74 shows the reaction equation of Preparation Example 42.

DETAILED DESCRIPTION

[Sulfhydryl-modified Polymer Compound]

As described above, the system to be gelled of the present disclosure requires the use of at least one of the series of compounds shown below:

a series of sulfhydryl-modified polymer compounds, polymer compounds to be modified comprising at least one selected from -COOH, -NH2, -OH, an acrylate group of formula a, an acrylamide group of formula b and an acryloyl group of formula c in the structure,

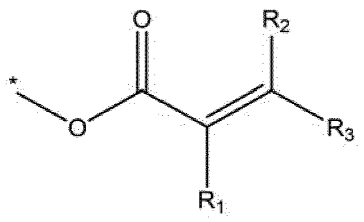
formula a

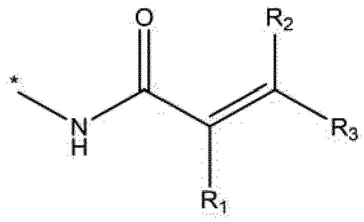
formula b

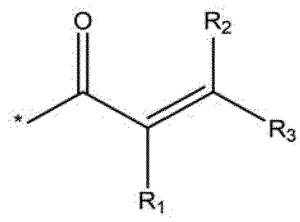
formula c

wherein part or all of the -COOH and/or the -NH2 and/or the -OH and/or the acrylate group and/or the acrylamide group and/or the acryloyl group are modified to form a side chain with the following terminal group:

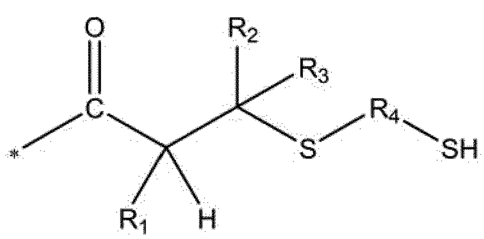

in the above group, * represents a linking site;

R1 is selected from hydrogen, halogen, an aliphatic group, an aromatic group, and the like; specifically, the halogen, the aliphatic group and the aromatic group are further defined as below; preferably, R1 is selected from hydrogen, halogen, and an aliphatic group; more preferably, R1 is selected from hydrogen, halogen and C1-6 alkyl (e.g., methyl and ethyl);
R2 and R3 are the same or different and independently from each other are selected from hydrogen, halogen, an aliphatic group, an aromatic group, and the like; specifically, the halogen, the aliphatic group and the aromatic group are further defined as below;
R4 is a fragment of a polysulfhydryl compound.

In a specific embodiment, part or all of the -COOH and/or the -NH2 and/or the -OH and/or the acrylate group and/or the acrylamide group and/or the acryloyl group are modified to form at least one of the following structures:

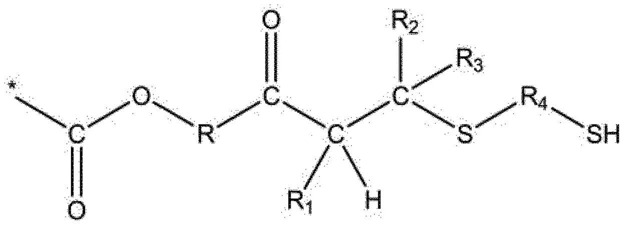

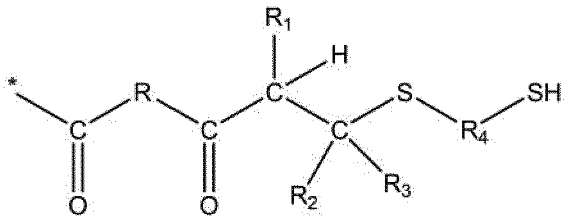

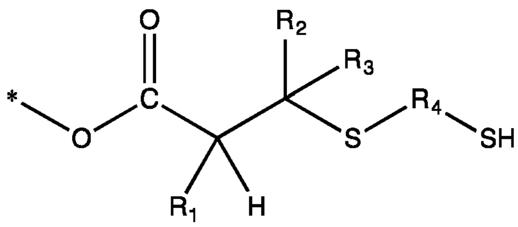

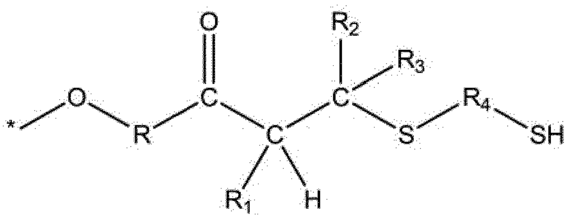

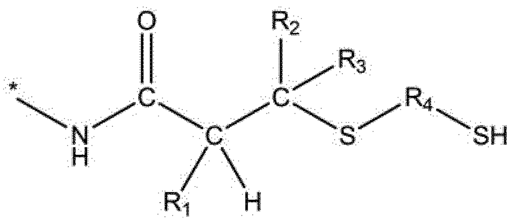

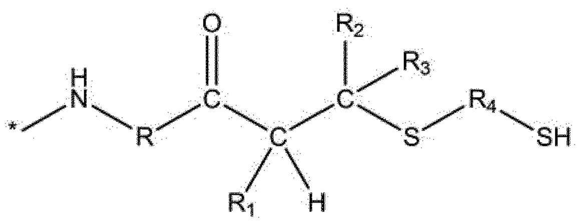

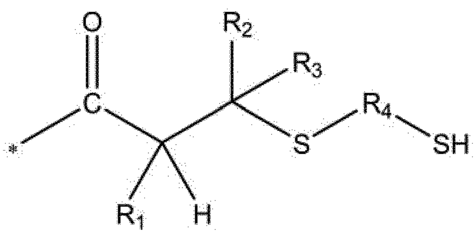

a.

wherein in the above structures, R is selected from

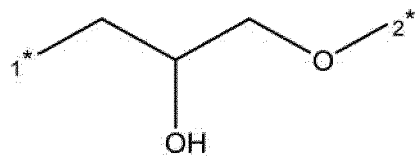

,

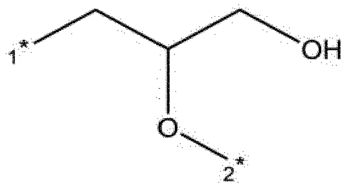

,

hydrocarbylene, arylene, an amide residue, a hydrazide residue, and the like; * represents a linking site; 1* represents a linking site to a left-hand group of R; 2* represents a linking site to a right-hand group of R; R1, R2, R3 and R4 are defined as above;
wherein at least one selected from the -COOH, the -NH2, the -OH, the acrylate group of formula a, the acrylamide group of formula b, and the acryloyl group of formula c can be directly linked to the main chain of the polymer compound, or connected to the main chain of the polymer compound via an R' group, and the R' group can be a heteroatom-containing group, hydrocarbylene, arylene or the following linker:

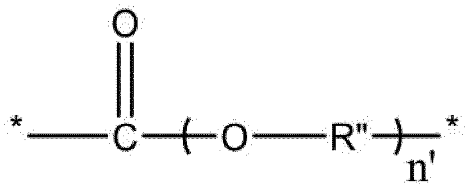

wherein in the above formula, R" is hydrocarbylene or arylene, n' is an integer from 1 to 1000, and * represents a linking site.

The heteroatom-containing group includes, but is not limited to an ester group, an amide residue or a hydrazide residue. Specifically, the ester group, the amide residue or the hydrazide residue are further defined as below.
The polymer compound to be modified comprises natural mucopolysaccharide polymers, such as at least one selected from chitosans (specifically chitosan, ethylene glycol chitosan, carboxymethyl chitosan, etc.), chondroitin sulfate, hyaluronic acid, and alginate; proteins such as gelatin, fibrin and serum proteins; and/or, synthetic polymers, such as at least one selected from polyvinyl alcohol, poly(meth)acrylic acid, polyhydroxyalkyl(meth)acrylate (e.g., polyhydroxyethyl(meth)acrylate), and hyperbranched polyethylene glycol.
A sulfhydryl content of the sulfhydryl-modified polymer compound as determined by the Ellman method is 0.01-30 mmol/g, for example, 0.1-10.0 mmol/g, for another example, 0.3-5.0 mmol/g, and for yet another example, 0.5-3.0 mmol/g.
The molecular weight of the sulfhydryl-modified polymer compound is substantially unchanged as compared to the molecular weight of the polymer compound before modification.
For example, the sulfhydryl-modified polymer compound of the present disclosure comprises at least one of the following structures:

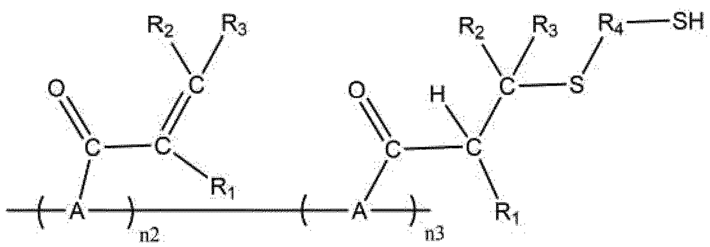

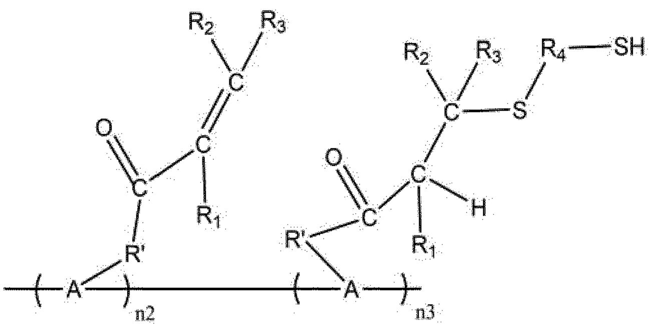

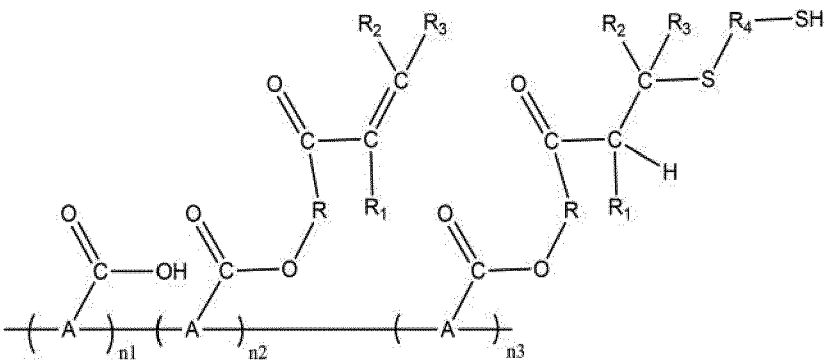

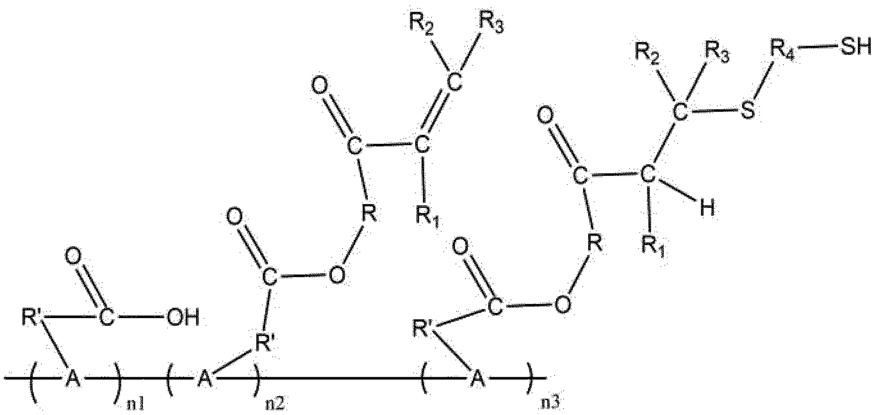

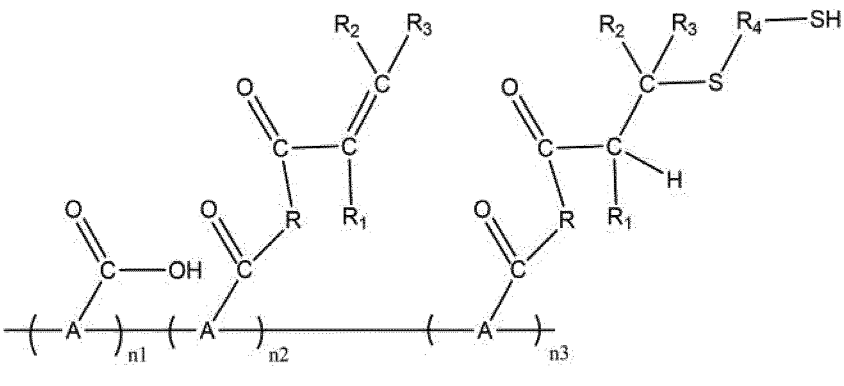

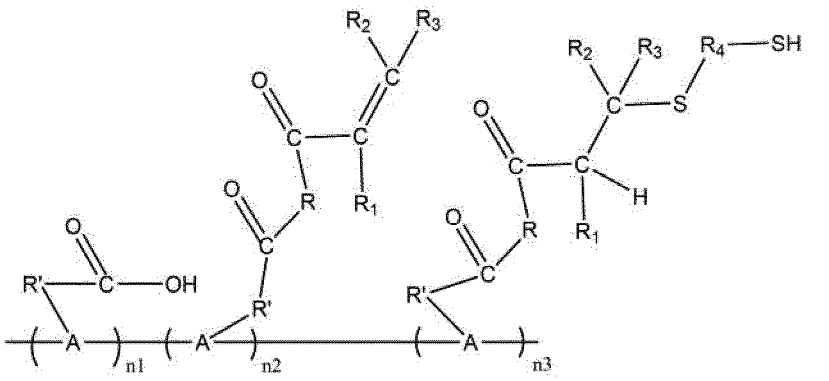

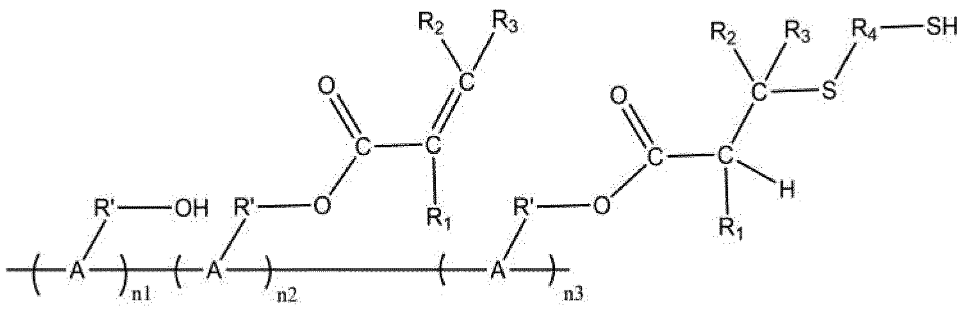

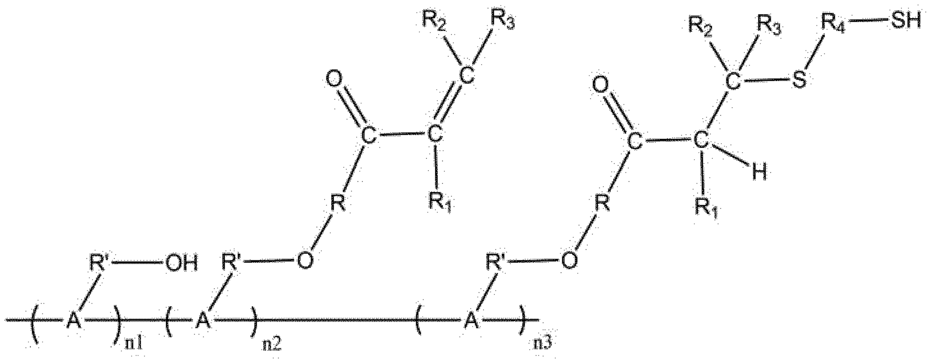

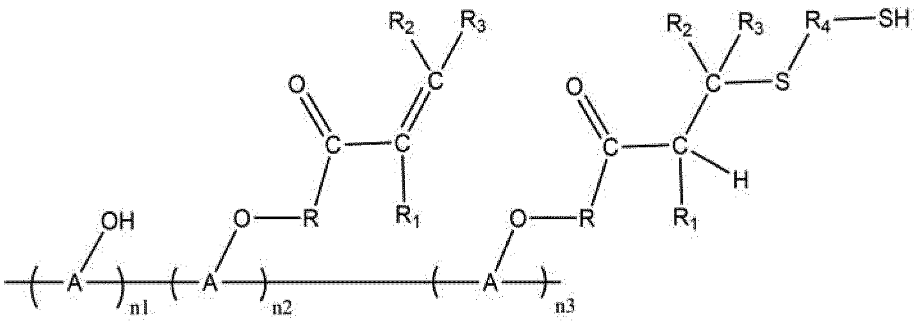

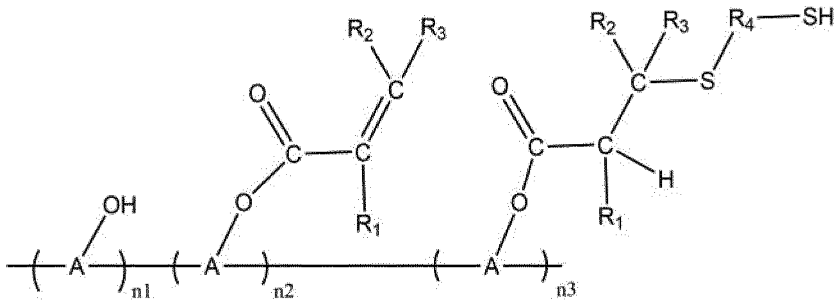

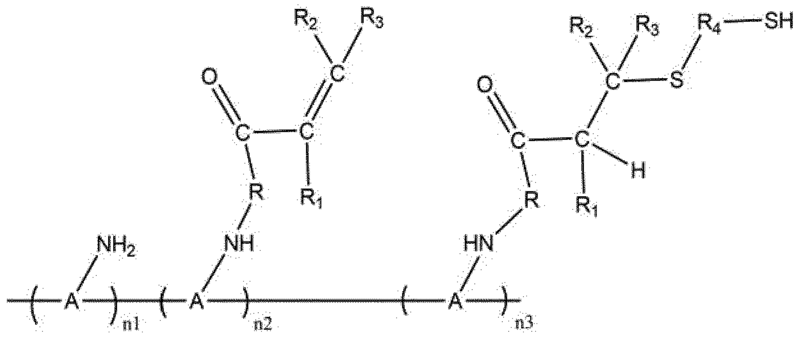

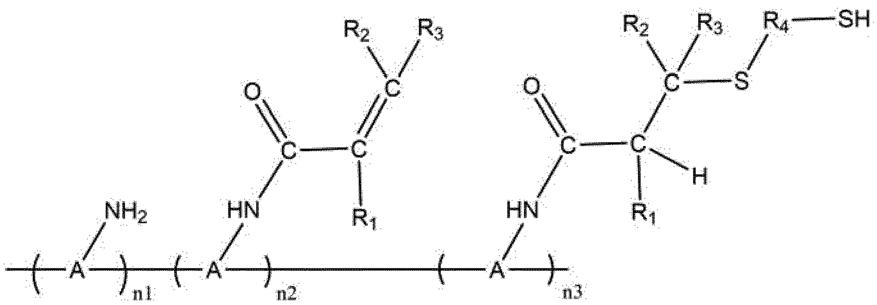

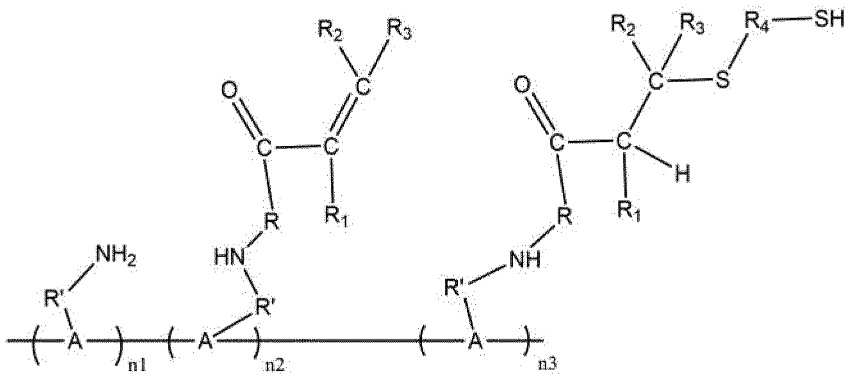

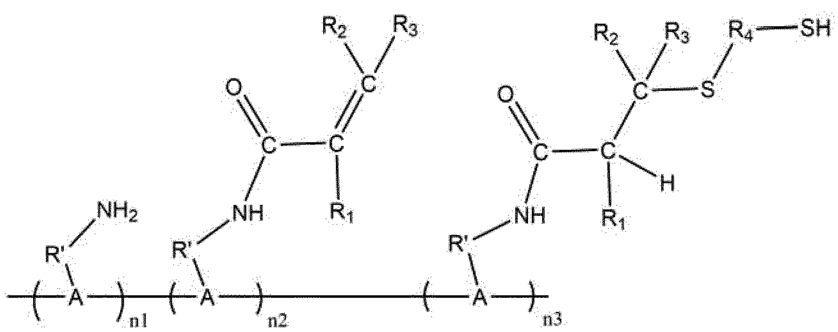

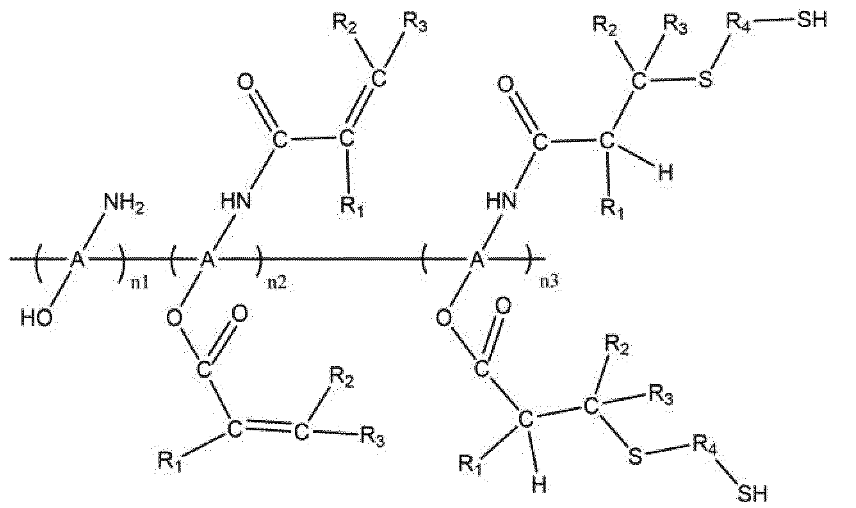

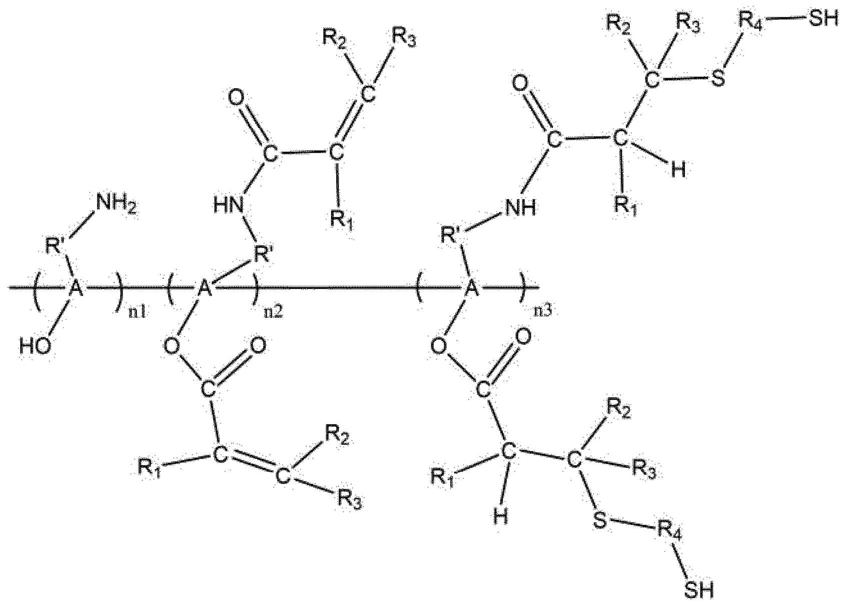

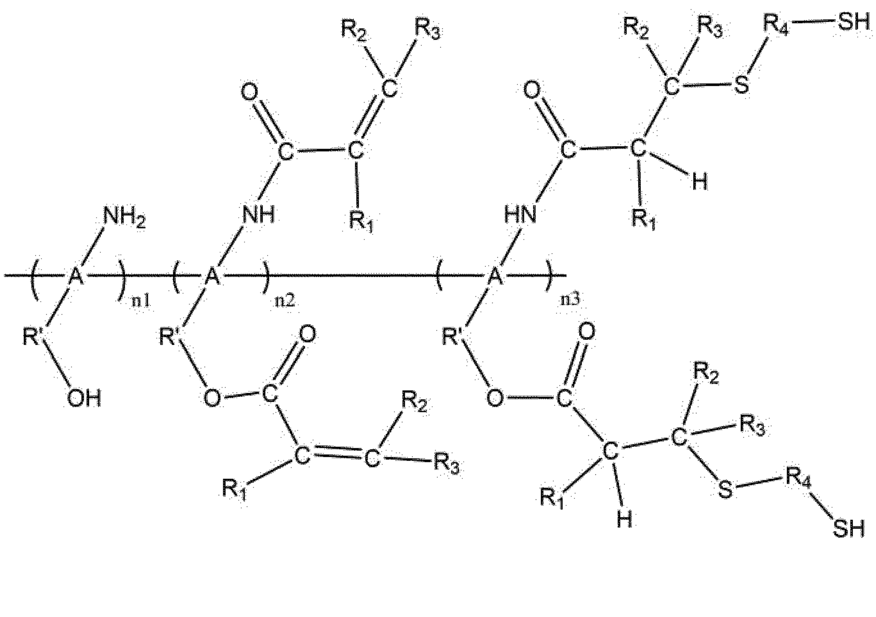

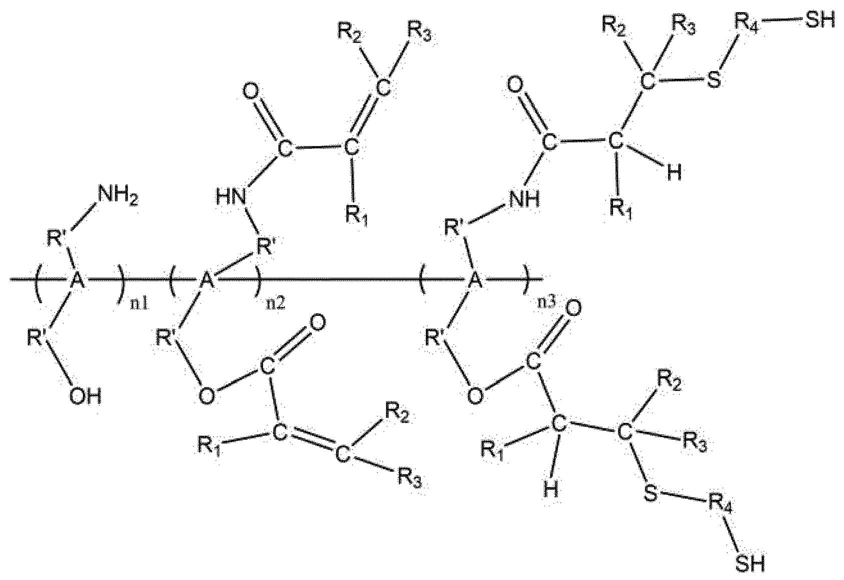

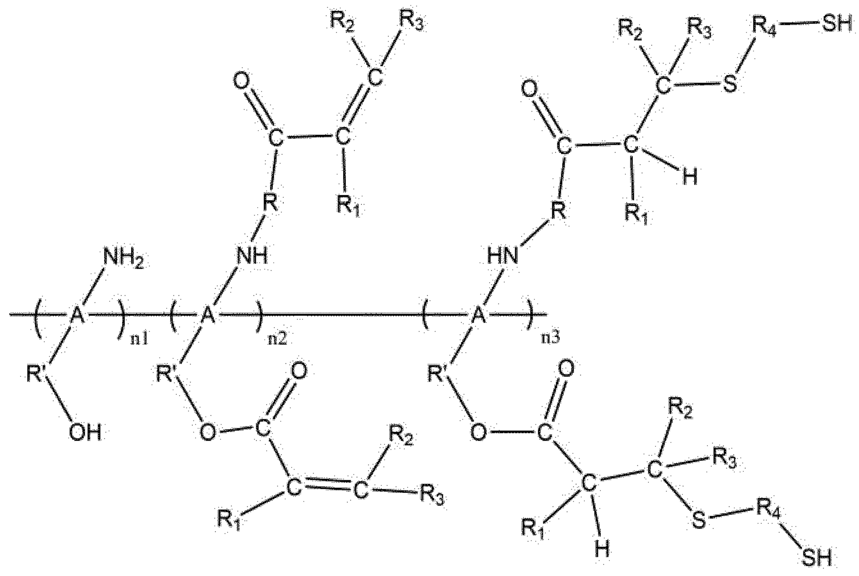

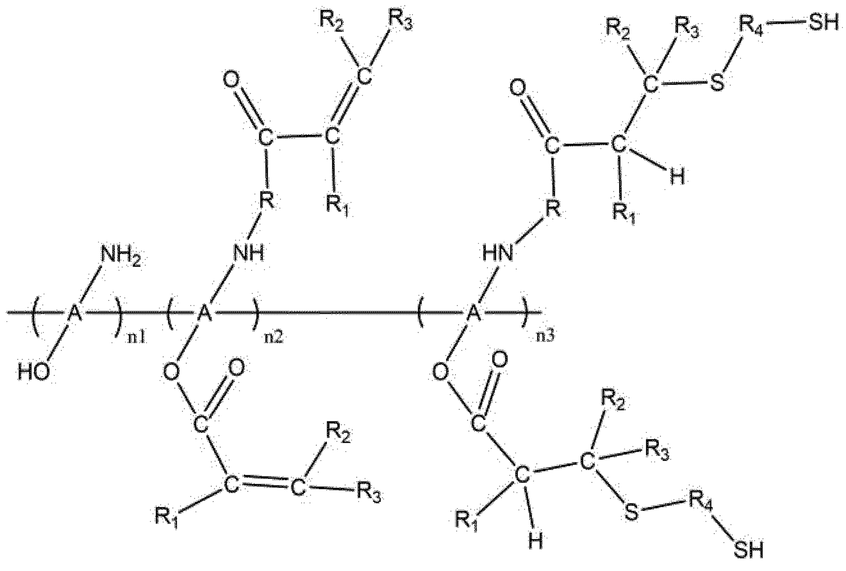

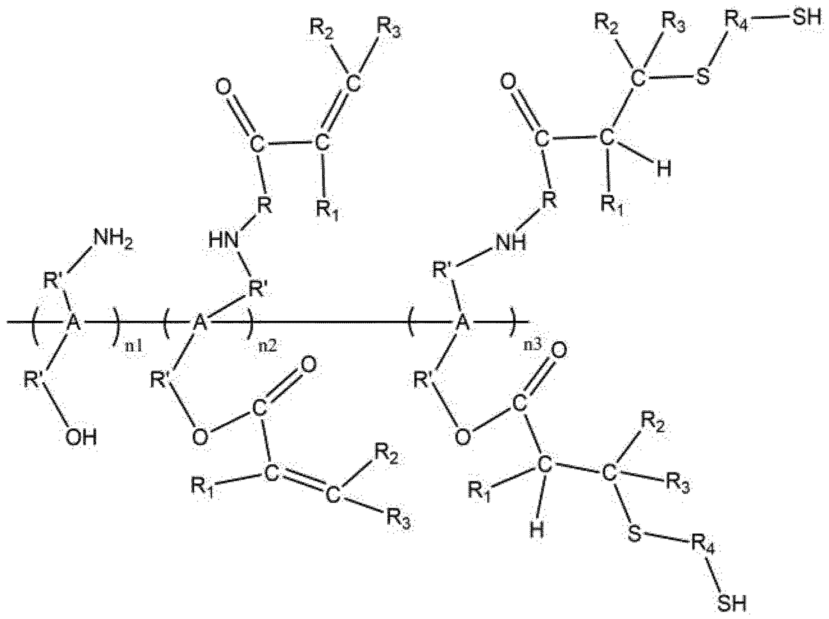

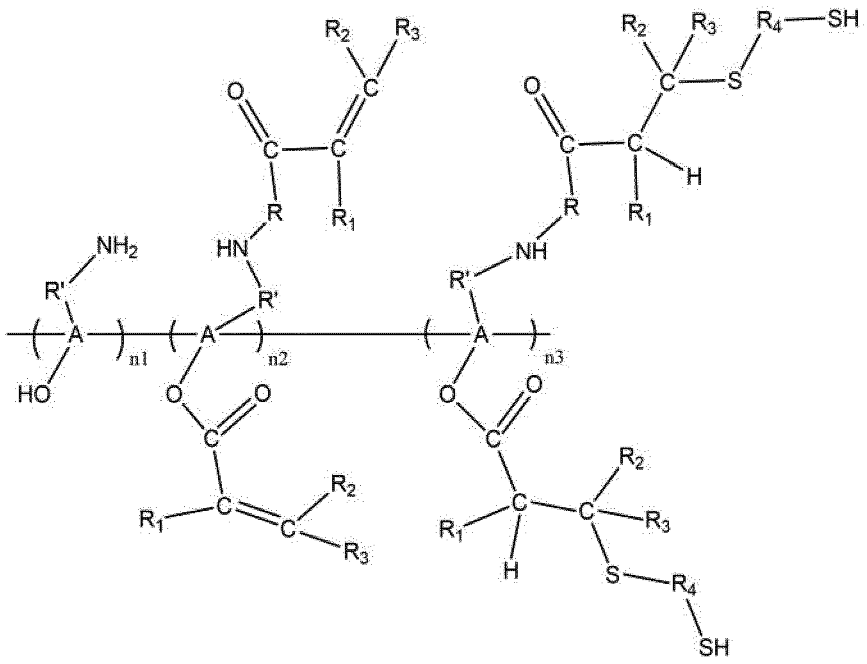

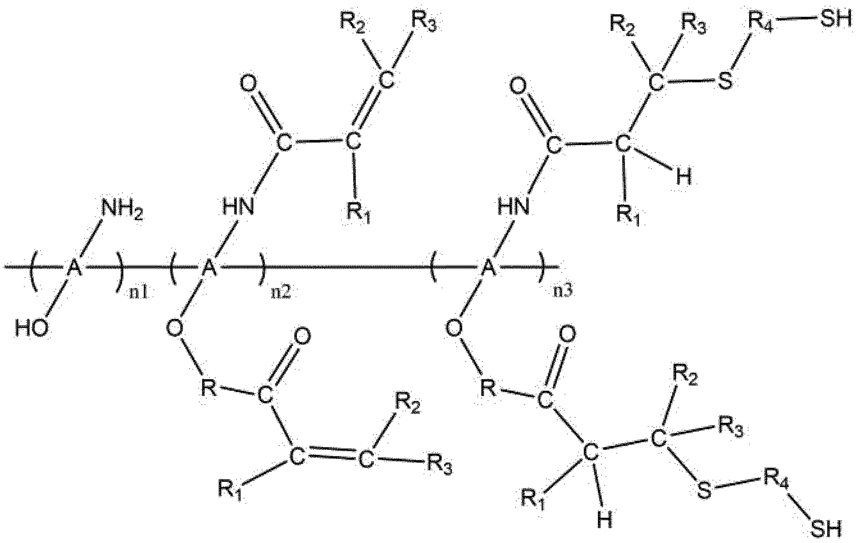

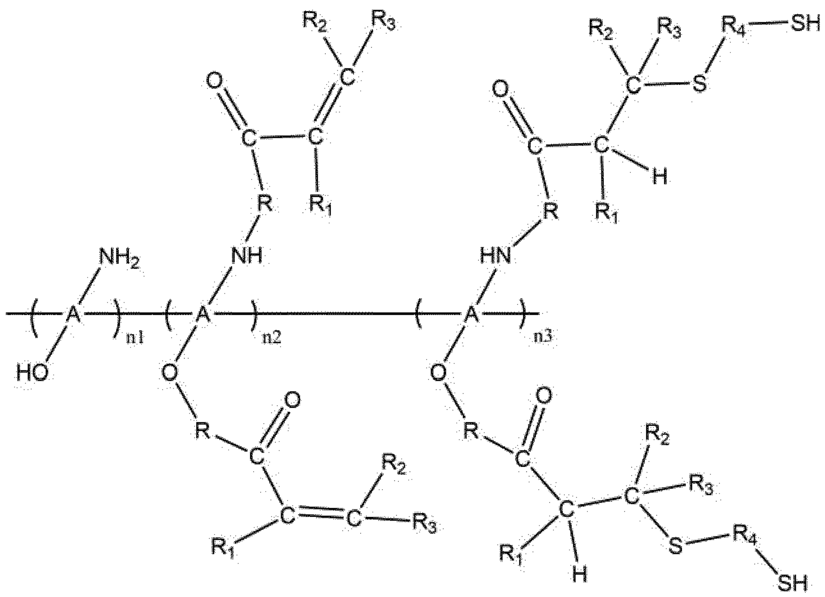

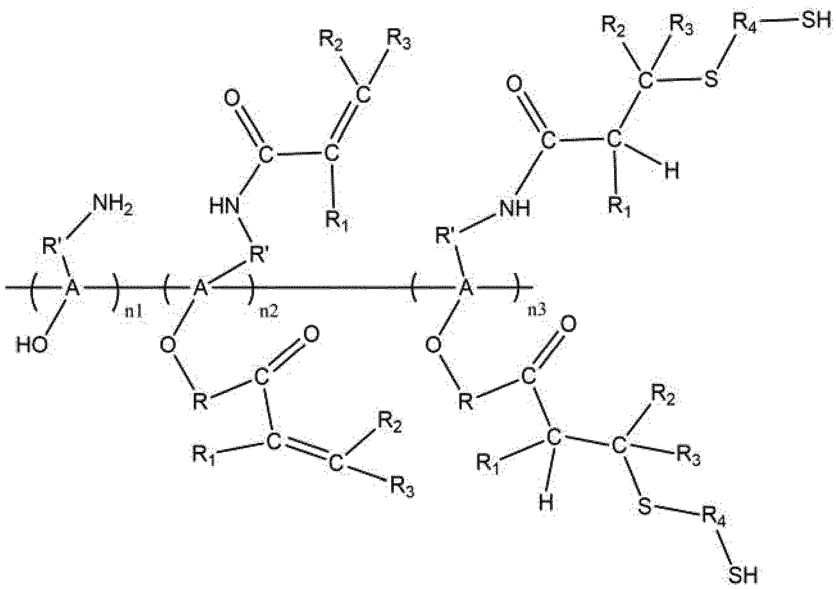

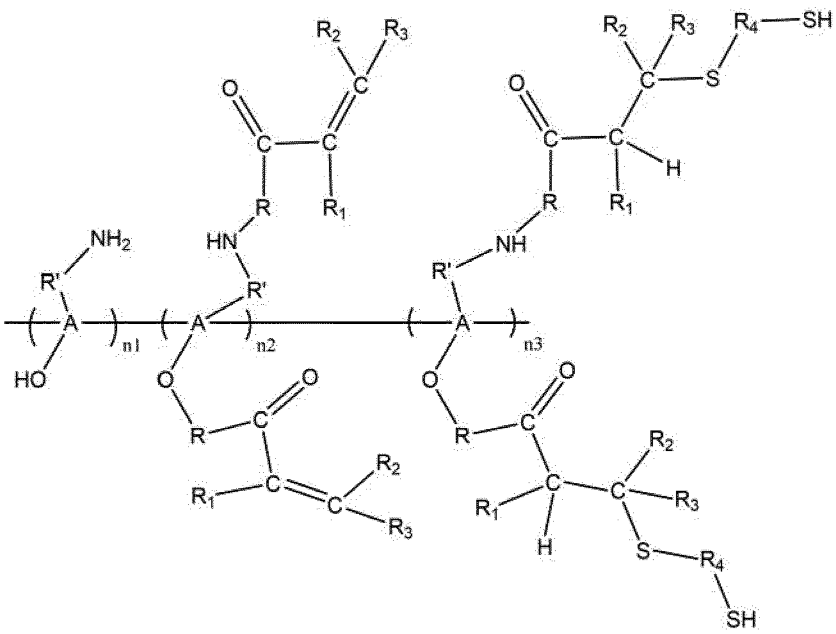

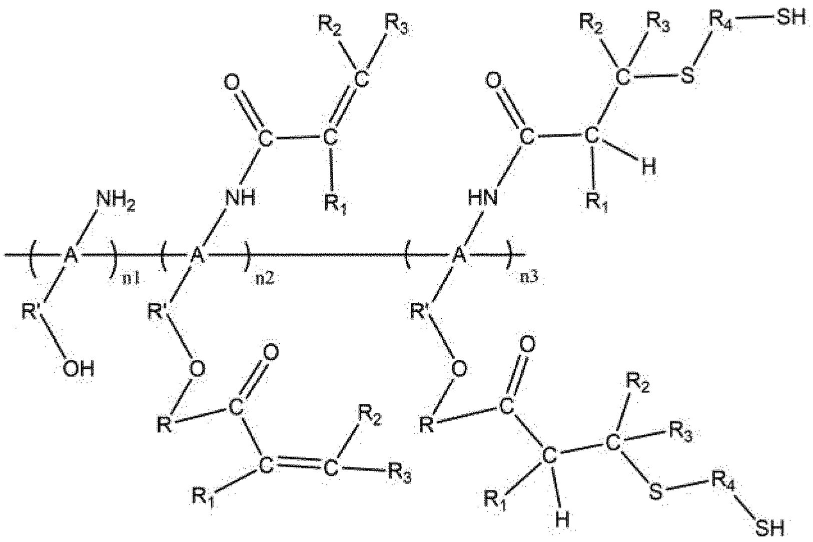

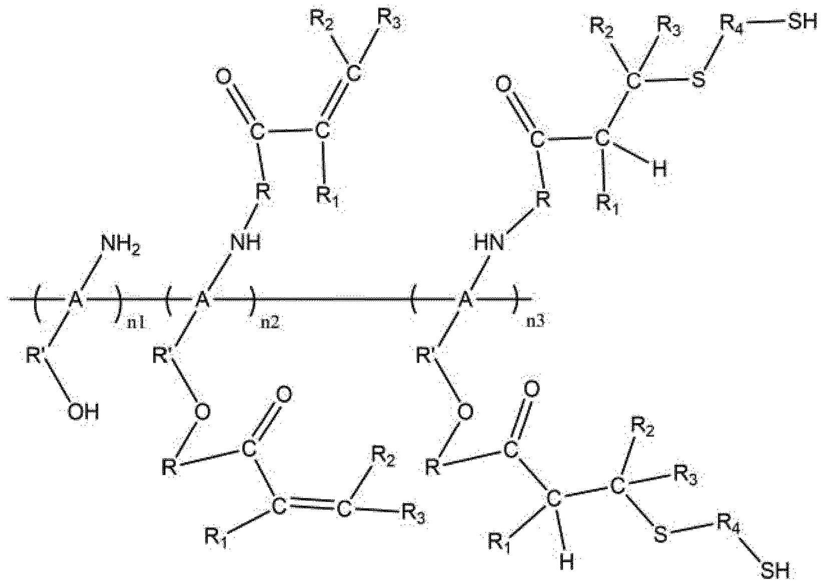

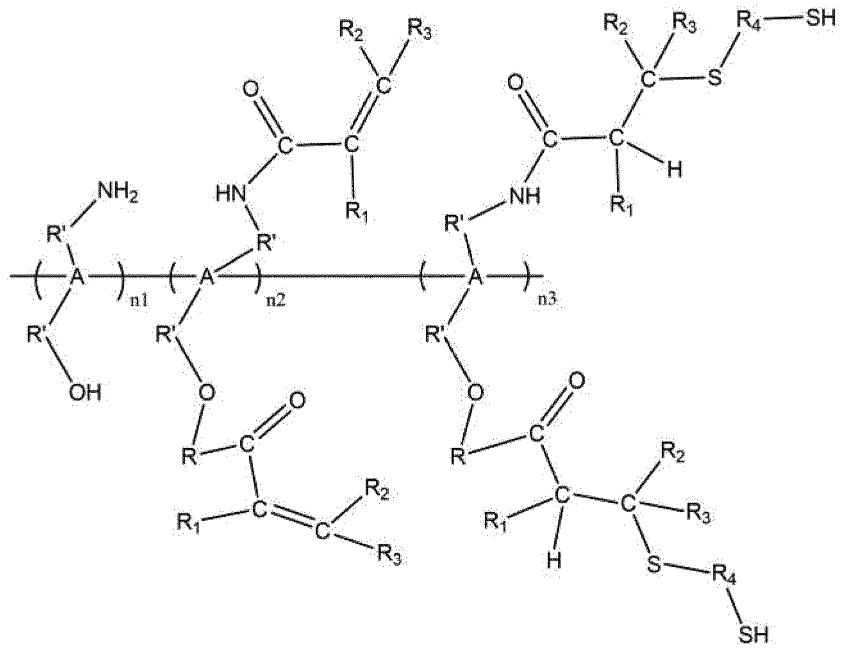

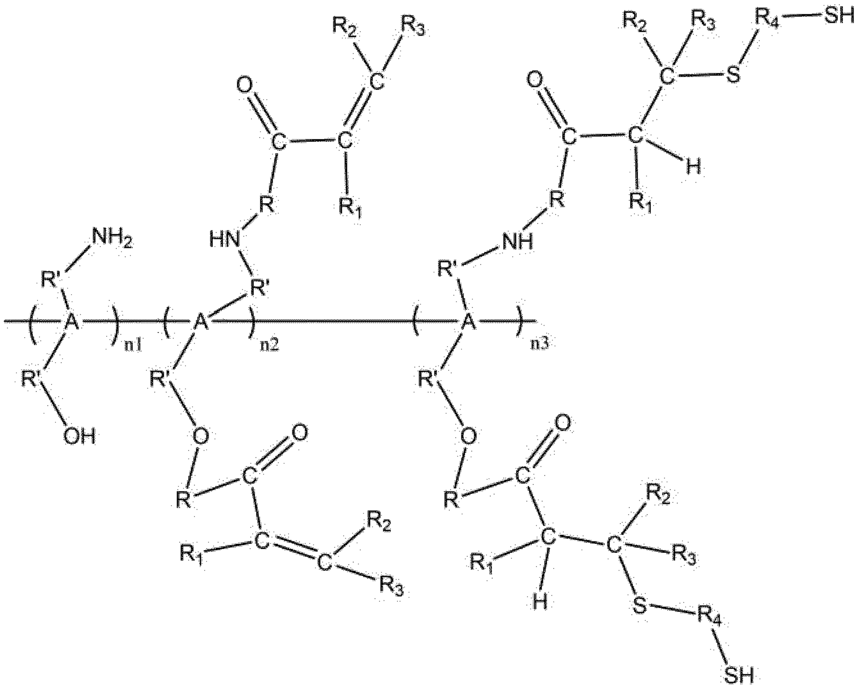

in the above structures, A is a fragment of the polymer compound to be modified comprising at least one selected from the -COOH, the -NH2, the -OH, the acrylate group of formula a, the acrylamide group of formula b and the acryloyl group of formula c in the structure; R, R', R1, R2, R3 and R4 are defined as above; (n2+n3)/(n1+n2+n3) represents a degree of acryloylation; n3/(n1+n2+n3) represents a degree of sulfhydrylation corresponding to the above sulfhydryl content of the sulfhydryl-modified polymer compound as determined by the Ellman method; the n1 can be 0, and if it is 0, the degree of acryloylation is not limited, and n3/(n2+n3) alone represents the degree of sulfhydrylation corresponding to the above sulfhydryl content of the sulfhydryl-modified polymer compound as determined by the Ellman method; the n2 can be 0, and if it is 0, n3/(n1+n3) represents both the degree of acryloylation and the degree of sulfhydrylation corresponding to the above sulfhydryl content of the sulfhydryl-modified polymer compound as determined by the Ellman method.

Specifically, the A can be a structure shown as follows:

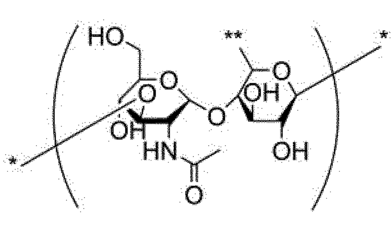

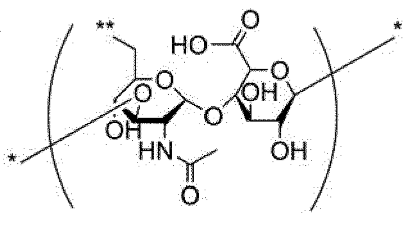

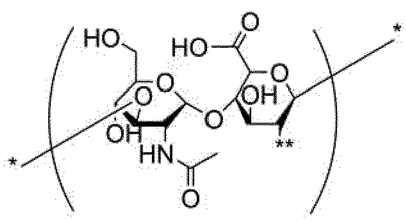

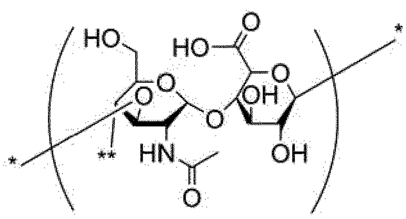

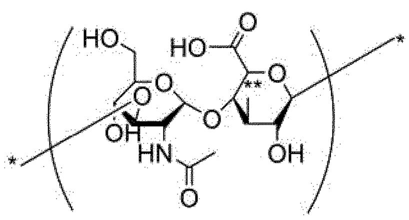

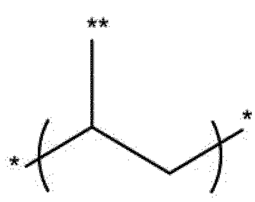

In each of the above structures, * represents a linking site between repeating units of the main chain; ** represents a linking site between -COOH, -NH2, -OH, an acrylate group of formula a, an acrylamide group of formula b or an acryloyl group of formula c and the fragment, or a linking site between an R' group and the fragment.

The A can also be a fragment or a repeating unit remaining in the following polymers Gelatin-A, Gelatin-MA, CTS-A, CTS-MA, PHEMA-A, PHEMA-MA, HB-PEG, PVA-A, PVA-MA, CHS-A or CHS-MA with the side chain containing the acrylamide group removed:

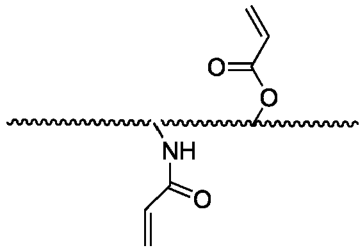

Gelatin-A

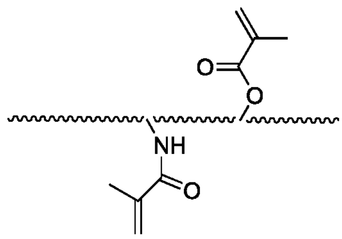

Gelatin-MA

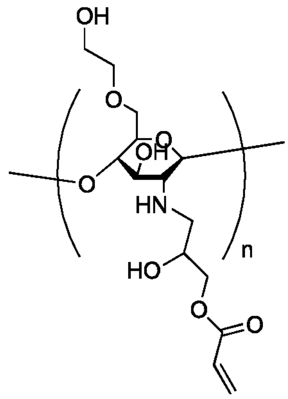

CTS-A

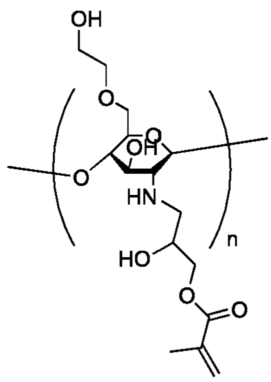

CTS-MA

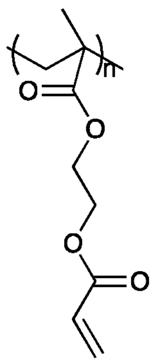

PHEMA-A

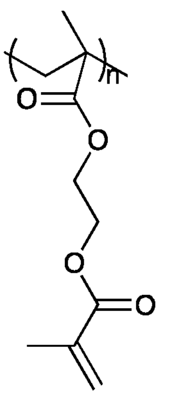

PHEMA-MA

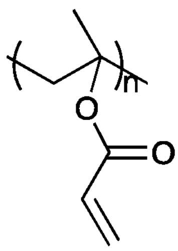

PVA-A

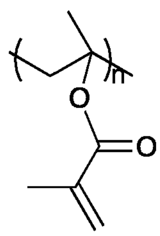

PVA-MA

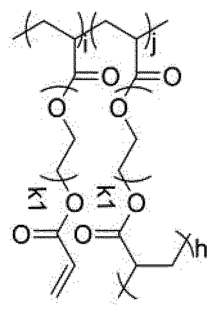

HB-PEG

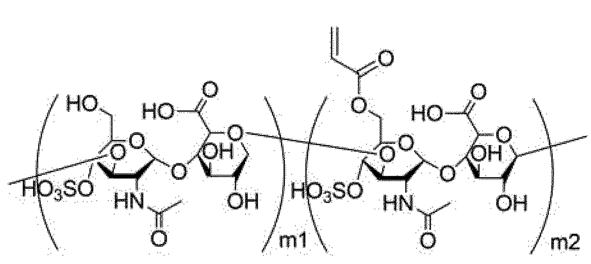

CHS-A

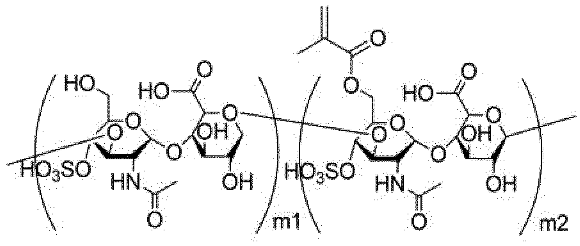

CHS-MA .

It should be noted that Gelatin-A, Gelatin-MA, CTS-A, CTS-MA, PHEMA-A, PHEMA-MA, HB-PEG, PVA-A, PVA-MA, CHS-A and CHS-MA are abbreviations for the names of polymers having the above structures, and letters therein, when being separated, are not related to the meaning of letters appearing elsewhere in the present disclosure.

In the present disclosure, n, n', n1, n2, n3, n4, n5, n6, m1, m2, i, j, k1 and h are the number of repeating units in the structural formula unless otherwise specified. The range of values falls within conventional ranges known in the art.

In one specific embodiment of the present disclosure, the series of sulfhydryl-modified polymer compounds are specifically:

a series of sulfhydryl-modified hyaluronic acid compounds, wherein part or all of -COOH and/or -OH contained in a side chain of a repeating unit of the hyaluronic acid are modified to form a side chain with the following terminal group:

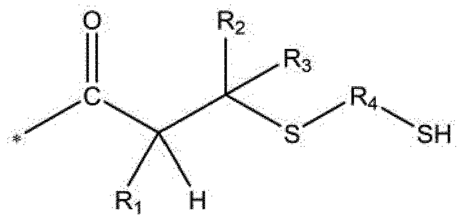

in the above group, * represents a linking site;

R1 is selected from hydrogen, halogen, an aliphatic group, an aromatic group, and the like;

specifically, the halogen, the aliphatic group and the aromatic group are further defined as below; preferably, R1 is selected from hydrogen, halogen, and an aliphatic group; more preferably, R1 is selected from hydrogen, halogen and C1-6 alkyl (e.g., methyl and ethyl);

R2 and R3 are the same or different and independently from each other are selected from hydrogen, halogen, an aliphatic group, an aromatic group, and the like; specifically, the halogen, the aliphatic group and the aromatic group are further defined as below;

R4 is a fragment of a polysulfhydryl compound.

In a specific embodiment, the terminal group is linked to the -COOH and/or the -OH through an R group or directly to the -COOH and/or the -OH to form a side chain of at least one of the following structures:

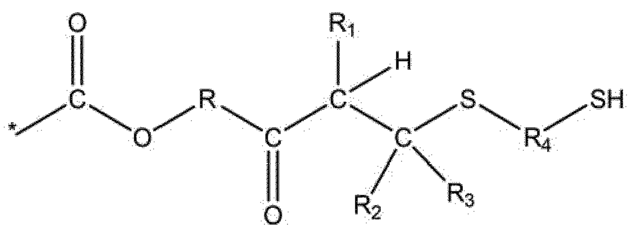

(structure a)

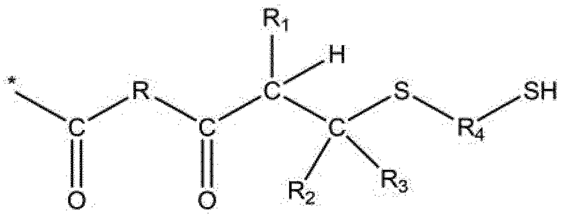

(structure b)

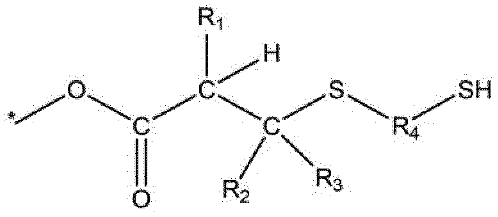

(structure c)

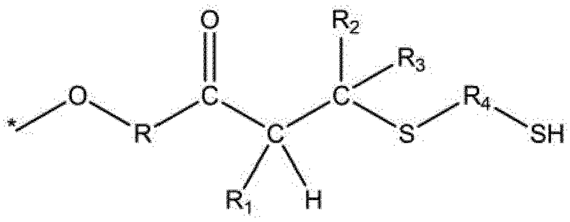

(structure d)

in the structures a, b, c and d, R is selected from

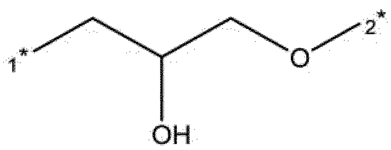

,

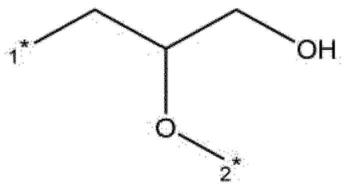

,

hydrocarbylene, arylene, an amide residue, a hydrazide residue, and the like; * represents a linking site; 1* represents a linking site to a left-hand group of R; 2* represents a linking site to a right-hand group of R; R1, R2, R3 and R4 are defined as above.

The sulfhydryl-modified hyaluronic acid has a molecular weight ranging from 5 kDa to 20 MDa. The molecular weight of the sulfhydryl-modified hyaluronic acid changes little or remains substantially unchanged before and after modification.

A sulfhydryl content of the sulfhydryl-modified hyaluronic acid as determined by the Ellman method is 0.01-30 mmol/g, for example, 0.1-10.0 mmol/g, for another example, 0.3-5.0 mmol/g, and for yet another example, 0.5-3.0 mmol/g.

For example, the sulfhydryl-modified hyaluronic acid of the present disclosure comprises at least one of the following structures:

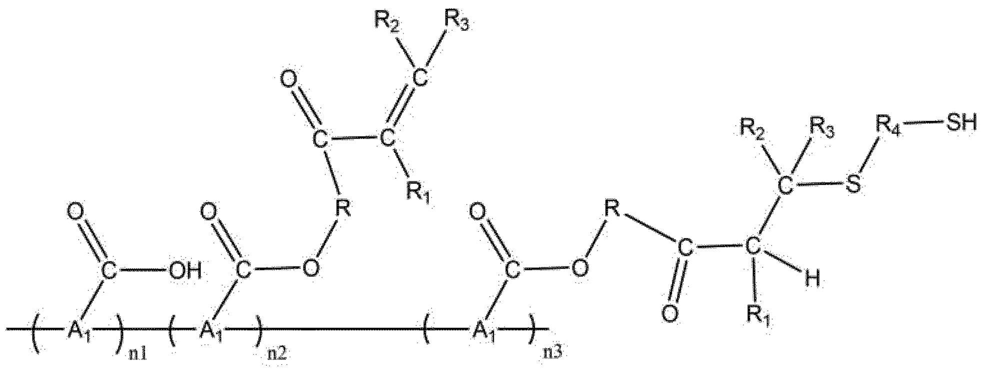

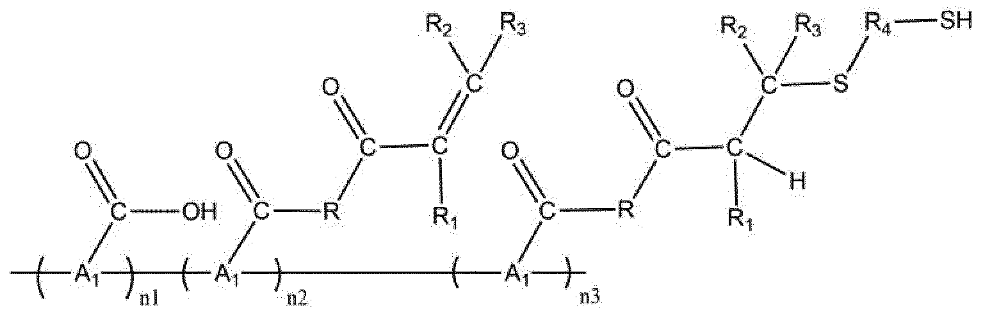

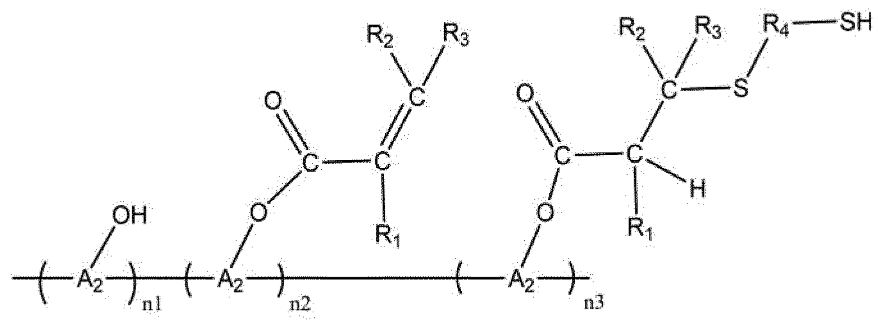

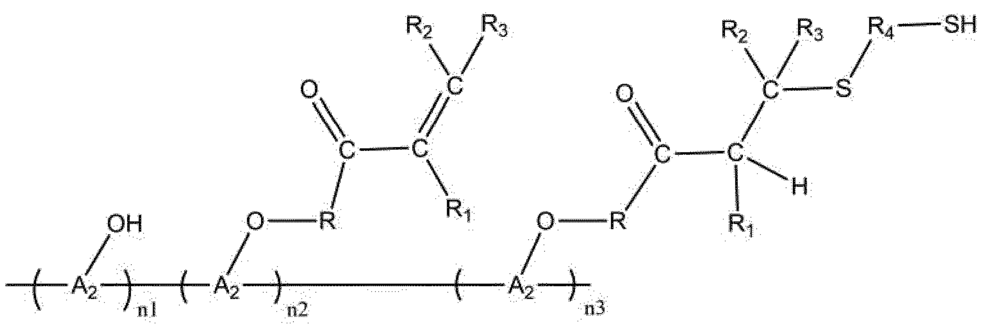

wherein in the above structures, R, R1, R2, R3 and R4 are defined as above; (n2+n3)/(n1+n2+n3) represents a degree of acryloylation; n3/(n1+n2+n3) represents a degree of sulfhydrylation corresponding to the above sulfhydryl content of the sulfhydryl-modified polymer compound as determined by the Ellman method; the n1 can be 0, and if it is 0, the degree of acryloylation is not limited, and n3/(n2+n3) alone represents the degree of sulfhydrylation corresponding to the above sulfhydryl content of the sulfhydryl-modified polymer compound as determined by the Ellman method; the n2 can be 0, and if it is 0, n3/(n1+n3) represents both the degree of acryloylation and the degree of sulfhydrylation corresponding to the above sulfhydryl content of the sulfhydryl-modified polymer compound as determined by the Ellman method;

the A1 is:

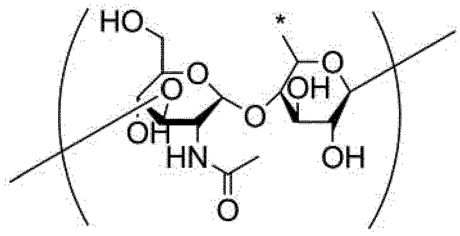

the A2 is one of the following structures:

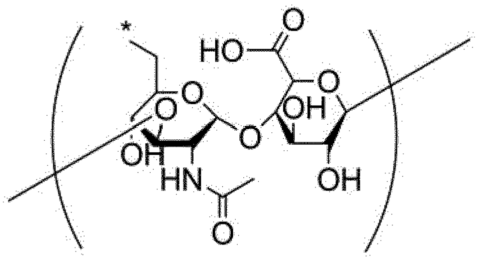

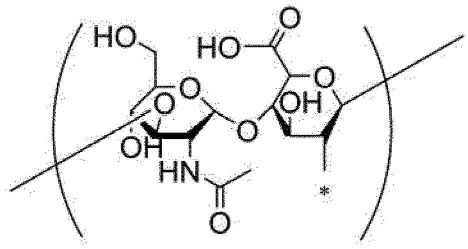

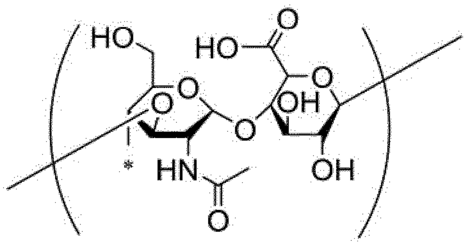

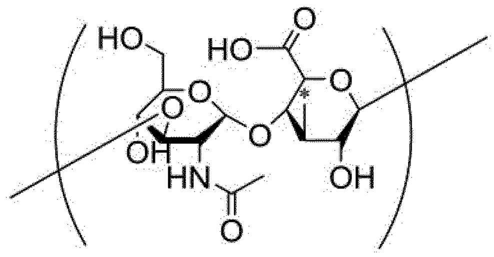

* in the structure of A1 and A2 represents a linking site to the COOH or the OH.

Specifically, the sulfhydryl-modified hyaluronic acid has at least one of the structures including but not limited to:

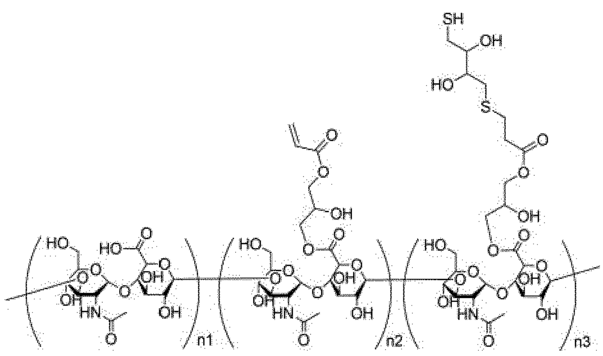

recorded as HA-A1-SH1

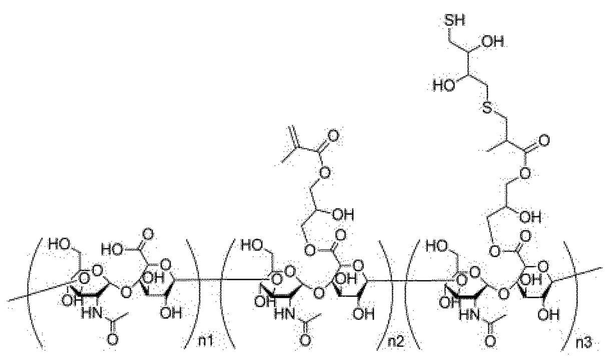

recorded as HA-MA1-SH1

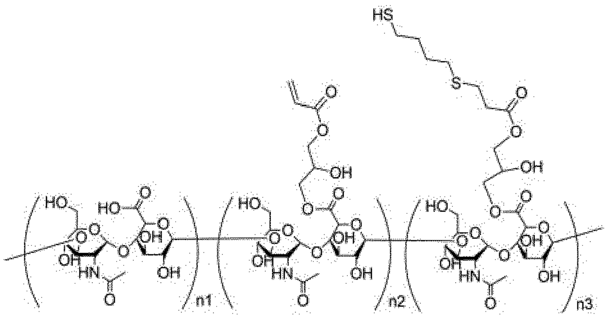

recorded as HA-A1-SH2

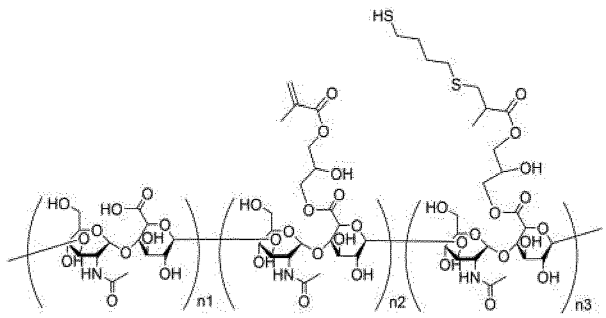

recorded as HA-MA1-SH2

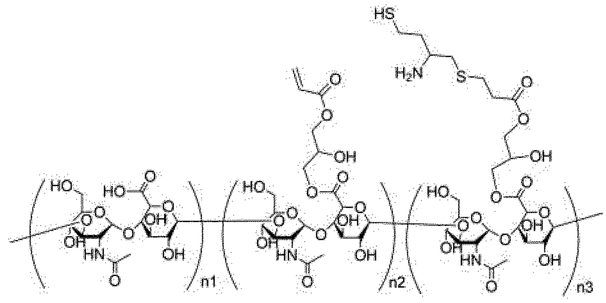

recorded as HA-A1-SH3

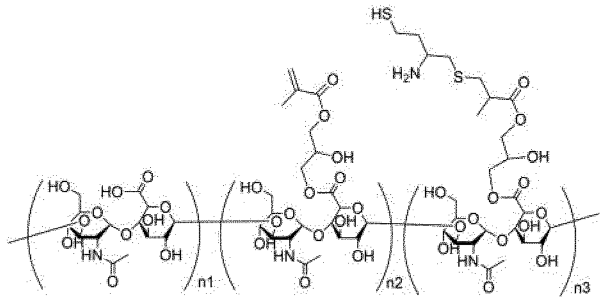

recorded as HA-MA1-SH3

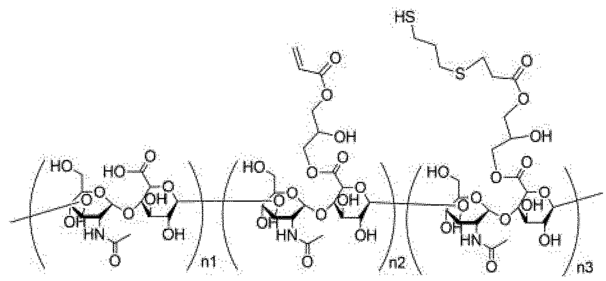

recorded as HA-A1-SH4

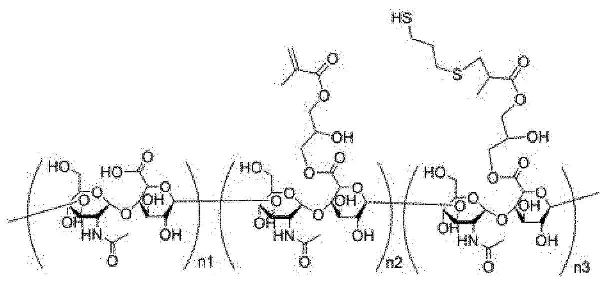

recorded as HA-MA1-SH4

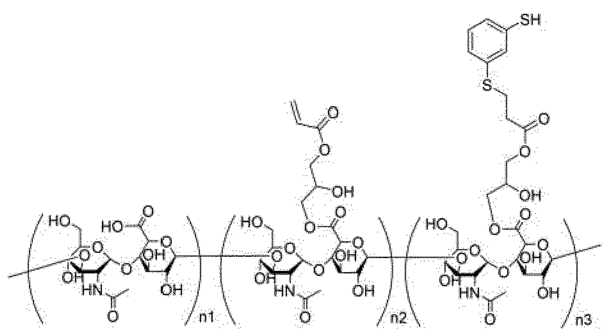

recorded as HA-A1-SH5

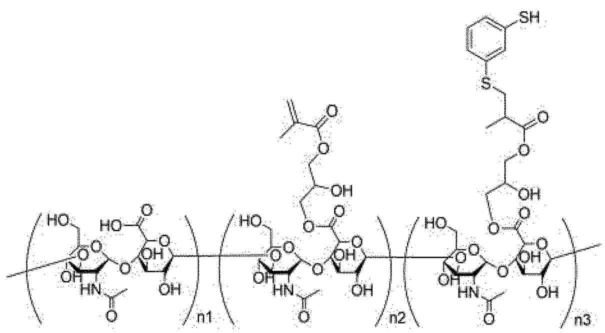

recorded as HA-MA1-SH5

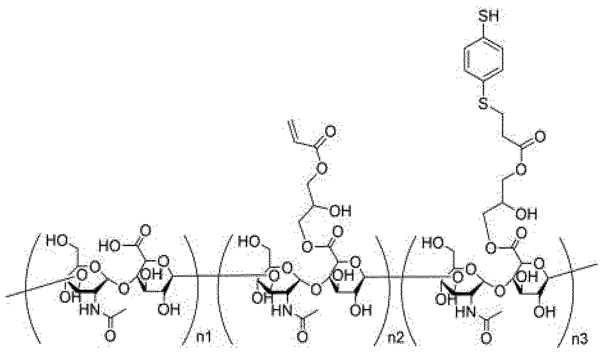

recorded as HA-A1-SH6

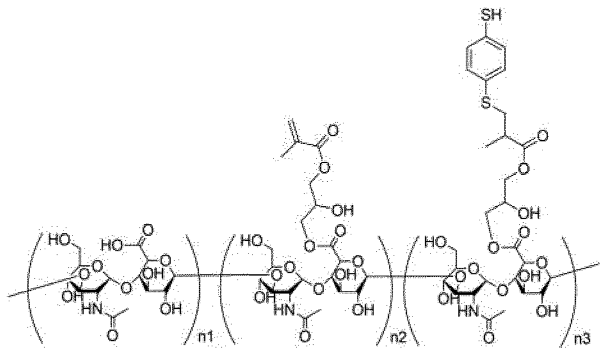

recorded as HA-MA1-SH6

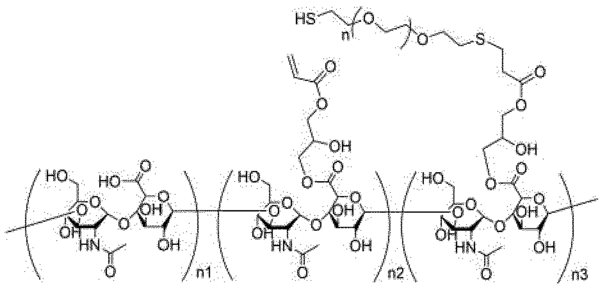

recorded as HA-A1-SH7

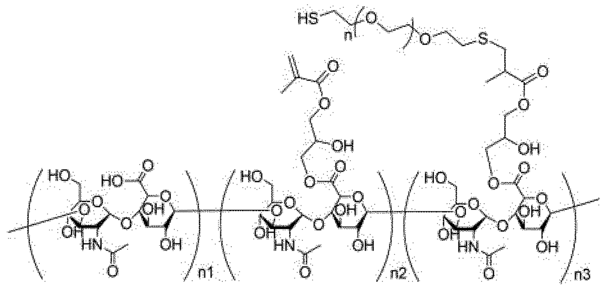

recorded as HA-MA1-SH7

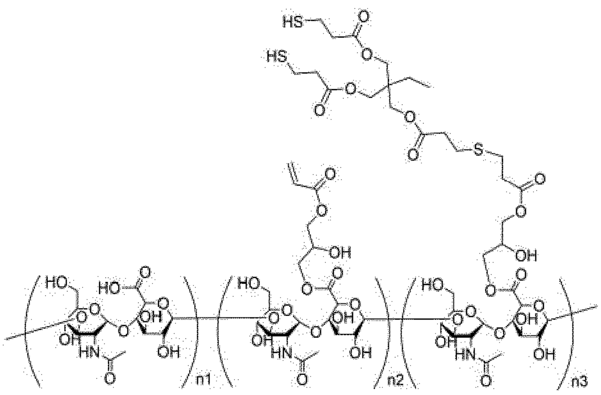

recorded as HA-A1-SH8

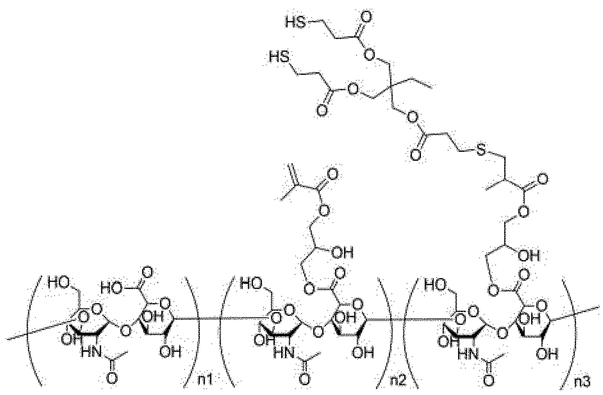

recorded as HA-MA1-SH8

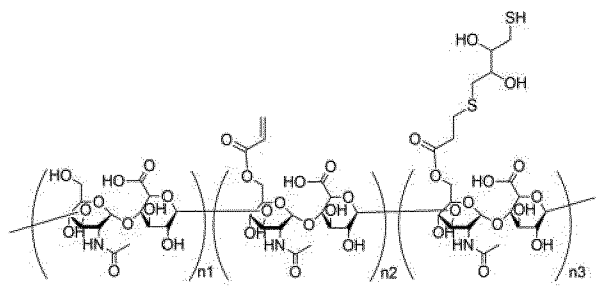

recorded as HA-A2-SH1

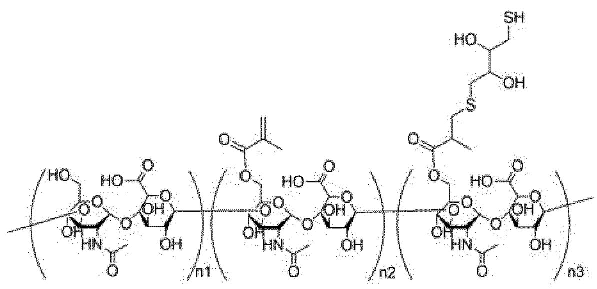

recorded as HA-MA2-SH1

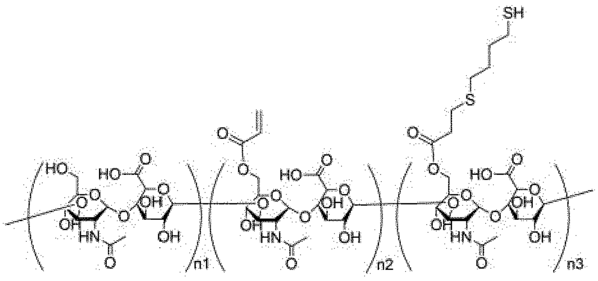

recorded as HA-A2-SH2

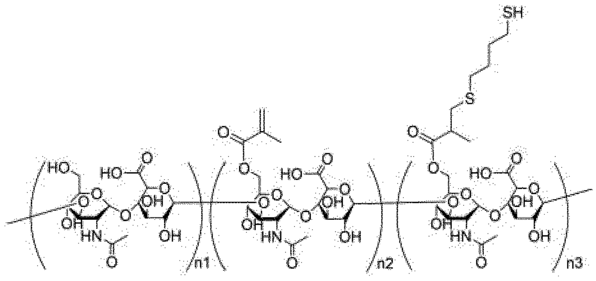

recorded as HA-MA2-SH2

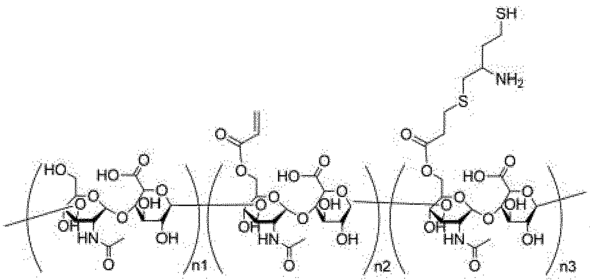

recorded as HA-A2-SH3

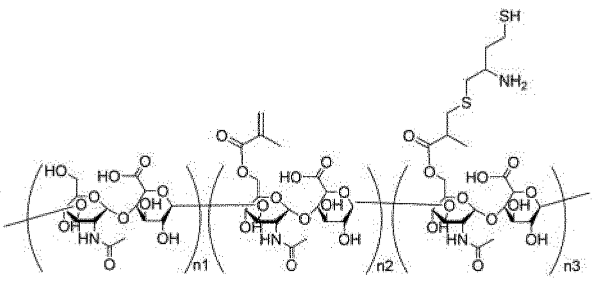

recorded as HA-MA2-SH3

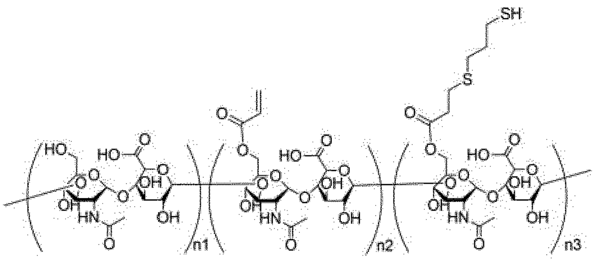

recorded as HA-A2-SH4

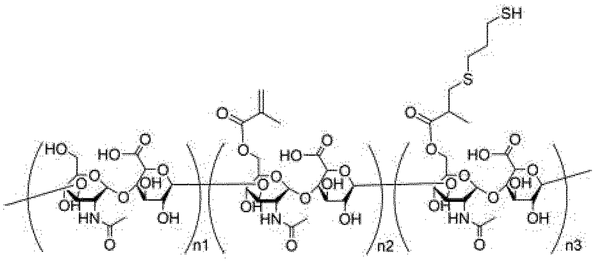

recorded as HA-MA2-SH4

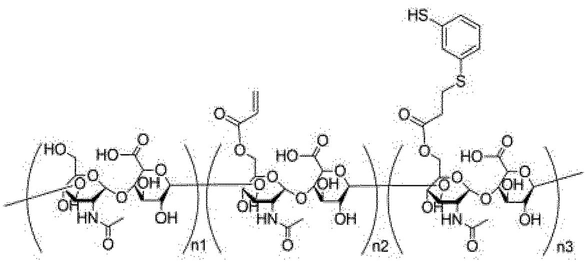

recorded as HA-A2-SH5

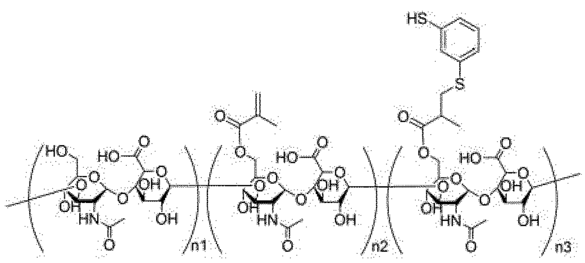

recorded as HA-MA2-SH5

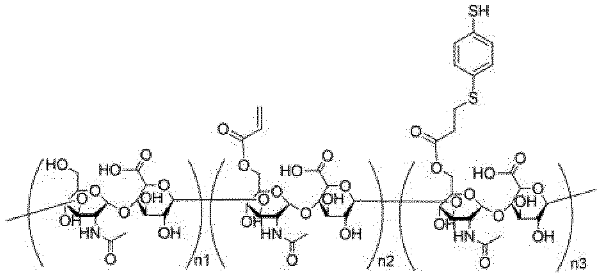

recorded as HA-A2-SH6

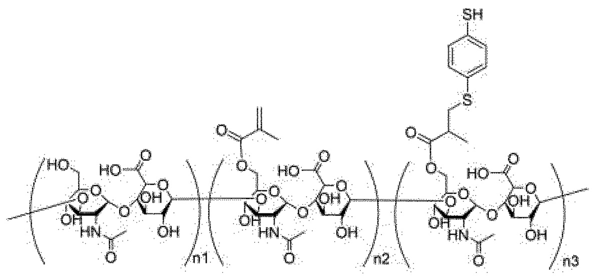

recorded as HA-MA2-SH6

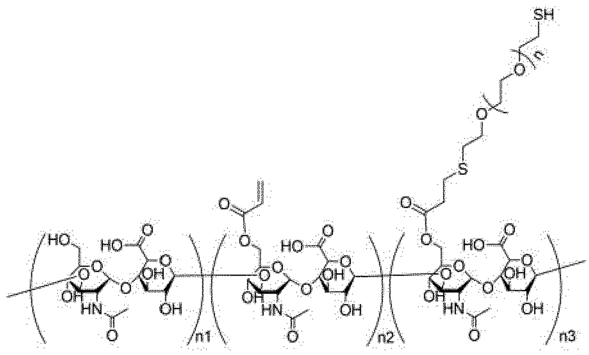

recorded as HA-A2-SH7

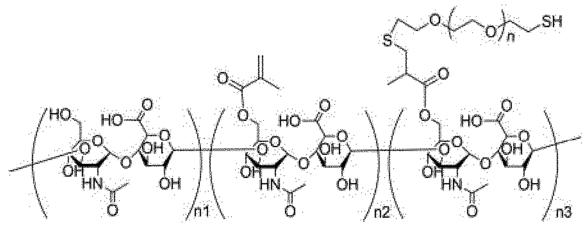

recorded as HA-MA2-SH7

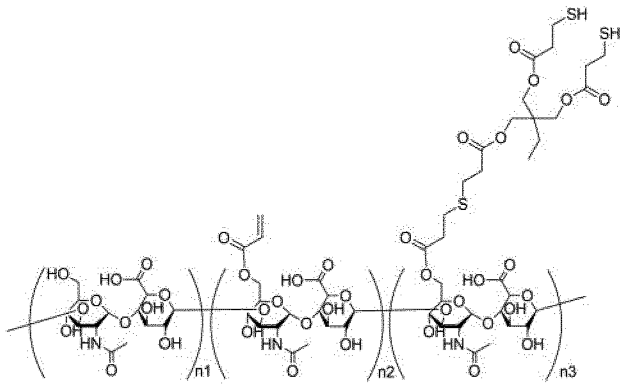

recorded as HA-A2-SH8

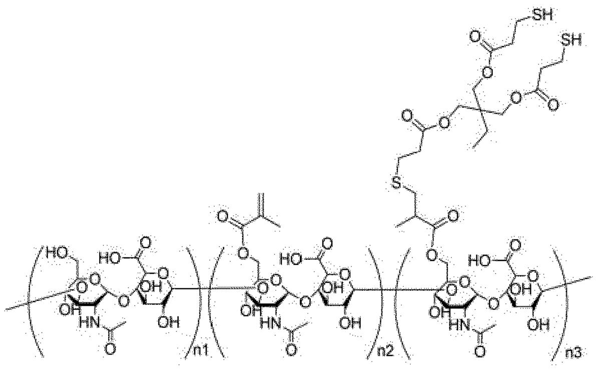

recorded as HA-MA2-SH8

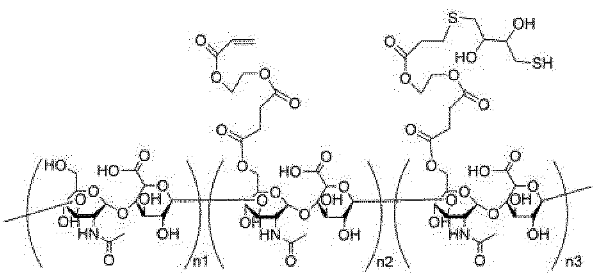

recorded as HA-A3-SH1

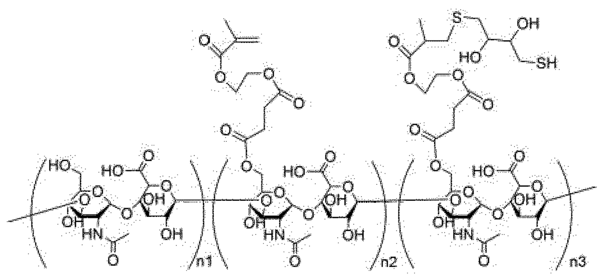

recorded as HA-MA3-SH1

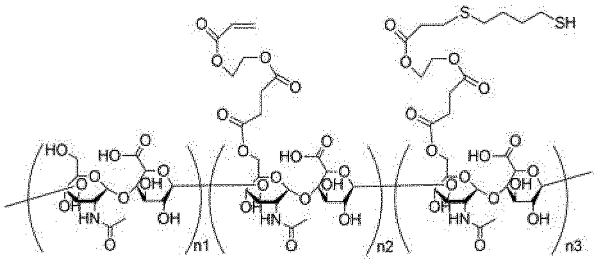

recorded as HA-A3-SH2

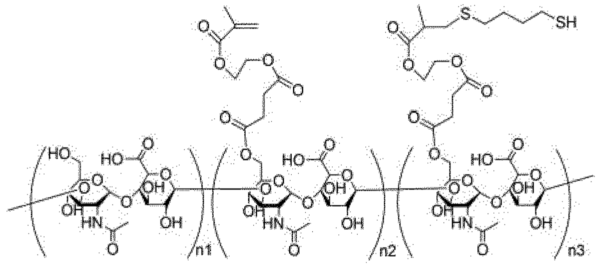

recorded as HA-MA3-SH2

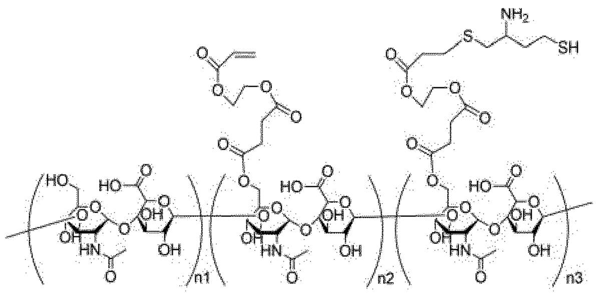

recorded as HA-A3-SH3

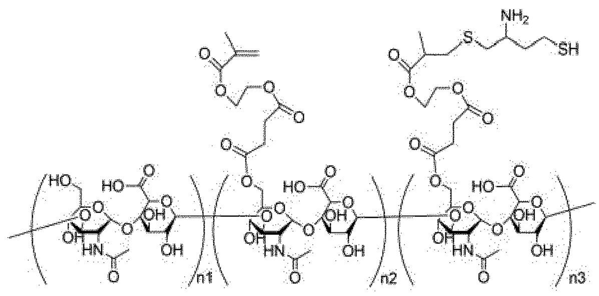

recorded as HA-MA3-SH3

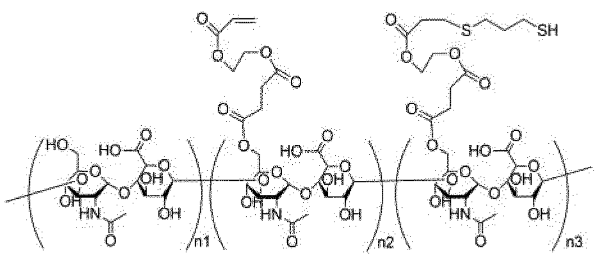

recorded as HA-A3-SH4

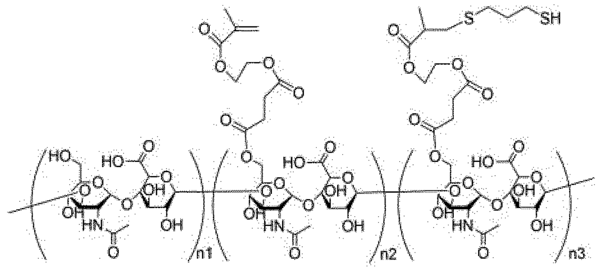

recorded as HA-MA3-SH4

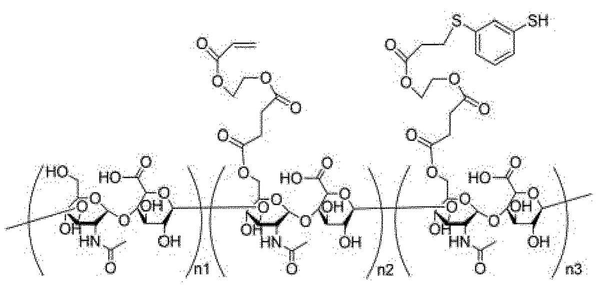

recorded as HA-A3-SH5

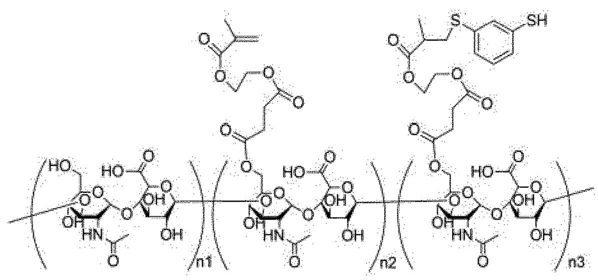

recorded as HA-MA3-SH5

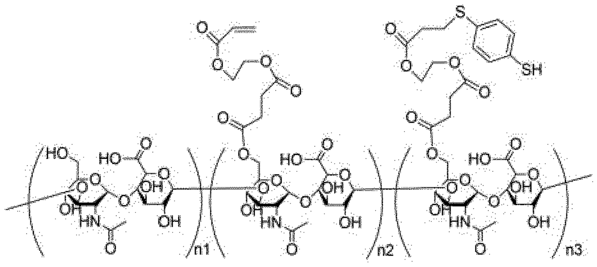

recorded as HA-A3-SH6

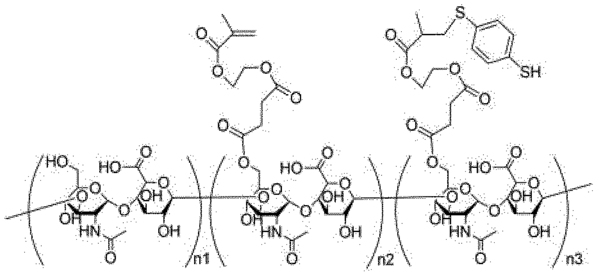

recorded as HA-MA3-SH6

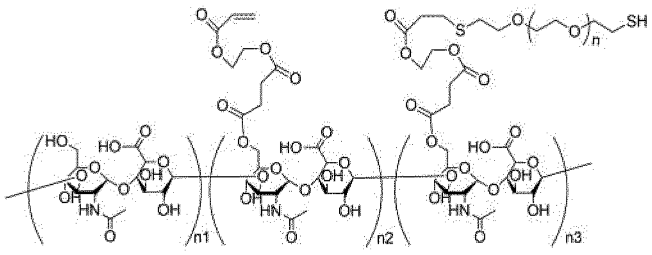

recorded as HA-A3-SH7

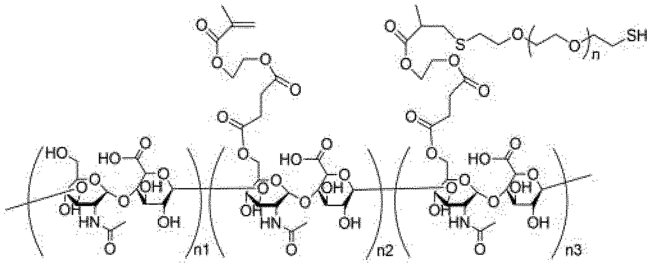

recorded as HA-MA3-SH7

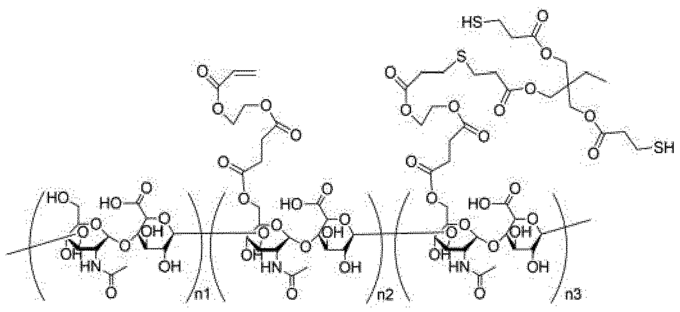

recorded as HA-A3-SH8

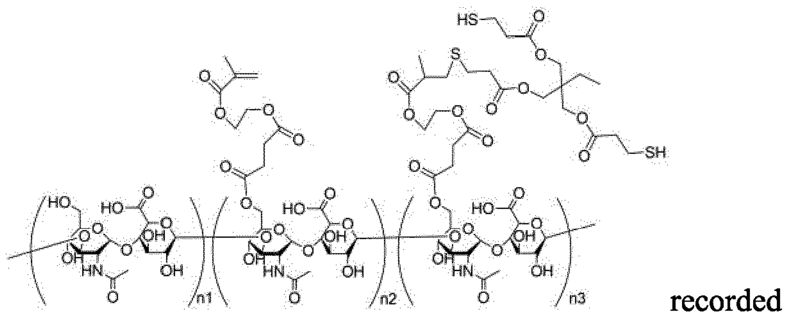

recorded as HA-MA3-SH8

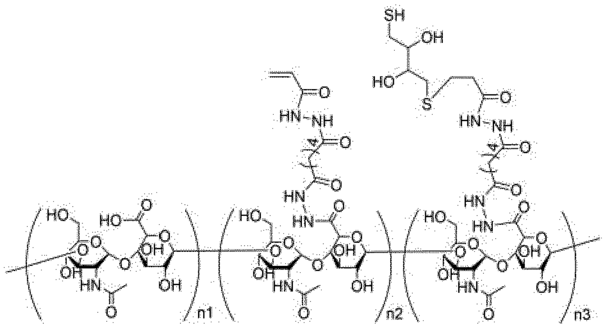

recorded as HA-A4-SH1

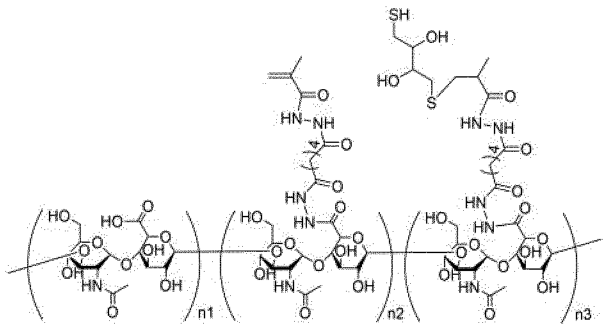

recorded as HA-MA4-SH1

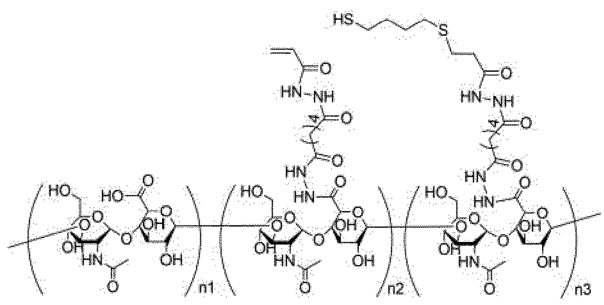

recorded as HA-A4-SH2

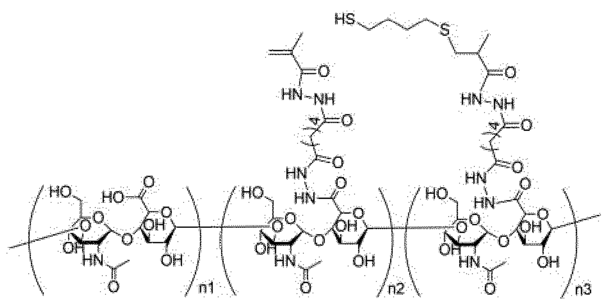

recorded as HA-MA4-SH2

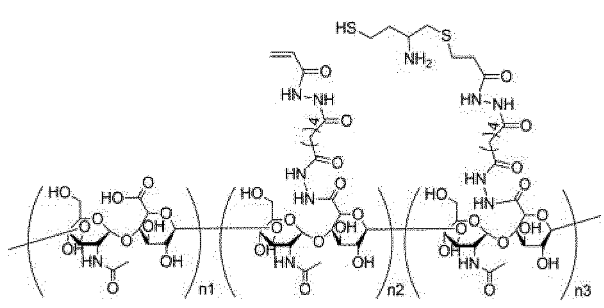

recorded as HA-A4-SH3

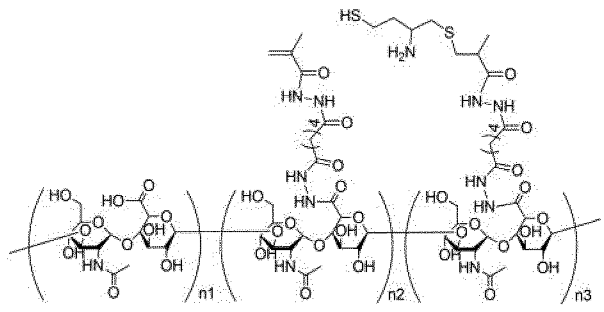

recorded as HA-MA4-SH3

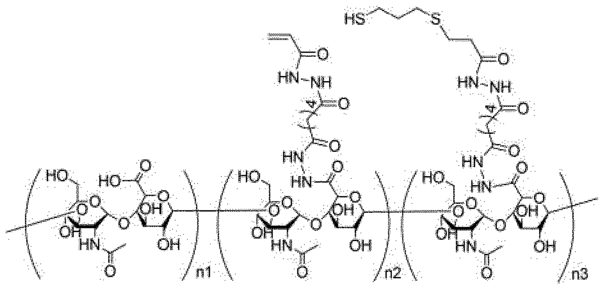

recorded as HA-A4-SH4

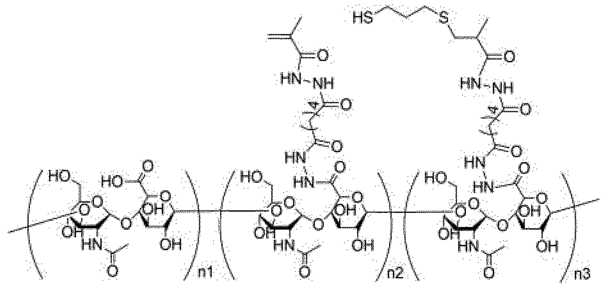

recorded as HA-MA4-SH4

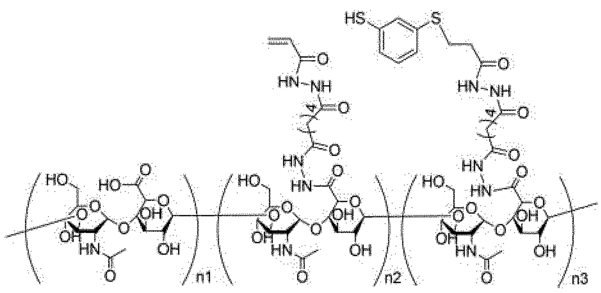

recorded as HA-A4-SH5

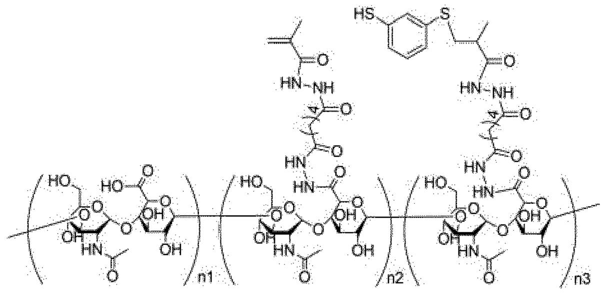

recorded as HA-MA4-SH5

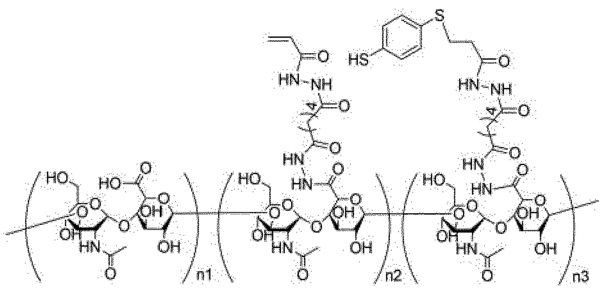

recorded as HA-A4-SH6

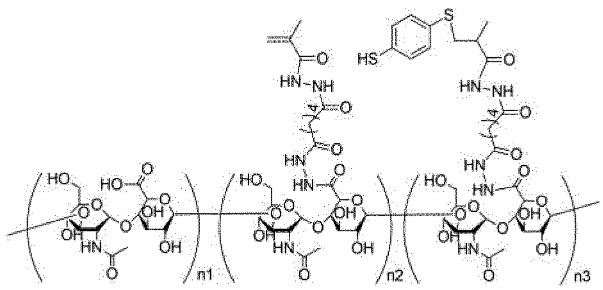

recorded as HA-MA4-SH6

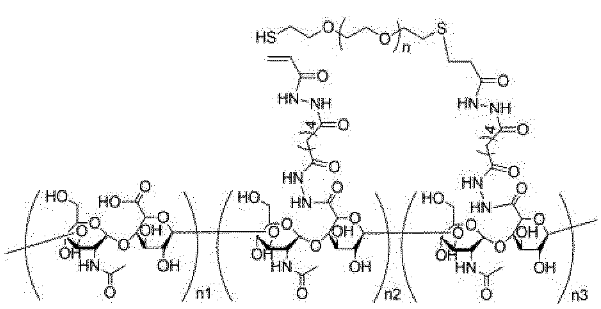

recorded as HA-A4-SH7

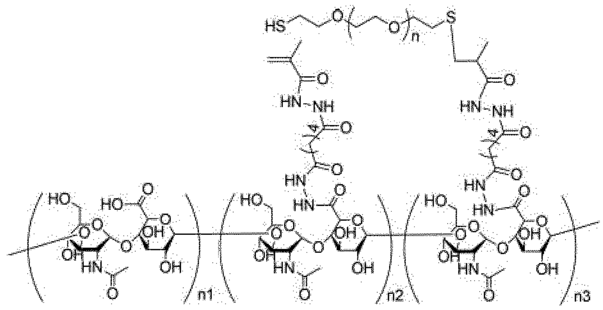

recorded as HA-MA4-SH7

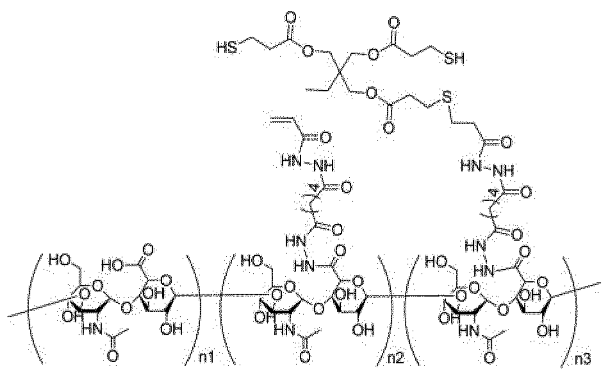

recorded as HA-A4-SH8

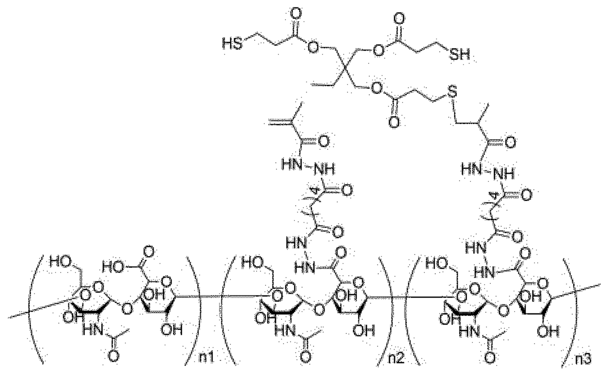

recorded as HA-MA4-SH8

in the above structural formulas, n1, n2 and n3 are defined as above.

As described above, R4 is a fragment of the polysulfhydryl compound, for example, an -S-R4-SH fragment can be introduced from the following polysulfhydryl compounds including but not limited to:

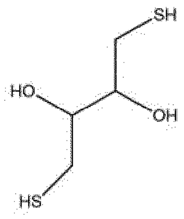

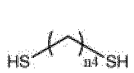

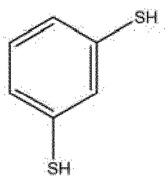

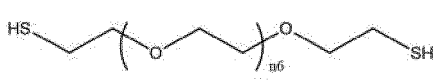

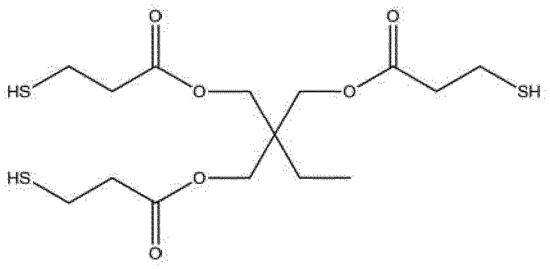

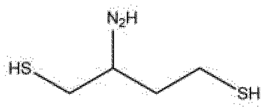

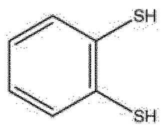

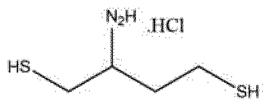

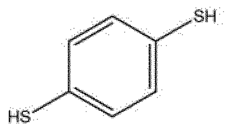

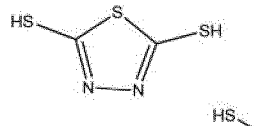

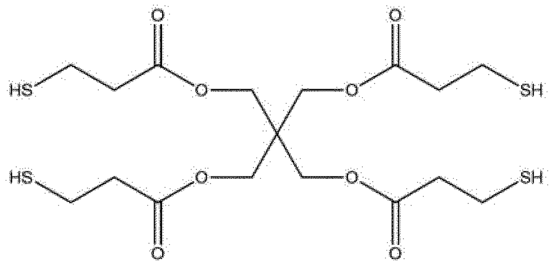

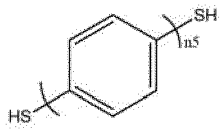

4-arm-PEG-SH

6-arm-PEG-SH

8-arm-PEG-SH

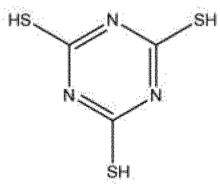

wherein n4 is an integer from 2 to 30, such as 2, 3, 4, 5 or 6 etc.; n5 is an integer from 1 to 30, such as 1, 2, 3, 4 or 5 etc.; n6 is an integer from 1 to 30, such as 1, 2, 3, 4 or 5 etc.; 4-arm-PEG-SH represents a PEG polymer containing four sulfhydryl groups; 6-arm-PEG-SH represents a PEG polymer containing six sulfhydryl groups; 8-arm-PEG-SH represents a PEG polymer containing eight sulfhydryl groups; the PEG is an abbreviation for polyethylene glycol.

[Terminologies and definitions]

As described above, R1 is selected from hydrogen, halogen, an aliphatic group, an aromatic group, and the like; R2 and R3 are the same or different and independently from each other are selected from hydrogen, halogen, an aliphatic group, an aromatic group, and the like.

As described above, the R may be selected from hydrocarbylene, arylene, an amide residue, a hydrazide residue, and the like.

As described above, the R' may be selected from a heteroatom-containing group, hydrocarbylene, arylene,

and the like.

As described above, the R" may be selected from hydrocarbylene, arylene, and the like.

The halogen refers to fluorine, chlorine, bromine or iodine.

The aliphatic group is, for example, a straight-chain or branched saturated/unsaturated aliphatic group, specifically may be alkyl, alkenyl or alkynyl.

The "hydrocarbyl" used herein alone or as a suffix or prefix is, for example, a straight-chain or branched saturated/unsaturated aliphatic group, specifically may be alkyl, alkenyl or alkynyl.

The "alkyl" used herein alone or as a suffix or prefix is intended to include both branched and straight-chain saturated aliphatic hydrocarbyl groups having 1-20, preferably 1-6, carbon atoms. For example, "C1-6 alkyl" refers to a straight-chain or branched alkyl group having 1, 2, 3, 4, 5 or 6 carbon atoms. Examples of alkyl include, but are not limited to, methyl, ethyl, n-propyl, isopropyl, n-butyl, isobutyl, sec-butyl, tert-butyl, pentyl, and hexyl.

The "alkenyl" used herein alone or as a suffix or prefix is intended to include both branched and straight-chain aliphatic hydrocarbyl groups comprising alkenyl or alkene having 2-20, preferably 2-6, carbon atoms (or the specific number of carbon atoms if provided). For example, "C2-6 alkenyl" refers to an alkenyl group having 2, 3, 4, 5 or 6 carbon atoms. Examples of alkenyl include, but are not limited to, ethenyl, allyl, 1-propenyl, 1-butenyl, 2-butenyl, 3-butenyl, 2-methylbut-2-enyl, 3-methylbut-1-enyl, 1-pentenyl, 3-pentenyl, and 4-hexenyl.

The "alkynyl" used herein alone or as a suffix or prefix is intended to include both branched and straight-chain aliphatic hydrocarbyl groups comprising alkynyl or alkyne having 2-20, preferably 2-6 carbon atoms (or the specific number of carbon atoms if provided). For example, ethynyl, propynyl (e.g., 1-propynyl, 2-propynyl), 3-butynyl, pentynyl, hexynyl and 1-methylpent-2-ynyl.

The aromatic group refers to an aromatic ring structure composed of 5-20 carbon atoms. For example, the aromatic ring structure containing 5, 6, 7 and 8 carbon atoms may be a monocyclic aromatic group, e.g., phenyl; the ring structure containing 8, 9, 10, 11, 12, 13 or 14 carbon atoms may be a polycyclic aromatic group, e.g., naphthyl. The aromatic ring may be substituted at one or more ring positions with substituents such as alkyl and halogen, e.g., tolyl. The term "aryl" also includes polycyclic ring systems having two or more rings in which two or more carbons are common to two adjacent rings (the rings are "fused rings"), and at least one of the rings is aromatic and the other rings may be, for example, cycloalkyl, cycloalkenyl, cycloalkynyl, aryl and/or heterocyclyl. Examples of polycyclic rings include, but are not limited to, 2,3-dihydro-1,4-benzodioxine and 2,3-dihydro-1-benzofuran.

The "hydrocarbylene" used herein is a group obtained by removing one hydrogen from the "hydrocarbyl".

The "arylene" used herein is a group obtained by removing one hydrogen from the "aromatic group".

The "alkylene" used herein is a group obtained by removing one hydrogen from the "alkyl".

The "amide group" used herein alone or as a suffix or prefix refers to the Ra-C(=O)-NH- group, wherein Ra is selected from the following groups unsubstituted or optionally substituted with one or more Rb: alkyl, cycloalkyl, alkenyl, cycloalkenyl, alkynyl, cycloalkynyl, heterocyclyl, aryl, heteroaryl, and the like; Rb is selected from the following groups unsubstituted or optionally substituted with one or more Rb1: halogen, hydroxyl, sulfhydryl, nitro, cyano, alkyl, alkoxy, cycloalkyl, alkenyl, alkynyl, heterocyclyl, aryl, heteroaryl, amino, carboxyl, an ester group, hydrazino, acyl, sulfinyl, sulfonyl, phosphoryl, and the like; each Rb1 independently selected from halogen, hydroxy, alkyl and aryl.

The "hydrazide group" used herein alone or as a suffix or prefix refers to the Ra-C(=O)-NH- group, wherein Ra is defined as above.

The "amide residue" used herein is a group obtained by removing one hydrogen from the "amide group".

The "hydrazide residue" used herein is a group obtained by removing one hydrogen from the "hydrazide group".

The term "cycloalkyl" used herein is intended to include saturated cyclic groups having the specified number of carbon atoms. These terms may include fused or bridged polycyclic ring systems. The cycloalkyl has 3-40 carbon atoms in its ring structure. In one embodiment, the cycloalkyl has 3, 4, 5 or 6 carbon atoms in its ring structure. For example, "C3-6 cycloalkyl" refers to a group such as cyclopropyl, cyclobutyl, cyclopentyl or cyclohexyl.

The term "cycloalkenyl" used herein is intended to include cyclic groups comprising at least one alkenyl group having the specified number of carbon atoms. These terms may include fused or bridged polycyclic ring systems. The cycloalkenyl has 3-40 carbon atoms in its ring structure. In one embodiment, the cycloalkenyl has 3, 4, 5 or 6 carbon atoms in its ring structure. For example, "C3-6 cycloalkenyl" refers to a group such as cyclopropenyl, cyclobutenyl, cyclopentenyl or cyclohexenyl.

The term "cycloalkynyl" used herein is intended to include cyclic groups comprising at least one alkynyl group having the specified number of carbon atoms. These terms may include fused or bridged polycyclic ring systems. The cycloalkynyl has 6-40 carbon atoms in its ring structure. In one embodiment, the cycloalkynyl has 6 carbon atoms in its ring structure. For example, "C3-6 cycloalkynyl" refers to a group such as cyclopropynyl, cyclobutynyl, cyclopentynyl or cyclohexynyl.

The "heteroaryl" used herein refers to a heteroaromatic heterocycle having at least one ring heteroatom (e.g., sulfur, oxygen, or nitrogen). The heteroaryl include monocyclic ring systems and polycyclic ring systems (e.g., having 2, 3 or 4 fused rings). Examples of heteroaryl include, but are not limited to, pyridinyl, pyrimidinyl, pyrazinyl, pyridazinyl,

triazinyl, furyl, quinolyl, isoquinolyl, thienyl, imidazolyl, thiazolyl, indolyl, pyrrolyl, oxazolyl, benzofuryl, benzothienyl, benzothiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, benzoxazolyl, azabenzoxazolyl, imidazothiazolyl, benzo[1,4]dioxanyl, benzo[1,3]dioxolyl, and the like. In some embodiments, the heteroaryl has 3-40 carbon atoms, and in other embodiments, 3-20 carbon atoms. In some embodiments, the heteroaryl contains 3-14, 4-14, 3-7, or 5-6 ring atoms. In some embodiments, the heteroaryl has 1-4, 1-3, or 1-2 heteroatoms. In some embodiments, the heteroaryl has 1 heteroatom.

The term "heterocyclyl" used herein refers to a saturated, unsaturated or partially saturated monocyclic, bicyclic or tricyclic ring containing 3-20 atoms, wherein 1, 2, 3, 4 or 5 ring atoms are selected from nitrogen, sulfur, oxygen or phosphorus, which, unless otherwise stated, may be linked through carbon or nitrogen, wherein the -CH2- group is optionally replaced by -C(O)-; wherein unless otherwise stated to the contrary, the ring nitrogen atom or the ring sulfur atom is optionally oxidized to form an N-oxide or S-oxide, or the ring nitrogen atom is optionally quaternized; wherein -NH in the ring is optionally substituted with acetyl, formyl, methyl, or methanesulfonyl; and the ring is optionally substituted with one or more halogens. It should be understood that when the total number of S and O atoms in the heterocyclic group exceeds 1, these heteroatoms are not adjacent to each other. If the heterocyclyl is a bicyclic or tricyclic ring, at least one ring may optionally be a heteroaromatic or aromatic ring, provided that at least one ring is non-heteroaromatic. If the heterocyclyl is a monocyclic ring, it cannot be aromatic. Examples of heterocyclyl include, but are not limited to, piperidyl, N-acetylpiperidyl, N-methylpiperidyl, N-formylpiperazinyl, N-methanesulfonylpiperazinyl, homopiperazinyl, piperazinyl, azetidinyl, oxetanyl, morpholinyl, tetrahydroisoquinolyl, tetrahydroquinolyl, indolinyl, tetrahydropyranyl, dihydro-2H-pyranyl, tetrahydrofuranyl, tetrahydrothiopyranyl, tetrahydrothiopyran-1-oxide, tetrahydrothiopyran-1,1-dioxide, 1H-pyridin-2-one, and 2,5-dioxoimidazolidinyl.

The term "acyl" used herein refers to the Ra-C(=O)- group, wherein Ra is defined as above.

The term "sulfinyl" used herein refers to the Ra-S(=O)- group, wherein Ra is defined as above.

The term "sulfonyl" used herein refers to the Ra-S(=O)2- group, wherein Ra is defined as above.

The term "phosphoryl" used herein refers to the Rc-P(=O)(Rd)- group, wherein Rc and Rd are the same or different and independently from each other are selected from the following groups, unsubstituted or optionally substituted with one or more Rb: alkyl, cycloalkyl, alkoxy, hydroxyl, alkenyl, cycloalkenyl, alkynyl, cycloalkynyl, heterocyclyl, aryl, heteroaryl, and the like; Rb is defined as above.

The term "hydrazino" used herein refers to the -NHNHRa group, wherein Ra is defined as above.

The term "amine group" used herein refers to the -NHRa group or the -N(Ra)2 group, wherein Ra is defined as above.

The term "amino" used herein refers to the -NH2 group.

The term "carboxyl" used herein refers to the -COOH group.

The term "ester group" used herein refers to the Ra-C(=O)-O- group or the Ra-O-C(=O)- group, wherein Ra is defined as above.

[Preparation Method for Sulfhydryl-modified Polymer Compound]

As described above, the present disclosure provides a preparation method for the sulfhydryl-modified polymer compound, which comprises the following steps:

1) acryloylating the polymer compound comprising at least one selected from the -COOH, the -NH2 and the -OH in the structure, namely linking at least one selected from the -COOH, the -NH2 and the -OH comprised in the structure of the polymer compound, directly or indirectly, to the following group:

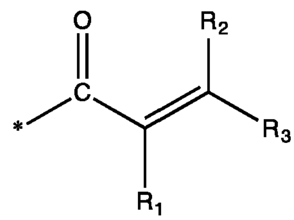

wherein R1, R2 and R3 are defined as above, and * represents a linking site;
alternatively, directly using the polymer compound comprising at least one selected from the acrylate group of formula a, the acrylamide group of formula b, and the acryloyl group of formula c in the structure as a reaction starting material;

2) reacting at least one of polymer compounds obtained in step 1) with a polysulfhydryl compound HS-R4-SH to

obtain the sulfhydryl-modified polymer compound, wherein R4 is defined as above.

In one specific embodiment of the present disclosure, the method comprises the following steps:

1) acryloylating the polymer compound comprising at least one selected from the -COOH, the -NH2 and the -OH in the structure, namely linking at least one selected from the -COOH, the -NH2 and the -OH comprised in the structure of the polymer compound, via an -R- group or directly, to the following group:

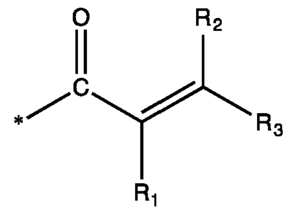

wherein R, R1, R2 and R3 are defined as above, and * represents a linking site;
alternatively, directly using the polymer compound comprising at least one selected from the acrylate group of formula a, the acrylamide group of formula b, and the acryloyl group of formula c in the structure as a reaction starting material;

2) reacting at least one of polymer compounds obtained in step 1) with a polysulfhydryl compound HS-R4-SH to obtain the sulfhydryl-modified polymer compound, wherein R4 is defined as above.

In one specific embodiment of the present disclosure, the present disclosure provides a preparation method for the sulfhydryl-modified hyaluronic acid, which comprises the following steps:

1) acryloylating the hyaluronic acid, namely linking at least one from the -COOH and the -OH comprised in the side chain of the repeating unit of the hyaluronic acid, directly or indirectly, to the following group:

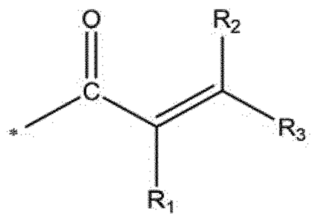

wherein R1, R2 and R3 are defined as above, and * represents a linking site;
2) reacting the acryloylated hyaluronic acid with a polysulfhydryl compound HS-R4-SH to obtain the sulfhydryl-modified hyaluronic acid, wherein R4 is defined as above.

Specifically, the step 1) is as follows: acryloylating the hyaluronic acid, namely linking at least one from the -COOH and the -OH comprised in the side chain of the repeating unit of the hyaluronic acid, via an R group or directly, to the terminal group to form the side chain of at least one of the following structures:

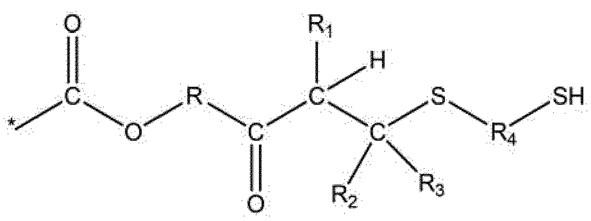

(structure a)

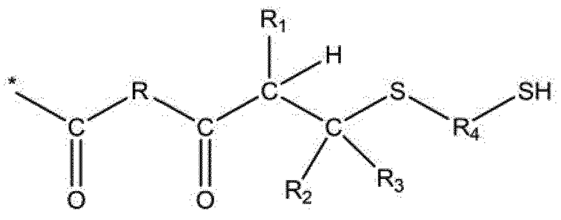

(structure b)

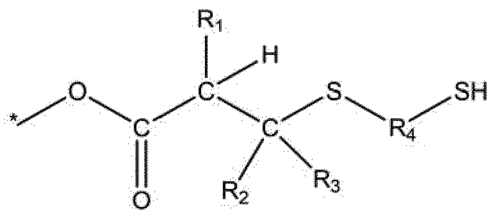

(structure c)

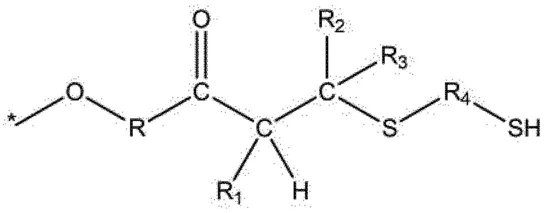

(structure d)

in the structures a, b, c and d, R, R1, R2, R3 and R4 are defined as above, and * represents a linking site.

In step 1), the acryloylating step can be performed by reacting the polymer compound to be modified with an acrylate compound, or by reacting the polymer compound to be modified with an acryloyl chloride compound or an acrylic anhydride compound.

The acrylate compound may be one or more of an alkyl acrylate compound, an aryl acrylate compound and a glycidyl acrylate polyol compound.

The polyol in the glycidyl acrylate polyol compound is, for example, a triol, specifically, glycerin, butanetriol, pentanetriol, and the like.

In step 1), the acryloylating step may be a conventional reaction step, which can be performed under existing conventional conditions. Generally, it is performed by reacting acryloyl chloride and derivatives thereof or acrylic anhydride and derivatives thereof with a polymer compound comprising at least one from -OH and -NH2. It can also be performed by reacting glycidyl acrylate and derivatives thereof with the polymer compound comprising at least one selected from -COOH, -OH and -NH2.

In step 1), the acryloylating step can be an unconventional reaction step, namely using a method other than the above method to synthesize a polymer compound comprising a structure of formula c.

In step 2), the reaction with the polysulfhydryl compound HS-R4-SH is performed in a solvent. The solvent is, for example, water or an organic solvent, and further can be deionized water or dimethylformamide.

In step 2), the reaction with the polysulfhydryl compound HS-R4-SH is performed under low to high temperature conditions. For example, the reaction temperature is 0-80 °C, and further can be 10-70 °C, and for example, the reaction can be performed at room temperature.

In step 2), the reaction time for the reaction with the polysulfhydryl compound HS-R4-SH is 0.1-100 h.

In step 2), the pH range for the reaction with the polysulfhydryl compound HS-R4-SH is -1-15. For example, the reaction pH can be 6-8, and for another example, 7.

In step 2), the reaction further comprises a post-treatment step.

In the post-treatment step, a dialysis method is adopted. Specifically, the solution after the reaction is filled into a dialysis bag (for example, a dialysis bag with a molecular weight cutoff of 2 kDa or more), dialyzed against a hydrochloric acid solution (for example, at pH 4) for several days (for example, 1-10 days, for another example, 5 days, and the like), optionally refreshed with water (for example, refreshed with water every day or every other day) for several times (for example, twice or more, and the like), and finally collected and dried (for example, lyophilized) to obtain a solid or viscous liquid, i.e., the sulfhydryl-modified polymer compound.

The present disclosure firstly provides a preparation method for the sulfhydryl-modified polymer compound by the Michael addition reaction of the sulfhydryl of the polysulfhydryl compound with the carbon-carbon double bond in

the acryloyl group. The method has a high degree of sulfhydrylation, mild conditions for the sulfhydrylation reaction (can be performed at room temperature in an aqueous solution) and no pollution, and the prepared sulfhydryl-modified polymer compound has high purity and is particularly suitable for further use in the fields such as pharmaceuticals, cosmetology and medicine.

[Acryloylated polymer compound]

As described above, the system to be gelled of the present disclosure may further comprise a substance C1: an acryloylated polymer compound, and the acryloylated polymer compound of the present disclosure may be selected from at least one of the following substances:

1) an acryloylated compound of a polymer compound comprising at least one selected from -COOH, -NH2 and -OH in the structure, namely, an acryloylated compound formed by linking at least one selected from -COOH, -NH2 and -OH comprised in the structure of the polymer compound, directly or indirectly, to the following group:

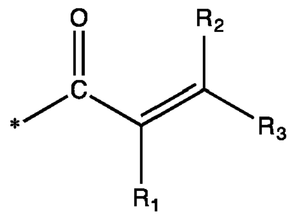

wherein R1, R2 and R3 are defined as above, and * represents a linking site;
2) a polymer compound comprising at least one selected from the acrylate group of formula a, the acrylamide group of formula b and the acryloyl group of formula c in the structure.

In the above substance 1), part or all of the -COOH and/or the -NH2 and/or the -OH are modified to form at least one of the following structures:

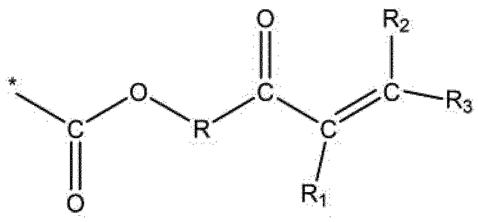

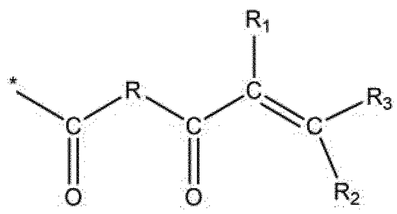

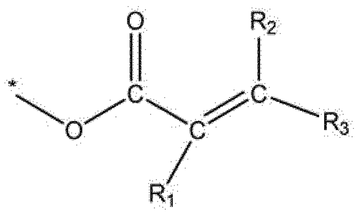

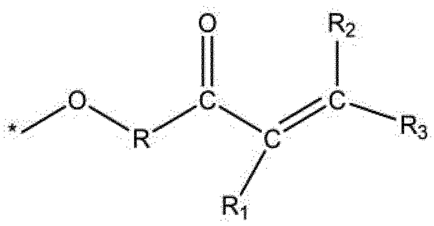

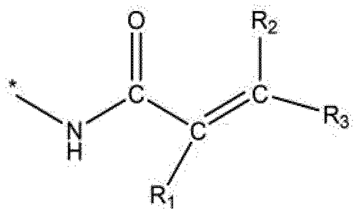

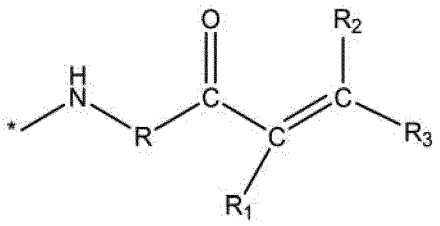

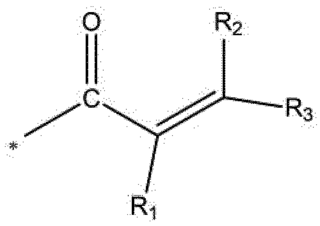

wherein in the above structures, R is selected from

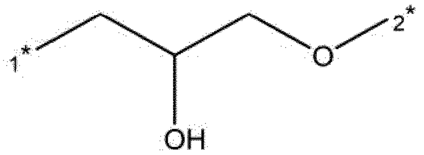

,

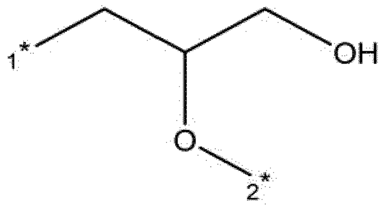

,

hydrocarbylene, arylene, an amide residue, a hydrazide residue, and the like; * represents a linking site; 1* represents a linking site to a left-hand group of R; 2* represents a linking site to a right-hand group of R; R1, R2, R3 and R4 are defined as above.

In the above substance 1), at least one selected from the -COOH, the-NH2 and the -OH may be directly linked to the main chain of the polymer compound, or linked to the main chain of the polymer compound via an R' group, and the R' group may be a heteroatom-containing group, hydrocarbylene, arylene or the following linker:

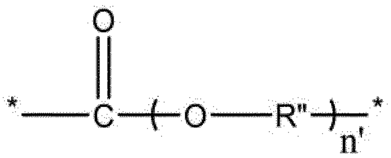

wherein in the above formula, R" is hydrocarbylene or arylene, n' is an integer from 1 to 1000, and * represents a linking site.

The heteroatom-containing group includes, but is not limited to an ester group, an amide residue or a hydrazide residue. Specifically, the ester group, the amide residue or the hydrazide residue are further defined herein.

In the above substance 1), the polymer compound to be acryloylated comprises natural mucopolysaccharide polymers, such as at least one selected from chitosans (specifically chitosan, ethylene glycol chitosan, carboxymethyl chitosan, etc.), chondroitin sulfate, hyaluronic acid, and alginate; proteins such as gelatin, fibrin and serum proteins; and/or, synthetic polymers, such as at least one selected from polyvinyl alcohol, poly(meth)acrylic acid, poly(meth)hydroxyalkyl acrylate (e.g., poly(meth)hydroxyethyl acrylate), and hyperbranched polyethylene glycol.

In the above substance 1), the acryloylated compound comprises at least one of the following structures:

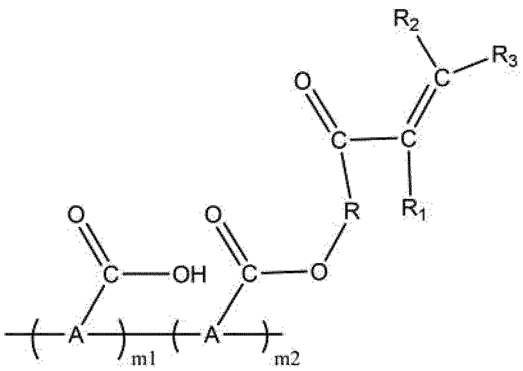

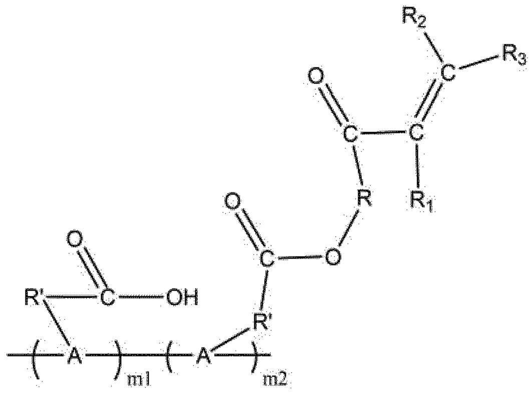

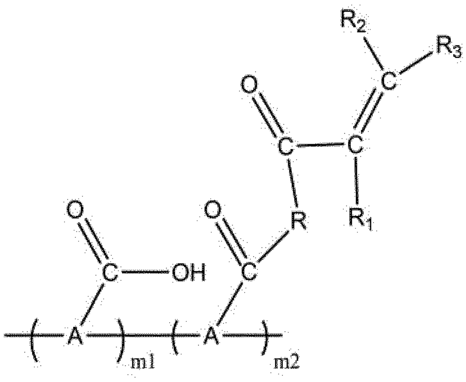

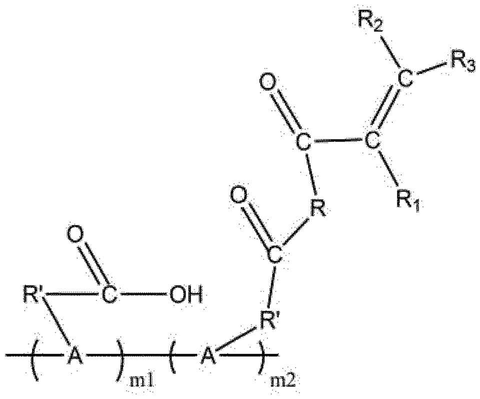

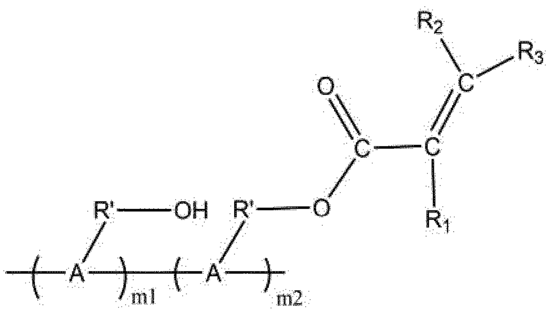

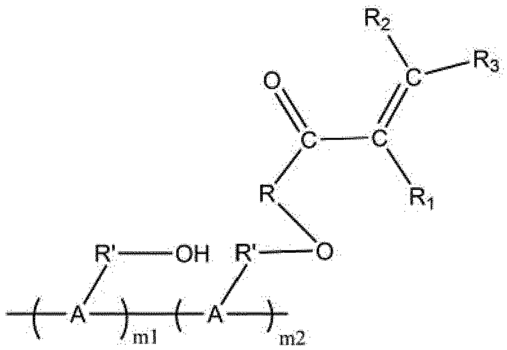

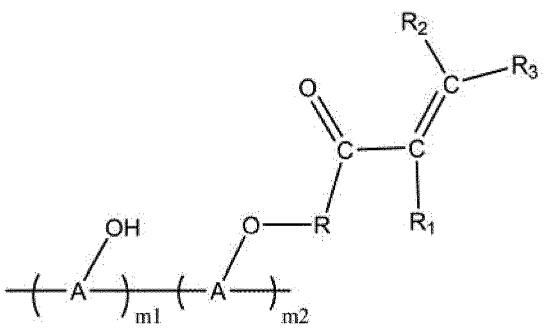

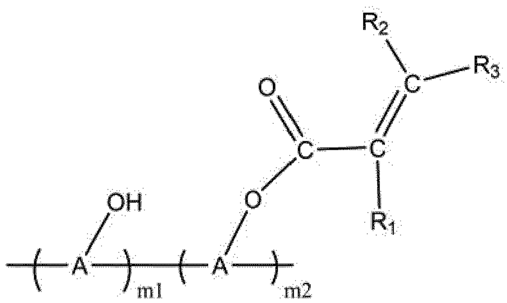

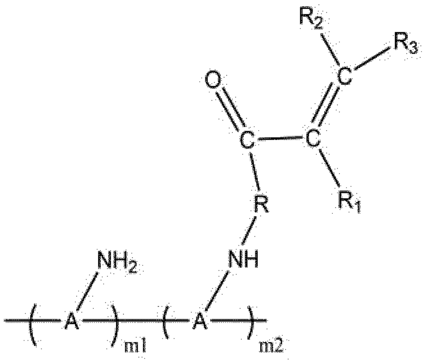

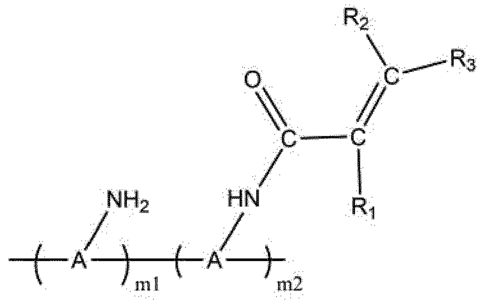

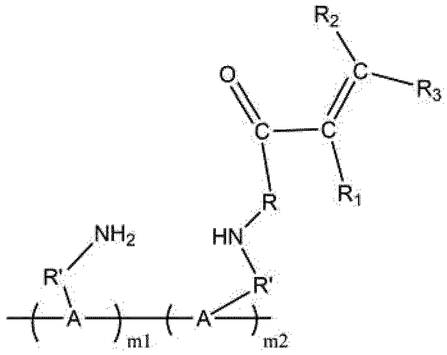

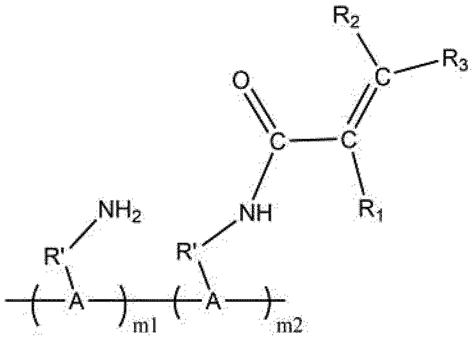

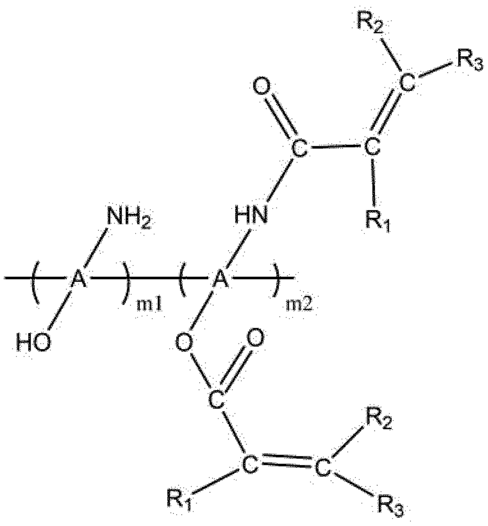

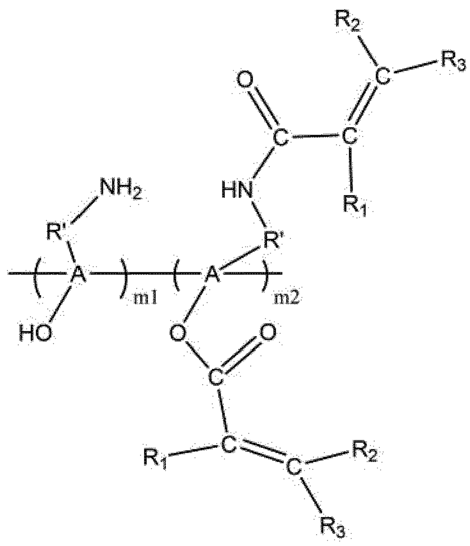

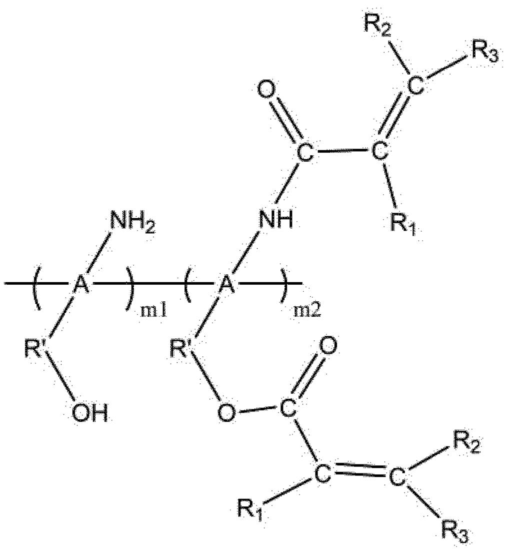

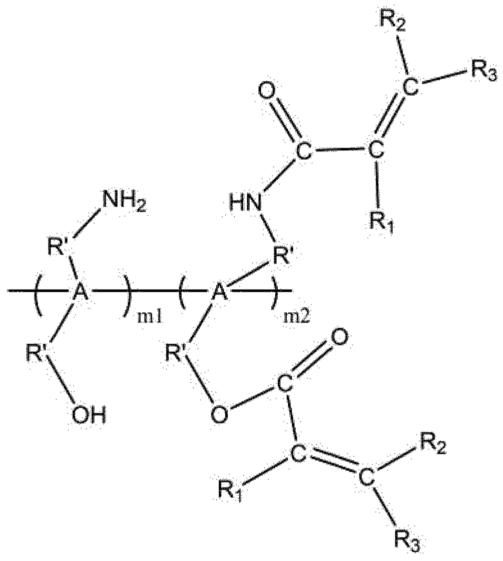

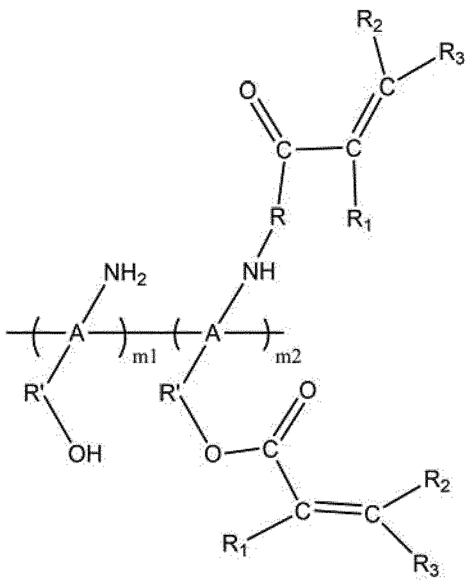

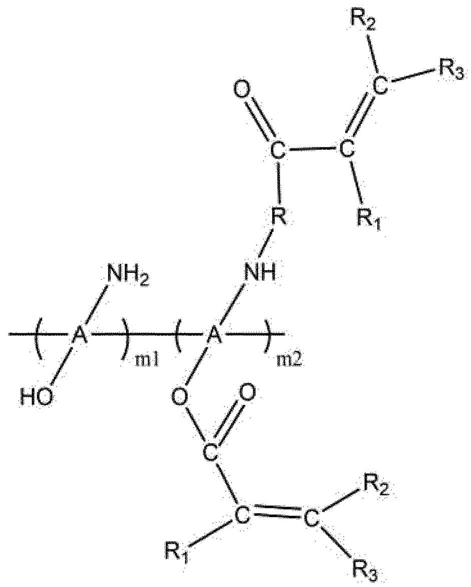

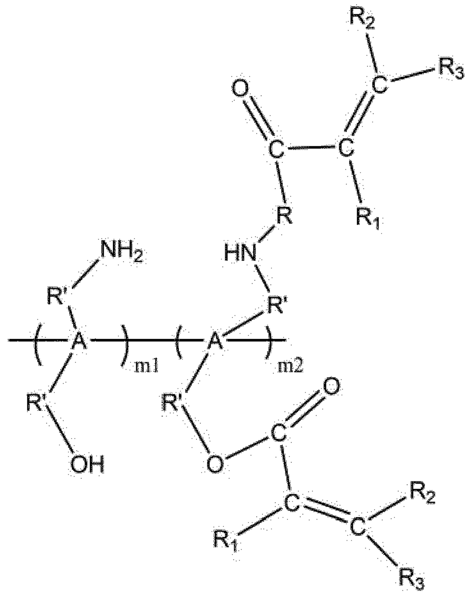

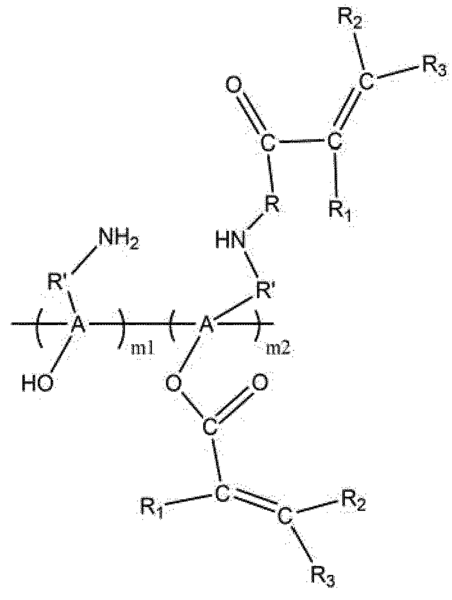

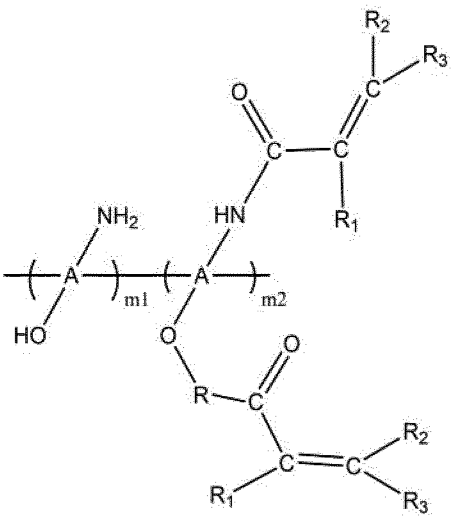

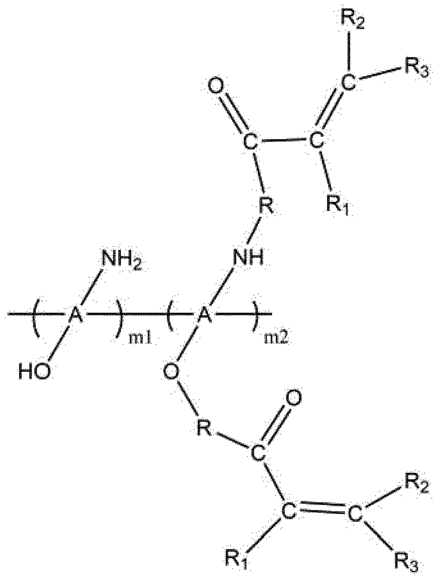

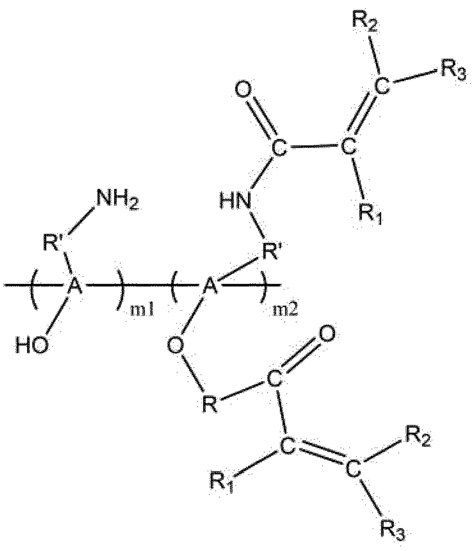

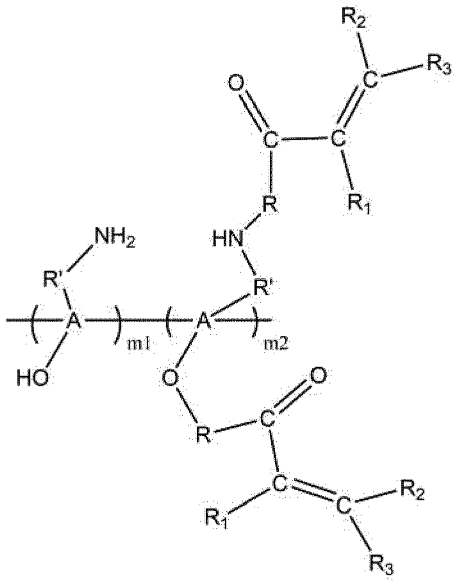

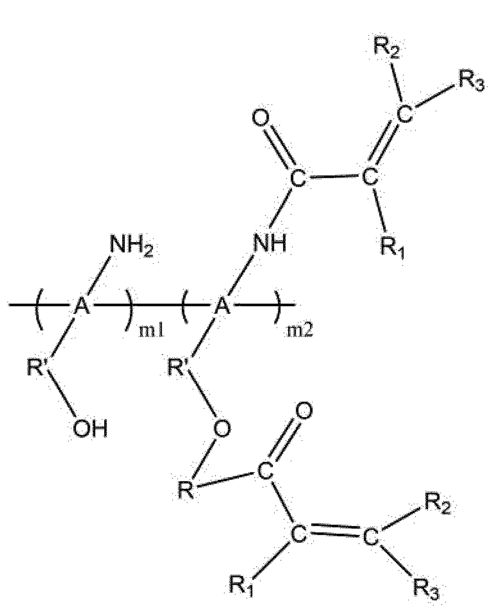

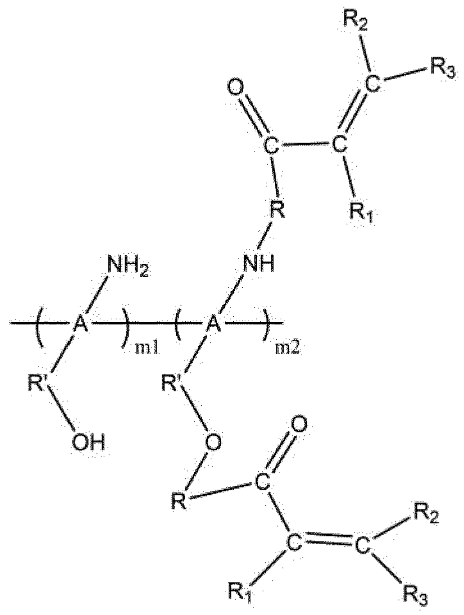

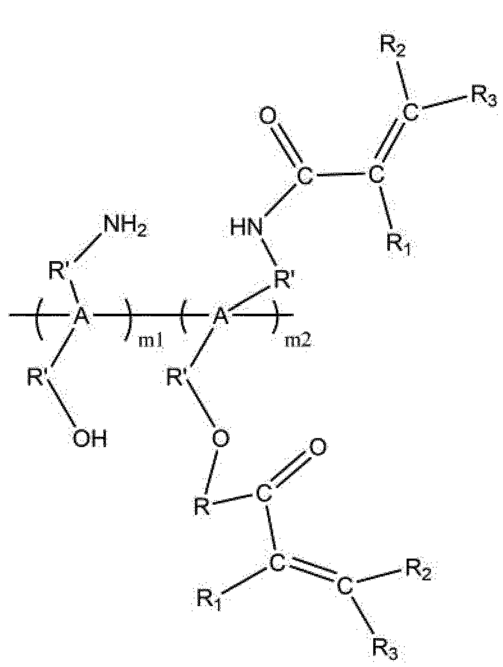

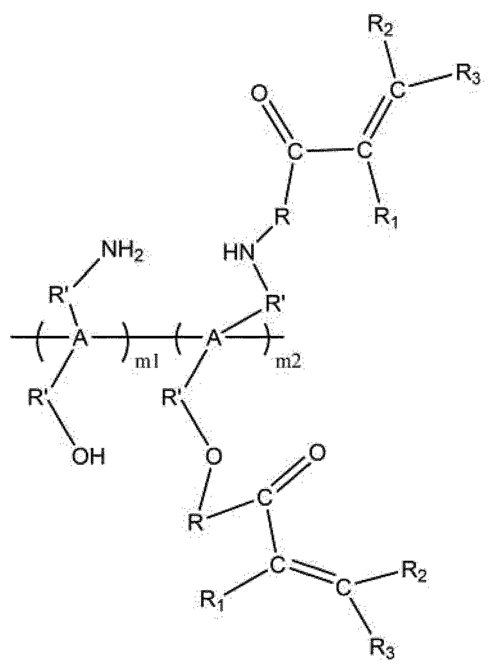

In the above structures, A is a fragment of a compound to be acryloylated comprising at least one selected from -COOH, -NH2 and -OH in the structure; R, R', R1, R2, R3 and R4 are defined as above; and (m2/(m1 + m2) represents the degree of acryloylation.

Specifically, the A can be a structure shown as follows:

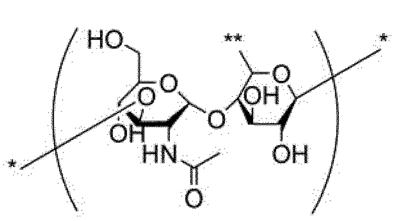

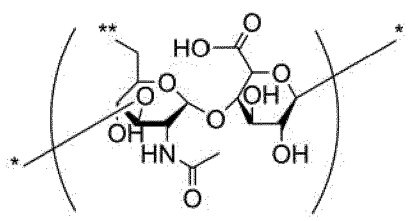

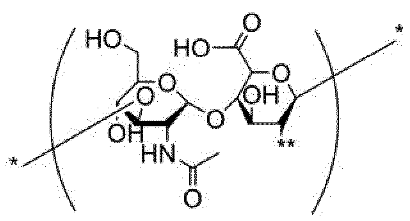

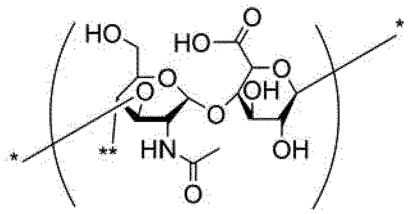

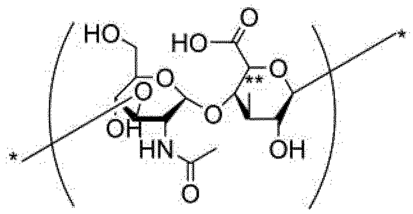

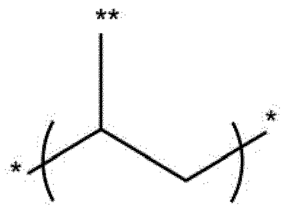

In each of the above structures, * represents a linking site between repeating units of the main chain; ** represents a linking between -COOH, -NH2 or -OH and the fragment, or a linking site between an R' group and the fragment.

The above substance 2) may be one of the following polymers Gelatin-A, Gelatin-MA, CTS-A, CTS-MA, PHEMA-A, PHEMA-MA, HB-PEG, PVA-A, PVA-MA, CHS-A and CHS-MA:

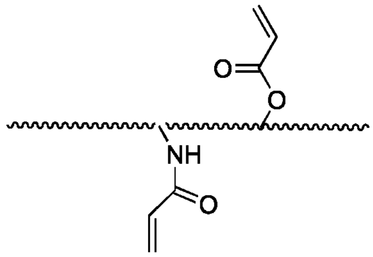

Gelatin-A

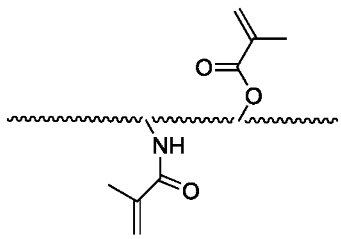

Gelatin-MA

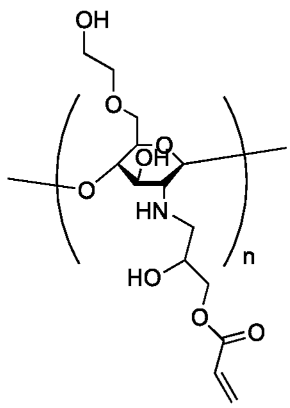

CTS-A

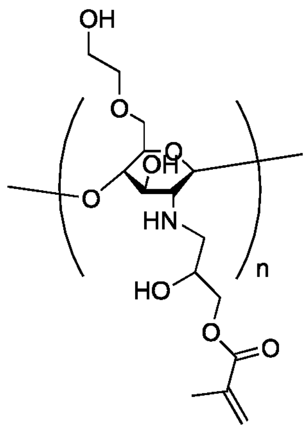

CTS-MA

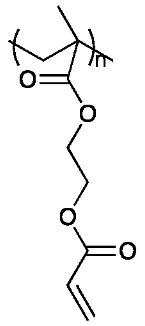

PHEMA-A

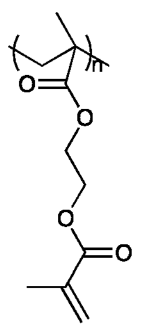

PHEMA-MA

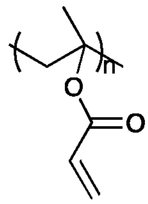

PVA-A

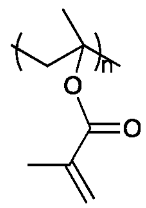

PVA-MA

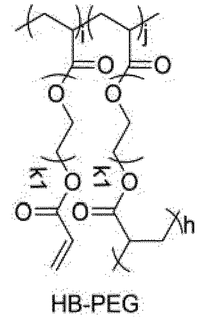

HB-PEG

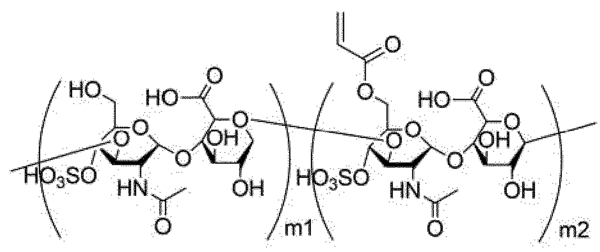

CHS-A

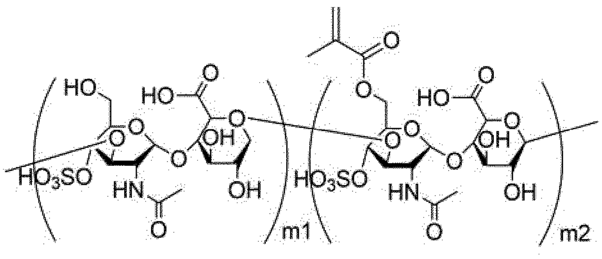

CHS-MA .

It should be noted that Gelatin-A, Gelatin-MA, CTS-A, CTS-MA, PHEMA-A, PHEMA-MA, HB-PEG, PVA-A, PVA-MA, CHS-A and CHS-MA are abbreviations for the names of polymers having the above structures, and letters therein, when being separated, are not related to the meaning of letters appearing elsewhere in the present disclosure.

[Small molecule cross-linking agent]

As described above, the system to be gelled of the present disclosure also comprises a substance C2: a small molecule cross-linking agent containing an acryloyl group, wherein the small molecule cross-linking agent includes, but is not limited to, small molecule compounds containing an acryloyl group or oligomers containing an acryloyl group, and specifically, is selected from ethylene glycol diacrylate (EGDA), polyethylene glycol diacrylate (PEGDA), trimethylolpropane triacrylate (TMPTA), pentaerythritol triacrylate (PTA), pentaerythritol tetraacrylate (PTTA), di(trimethylolpropane) tetraacrylate (DTTA), and the like.

[Hydrogel]

As described above, the present disclosure provides a hydrogel prepared by gelation of a system comprising the following substances:

(i) the sulfhydryl-modified polymer compound above, and
(ii) at least one selected from the substance C1 and the substance C2.

In one specific embodiment of the present disclosure, the hydrogel is prepared by gelation of the sulfhydryl-modified polymer compound above and the acryloylated polymer compound above,
wherein the sulfhydryl-modified polymer compound and the acryloylated polymer compound are subjected to cross-linking reaction after being fully contacted, the viscosity of the mixed system is increased immediately, and finally a uniform gel system is formed.

In one specific embodiment of the present disclosure, the hydrogel is prepared by gelation of the sulfhydryl-modified polymer compound above and the small molecule cross-linking agent above,
wherein the sulfhydryl-modified polymer compound and the small molecule cross-linking agent are subjected to cross-linking reaction after being fully contacted, the viscosity of the mixed system is increased immediately, and finally a uniform gel system is formed.

In one specific embodiment of the present disclosure, the hydrogel is prepared by gelation of the sulfhydryl-modified polymer compound above, the acryloylated polymer compound above and the small molecule cross-linking agent above,
wherein the sulfhydryl-modified polymer compound and the acryloylated polymer compound and small molecule cross-linking agent are subjected to cross-linking reaction after being fully contacted, the viscosity of the mixed system is increased immediately, and finally a uniform gel system is formed.

The hydrogel comprises the following characteristic structural unit:

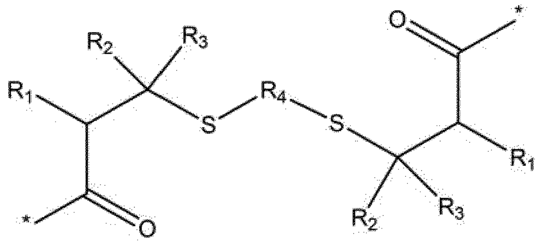

wherein in the above unit, R1, R2, R3 and R4 are defined as above, and * represents a linking site.

The amount ratio (1 part by mass in total) of the sulfhydryl-modified polymer compound to the acryloylated polymer compound is 0.01:0.99-0.99:0.01. For example, it may be 0.1:0.9-0.9:0.1, such as 0.01:0.99, 0.1:0.9, 0.15:0.85, 0.2:0.8, 0.3:0.7, 0.4:0.6, 0.5:0.5, 0.6:0.4, 0.7:0.3, 0.8:0.2, 0.85:0.15, 0.9:0.1, 0.99:0.01 or any ratio within the interval.

The amount ratio (1 part by mass in total) of the sulfhydryl-modified polymer compound to the small molecule cross-linking agent is 0.01:0.99-0.99:0.01. For example, it may be 0.1:0.9-0.9:0.1, such as 0.01:0.99, 0.1:0.9, 0.15:0.85, 0.2:0.8, 0.3:0.7, 0.4:0.6, 0.5:0.5, 0.6:0.4, 0.7:0.3, 0.8:0.2, 0.85:0.15, 0.9:0.1, 0.99:0.01 or any ratio within the interval.

The amount ratio (1 part by mass in total) of the sulfhydryl-modified polymer compound to the acryloylated polymer compound and the small molecule cross-linking agent is 0.01:0.99-0.99:0.01. For example, it may be 0.1:0.9-0.9:0.1, such as 0.01:0.99, 0.1:0.9, 0.15:0.85, 0.2:0.8, 0.3:0.7, 0.4:0.6, 0.5:0.5, 0.6:0.4, 0.7:0.3, 0.8:0.2, 0.85:0.15, 0.9:0.1, 0.99:0.01 or any ratio within the interval. The acryloylated polymer compound and the small molecule cross-linking agent can be mixed in any proportion.

The hydrogel of the present disclosure is a stable cross-linking material formed by the addition reaction of a thiol group (-SH) of the sulfhydryl-modified polymer compound and a carbon-carbon double bond of the substance C1 and/or the substance C2, and the cross-linking material (namely the hydrogel) has excellent mechanical properties and good physical stability and mechanical strength; in addition, the rate of in vivo metabolism is controllable. If two substances, namely a C1 and a C2, are introduced into the system at the same time, the C2 (a small molecule cross-linking agent) can participate in the cross-linking reaction of the sulfhydryl-modified polymer compound with the C1 (an acryloylated polymer compound); namely, the three substances are cross-linked together to form a stable cross-linking material. Meanwhile, the substance C1 can also be added to the gel system by physical mixing, thereby achieving different application purposes. The sulfhydryl-modified polymer compound is used with the C1 (an acryloylated polymer compound) and/or the C2 (a small molecule cross-linking agent) and mutual complementarity is achieved, thereby obtaining a three-dimensional scaffold material with excellent properties, which can meet most application requirements of tissue engineering.

In one specific embodiment of the present disclosure, the system may be further added with at least one selected from other biological functional materials (such as hyaluronic acid, collagen, gelatin, chondroitin sulfate, chitosan and sodium alginate), drugs, growth factors, cell suspensions, and the like. Additional effect can be brought to the hydrogel of the present disclosure by adding other biological functional materials. For example, the introduction of unmodified hyaluronic acid can enhance the hydrogel's promotion effect in wound healing, the introduction of collagen or gelatin can make the hydrogel system more similar to the composition of soft tissues of an organism, the introduction of chondroitin sulfate can enhance the hydrogel system's promotion effect in cartilage repair, the introduction of positively charged biomaterials such as chitosan can promote the antibacterial effect of the hydrogel, and the introduction of sodium alginate can enhance the mechanical strength of the hydrogel system.

[Preparation of hydrogel]

As described above, the present disclosure provides a preparation method for the hydrogel, which comprises the following steps:

gelling a system comprising the following substances:

(i) the sulfhydryl-modified polymer compound, and

(ii) at least one selected from the substance C1 and the substance C2,

thus obtaining the hydrogel.

In one specific embodiment, the method comprises the following step: gelling a system comprising the following substances:

(a) the sulfhydryl-modified polymer compound,
(b) at least one of the following substances: C1. an acryloylated polymer compound, and C2. a small molecule cross-linking agent containing an acryloyl group, and
(c) optionally at least one of the following substances: other biological functional materials, drugs, growth factors and cell suspensions,

thus obtaining the hydrogel.

Specifically, a solution of the sulfhydryl-modified polymer compound, a solution of the acryloylated polymer compound, a solution of the small molecule cross-linking agent and optionally a solution of at least one selected from other biological functional materials, drugs, growth factors and cell suspensions were prepared, and then these solutions were mixed and gelled to obtain the hydrogel. In addition, at least one selected from the other biological functional materials, the drugs, the growth factors and the cell suspensions may be introduced by directly addition into the solution of the sulfhydryl-modified polymer compound, the solution of the acryloylated polymer compound or the solution of the small molecule cross-linking agent.

The preparation process of the hydrogel can be performed by adding the solution of the sulfhydryl-modified polymer compound into the solution of the acryloylated polymer compound and/or the solution of the small molecule cross-linking agent, or by adding the solution of the acryloylated polymer compound and/or the solution of the small molecule cross-linking agent into the solution of the sulfhydryl-modified polymer compound. Specifically, the two solutions can be mixed by a common syringe, by a double-needle syringe, or other means.

The solution of the sulfhydryl-modified polymer compound has a concentration of 0.1% to 95% (w/v), for example, 1% to 90% (w/v), and further, for example, may have a concentration of 0.1%, 1%, 5%, 10%, 20%, 30%, 40%, 50%, 60%, 70%, 80%, 90%, or 95% (w/v). The solution can be added with an acid, a base or a buffer solution to adjust pH to 7.4. The buffer solution may be a phosphate buffer.

The solution of the acryloylated polymer compound has a concentration of 0.1% to 95% (w/v), for example, 1% to 90% (w/v), and further, for example, may have a concentration of 0.1%, 1%, 5%, 10%, 20%, 30%, 40%, 50%, 60%, 70%, 80%, 90%, or 95% (w/v). The solution can be added with an acid, a base or a buffer solution to adjust pH to 7.4. The buffer solution may be a phosphate buffer.

The solution of the small molecule cross-linking agent has a concentration of 0.1% to 95% (w/v), for example, 1% to 90% (w/v), and further, for example, may have a concentration of 0.1%, 1%, 5%, 10%, 20%, 30%, 40%, 50%, 60%, 70%, 80%, 90%, or 95% (w/v). The solution can be added with an acid, a base or a buffer solution to adjust pH to 7.4. The buffer solution may be a phosphate buffer.

The two solutions may be mixed in any proportion, for example, in equal volume.

[Use of hydrogel]

Hydrogel is known as a three-dimensional network that is formed by cross-linking of hydrophilic polymer chain segments and can swell in water. The gelation process can be achieved by different reaction mechanisms, including physical entanglement, electrostatic interaction, covalent chemical cross-linking, reversible chemical cross-linking, supramolecular chemical cross-linking, hydrophilic-hydrophobic interaction cross-linking, etc., of polymer chain segments. In recent years, with the in-depth research on the functions of hydrogel, hydrogel has been widely used in the pharmaceutical field, such as in preparation of drug delivery systems, dressings for soft tissue wound repair, scaffold materials for bone repair, viscoelastic agents for supporting in ophthalmic surgery, materials for preventing tissue adhesion after surgery, and scaffold materials for 3D bioprinting, which has become a research hot spot in the fields of tissue engineering and regenerative medicine.

The hydrogel of the present disclosure is particularly suitable for use in the fields of biopharmaceuticals, medical cosmetology, cosmetics and the like. Specifically, the hydrogel can be used in preparation of drug delivery systems, dressings for soft tissue wound repair, scaffold materials for bone repair, viscoelastic agents for supporting in ophthalmic surgery, materials for preventing tissue adhesion after surgery, scaffold materials for 3D bioprinting, and the like.

The hydrogel of the present application realizes in situ cross-linking under physiological conditions in the true sense; namely, the cross-linking reaction can be completed under the conditions of room temperature and normal pressure; or after the hydrogel is injected into tissues of an animal or a human, the cross-linking reaction can still be

realized in the tissues, so that the degradation resistance and metabolism resistance of the hydrogel product are significantly improved, and thus the using effect of the hydrogel injection product is remarkably improved. Besides, due to the unique technology of the present disclosure, the controllability of the cross-linking degree of the hydrogel product injected into the animal or human can be realized before the in vitro cross-linking or mixing stage; namely, the cross-linking reaction with controllable cross-linking reaction end point can be realized after the hydrogel product is injected into the animal or human, and thus the safety and the therapeutic effect of the product are ensured.

The present disclosure will be further illustrated with reference to the following specific examples. It should be understood that these examples are merely intended to illustrate the present disclosure rather than limit the protection scope of the present disclosure.

In the present disclosure, the 1H-NMR spectrum is determined by a Varian 400 MHz nuclear magnetic resonance spectrometer, with the test temperature of 25 °C, the relaxation time of 1 s, and the number of scanning of 8. Specifically, 8-10 mg of the test sample is dissolved in 750 μL of deuterated water, and the obtained sample solution is determined for the 1H-NMR spectrum.

The storage modulus of the present disclosure is determined based on the rheological mechanical properties of hydrogel. Specifically, the detection instrument is a TA-DHR2 rheometer, the detection probe is a 20 mm parallel plate probe, the detection temperature is 25 °C, the shear frequency is 1 Hz, and the shear strain is 1%.

The cellular activity and biocompatibility of polymer compounds of the present disclosure were tested with reference to the criteria set forth in "GBT 16886.5-2017 Biological evaluation of medical devices--Part 5. Tests for in vitro cytotoxicity". Specifically, the following MTT method is a method for determining the survival rate of cells by metabolic activity. A yellow aqueous solution MTT [3-(4,5-dimethylthizol-2-yl)-2,5-diphenyltetrazolium bromide] is metabolically reduced in living cells to generate blue-violet insoluble formazan. The number of living cells correlates with the chroma determined by a photometer after formazan dissolves in alcohol.

Preparation Example 1. Synthesis of Acrylate-Modified Hyaluronic Acid (HA-A1)

To a 200 mL beaker were added 1 g of hyaluronic acid (Bloomage Freda, weight-average molecular weight: about 300 kDa), 50 mL of deionized water, 50 mL of dimethylformamide, 12 mL of triethylamine, and 14 mL of glycidyl acrylate. After being stirred at room temperature until uniform and transparent, the mixture was stirred for an additional 48 h. 300 mL of acetone was added, and a large amount of white precipitate was generated. The reaction solution was centrifuged, and the resulting precipitate was dissolved in 100 mL of deionized water to obtain a colorless transparent solution. The resulting solution was filled into a dialysis bag (molecular weight cutoff: 8 kDa) and dialyzed against 5 L of deionized water for 5 days, with water refreshed twice a day. Finally, the solution in the dialysis bag was collected and lyophilized to obtain HA-A1 (921 mg, yield 92.1%) as a white flocculent solid.

The structural formula of HA-A1 is shown in FIG. 15. FIG. 15 is a schematic diagram only, showing the esterification of COOH in some of the repeating units of the hyaluronic acid with glycidyl acrylate, wherein m2/(m1+m2) represents the degree of acryloylation, m1 + m2 = n, and n is the number of repeating units of an hyaluronic acid to be. The meanings of the structural formulas in the following preparation examples un-modified and examples are the same as that of Preparation Example 1, and will not be repeated.

The 1H-NMR spectrum of HA-A1 is shown in FIG. 15, wherein a nuclear magnetic peak belonging to the acrylic functional group located between 6 ppm and 6.5 ppm can be seen, demonstrating that the group is successfully grafted into the structure of the hyaluronic acid.

Preparation Example 2. Synthesis of Acrylate-Modified Hyaluronic Acid (HA-A2)

To a 200 mL beaker were added 1 g of hyaluronic acid (Bloomage Freda, weight-average molecular weight: about 400 kDa), 50 mL of deionized water, 50 mL of dimethylformamide, and 6.3 g of acrylic anhydride, and the mixture was dissolved with stirring. The solution was maintained at pH 8 ± 0.5 with 1 mol/L NaOH, and stirred for an additional 24 h. 300 mL of acetone was added, and a large amount of white precipitate was generated. The reaction solution was centrifuged, and the resulting precipitate was dissolved in 100 mL of deionized water to obtain a colorless transparent solution. The resulting solution was filled into a dialysis bag (Spectrumlabs, molecular weight cutoff: 8 kDa) and dialyzed against 5 L of deionized water for 5 days, with water refreshed twice a day. Finally, the solution in the dialysis bag was collected and lyophilized to obtain HA-A2 (789 mg, yield 78.9%) as a white flocculent solid.

The structural formula of HA-A2 is shown in FIG. 16.

The 1H-NMR spectrum of HA-A2 is shown in FIG. 16, wherein a nuclear magnetic peak belonging to the acrylic functional group located between 5.8 ppm and 6.4 ppm can be seen, demonstrating that the group is successfully grafted into the structure of the hyaluronic acid.

Preparation Example 3. Synthesis of Methacrylate-Modified Hyaluronic Acid (HA-MA1)

To a 200 mL beaker were added 1 g of hyaluronic acid (Bloomage Freda, weight-average molecular weight: about 400 kDa), 50 mL of deionized water, 50 mL of dimethylformamide (Sigma), 12 mL of triethylamine (Sigma), and 15 mL of glycidyl methacrylate. After being stirred at room temperature until uniform and transparent, the mixture was stirred for an additional 48 h. 300 mL of acetone (Sigma) was added, and a large amount of white precipitate was generated. The reaction solution was centrifuged, and the resulting precipitate was dissolved in 100 mL of deionized water to obtain a colorless solution. The resulting solution was filled into a dialysis bag (Spectrumlabs, molecular weight cutoff 8 kDa) and dialyzed against 5 L of deionized water for 5 days, with water refreshed twice a day. Finally, the solution in the dialysis bag was collected and lyophilized to obtain HA-MA1 (859 mg, yield 85.9%) as a white flocculent solid.
The structural formula of HA-MA1 is shown in FIG. 17.
The 1H-NMR spectrum of HA-MA1 is shown in FIG. 17, wherein a nuclear magnetic peak belonging to the methacrylic functional group located between 5.8 ppm and 6.2 ppm can be seen, demonstrating that the group is successfully grafted into the structure of the hyaluronic acid.

Preparation Example 4. Synthesis of Methacrylate-Modified Hyaluronic Acid (HA-MA2)

To a 200 mL beaker were added 1 g of hyaluronic acid (Bloomage Freda, weight-average molecular weight: about 400 kDa) and 100 mL of deionized water, and the mixture was dissolved with stirring at room temperature. Further, 7.7 g of methacrylic anhydride was added and dissolved with stirring. The solution was maintained at pH 8 ± 0.5 with 1 mol/L NaOH, and stirred for an additional 24 h. 200 mL of acetone (Sigma) was added, and a large amount of white precipitate was generated. The reaction solution was centrifuged, and the resulting precipitate was dissolved in 100 mL of deionized water to obtain a colorless transparent solution. The resulting solution was filled into a dialysis bag (Spectrumlabs, molecular weight cutoff: 8 kDa) and dialyzed against 5 L of deionized water for 5 days, with water refreshed twice a day. Finally, the solution in the dialysis bag was collected and lyophilized to obtain HA-MA2 (846 mg, yield 84.6%) as a white flocculent solid.
The structural formula of HA-MA2 is shown in FIG. 18.
The 1H-NMR spectrum of HA-MA2 is shown in FIG. 18, wherein a nuclear magnetic peak belonging to the methacrylic functional group located between 5.8 ppm and 6.2 ppm can be seen, demonstrating that the group is successfully grafted into the structure of the hyaluronic acid.

Preparation Example 5. Synthesis of Sulfhydryl-Acrylate-Modified Hyaluronic Acid (HA-A1-SH1)

To a 200 mL beaker were added 1 g of HA-A1 prepared according to the method of Preparation Example 1, 0.3 g of dithiothreitol (VWR) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A1-SH1 (842 mg, yield 84.2%) as a white flocculent solid.
The reaction equation for HA-A1-SH1 is shown in FIG. 1, and the structural formula is shown in FIGs. 1 and 25.
The 1H-NMR spectrum of HA-A1-SH1 is shown in FIG. 25, wherein a nuclear magnetic peak belonging to a sulfhydryl side chain located between 2.3 ppm and 2.8 ppm can be seen, demonstrating that the sulfhydryl group is successfully grafted into the structure of the hyaluronic acid.

Preparation Example 6. Synthesis of Sulfhydryl-Acrylate-Modified Hyaluronic Acid (HA-A2-SH1)

To a 200 mL beaker were added 1 g of HA-A2 prepared according to the method of Preparation Example 2, 0.3 g of dithiothreitol (VWR) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH1 (827 mg, yield 82.7%) as a white flocculent solid.
The reaction equation for HA-A2-SH1 is shown in FIG. 2, and the structural formula is shown in FIGs. 2 and 26.
The 1H-NMR spectrum of HA-A2-SH1 is shown in FIG. 26, wherein a nuclear magnetic peak belonging to a sulfhydryl side chain located between 2.6 ppm and 2.9 ppm can be seen, demonstrating that the sulfhydryl group is successfully grafted into the structure of the hyaluronic acid.

Preparation Example 7. Synthesis of Sulfhydryl-Methacrylate-Modified Hyaluronic Acid (HA-MA1-SH1)

To a 200 mL beaker were added 1 g of HA-MA1 prepared according to the method of Preparation Example 3, 0.3 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-MA1-SH1 (854 mg, yield 85.4%) as a white flocculent solid.
The reaction equation for HA-MA1-SH1 is shown in FIG. 3, and the structural formula is shown in FIGs. 3 and 27.
The 1H-NMR spectrum of HA-MA1-SH1 is shown in FIG. 27, wherein a nuclear magnetic peak belonging to a sulfhydryl side chain located between 2.6 ppm and 3.0 ppm can be seen, demonstrating that the sulfhydryl group is successfully grafted into the structure of the hyaluronic acid.

Preparation Example 8. Synthesis of Sulfhydryl-Methacrylate-Modified Hyaluronic Acid (HA-MA2-SH1)

To a 200 mL beaker were added 1 g of HA-MA2 prepared according to the method of Preparation Example 4, 0.3 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-MA2-SH1 (833 mg, yield 83.3%) as a white flocculent solid.
The reaction equation for HA-MA2-SH1 is shown in FIG. 4, and the structural formula is shown in FIGs. 4 and 28.
The 1H-NMR spectrum of HA-MA2-SH1 is shown in FIG. 28, wherein a nuclear magnetic peak belonging to a sulfhydryl side chain located between 2.6 ppm and 3.0 ppm can be seen, demonstrating that the sulfhydryl group is successfully grafted into the structure of the hyaluronic acid.

Preparation Example 9. Synthesis of Acrylate-Modified Chondroitin Sulfate (CHS-A)

To a 200 mL beaker were added 1.2 g of chondroitin sulfate (weight-average molecular weight: about 80 kDa), 50 mL of deionized water, 50 mL of dimethylformamide, and 5.4 g of acrylic anhydride, and the mixture was dissolved with stirring. The solution was maintained at pH 8 $\pm$ 0.5 with 1 mol/L NaOH, and stirred for an additional 24 h. The resulting solution was filled into a dialysis bag (Spectrumlabs, molecular weight cutoff: 3.5 kDa) and dialyzed against 5 L of deionized water for 5 days, with water refreshed twice a day. Finally, the solution in the dialysis bag was collected and lyophilized to obtain CHS-A (781 mg, yield 65.1%) as a light yellow flocculent solid.
The structural formula of CHS-A is shown in FIG. 19.
The 1H-NMR spectrum of CHS-A is shown in FIG. 19, wherein a nuclear magnetic peak belonging to the acrylic functional group located between 6.0 ppm and 6.5 ppm can be seen, demonstrating that the group is successfully grafted into the structure of the chondroitin sulfate.

Preparation Example 10. Synthesis of Methacrylate-Modified Chondroitin Sulfate (CHS-MA)

To a 200 mL beaker were added 1.2 g of chondroitin sulfate (weight-average molecular weight: about 90 kDa), 50 mL of deionized water, and 50 mL of dimethylformamide, followed by 6.5 g of methacrylic anhydride, and the mixture was dissolved with stirring. The solution was maintained at pH 8 $\pm$ 0.5 with 1 mol/L NaOH, and stirred for an additional 24 h. The resulting solution was filled into a dialysis bag (Spectrumlabs, molecular weight cutoff: 3.5 kDa) and dialyzed against 5 L of deionized water for 5 days, with water refreshed twice a day. Finally, the solution in the dialysis bag was collected and lyophilized to obtain CHS-MA (776 mg, yield 64.7%) as a light yellow flocculent solid.
The structural formula of CHS-MA is shown in FIG. 20.
The 1H-NMR spectrum of CHS-MA is shown in FIG. 20, wherein a nuclear magnetic peak belonging to the methacrylic functional group located between 6.0 ppm and 6.5 ppm can be seen, demonstrating that the group is successfully grafted into the structure of the chondroitin sulfate.

Preparation Example 11. Synthesis of Acrylate-Modified Gelatin (Gelatin-A)

To a 200 mL beaker were added 1 g of gelatin (strength: 300 Blooms), 50 mL of deionized water, and 50 mL of dimethylformamide, followed by 10 g of acrylic anhydride, and the mixture was dissolved with stirring. The solution was maintained at pH 8 $\pm$ 0.5 with 1 mol/L NaOH, and stirred for an additional 24 h. The resulting solution was filled into a dialysis bag (Spectrumlabs, molecular weight cutoff: 8 kDa) and dialyzed against 5 L of deionized water for 5 days,

with water refreshed twice a day. Finally, the solution in the dialysis bag was collected and lyophilized to obtain Gelatin-A (781 mg, yield 78.1%) as a light yellow flocculent solid.

The condensed structural formula of Gelatin-A is shown in FIG. 21 (the wavy line therein represents the main chain of Gelatin).

The 1H-NMR spectrum of Gelatin-A is shown in FIG. 21, wherein a nuclear magnetic peak belonging to the acrylic functional group located between 6.0 ppm and 6.5 ppm can be seen, demonstrating that the group is successfully grafted into the structure of the gelatin.

Preparation Example 12. Synthesis of Methacrylate-Modified Gelatin (Gelatin-MA)

To a 200 mL beaker were added 1 g of gelatin (strength: 300 Blooms), 50 mL of deionized water, and 50 mL of dimethylformamide, followed by 10 g of methacrylic anhydride, and the mixture was dissolved with stirring. The solution was maintained at pH 8 ± 0.5 with 1 mol/L NaOH, and stirred for an additional 24 h. The resulting solution was filled into a dialysis bag (Spectrumlabs, molecular weight cutoff: 8 kDa) and dialyzed against 5 L of deionized water for 5 days, with water refreshed twice a day. Finally, the solution in the dialysis bag was collected and lyophilized to obtain Gelatin-MA (824 mg, yield 82.4%) as a light yellow flocculent solid.

The condensed structural formula of Gelatin-MA is shown in FIG. 22 (the wavy line therein represents the main chain of Gelatin).

The 1H-NMR spectrum of Gelatin-MA is shown in FIG. 22, wherein a nuclear magnetic peak belonging to the methacrylic functional group located between 5.7 ppm and 6.2 ppm can be seen, demonstrating that the group is successfully grafted into the structure of the gelatin.

Preparation Example 13. Synthesis of Acrylate-Modified Ethylene Glycol Chitosan (CTS-A)

To a 200 mL beaker were added 1 g of ethylene glycol chitosan (weight-average molecular weight: about 250 kDa), 50 mL of deionized water, 50 mL of dimethylformamide, 8 mL of triethylamine (Sigma), and 13 mL of glycidyl acrylate. After being stirred at room temperature until uniform and transparent, the mixture was stirred for an additional 48 h. The resulting solution was filled into a dialysis bag (Spectrumlabs, molecular weight cutoff: 3.5 kDa) and dialyzed against 5 L of deionized water for 5 days, with water refreshed twice a day. Finally, the solution in the dialysis bag was collected and lyophilized to obtain CTS-A (694 mg, yield 69.4%) as a light yellow flocculent solid.

The structural formula of CTS-A is shown in FIG. 23.

The 1H-NMR spectrum of CTS-A is shown in FIG. 23, wherein a nuclear magnetic peak belonging to the acrylic functional group located between 5.8 ppm and 6.4 ppm can be seen, demonstrating that the group is successfully grafted into the structure of the ethylene glycol chitosan.

Preparation Example 14. Synthesis of Methacrylate-Modified Ethylene Glycol Chitosan (CTS-MA)

To a 200 mL beaker were added 1 g of ethylene glycol chitosan (weight-average molecular weight: about 200 kDa), 50 mL of deionized water, 50 mL of dimethylformamide, 8 mL of triethylamine (Sigma), and 13 mL of glycidyl methacrylate. After being stirred at room temperature until uniform and transparent, the mixture was stirred for an additional 48 h. The resulting solution was filled into a dialysis bag (Spectrumlabs, molecular weight cutoff: 3.5 kDa) and dialyzed against 5 L of deionized water for 5 days, with water refreshed twice a day. Finally, the solution in the dialysis bag was collected and lyophilized to obtain CTS-MA (726 mg, yield 72.6%) as a light yellow flocculent solid.

The structural formula of CTS-MA is shown in FIG. 24.

The 1H-NMR spectrum of CTS-MA is shown in FIG. 24, wherein a nuclear magnetic peak belonging to the methacrylic functional group located between 5.7 ppm and 6.2 ppm can be seen, demonstrating that the group is successfully grafted into the structure of the ethylene glycol chitosan.

Preparation Example 15. Synthesis of Sulfhydryl-Acrylate-Modified Chondroitin Sulfate (CHS-A-SH1)

To a 200 mL beaker were added 1 g of CHS-A prepared according to the method of Preparation Example 9, 0.25 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff 3.5 kDa) and dialyzed against 5 L of hydrochloric acid solution at pH 4 for 5 days, with water refreshed twice a day. Finally, the solution in the dialysis bag was collected and lyophilized to obtain CHS-A-SH1 (629 mg, yield 62.9%) as a light yellow flocculent solid.

The reaction equation for CHS-A-SH1 is shown in FIG. 5, and the structural formula is shown in FIGs. 5 and 29.

The 1H-NMR spectrum of CHS-A-SH1 is shown in FIG. 29, wherein a nuclear magnetic peak belonging to a

sulfhydryl side chain located between 2.6 ppm and 3.0 ppm can be seen, demonstrating that the sulfhydryl group is successfully grafted into the structure of the chondroitin sulfate.

Preparation Example 16. Synthesis of Sulfhydryl-Methacrylate-Modified Chondroitin Sulfate (CHS-MA-SH1)

To a 200 mL beaker were added 1 g of CHS-MA prepared according to the method of Preparation Example 10, 0.25 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff: 3.5 kDa) and dialyzed against 5 L of hydrochloric acid solution at pH 4 for 5 days, with water refreshed twice a day. Finally, the solution in the dialysis bag was collected and lyophilized to obtain CHS-MA-SH1 (642 mg, yield 64.2%) as a light yellow flocculent solid.

The reaction equation for CHS-MA-SH1 is shown in FIG. 6, and the structural formula is shown in FIGs. 6 and 30.

The 1H-NMR spectrum of CHS-MA-SH1 is shown in FIG. 30, wherein a nuclear magnetic peak belonging to a sulfhydryl side chain located between 2.6 ppm and 3.0 ppm can be seen, demonstrating that the sulfhydryl group is successfully grafted into the structure of the chondroitin sulfate.

Preparation Example 17. Synthesis of Sulfhydryl-Acrylate-Modified Gelatin (Gelatin-A-SH1)

To a 200 mL beaker were added 1 g of Gelatin-A prepared according to the method of Preparation Example 11, 0.19 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain Gelatin-A-SH1 (763 mg, yield 76.3%) as a light yellow flocculent solid.

The reaction equation for Gelatin-A-SH1 is shown in FIG. 7, and the structural formula is shown in FIGs. 7 and 31.

The 1H-NMR spectrum of Gelatin-A-SH1 is shown in FIG. 31, wherein a nuclear magnetic peak belonging to a sulfhydryl side chain located between 2.6 ppm and 2.8 ppm can be seen, demonstrating that the sulfhydryl group is successfully grafted into the structure of the gelatin.

Preparation Example 18. Synthesis of Sulfhydryl-Methacrylate-Modified Gelatin (Gelatin-MA-SH1)

To a 200 mL beaker were added 1 g of Gelatin-MA prepared according to the method of Preparation Example 12, 0.19 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain Gelatin-MA-SH1 (787 mg, yield 78.7%) as a light yellow flocculent solid.

The reaction equation for Gelatin-MA-SH1 is shown in FIG. 8, and the structural formula is shown in FIGs. 8 and 32.

The 1H-NMR spectrum of Gelatin-MA-SH1 is shown in FIG. 32, wherein a nuclear magnetic peak belonging to a sulfhydryl side chain located between 2.6 ppm and 2.7 ppm can be seen, demonstrating that the sulfhydryl group is successfully grafted into the structure of the gelatin.

Preparation Example 19. Synthesis of Sulfhydryl-Acrylate-Modified Ethylene Glycol Chitosan (CTS-A-SH1)

To a 200 mL beaker were added 1 g of CTS-A prepared according to the method of Preparation Example 13, 0.25 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff 3.5 kDa) and dialyzed against 5 L of hydrochloric acid solution at pH 4 for 5 days, with water refreshed twice a day. Finally, the solution in the dialysis bag was collected and lyophilized to obtain CTS-A-SH1 (602 mg, yield 60.2%) as a light yellow flocculent solid.

The reaction equation for CTS-A-SH1 is shown in FIG. 9, and the structural formula is shown in FIGs. 9 and 33.

The 1H-NMR spectrum of CTS-A-SH1 is shown in FIG. 33, wherein a nuclear magnetic peak belonging to a sulfhydryl side chain located between 2.6 ppm and 3.0 ppm can be seen, demonstrating that the sulfhydryl group is successfully grafted into the structure of the ethylene glycol chitosan.

Preparation Example 20. Synthesis of Sulfhydryl-Methacrylate-Modified Chitosan (CTS-MA-SH1)

To a 200 mL beaker were added 1 g of CTS-MA prepared according to Preparation Example 14, 0.25 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff 3.5 kDa) and dialyzed against 5 L of hydrochloric acid solution at pH 4 for 5 days, with water refreshed twice a day. Finally, the solution in the dialysis bag was collected and lyophilized to obtain CTS-MA-SH1 (643 mg, yield 64.3%) as a white flocculent solid.

The reaction equation for CTS-MA-SH1 is shown in FIG. 10, and the structural formula is shown in FIGs. 10 and 34.

The 1H-NMR spectrum of CTS-MA-SH1 is shown in FIG. 34, wherein a nuclear magnetic peak belonging to a sulfhydryl side chain located between 2.5 ppm and 2.9 ppm can be seen, demonstrating that the sulfhydryl group is successfully grafted into the structure of the chitosan.

Preparation Example 21. Synthesis of Acrylate-Modified Polyhydroxyethyl Methacrylate (PHEMA-A)

To a 200 mL beaker were added 2 g of polyhydroxyethyl methacrylate (Sigma, Mv: 20 kDa), 50 mL of deionized water, and 50 mL of dimethylformamide, followed by 16.5 g of acrylic anhydride, and the mixture was dissolved with stirring. The solution was maintained at pH 8 ± 0.5 with 1 mol/L NaOH, and stirred for an additional 24 h. The resulting solution was filled into a dialysis bag (Spectrumlabs, molecular weight cutoff: 2 kDa) and dialyzed against 5 L of deionized water for 5 days, with water refreshed twice a day. Finally, the solution in the dialysis bag was collected and lyophilized to obtain PHEMA-A (1.42 g, yield 71.0%) as a white solid.

The structural formula of PHEMA-A is shown in FIG. 35.

The 1H-NMR spectrum of PHEMA-A is shown in FIG. 35, wherein a nuclear magnetic peak belonging to the acrylic functional group located between 5.9 ppm and 6.4 ppm can be seen, demonstrating that the group is successfully grafted into the structure of the polyhydroxyethyl methacrylate.

Preparation Example 22. Synthesis of Methacrylate-Modified Polyhydroxyethyl Methacrylate (PHEMA-MA)

To a 200 mL beaker were added 2 g of polyhydroxyethyl methacrylate (Sigma, Mv: 20 kDa), 50 mL of deionized water, and 50 mL of dimethylformamide, followed by 16.8 g of methacrylic anhydride, and the mixture was dissolved with stirring. The solution was maintained at pH 8 ± 0.5 with 1 mol/L NaOH, and stirred for an additional 24 h. The resulting solution was filled into a dialysis bag (Spectrumlabs, molecular weight cutoff: 2 kDa) and dialyzed against 5 L of deionized water for 5 days, with water refreshed twice a day. Finally, the solution in the dialysis bag was collected and lyophilized to obtain PHEMA-MA (1.48 g, yield 74.0%) as a white solid.

The structural formula of PHEMA-MA is shown in FIG. 36.

The 1H-NMR spectrum of PHEMA-MA is shown in FIG. 36, wherein a nuclear magnetic peak belonging to the methacrylic functional group located between 5.7 ppm and 6.3 ppm can be seen, demonstrating that the group is successfully grafted into the structure of the polyhydroxyethyl methacrylate.

Preparation Example 23. Synthesis of Acrylate-Modified Polyvinyl Alcohol (PVA-A)

To a 200 mL beaker were added 2 g of polyvinyl alcohol (Sigma, Mw: 61 kDa), 50 mL of deionized water, and 50 mL of dimethylformamide, followed by 13 g of acrylic anhydride, and the mixture was dissolved with stirring. The solution was maintained at pH 8 ± 0.5 with 1 mol/L NaOH, and stirred for an additional 24 h. The resulting solution was filled into a dialysis bag (Spectrumlabs, molecular weight cutoff: 3.5 kDa) and dialyzed against 5 L of deionized water for 5 days, with water refreshed twice a day. Finally, the solution in the dialysis bag was collected and lyophilized to obtain PVA-A (1.57 g, yield 78.5%) as a white solid.

The structural formula of PVA-A is shown in FIG. 37.

The 1H-NMR spectrum of PVA-A is shown in FIG. 37, wherein a nuclear magnetic peak belonging to the acrylic functional group located between 6.0 ppm and 6.5 ppm can be seen, demonstrating that the group is successfully grafted into the structure of the polyvinyl alcohol.

Preparation Example 24. Synthesis of Methacrylate-Modified Polyvinyl Alcohol (PVA-MA)

To a 200 mL beaker were added 2 g of polyvinyl alcohol (Sigma, Mw: 61 kDa), 50 mL of deionized water, and 50 mL of dimethylformamide, followed by 13.4 g of methacrylic anhydride, and the mixture was dissolved with stirring. The solution was maintained at pH 8 ± 0.5 with 1 mol/L NaOH, and stirred for an additional 24 h. The resulting solution

was filled into a dialysis bag (Spectrumlabs, molecular weight cutoff: 3.5 kDa) and dialyzed against 5 L of deionized water for 5 days, with water refreshed twice a day. Finally, the solution in the dialysis bag was collected and lyophilized to obtain PVA-MA (1.51 g, yield 75.5%) as a white solid.

The structural formula of PVA-MA is shown in FIG. 38.

The 1H-NMR spectrum of PVA-MA is shown in FIG. 38, wherein a nuclear magnetic peak belonging to the methacrylic functional group located between 5.7 ppm and 6.3 ppm can be seen, demonstrating that the group is successfully grafted into the structure of the polyvinyl alcohol.

Preparation Example 25. Synthesis of Sulfhydryl-Acrylate-Modified Polyhydroxyethyl Methacrylate (PHEMA-A-SH1)

To a 200 mL beaker were added 2 g of PHEMA-A prepared according to the method of Preparation Example 21, 0.42 g of dithiothreitol (VWR), 50 mL of deionized water and 50 mL of dimethylformamide, and the mixture was dissolved with stirring at room temperature. The resulting transparent solution was stirred for an additional 12 h. The resulting solution was filled into a dialysis bag (Spectrumlabs, molecular weight cutoff: 2 kDa) and dialyzed against 5 L of hydrochloric acid solution at pH 4 for 5 days, with water refreshed twice a day. Finally, the solution in the dialysis bag was collected and lyophilized to obtain PHEMA-A-SH1 (1.67 g, yield 83.5%) as a white solid.

The reaction equation for PHEMA-A-SH1 is shown in FIG. 57, and the structural formula is shown in FIGs. 57 and 39.

The 1H-NMR spectrum of PHEMA-A-SH1 is shown in FIG. 39, wherein a nuclear magnetic peak belonging to a sulfhydryl side chain located between 2.6 ppm and 2.9 ppm can be seen, demonstrating that the sulfhydryl group is successfully grafted into the structure of the polyhydroxyethyl methacrylate.

Preparation Example 26. Synthesis of Sulfhydryl-Methacrylate-Modified Polyhydroxyethyl Methacrylate (PHEMA-MA-SH1)

To a 200 mL beaker were added 2 g of PHEMA-MA prepared according to the method of Preparation Example 22, 0.41 g of dithiothreitol (VWR), 50 mL of deionized water and 50 mL of dimethylformamide, and the mixture was dissolved with stirring at room temperature. The resulting transparent solution was stirred for an additional 12 h. The resulting solution was filled into a dialysis bag (Spectrumlabs, molecular weight cutoff: 2 kDa) and dialyzed against 5 L of hydrochloric acid solution at pH 4 for 5 days, with water refreshed twice a day. Finally, the solution in the dialysis bag was collected and lyophilized to obtain PHEMA-MA-SH1 (1.62 g, yield 81%) as a white solid.

The reaction equation for PHEMA-MA-SH1 is shown in FIG. 58, and the structural formula is shown in FIGs. 58 and 40.

The 1H-NMR spectrum of PHEMA-MA-SH1 is shown in FIG. 40, wherein a nuclear magnetic peak belonging to a sulfhydryl side chain located between 2.6 ppm and 3.0 ppm can be seen, demonstrating that the sulfhydryl group is successfully grafted into the structure of the polyhydroxyethyl methacrylate.

Preparation Example 27. Synthesis of Sulfhydryl-Acrylate-Modified Polyvinyl Alcohol (PVA-A-SH1)

To a 200 mL beaker were added 1 g of PVA-A prepared according to the method of Preparation Example 23 and 100 mL of deionized water, and the solution was heated with stirring until the PVA-A was completely dissolved. Subsequently, the solution was added with 0.47 g of dithiothreitol (VWR) and dissolved with stirring at room temperature. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain PVA-A-SH1 (737 mg, yield 73.7%) as a white solid.

The reaction equation of PVA-A-SH1 is shown in FIG. 59, and the structural formula is shown in FIGs. 59 and 41.

The 1H-NMR spectrum for PVA-A-SH1 is shown in FIG. 41, wherein a nuclear magnetic peak belonging to a sulfhydryl side chain located between 2.6 ppm and 3.0 ppm can be seen, demonstrating that the sulfhydryl group is successfully grafted into the structure of the polyvinyl alcohol.

Preparation Example 28. Synthesis of Sulfhydryl-Methacrylate-Modified Polyvinyl Alcohol (PVA-MA-SH1)

To a 200 mL beaker were added 1 g of PVA-MA prepared according to the method of Preparation Example 24 and 100 mL of deionized water, and the solution was heated with stirring until the PVA-MA was completely dissolved. Subsequently, the solution was added with 0.47 g of dithiothreitol (VWR) and dissolved with stirring at room temperature. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days,

with water refreshed twice a day. Finally, the solution in the dialysis bag was collected and lyophilized to obtain PVA-MA-SH1 (718 mg, yield 71.8%) as a white solid.

The reaction equation for PVA-MA-SH1 is shown in FIG. 60, and the structural formula is shown in FIGs. 60 and 42.

The 1H-NMR spectrum of PVA-MA-SH1 is shown in FIG. 42, wherein a nuclear magnetic peak belonging to a sulfhydryl side chain located between 2.5 ppm and 3.0 ppm can be seen, demonstrating that the sulfhydryl group is successfully grafted into the structure of the polyvinyl alcohol.

Preparation Example 29. Synthesis of Sulfhydryl-Modified Hyperbranched PEG Polymer (HB-PEG-SH1)

To a 200 mL beaker were added 5 g of hyperbranched PEG (HB-PEG, Blafar Ltd., Mw: 20 kDa), 0.86 g of dithiothreitol (VWR) and 100 mL of deionized water, and the mixture was dissolved with stirring at room temperature. The resulting transparent solution was stirred for an additional 12 h. The resulting solution was filled into a dialysis bag (Spectrumlabs, molecular weight cutoff 2 kDa) and dialyzed against 5 L of hydrochloric acid solution at pH 4 for 5 days, with water refreshed twice a day. Finally, the solution in the dialysis bag was collected and lyophilized to obtain HB-PEG-SH1 (3.84 g, yield 76.8%) as a colorless viscous liquid.

The reaction equation for HB-PEG-SH1 is shown in FIG. 61, and the structural formula is shown in FIGs. 61 and 43.

The 1H-NMR spectrum of HB-PEG-SH1 is shown in FIG. 43, wherein a nuclear magnetic peak belonging to a sulfhydryl side chain located between 2.5 ppm and 2.6 ppm can be seen, demonstrating that the sulfhydryl group is successfully grafted into the structure of the hyperbranched PEG polymer.

Preparation Example 30. Synthesis of Sulfhydryl-Acrylate-Modified Hyaluronic Acid (HA-A1-SH2)

To a 200 mL beaker were added 1 g of HA-A1 prepared according to the method of Preparation Example 1, 0.42 g of 1,4-butanedithiol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A1-SH2 (852 mg, yield 85.2%) as a white flocculent solid.

The reaction equation for HA-A1-SH2 is shown in FIG. 62, and the structural formula is shown in FIGs. 62 and 44.

The 1H-NMR spectrum of HA-A1-SH2 is shown in FIG. 44, wherein a nuclear magnetic peak belonging to a sulfhydryl side chain located between 1.6 ppm and 1.9 ppm can be seen, demonstrating that the sulfhydryl group is successfully grafted into the structure of the hyaluronic acid.

Preparation Example 31. Synthesis of Sulfhydryl-Acrylate-Modified Hyaluronic Acid (HA-A1-SH3)

To a 200 mL beaker were added 1 g of HA-A1 prepared according to the method of Preparation Example 1, 0.43 g of 2-amino-1,4-butanedithiol hydrochloride (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A1-SH3 (843 mg, yield 84.3%) as a white flocculent solid.

The reaction equation for HA-A1-SH3 is shown in FIG. 63, and the structural formula is shown in FIGs. 63 and 45.

The 1H-NMR spectrum of HA-A1-SH3 is shown in FIG. 45, wherein a nuclear magnetic peak belonging to a sulfhydryl side chain located between 3.0 ppm and 3.2 ppm can be seen, demonstrating that the sulfhydryl group is successfully grafted into the structure of the hyaluronic acid.

Preparation Example 32. Synthesis of Sulfhydryl-Acrylate-Modified Hyaluronic Acid (HA-A2-SH2)

To a 200 mL beaker were added 1 g of HA-A2 prepared according to the method of Preparation Example 2, 0.42 g of 1,4-butanedithiol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH2 (827 mg, yield 82.7%) as a white flocculent solid.

The reaction equation for HA-A2-SH2 is shown in FIG. 64, and the structural formula is shown in FIGs. 64 and 46.

The 1H-NMR spectrum of HA-A2-SH2 is shown in FIG. 46, wherein a nuclear magnetic peak belonging to a

sulfhydryl side chain located between 1.6 ppm and 1.9 ppm can be seen, demonstrating that the sulfhydryl group is successfully grafted into the structure of the hyaluronic acid.

Preparation Example 33. Synthesis of Sulfhydryl-Acrylate-Modified Hyaluronic Acid (HA-A2-SH3)

To a 200 mL beaker were added 1 g of HA-A2 prepared according to the method of Preparation Example 2, 0.43 g of 2-amino-1,4-butanedithiol hydrochloride (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH3 (833 mg, yield 83.3%) as a white flocculent solid.
The reaction equation for HA-A2-SH3 is shown in FIG. 65, and the structural formula is shown in FIGs. 65 and 47.
The 1H-NMR spectrum of HA-A2-SH3 is shown in FIG. 47, wherein a nuclear magnetic peak belonging to a sulfhydryl side chain located between 3.0 ppm and 3.2 ppm can be seen, demonstrating that the sulfhydryl group is successfully grafted into the structure of the hyaluronic acid.

Preparation Example 34. Synthesis of Sulfhydryl-Acrylate-Modified Hyaluronic Acid (HA-A2-SH4)

To a 200 mL beaker were added 1 g of HA-A2 prepared according to the method of Preparation Example 2, 0.38 g of 1,3-propanedithiol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH4 (814 mg, yield 81.4%) as a white flocculent solid.
The reaction equation for HA-A2-SH4 is shown in FIG. 66, and the structural formula is shown in FIGs. 66 and 48.
The 1H-NMR spectrum of HA-A2-SH4 is shown in FIG. 48, wherein a nuclear magnetic peak belonging to a sulfhydryl side chain located between 2.5 ppm and 2.8 ppm can be seen, demonstrating that the sulfhydryl group is successfully grafted into the structure of the hyaluronic acid.

Preparation Example 35. Synthesis of Sulfhydryl-Acrylate-Modified Hyaluronic Acid (HA-A2-SH5)

To a 200 mL beaker were added 1 g of HA-A2 prepared according to the method of Preparation Example 2, 0.52 g of 1,3-phenyldithiophenol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH5 (836 mg, yield 83.6%) as a white flocculent solid.
The reaction equation for HA-A2-SH5 is shown in FIG. 67, and the structural formula is shown in FIGs. 67 and 49.
The 1H-NMR spectrum of HA-A2-SH5 is shown in FIG. 49, wherein a nuclear magnetic peak belonging to a sulfhydryl side chain located between 6.9 ppm and 7.4 ppm can be seen, demonstrating that the sulfhydryl group is successfully grafted into the structure of the hyaluronic acid.

Preparation Example 36. Synthesis of Sulfhydryl-Acrylate-Modified Hyaluronic Acid (HA-A2-SH6)

To a 200 mL beaker were added 1 g of HA-A2 prepared according to the method of Preparation Example 2, 0.52 g of 1,4-phenyldithiophenol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH6 (831 mg, yield 83.1%) as a white flocculent solid.
The reaction equation for HA-A2-SH6 is shown in FIG. 68, and the structural formula is shown in FIGs. 68 and 50.
The 1H-NMR spectrum of HA-A2-SH6 is shown in FIG. 50, wherein a nuclear magnetic peak belonging to a sulfhydryl side chain located between 6.8 ppm and 7.0 ppm can be seen, demonstrating that the sulfhydryl group is successfully grafted into the structure of the hyaluronic acid.

Preparation Example 37. Synthesis of Sulfhydryl-Acrylate-Modified Hyaluronic Acid (HA-A2-SH7)

To a 200 mL beaker were added 1 g of HA-A2 prepared according to the method of Preparation Example 2,

0.96 g of sulfhydryl polyethylene glycol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff 8 kDa) and dialyzed against 5 L of hydrochloric acid solution at pH 4 for 5 days, with water refreshed twice a day. Finally, the solution in the dialysis bag was collected and lyophilized to obtain HA-A2-SH7 (894 mg, yield 89.4%) as a white flocculent solid.
The reaction equation for HA-A2-SH7 is shown in FIG. 69, and the structural formula is shown in FIGs. 69 and 51.
The 1H-NMR spectrum of HA-A2-SH7 is shown in FIG. 51, wherein a nuclear magnetic peak belonging to a sulfhydryl side chain located at 3.6 ppm can be seen, demonstrating that the sulfhydryl group is successfully grafted into the structure of the hyaluronic acid.

Preparation Example 38. Synthesis of Sulfhydryl-Acrylate-Modified Hyaluronic Acid (HA-A2-SH8)

To a 200 mL beaker were added 1 g of HA-A2 prepared according to the method of Preparation Example 2, 0.74 g of trimethylolpropane-tris(3-sulfhydrylpropionate) (Sigma), 50 mL of deionized water and 50 mL of dimethylformamide, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-A2-SH8 (785 mg, yield 78.5%) as a white flocculent solid.
The reaction equation for HA-A2-SH8 is shown in FIG. 70, and the structural formula is shown in FIGs. 70 and 52.
The 1H-NMR spectrum of HA-A2-SH8 is shown in FIG. 52, wherein nuclear magnetic peaks belonging to a sulfhydryl side chain located between 0.8 ppm and 1.0 ppm, at 1.5 ppm, and between 2.6 ppm and 2.9 ppm can be seen, demonstrating that the sulfhydryl group is successfully grafted into the structure of the hyaluronic acid.

Preparation Example 39. Synthesis of Sulfhydryl-Methacrylate Modified Hyaluronic Acid (HA-MA1-SH5)

To a 200 mL beaker were added 1 g of HA-MA1 prepared according to the method of Preparation Example 3, 0.50 g of 1,3-phenyldithiophenol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-MA1-SH5 (828 mg, yield 82.8%) as a white flocculent solid.
The reaction equation for HA-MA1-SH5 is shown in FIG. 71, and the structural formula is shown in FIGs. 71 and 53.
The 1H-NMR spectrum of HA-MA1-SH5 is shown in FIG. 53, wherein a nuclear magnetic peak belonging to a sulfhydryl side chain located between 6.9 ppm and 7.4 ppm can be seen, demonstrating that the sulfhydryl group is successfully grafted into the structure of the hyaluronic acid.

Preparation Example 40. Synthesis of Sulfhydryl-Methacrylate-Modified Hyaluronic Acid (HA-MA1-SH6)

To a 200 mL beaker were added 1 g of HA-MA1 prepared according to the method of Preparation Example 3, 0.50 g of 1,4-phenyldithiophenol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-MA1-SH6 (833 mg, yield 83.3%) as a white flocculent solid.
The reaction equation for HA-MA1-SH6 is shown in FIG. 72, and the structural formula is shown in FIGs. 72 and 54.
The 1H-NMR spectrum of HA-MA1-SH6 is shown in FIG. 54, wherein a nuclear magnetic peak belonging to a sulfhydryl side chain located between 6.9 ppm and 7.0 ppm can be seen, demonstrating that the sulfhydryl group is successfully grafted into the structure of the hyaluronic acid.

Preparation Example 41. Synthesis of Sulfhydryl-Methacrylate-Modified Hyaluronic Acid (HA-MA2-SH7)

To a 200 mL beaker were added 1 g of HA-MA2 prepared according to the method of Preparation Example 4, 0.92 g of sulfhydryl polyethylene glycol (Sigma) and 100 mL of deionized water, and the mixture was dissolved with stirring at room temperature to obtain a transparent solution. The resulting transparent solution was stirred for an additional 12 h. The resulting solution was filled into a dialysis bag (Spectrumlabs, molecular weight cutoff 8 kDa) and dialyzed

against 5 L of hydrochloric acid solution at pH 4 for 5 days, with water refreshed twice a day. Finally, the solution in the dialysis bag was collected and lyophilized to obtain HA-MA2-SH7 (876 mg, yield 87.6%) as a white flocculent solid.

The reaction equation for HA-MA2-SH7 is shown in FIG. 73, and the structural formula is shown in FIGs. 73 and 55.

The 1H-NMR spectrum of HA-MA2-SH7 is shown in FIG. 55, wherein a nuclear magnetic peak belonging to a sulfhydryl side chain located at 3.6 ppm can be seen, demonstrating that the sulfhydryl group is successfully grafted into the structure of the hyaluronic acid.

Preparation Example 42. Synthesis of Sulfhydryl-Methacrylate 2-Modified Hyaluronic Acid (HA-MA2-SH8)

To a 200 mL beaker were added 1 g of HA-MA2 prepared according to the method of Preparation Example 4, 0.68 g of trimethylolpropane-tris(3-sulfhydrylpropionate) (Sigma), 50 mL of deionized water and 50 mL of dimethylformamide, and the mixture was dissolved with stirring at room temperature to obtain a transparent solution. The resulting transparent solution was stirred for an additional 12 h. The resulting solution was filled into a dialysis bag (Spectrumlabs, molecular weight cutoff: 8 kDa) and dialyzed against 5 L of hydrochloric acid solution at pH 4 for 5 days, with water refreshed twice a day. Finally, the solution in the dialysis bag was collected and lyophilized to obtain HA-MA2-SH8 (825 mg, yield 82.5%) as a white flocculent solid.

The reaction equation for HA-MA2-SH8 is shown in FIG. 74, and the structural formula is shown in FIGs. 74 and 56.

The 1H-NMR spectrum of HA-MA2-SH8 is shown in FIG. 56, wherein nuclear magnetic peaks belonging to a sulfhydryl side chain located between 0.8 ppm and 1.0 ppm, at 1.5 ppm, and between 2.6 ppm and 2.9 ppm can be seen, demonstrating that the sulfhydryl group is successfully grafted into the structure of the hyaluronic acid.

Example 1. Preparation of Hydrogels of Thiol-Ethenyl Cross-linked Hyaluronic Acid

10 mg of any one of the acryloylated polymer compounds prepared in Preparation Examples 1, 2, 9, 11 and 13 or ethylene glycol diacrylate (EGDA) was dissolved in 1 mL of a phosphate buffer (pH = 7.4) to obtain a series of solutions A having a concentration of 1% (w/v).

10 mg of any one of the sulfhydryl-modified polymer compounds prepared in Preparation Examples 5-8 and 15-20 was dissolved in 1 mL of a phosphate buffer (pH = 7.4) to obtain a series of solutions B having a concentration of 1% (w/v).

Any one of the solutions A and any one of the solutions B were uniformly mixed in equal volume, and the physiological in situ cross-linking reaction between the two polymer compounds occurred immediately. The viscosity of the solution was gradually increased with the increase of the mixing time, and finally the hydrogel was formed.

Each hydrogel in Example 1 comprises the following characteristic structural unit:

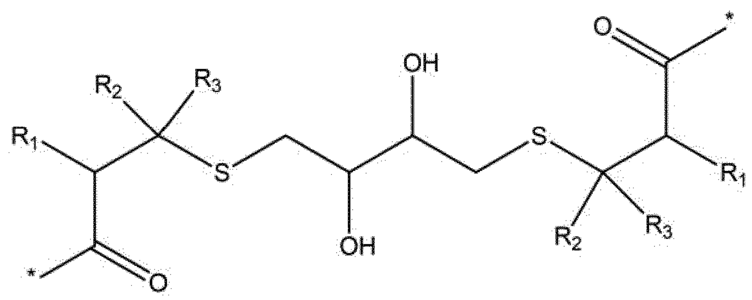

wherein R1, R2 and R3 are defined as above; and * represents a linking site.

Gelling time of hydrogel of each group is listed in Table 1.

Table 1. Gelling time of hydrogels

| Sample | Gelling time |
|---|---|
| | Minutes (min) |
| HA-A1 / HA-A1-SH1 | 14±3 |
| HA-A2 / HA-A2-SH1 | 18±5 |
| HA-A1 / HA-MA1-SH1 | 15±3 |

(continued)

| Sample | Gelling time |
|---|---|
| | Minutes (min) |
| HA-A2 / HA-MA2-SH1 | 15±4 |
| CHS-A / CHS-A-SH1 | 22±5 |
| CHS-A / CHS-MA-SH1 | 24±4 |
| Gelatin-A / Gelatin-A-SH1 | 27±4 |
| Gelatin-A / Gelatin-MA-SH1 | 29±5 |
| CTS-A / CTS-A-SH1 | 24±6 |
| CTS-A / CTS-MA-SH1 | 22±5 |
| EGDA/HA-A1-SH1 | 66±15 |

Example 2. Detection of Storage Modulus of Hydrogels

2 mL of the mixed solution of hydrogel prepared in Example 1 was placed in a cylindrical mold, cross-linked at room temperature for 24 h, and then taken out to detect the storage modulus of the cross-linked samples, and the sample of each group was detected three times. The detection instrument was a TA-DHR2 rheometer, the detection probe was a 20 mm parallel plate probe, the detection temperature was 25 °C, and the shear frequency was 1 Hz. The test results are listed in Table 2.

Table 2. Comparison table of storage modulus

| Sample | Storage modulus G’ |
|---|---|
| | Pascal (Pa) |
| HA-A1 / HA-A1-SH1 | 1092±37 |
| HA-A2 / HA-A2-SH1 | 1133±51 |
| HA-A1 / HA-MA1-SH1 | 1040±39 |
| HA-A2 / HA-MA2-SH1 | 1269±44 |
| CHS-A / CHS-A-SH1 | 905±58 |
| CHS-A / CHS-MA-SH1 | 856±41 |
| Gelatin-A / Gelatin-A-SH1 | 784±47 |
| Gelatin-A / Gelatin-MA-SH1 | 812±34 |
| CTS-A / CTS-A-SH1 | 931±62 |
| CTS-A / CTS-MA-SH1 | 898±54 |
| EGDA/HA-A1-SH1 | 621±34 |

Example 3. Determination of Water Retention of Hydrogels

The hydrogel prepared in the Example 1 was added to a 20 mL glass bottle weighed in advance, and the mass of the hydrogel was obtained by the mass subtraction method and recorded as m0. The glass bottle was placed in a shaker at 37 °C and weighed at regular intervals to obtain a real-time mass of the hydrogel, which was recorded as mt. The water retention of hydrogel was calculated according to the following formula:

$$\text{Water retention rate (\%)} = mt / m0 \times 100\%$$

The results of water retention rate are shown in Table 3.

Table 3: Comparison table of water retention rate index

| Sample | Water retention rate | | | | |
|---|---|---|---|---|---|
| | Time point (day) | | | | |
| | 0 | 1 | 3 | 7 | 10 |
| HA-A1 / HA-A1-SH1 | 100% | 97.6% | 85.4% | 66.1% | 34.2% |
| HA-A2 / HA-A2-SH1 | 100% | 98.4% | 86.1% | 64.7% | 36.5% |
| HA-A1 / HA-MA1-SH1 | 100% | 96.5% | 83.3% | 61.8% | 33.5% |
| HA-A2 / HA-MA2-SH1 | 100% | 97.5% | 86.7% | 63.9% | 37.1% |
| CHS-A / CHS-A-SH1 | 100% | 94.2% | 80.2% | 52.1% | 22.4% |
| CHS-A / CHS-MA-SH1 | 100% | 93.7% | 81.7% | 54.8% | 24.5% |
| Gelatin-A / Gelatin-A-SH1 | 100% | 93.3% | 78.4% | 48.4% | 20.7% |
| Gelatin-A / Gelatin-MA-SH1 | 100% | 91.5% | 81.9% | 43.8% | 18.4% |
| CTS-A / CTS-A-SH1 | 100% | 96.4% | 85.8% | 50.5% | 28.3% |
| CTS-A / CTS-MA-SH1 | 100% | 94.2% | 84.3% | 51.2% | 27.8% |

Example 4: In Vitro Degradation Experiment of Hydrogels

Test of degradation stability: 10 mL of PBS solution was added to the hydrogel prepared in the Example 1 under experimental conditions of 37±0.1 °C and 65±5% relative humidity. The weight of the hydrogel at the initial time point was recorded as m0, the weight of the hydrogel measured at weeks 1, 4, 8, and 16 after the start of the degradation experiment was recorded as mt, and the degradation ratio of the hydrogel was calculated according to the following formula:

$$\text{Degradation rate (\%)} = (m0 - mt) / m0 \times 100\%$$

The results of the in vitro degradation test of the hydrogels are shown in Table 4.

Table 4. In vitro degradation test of hydrogels

| Sample | Degradation ratio | | | | |
|---|---|---|---|---|---|
| | Time points (week) | | | | |
| | 0 | 4 | 8 | 16 | 32 |
| HA-A1 / HA-A1-SH1 | 0% | 6.7% | 10.8% | 25.8% | 39.5% |
| HA-A2 / HA-A2-SH1 | 0% | 8.5% | 9.1% | 28.1% | 40.8% |
| HA-A1 / HA-MA1-SH1 | 0% | 3.6% | 7.1% | 24.7% | 35.8% |
| HA-A2 / HA-MA2-SH1 | 0% | 5.8% | 7.8% | 24.4% | 36.1% |
| CHS-A / CHS-A-SH1 | 0% | 2.4% | 7.3% | 16.9% | 32.9% |
| CHS-A / CHS-MA-SH1 | 0% | 1.6% | 6.9% | 17.6% | 31.7% |
| Gelatin-A / Gelatin-A-SH1 | 0% | 3.5% | 8.3% | 19.1% | 33.2% |
| Gelatin-A / Gelatin-MA-SH1 | 0% | 2.5% | 6.6% | 18.5% | 31.4% |
| CTS-A / CTS-A-SH1 | 0% | 5.8% | 9.9% | 23.9% | 38.8% |
| CTS-A / CTS-MA-SH1 | 0% | 6.3% | 9.1% | 22.6% | 37.7% |

Example 5. Cell Activity Assay of Hydrogels

The cellular activity and biocompatibility of HA-SH of the present disclosure were tested with reference to the criteria set forth in "GBT 16886.5-2017 Biological evaluation of medical devices-Part 5. Tests for in vitro cytotoxicity". Specifically, the following MTT method, also known as MTT colorimetric method, is a method used to detect the survival and growth of cells. The detection principle is that succinate dehydrogenase in mitochondria of living cells can reduce exogenous MTT into water-insoluble purple crystalline Formazan and deposit the Formazan in the cells, while dead cells do not have this function. Dimethyl sulfoxide (DMSO) can dissolve formazan in cells, and its absorbance at 490 nm can be determined by using an enzyme linked immunosorbent assay detector, so that the number of living cells can

be indirectly reflected. Within a certain range of cell number, MTT crystals are formed in an amount proportional to the cell number. The specific test procedures and results are as follows:
A solution of HA-A1 prepared in Preparation Example 1 (with a concentration of 10 mg/mL, a phosphate buffer as a solvent, pH = 7.4) was taken and recorded as solution A for later use.

A solution of HA-A1-SH1 prepared in Preparation Example 5 (with a concentration of 10 mg/mL, a phosphate buffer as a solvent, pH = 7.4) was taken and recorded as solution B for later use.

Cell culture medium was prepared with Dulbecco's Modified Eagle Medium, 10% fetal bovine serum and 1% penicillin/streptomycin solution. L929 cells were cultured conventionally, and after the cells were cultured to near confluency, the cells were digested to obtain a cell suspension.

The three components, namely the solution A, the solution B and the cell suspension, were uniformly mixed to prepare a cell/hydrogel composite system with a volume of 50 μL, wherein the final concentration of the cells is 1 × 106 cells/mL. The system was placed in a 24-well cell culture plate and 1 mL of cell culture medium was added to each well for culture, wherein cells with an equal number as those at the bottom of the plate served as a negative control group. The samples were incubated in a cell incubator at 5% CO2, 37 °C and > 90% humidity for 24 h. The survival states of cells in different hydrogel samples were detected by using the MTT assay, and the cell activity of the hydrogel group was compared with that of the negative control group. The negative control group was 100% active. The medium was removed, 100 μL of MTT was added to each well, and the mixture was further incubated for 4 h. Then the MTT solution was discarded, and 200 μL of DMSO solution was added to each well. After the plate was shaken to mix well, the absorbance at 490 nm was determined by a microplate reader. The test results are shown in FIG. 11. Materials with cell viability below 70% in MTT experiments are considered potentially cytotoxic. The results show that the survival rate of cells in hydrogels of the present disclosure is over 70%, suggesting that the materials have no significant cytotoxicity and have good biocompatibility.

Example 6. Animal Experiment for Shaping and Supporting Effect of Hydrogel

C57BL/6 mice were anesthetized and dehaired in the back, and conventional sterilization was performed. 120 μL of each of the HA-A1 and HA-A1-SH1 solution and the HA-A2 and HA-A2-SH1 solution (a concentration of 10 mg/mL for each) prepared as described in Example 1 was taken; namely, 12 mg of each material was dissolved in 1.2 mL of PBS (phosphate buffered saline) solution and shaken well to obtain samples for later use. 120 μL of each of the samples was sucked out by a syringe and mixed well, and the obtained hydrogel precursor solutions were each injected into the subcutaneous part of the back of a mouse using a 24G needle. The same volume of normal saline was injected into the subcutaneous part of the back of a mouse in the same manner.

The bulge site was photographed, measured using a vernier caliper and recorded in detail before injection, immediately after injection and at weeks 4, 8, and 12. The shaping effect of the hydrogels was evaluated by comparing the maintenance and change of the three-dimensional morphology of the different injection samples after being injected into an animal. The higher the height of the bulge at the injection site and the smaller the basal area, the better the shaping and supporting effect. The results are shown in FIGs. 12 and 13. The data shows that the hydrogel of the present disclosure forms a support body capable of maintaining a certain morphology after being injected into an animal, and the morphology stability of the injectant can be well kept.

Example 7: Animal Experiment for Shaping Effect of Hydrogel

C57BL/6 mice were anesthetized and dehaired in the back, and conventional sterilization was performed. 120 μL of each of the HA-A1 and HA-A1-SH1 solution and the HA-A2 and HA-A2-SH1 solution (a concentration of 10 mg/mL for each) prepared as described in Example 1 was taken; namely, 12 mg of each material was dissolved in 1.2 mL of PBS solution and shaken well to obtain samples for later use. 120 μL of each of the samples was sucked out by a syringe and mixed well, and the obtained hydrogel precursor solutions were each injected into the subcutaneous part of the back of a mouse using a 24G needle. A mouse was euthanized 60 min after injection, and the in situ formation condition and the morphology of the hydrogel under the skin of the mouse were observed. The remaining animals were still fed in a conventional way and the injection sites were photographed and observed at weeks 4, 8 and 12 after injection and the changes of gel volumes were recorded. The same volume of normal saline was injected into the subcutaneous part of the back of a mouse in the same manner.

After the hydrogel precursor mixed solution was injected into the subcutaneous part of the back of a mouse, a round bulge was visible at the injection site. It was observed, 60 min after injection, that the hydrogel formed subcutaneously in the mouse was a transparent intact hemisphere. The results show that the mixed precursor solution can rapidly be subjected to a cross-linking reaction, form hydrogel at the injection site and maintain a certain morphology. 12 weeks after injection, a significant bulge was still observed in the subcutaneous part of the back of the animal. The hydrogel and the local state of the surrounding tissues were observed by cutting the skin tissues, and it was found that

the hydrogel was in good morphology, and the surrounding tissues had no abnormalities such as inflammation, infection and necrosis. The hydrogel was observed and weighed after being taken out, and it was found that the hydrogel was still in the shape of an intact hemisphere, the weight was slightly reduced after weighing (see results in FIG. 14), and no significant gel fracture, disintegration and the like were observed.

The results show that the hydrogel of the present disclosure has relatively superior performance in degradation resistance and maintenance of gel stability.

The examples of the present disclosure have been described above. However, the present disclosure is not limited to the above examples.

## Claims

1. A hydrogel, **characterized in that**, the hydrogel is prepared by gelation of a system comprising a sulfhydryl-modified polymer compound, wherein
   the sulfhydryl-modified polymer compound is at least one of the following series of compounds:

   a series of sulfhydryl-modified polymer compounds, wherein to-be-modified polymer compounds comprise at least one selected from -COOH, $-NH_2$, -OH, an acrylate group of formula a, an acrylamide group of formula b and an acryloyl group of formula c in the structure,

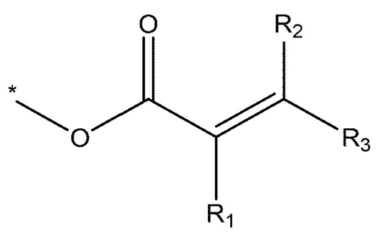
formula a

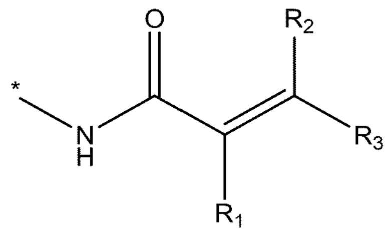
formula b

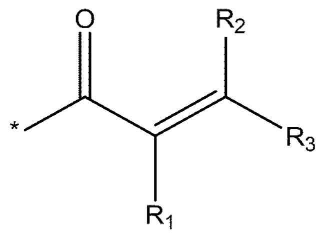
formula c,

   wherein part or all of the -COOH and/or the $-NH_2$ and/or the -OH and/or the acrylate group and/or the acrylamide group and/or the acryloyl group are modified to form a side chain with the following terminal group:

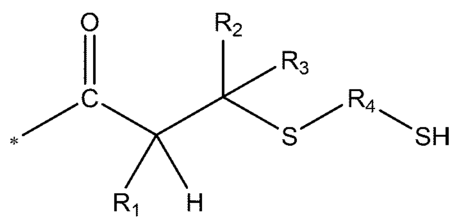

   wherein in the above group, * represents a linking site; $R_1$ is selected from hydrogen, halogen, an aliphatic group, an aromatic group and the like; $R_2$ and $R_3$ are the same or different and independently from each other, and are selected from hydrogen, halogen, an aliphatic group, an aromatic group and the like; $R_4$ is a polysulfhydryl compound fragment;
   the system further comprises at least one of the following substances:

   C1. an acryloylated polymer compound, and
   C2. a small molecule cross-linking agent containing an acryloyl group;
   wherein the substance C2, the small molecule cross-linking agent containing an acryloyl group, includes

at least one of the following substanes: ethylene glycol diacrylate (EGDA), polyethylene glycol diacrylate (PEGDA), trimethylolpropane triacrylate (TMPTA), pentaerythritol triacrylate (PTA), pentaerythritol tetraacrylate (PTTA), di(trimethylolpropane) tetraacrylate (DTTA).

**2.** The hydrogel according to claim 1, wherein part or all of the -COOH and/or the -$NH_2$ and/or the -OH and/or the acrylate group and/or the acrylamide group and/or the acryloyl group are modified to form at least one of the following structures:

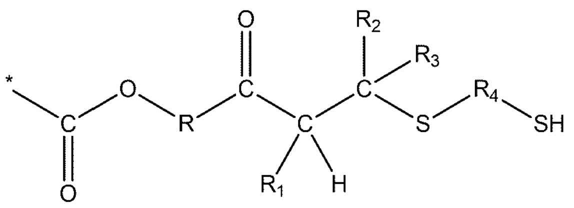

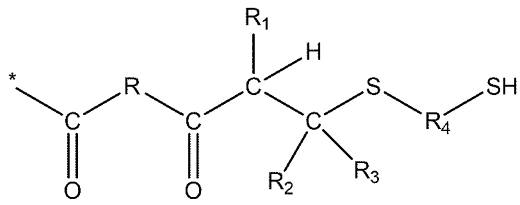

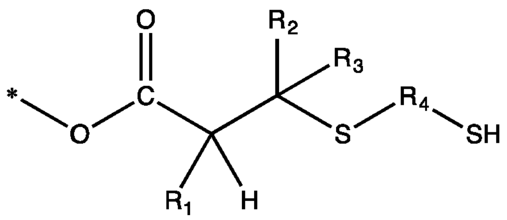

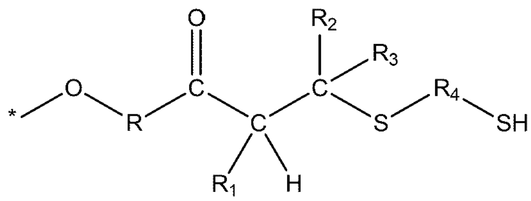

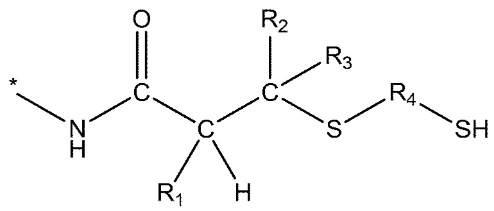

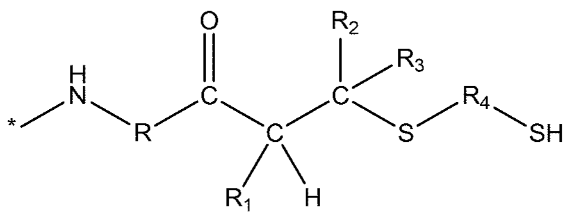

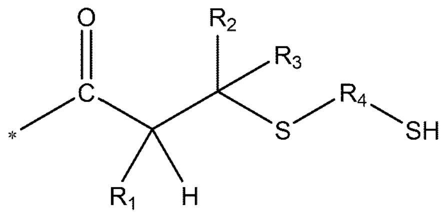

wherein in the above structures, R is selected from

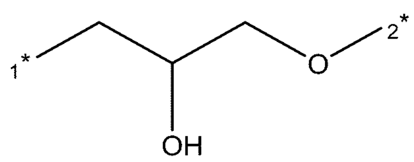
,

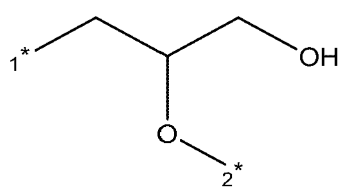
,

hydrocarbylene, arylene, an amide residue, a hydrazide residue, and the like; * represents a linking site; $_1$* represents a linking site to a left-hand group of R; $_2$* represents a linking site to a right-hand group of R; $R_1$, $R_2$, $R_3$ and $R_4$ are defined as above.

3. The hydrogel according to claim 1 or 2, wherein at least one selected from the -COOH, the -$NH_2$, the -OH, the acrylate group of formula a, the acrylamide group of formula b, and the acryloyl group of formula c can be directly linked to a main chain of the polymer compound, or linked to the main chain of the polymer compound via an R' group, and the R' group can be a heteroatom-containing group, hydrocarbylene, arylene or the following linker:

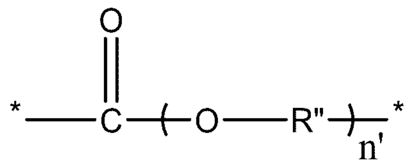

wherein in the above formula, R" is hydrocarbylene or arylene, n' is an integer from 1 to 1000, and * represents a linking site.

4. The hydrogel according to any one of claims 1-3, wherein the acryloylated polymer compound is selected from at least one of the following substances:

1) an acryloylated compound of a polymer compound comprising at least one selected from -COOH, -$NH_2$ and -OH in the structure, namely, an acryloylated compound formed by linking at least one selected from -COOH, -$NH_2$ and -OH comprised in the structure of the polymer compound, directly or indirectly, to the following group:

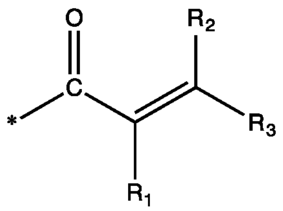

wherein $R_1$, $R_2$ and $R_3$ are defined as above;
2) a polymer compound comprising at least one selected from the acrylate group of formula a, the acrylamide group of formula b and the acryloyl group of formula c in the structure.

5. The hydrogel according to claim 4, wherein in the above substance 1), part or all of the -COOH and/or the -$NH_2$ and/or the -OH are modified to form at least one of the following structures:

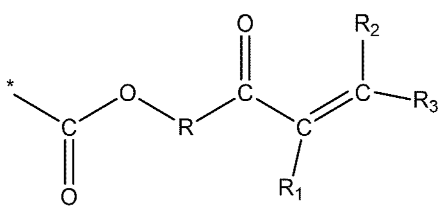

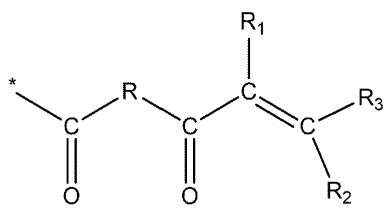

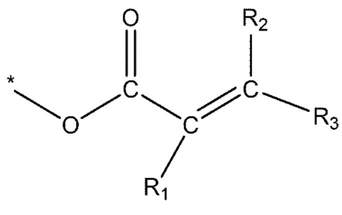

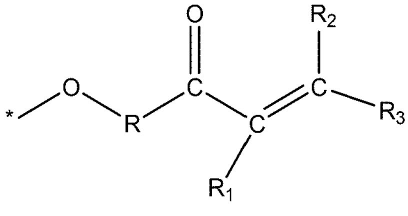

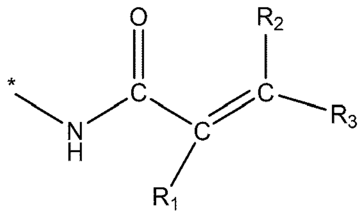

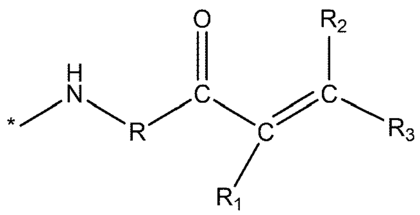

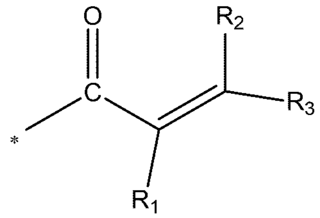

wherein in the above structures, R is selected from

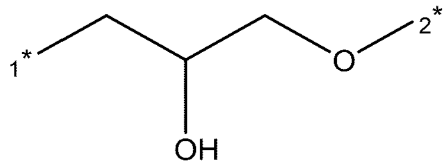

,

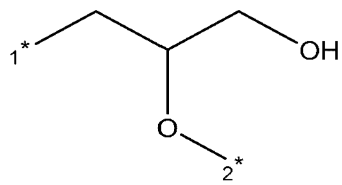

,

hydrocarbylene, arylene, an amide residue, a hydrazide residue, and the like; * represents a linking site; $_1$* represents a linking site to a left-hand group of R; $_2$* represents a linking site to a right-hand group of R; $R_1$, $R_2$, $R_3$ and $R_4$ are defined as above.

**6.** The hydrogel according to claim 4 or 5, wherein in the above substance 1), at least one selected from the -COOH, the-$NH_2$ and the -OH can be directly linked to the main chain of the polymer compound, or linked to the main chain of the polymer compound via an R' group, and the R' group can be a heteroatom-containing group, hydrocarbylene, arylene or the following linker:

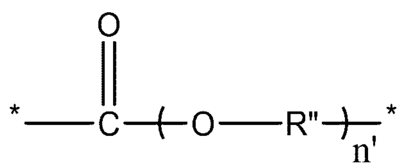

wherein in the above formula, R" is hydrocarbylene or arylene, n' is an integer from 1 to 1000, and * represents a linking site.

**7.** The hydrogel according to any one of claims 1-6, wherein the hydrogel comprises the following characteristic structural unit:

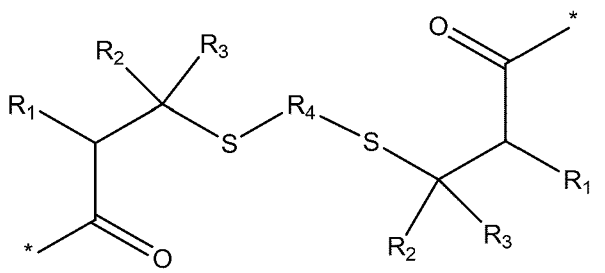

wherein in the above unit, $R_1$, $R_2$, $R_3$ and $R_4$ are defined as above, and * represents a linking site.

**8.** A preparation method for the hydrogel according to any one of claims 1-7, comprising the following step:

gelling a system comprising the following substances:

(i) the sulfhydryl-modified polymer compound, and
(ii) at least one selected from the substance C1 and the substance C2,

thus obtaining the hydrogel.

**9.** The preparation method according to claim 8, wherein
a solution of the sulfhydryl-modified polymer compound, a solution of the acryloylated polymer compound, a solution of the small molecule cross-linking agent and optionally a solution of at least one selected from other biological functional materials, drugs, growth factors and cell suspensions were prepared, and then these solutions were mixed and gelled to obtain the hydrogel.

**10.** The preparation method according to claim 9, wherein at least one selected from the other biological functional materials, the drugs, the growth factors and the cell suspensions can be introduced by directly addition into the solution of the sulfhydryl-modified polymer compound, the solution of the acryloylated polymer compound or the solution of the small molecule cross-linking agent.

**11.** The preparation method according to claim 9, wherein a preparation process of the hydrogel can be performed by adding the solution of the sulfhydryl-modified polymer compound into the solution of the acryloylated polymer compound and/or the solution of the small molecule cross-linking agent, or by adding the solution of the acryloylated polymer compound and/or the solution of the small molecule cross-linking agent into the solution of the sulfhydryl-modified polymer compound; specifically, the two solutions can be mixed by a common syringe, by a double-needle syringe, or by other means.

**12.** Use of the hydrogel according to any one of claims 1-7 in the fields of biopharmaceuticals, medical cosmetology, cosmetics and the like, wherein specifically, the hydrogel can be used in preparation of drug delivery systems, dressings for soft tissue wound repair, scaffold materials for bone repair, viscoelastic agents for supporting in ophthalmic surgery, materials for preventing tissue adhesion after surgery, scaffold materials for 3D bioprinting, and the like.

## Patentansprüche

**1.** Hydrogel, **dadurch gekennzeichnet, dass** das Hydrogel durch Gelierung eines Systems hergestellt wird, das eine sulfhydryl-modifizierte Polymerverbindung umfasst, wobei
es sich bei der sulfhydryl-modifizierten Polymerverbindung zumindest um eine der folgenden Reihen von Verbindungen handelt:

eine Reihe von sulfhydryl-modifizierten Polymerverbindungen, wobei die zu modifizierenden Polymerverbindungen zumindest eine ausgewählt aus -COOH, $-NH_2$, -OH, einer Acrylatgruppe von Formel a, einer Acrylamidgruppe von Formel b und einer Acryloylgruppe von Formel c in der Struktur umfasst,

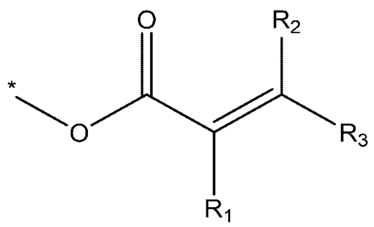

Formel a

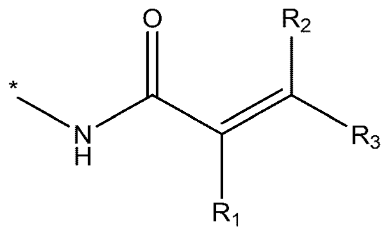

Formel b

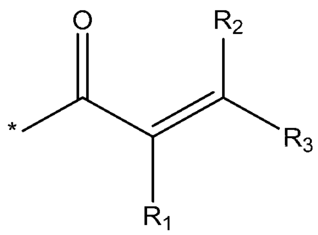

Formel c,

wobei ein Teil oder alle der -COOH und/oder der $-NH_2$ und/oder der -OH und/oder der Acrylatgruppe und/oder der Acrylamidgruppe und/oder der Acryloylgruppe modifiziert sind, um eine Seitenkette mit der folgenden Endgruppe zu bilden:

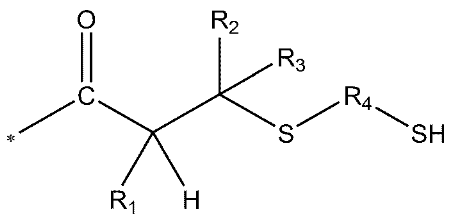

wobei in der obenstehenden Gruppe * eine Verknüpfungsstelle darstellt; $R_1$ aus Wasserstoff, Halogen, einer aliphatischen Gruppe, einer aromatischen Gruppe und dergleichen ausgewählt ist; $R_2$ und $R_3$ gleich oder unterschiedlich und unabhängig voneinander sind und aus Wasserstoff, Halogen, einer aliphatischen Gruppe, einer aromatischen Gruppe und dergleichen ausgewählt sind; es sich bei $R_4$ um ein Polysulfhydryl-Verbindungsfragment handelt;
das System ferner zumindest eine der folgenden Substanzen umfasst:

C1. eine acryloylierte Polymerverbindung und
C2. ein niedermolekulares Vernetzungsmittel, das eine Acryloylgruppe enthält;

wobei die Substanz C2, das niedermolekulare Vernetzungsmittel, das eine Acryloylgruppe enthält, zumindest eine der folgenden Substanzen umfasst: Ethylenglykoldiacrylat (EGDA), Polyethylenglykoldiacrylat (PEGDA), Trimethylolpropantriacrylat (TMPTA), Pentaerythritol-Triacrylat (PTA), Pentaerythritol-Tetraacrylat (PTTA), Di(trimethylolpropan)tetraacrylat (DTTA).

**2.** Hydrogel nach Anspruch 1, wobei ein Teil oder alle der -COOH und/oder der $-NH_2$ und/oder der -OH und/oder der Acrylatgruppe und/oder der Acrylamidgruppe und/oder der Acryloylgruppe modifiziert sind, um zumindest eine der folgenden Strukturen zu bilden:

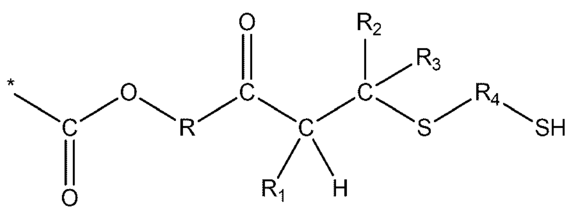
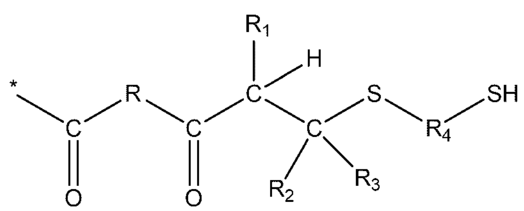

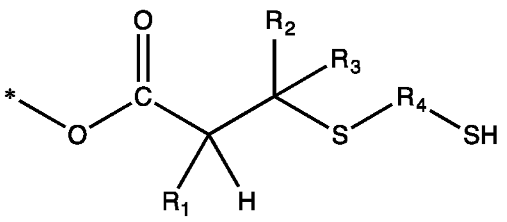

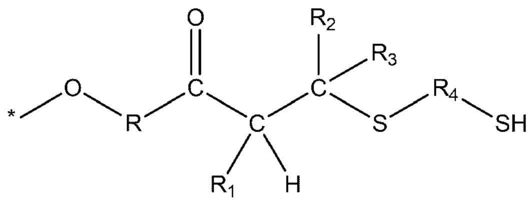

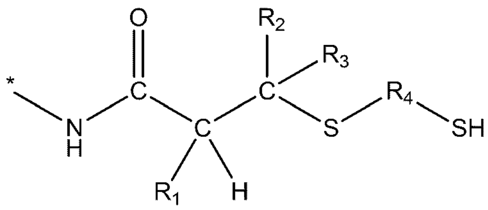

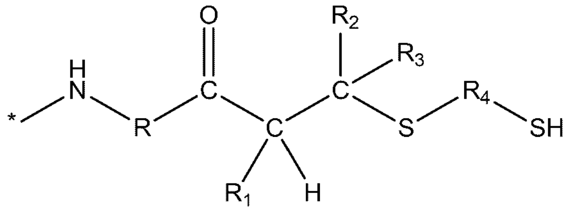

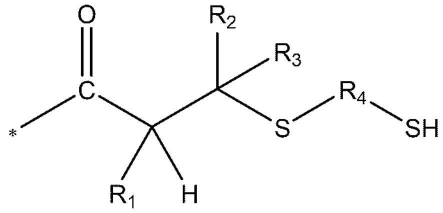

wobei in den obenstehenden Strukturen R aus

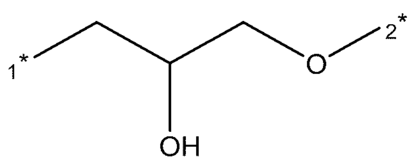

,

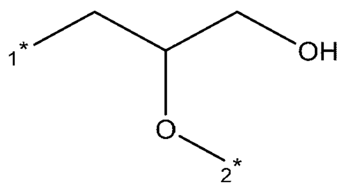

,

Hydrocarbylen, Arylen, einem Amidrest, einem Hydrazidrest und dergleichen ausgewählt ist; * eine Verknüpfungsstelle darstellt; $_1$* eine Verknüpfungsstelle zu einer linken Gruppe von R darstellt; $_2$* eine Verknüpfungsstelle zu einer rechten Gruppe von R darstellt; $R_1$, $R_2$, $R_3$ und $R_4$ wie obenstehend definiert sind.

**3.** Hydrogel nach Anspruch 1 oder 2, wobei zumindest eine ausgewählt aus der -COOH, der $-NH_2$, der -OH, der Acrylatgruppe von Formel a, der Acrylamidgruppe von Formel b, und der Acryloylgruppe von Formel c direkt mit einer Hauptkette der Polymerverbindung verknüpfbar ist, oder mit der Hauptkette der Polymerverbindung über eine R'-Gruppe verknüpfbar ist, und die R'-Gruppe in der Lage ist, eine ein Heteroatom enthaltende Gruppe, Hydrocarbylen, Arylen oder der folgende Linker zu sein:

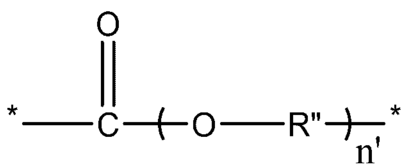

wobei es sich in der obenstehenden Formel bei R" um Hydrocarbylen oder Arylen handelt, es sich bei n' um eine ganze Zahl von 1 bis 1000 handelt, und * eine Verknüpfungsstelle darstellt.

**4.** Hydrogel nach einem der Ansprüche 1-3, wobei die acryloylierte Polymerverbindung aus zumindest einer der folgenden Substanzen ausgewählt ist:

1) einer acryloylierten Verbindung einer Polymerverbindung, umfassend zumindest eine ausgewählt aus -COOH, $-NH_2$ und -OH in der Struktur, nämlich eine acryloylierte Verbindung, die durch direkte oder indirekte Verknüpfung von zumindest einer ausgewählt aus -COOH, $-NH_2$ und -OH, die in der Struktur der Polymerverbindung enthalten ist, mit der folgenden Gruppe gebildet wird:

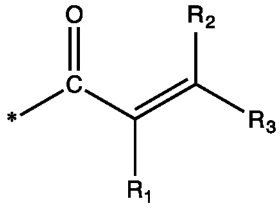

wobei $R_1$, $R_2$ und $R_3$ wie obenstehend definiert sind;
2) eine Polymerverbindung, umfassend zumindest eine ausgewählt aus der Acrylatgruppe von Formel a, der Acrylamidgruppe von Formel b und der Acryloylgruppe von Formel c in der Struktur.

**5.** Hydrogel nach Anspruch 4, wobei in der obenstehenden Substanz 1), ein Teil oder alle der -COOH und/oder der $-NH_2$ und/oder der -OH modifiziert sind, um zumindest eine der folgenden Strukturen zu bilden:

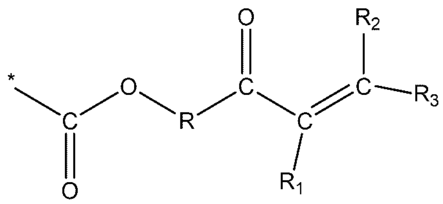 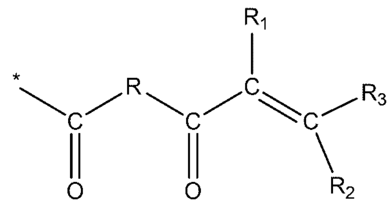

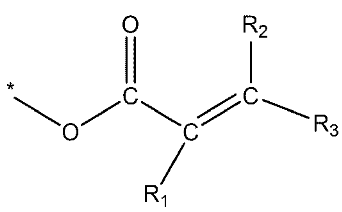 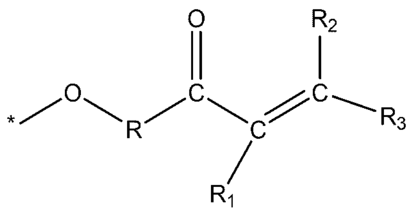

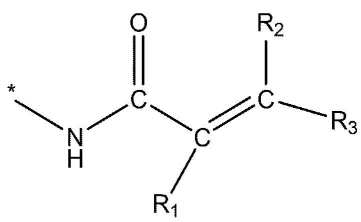 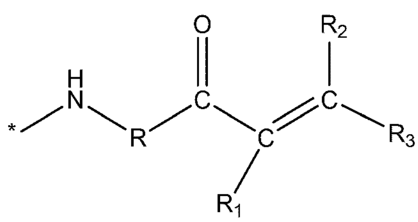

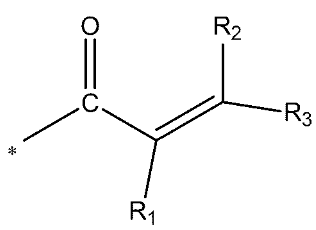

wobei in den obenstehenden Strukturen R aus

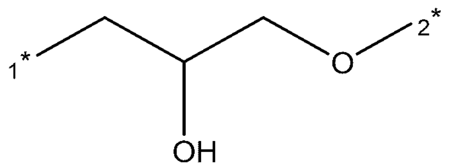

,

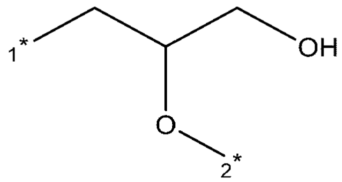

,

Hydrocarbylen, Arylen, einem Amidrest, einem Hydrazidrest und dergleichen ausgewählt ist; * eine Verknüpfungsstelle darstellt; $_1$* eine Verknüpfungsstelle zu einer linken Gruppe von R darstellt; $_2$* eine Verknüpfungsstelle zu einer rechten Gruppe von R darstellt; $R_1$, $R_2$, $R_3$ und $R_4$ wie obenstehend definiert sind.

**6.** Hydrogel nach Anspruch 4 oder 5, wobei in der obenstehenden Substanz 1) zumindest eine ausgewählt aus der -COOH, der $-NH_2$, der -OH direkt mit der Hauptkette der Polymerverbindung verknüpfbar ist, oder mit der Hauptkette der Polymerverbindung über eine R'-Gruppe verknüpfbar ist, und die R'-Gruppe in der Lage ist, eine ein Heteroatom enthaltende Gruppe, Hydrocarbylen, Arylen oder der folgende Linker zu sein:

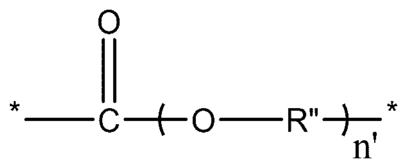

wobei es sich in der obenstehenden Formel bei R" um Hydrocarbylen oder Arylen handelt, es sich bei n' um eine ganze Zahl von 1 bis 1000 handelt, und * eine Verknüpfungsstelle darstellt.

**7.** Hydrogel nach einem der Ansprüche 1-6, wobei das Hydrogel die folgende charakteristische Struktureinheit umfasst:

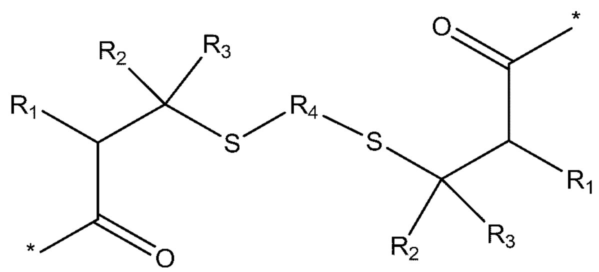

wobei die obenstehende Einheit, $R_1$, $R_2$, $R_3$ und $R_4$ wie obenstehend definiert sind und * eine Verknüpfungsstelle darstellt.

**8.** Herstellungsverfahren für das Hydrogel nach einem der Ansprüche 1-7, umfassend die folgenden Schritte:

Gelieren eines Systems, umfassend die folgenden Substanzen:

(i) die Sulfhydryl-modifizierte Polymerverbindung, und
(ii) zumindest eine ausgewählt aus der Substanz C1 und der Substanz C2,

dadurch Erhalten des Hydrogels.

**9.** Herstellungsverfahren nach Anspruch 8, wobei eine Lösung der sulfhydryl-modifizierten Polymerverbindung, eine Lösung der acryloylierten Polymerverbindung, eine Lösung des niedermolekularen Vernetzungsmittels und optional eine Lösung von zumindest einem ausgewählt aus anderen biologischen Funktionsmaterialien, Arzneimitteln, Wachstumsfaktoren und Zellsuspensionen hergestellt werden und dann diese Lösungen gemischt und geliert werden, um das Hydrogel zu erhalten.

**10.** Herstellungsverfahren nach Anspruch 9, wobei sich zumindest eines ausgewählt aus den anderen biologischen Funktionsmaterialien, den Arzneimitteln, den Wachstumsfaktoren und den Zellsuspensionen durch direkte Zugabe in die Lösung der sulfhydryl-modifizierten Polymerverbindung, die Lösung der acryloylierten Polymerverbindung oder die Lösung des niedermolekularen Vernetzungsmittels einbringen lässt.

**11.** Herstellungsverfahren nach Anspruch 9, wobei ein Herstellungsprozess des Hydrogels durch Zugabe der Lösung der sulfhydryl-modifizierten Polymerverbindung zu der Lösung der acryloylierten Polymerverbindung und/oder der Lösung des niedermolekularen Vernetzungsmittels oder durch Zugabe der Lösung der acryloylierten Polymerverbindung und/oder der Lösung des niedermolekularen Vernetzungsmittels zu der Lösung der sulfhydryl-modifizierten Polymerverbindung durchführbar ist; insbesondere sich die zwei Lösungen durch eine gemeinsame Spritze, durch eine Doppelnadelspritze oder durch andere Mittel mischen lässt.

**12.** Verwendung des Hydrogels nach einem der Ansprüche 1-7 in den Bereichen Biopharmazeutika, medizinische Kosmetik, Kosmetik und dergleichen, wobei das Hydrogel insbesondere bei der Herstellung von Arzneimittelabgabesystemen, Verbänden für die Reparatur von Weichgewebewunden, Scaffold-Materialien für die Knochenreparatur, viskoelastischen Mitteln zur Unterstützung in der Augenchirurgie, Materialien zur Verhinderung von Gewebeadhäsion nach einer Operation, Scaffold-Materialien für das 3D-Bioprinting und dergleichen verwendbar ist.

**Revendications**

**1.** Hydrogel, **caractérisé en ce que** l'hydrogel est préparé par gélification d'un système comprenant un composé polymère modifié par un sulfhydryle, dans lequel
le composé polymère modifié par un sulfhydryle est au moins l'une des séries de composés suivantes :

une série de composés polymères modifiés par un sulfhydryle, dans lequel les composés polymères à modifier comprennent au moins un composé choisi parmi -COOH, -$NH_2$, -OH, un groupe acrylate de formule a, un groupe acrylamide de formule b et un groupe acryloyle de formule c dans la structure,

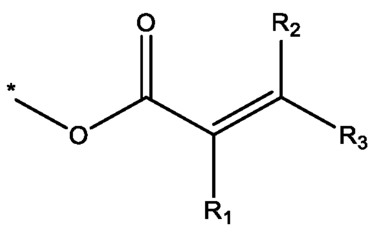
formule a

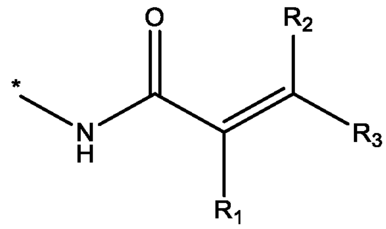
formule b

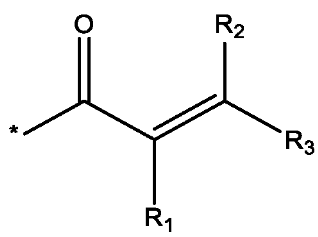
formule c,

dans lequel une partie ou la totalité du -COOH et/ou du -$NH_2$ et/ou du -OH et/ou du groupe acrylate et/ou du groupe acrylamide et/ou du groupe acryloyle sont modifiés pour former une chaîne latérale avec le groupe terminal suivant :

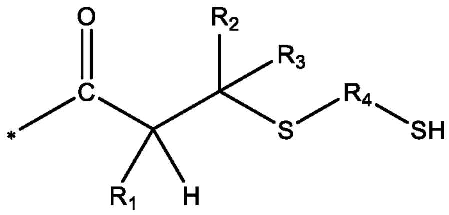

dans lequel dans le groupe ci-dessus, * représente un site de liaison ; $R_1$ est choisi parmi l'hydrogène, un halogène, un groupe aliphatique, un groupe aromatique et similaires ; $R_2$ et $R_3$ sont identiques ou différents et indépendamment l'un de l'autre, et sont choisis parmi l'hydrogène, un halogène, un groupe aliphatique, un groupe aromatique et similaires ; $R_4$ est un fragment de composé polysulfhydryle ;
le système comprend en outre au moins l'une des substances suivantes :

C1. un composé polymère acryloylé, et
C2. un agent de réticulation à petites molécules contenant un groupe acryloyle ;

dans lequel la substance C2, l'agent de réticulation à petites molécules contenant un groupe acryloyle, comprend au moins l'une des substances suivantes : diacrylate d'éthylène glycol (EGDA), diacrylate de polyéthylène glycol (PEGDA), triacrylate de triméthylolpropane (TMPTA), triacrylate de pentaérythritol (PTA), tétraacrylate de pentaérythritol (PTTA), tétraacrylate de di(triméthylolpropane) (DTTA).

**2.** Hydrogel selon la revendication 1, dans lequel une partie ou la totalité du -COOH et/ou du -$NH_2$ et/ou du -OH et/ou du groupe acrylate et/ou du groupe acrylamide et/ou du groupe acryloyle sont modifiées pour former au moins une des structures suivantes :

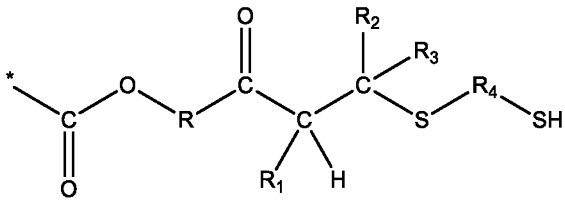

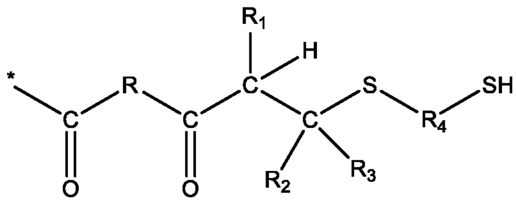

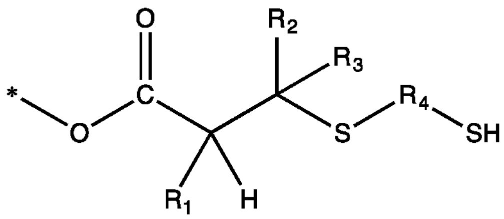

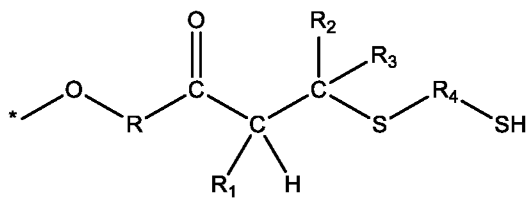

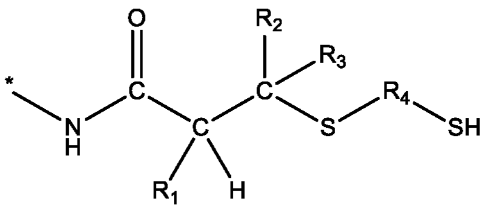

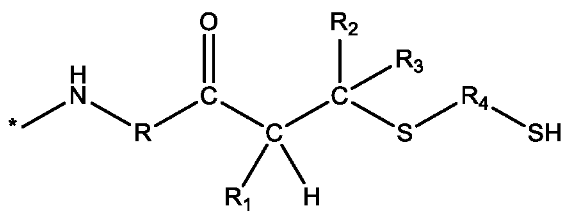

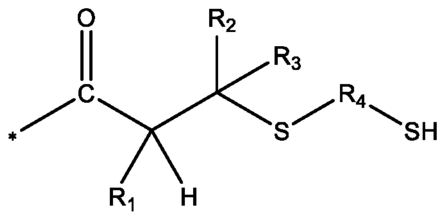

dans lequel dans les structures ci-dessus, R est choisi parmi

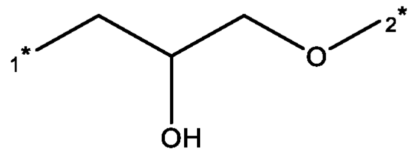

,

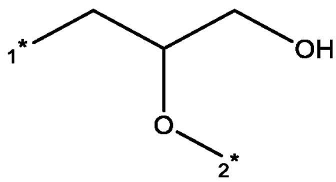

,

hydrocarbylène, arylène, un résidu amide, un résidu hydrazide, et similaires ; * représente un site de liaison ; $_1$* représente un site de liaison vers un groupe de gauche de R ; $_2$* représente un site de liaison vers un groupe de droite de R ; $R_1$, $R_2$, $R_3$ et $R_4$ sont définis comme ci-dessus.

**3.** Hydrogel selon la revendication 1 ou 2, dans lequel au moins un choisi parmi -COOH, $-NH_2$, -OH, le groupe acrylate de formule a, le groupe acrylamide de formule b et le groupe acryloyle de formule c peut être directement lié à une chaîne principale du composé polymère, ou lié à la chaîne principale du composé polymère via un groupe R', et le groupe R' peut être un groupe contenant un hétéroatome, un hydrocarbylène, un arylène ou le lieur suivant :

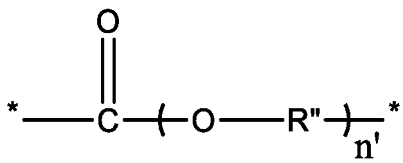

dans lequel dans la formule ci-dessus, R" est un hydrocarbylène ou un arylène, n' est un nombre entier de 1 à 1000 et * représente un site de liaison.

**4.** Hydrogel selon l'une quelconque des revendications 1 à 3, dans lequel le composé polymère acryloylé est choisi parmi au moins l'une des substances suivantes :

1) un composé acryloylé d'un composé polymère comprenant au moins un choisi parmi -COOH, $-NH_2$ et -OH dans la structure, à savoir un composé acryloylé formé en liant au moins un composé choisi parmi -COOH, $-NH_2$ et -OH compris dans la structure du composé polymère, directement ou indirectement, au groupe suivant :

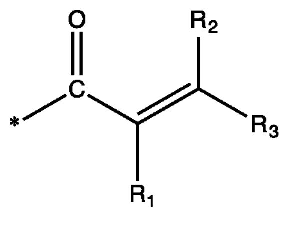

dans lequel $R_1$, $R_2$ et $R_3$ sont définis comme ci-dessus ;
2) un composé polymère comprenant au moins un choisi parmi le groupe acrylate de formule a, le groupe acrylamide de formule b et le groupe acryloyle de formule c dans la structure.

**5.** Hydrogel selon la revendication 4, dans lequel dans la substance 1) ci-dessus, une partie ou la totalité du -COOH et/ou du $-NH_2$ et/ou du -OH sont modifiées pour former au moins une des structures suivantes :

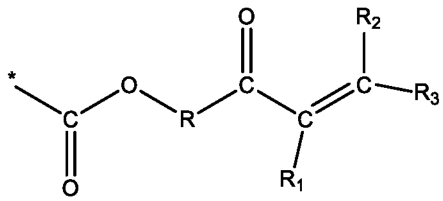

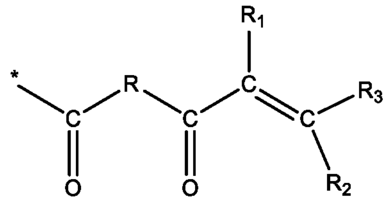

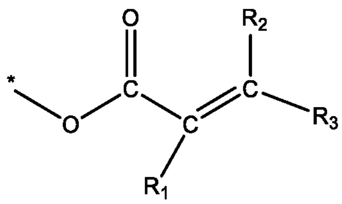

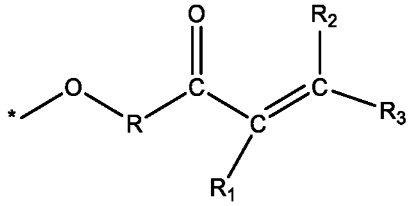

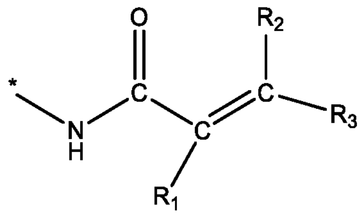

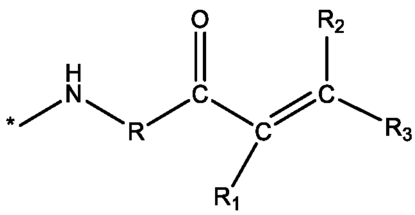

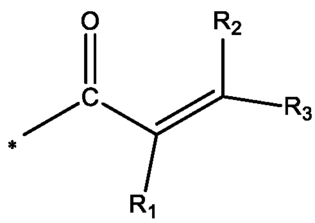

dans lequel dans les structures ci-dessus, R est choisi parmi

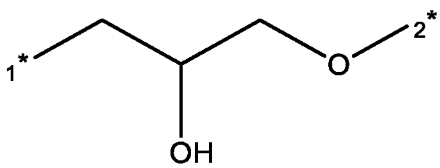

,

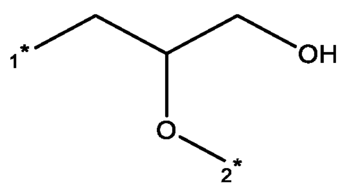

,

hydrocarbylène, arylène, un résidu amide, un résidu hydrazide, et similaires ; * représente un site de liaison ; $_1$* représente un site de liaison vers un groupe de gauche de R ; $_2$* représente un site de liaison vers un groupe de droite de R ; $R_1$, $R_2$, $R_3$ et $R_4$ sont définis comme ci-dessus.

**6.** Hydrogel selon la revendication 4 ou 5, dans lequel dans la substance 1) ci-dessus, au moins un choisi parmi -COOH, le -$NH_2$ et le -OH peut être directement lié à la chaîne principale du composé polymère, ou lié à la chaîne principale du composé polymère via un groupe R', et le groupe R' peut être un groupe contenant un hétéroatome, un hydrocarbylène, un arylène ou le lieur suivant :

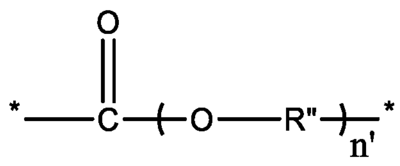

dans lequel dans la formule ci-dessus, R" est un hydrocarbylène ou un arylène, n' est un nombre entier de 1 à 1000 et * représente un site de liaison.

**7.** Hydrogel selon l'une quelconque des revendications 1 à 6, l'hydrogel comprenant l'unité structurelle caractéristique suivante :

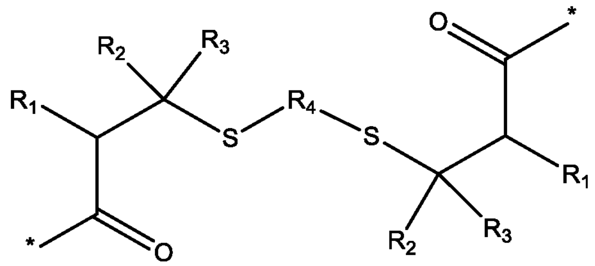

dans lequel dans l'unité ci-dessus, $R_1$, $R_2$, $R_3$ et $R_4$ sont définis comme ci-dessus, et * représente un site de liaison.

**8.** Procédé de préparation de l'hydrogel selon l'une quelconque des revendications 1 à 7, comprenant l'étape suivante :

la gélification d'un système comprenant les substances suivantes :

(i) le composé polymère modifié par un sulfhydryle, et
(ii) au moins une substance choisie parmi la substance C1 et la substance C2,

obtenant ainsi l'hydrogel.

**9.** Procédé de préparation selon la revendication 8, dans lequel
une solution du composé polymère modifié par sulfhydryle, une solution du composé polymère acryloylé, une solution de l'agent de réticulation à petite molécule et éventuellement une solution d'au moins un élément sélectionné parmi d'autres matériaux fonctionnels biologiques, médicaments, facteurs de croissance et suspensions cellulaires ont été préparées, puis ces solutions ont été mélangées et gélifiées pour obtenir l'hydrogel.

**10.** Procédé de préparation selon la revendication 9, dans lequel au moins un choisi parmi les autres matériaux fonctionnels biologiques, les médicaments, les facteurs de croissance et les suspensions cellulaires peuvent être introduits par addition directe dans la solution du composé polymère modifié par un sulfhydryle, la solution de le composé polymère acryloylé ou la solution de l'agent de réticulation à petite molécule.

**11.** Procédé de préparation selon la revendication 9, dans lequel un processus de préparation de l'hydrogel peut être effectué en ajoutant la solution du composé polymère modifié par un sulfhydryle à la solution du composé polymère acryloylé et/ou à la solution de l'agent de réticulation à petite molécules, ou en ajoutant la solution du composé polymère acryloylé et/ou la solution de l'agent de réticulation à petite molécule dans la solution du composé polymère modifié par un sulfhydryle ; en effet, les deux solutions peuvent être mélangées par une seringue commune, par une seringue à double aiguille ou par d'autres moyens.

**12.** Utilisation de l'hydrogel selon l'une quelconque des revendications 1 à 7 dans les domaines des produits biopharmaceutiques, de la cosmétologie médicale, des cosmétiques et similaires, dans laquelle spécifiquement, l'hydrogel peut être utilisé dans la préparation de systèmes d'administration de médicaments, de pansements pour la réparation de plaies des tissus mous, de matériaux d'échafaudage pour la réparation osseuse, d'agents viscoélastiques comme adjuvants en chirurgie ophtalmologique, des matériaux pour empêcher l'adhérence des tissus après une chirurgie, de matériaux d'échafaudage pour la bio-impression 3D, et similaires.

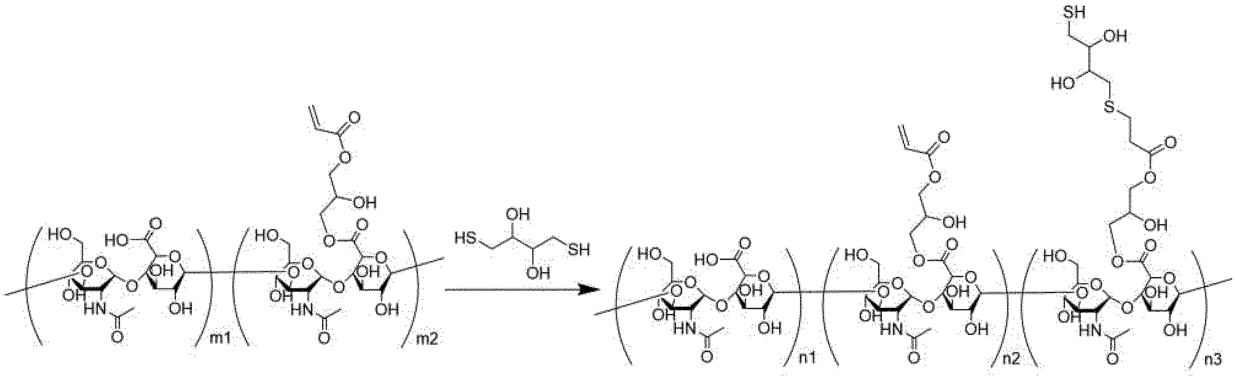

HA-A1

HA-A1-SH1

FIG. 1

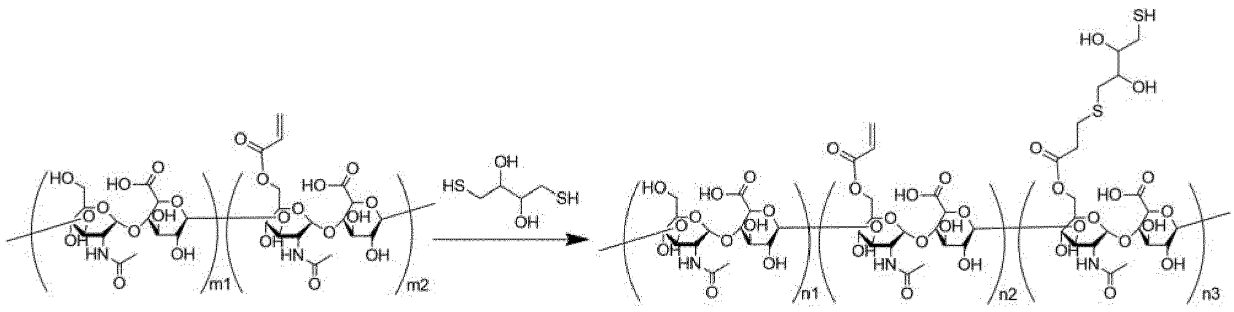

HA-A2

HA-A2-SH1

FIG. 2

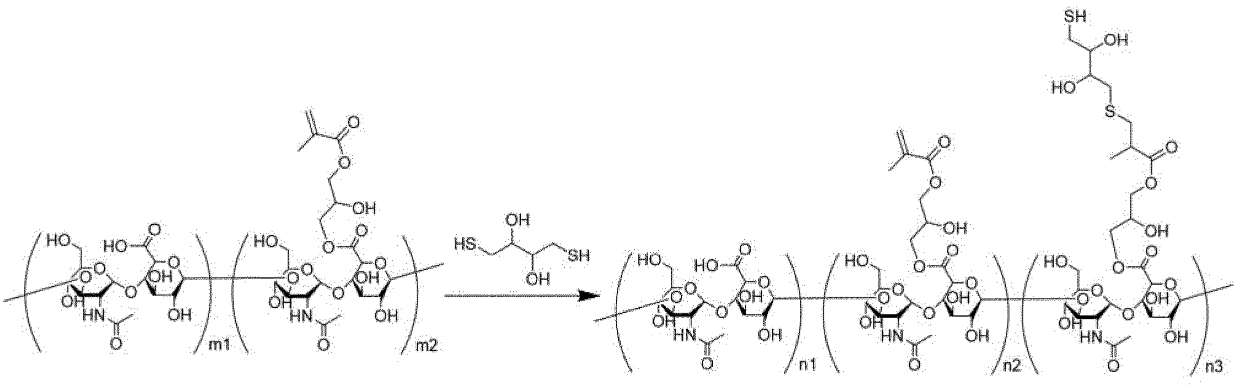

HA-MA1

HA-MA1-SH1

FIG. 3

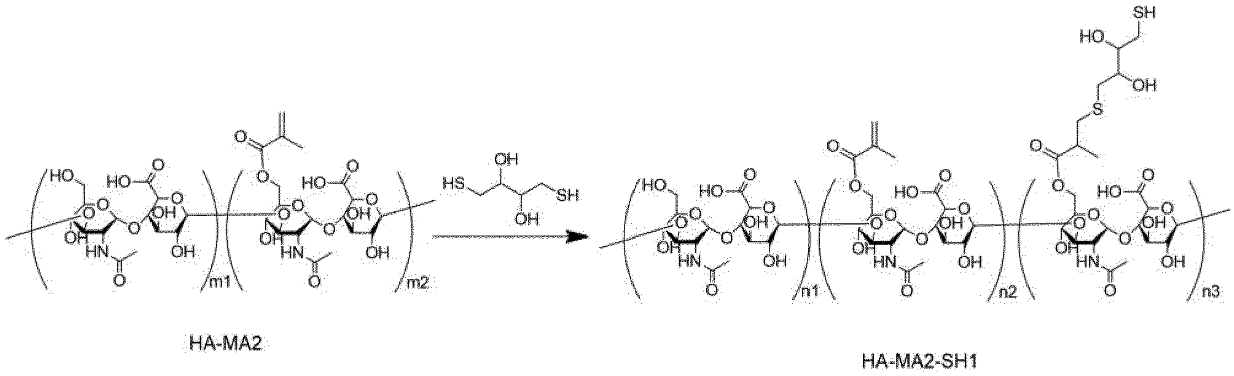


FIG. 4

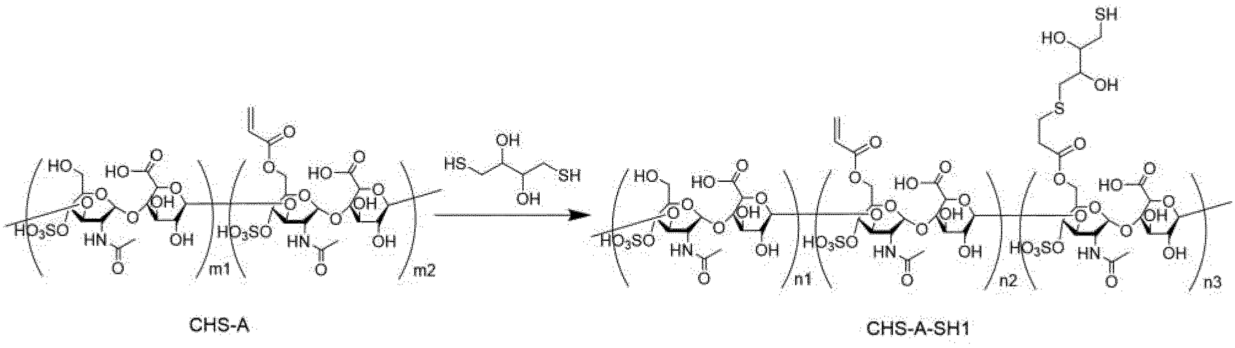


FIG. 5

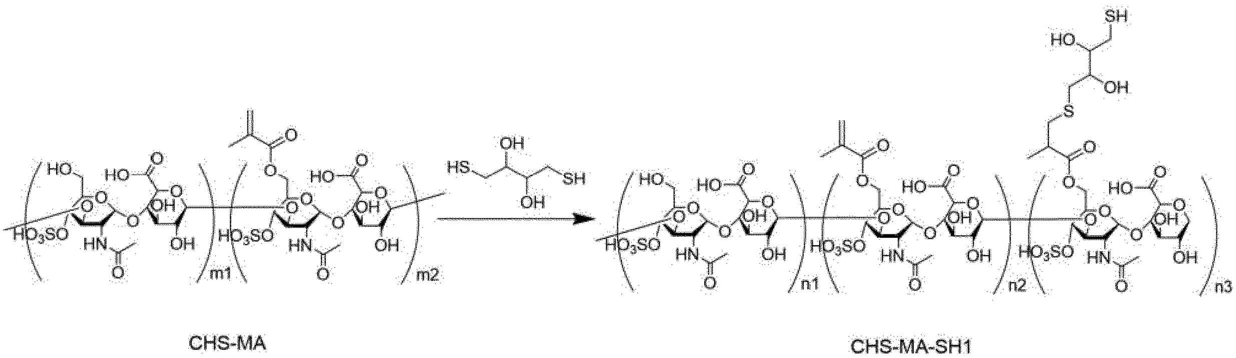


FIG. 6

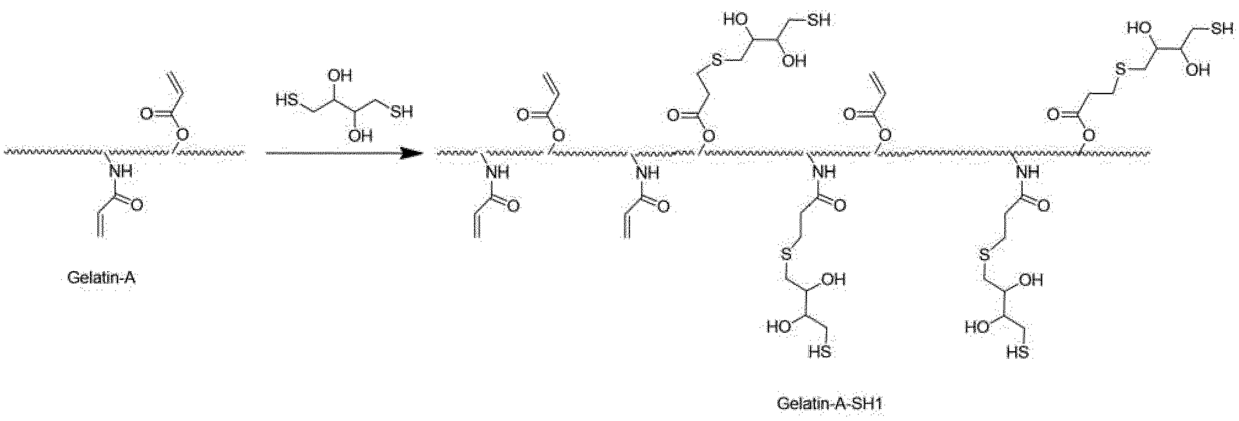


FIG. 7

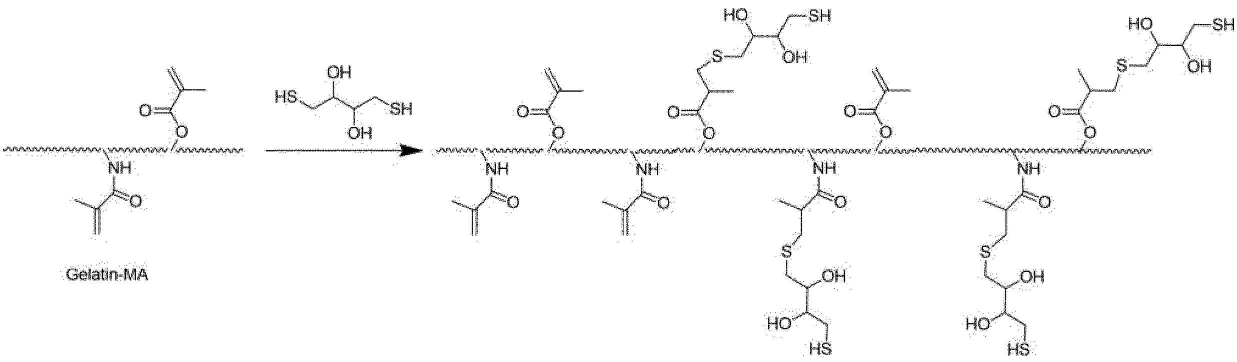

Gelatin-MA

Gelatin-MA-SH1

FIG. 8

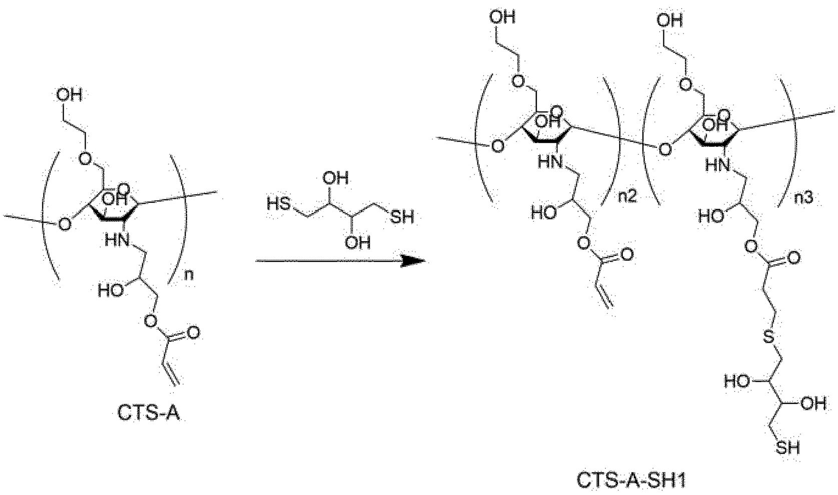

CTS-A

CTS-A-SH1

FIG. 9

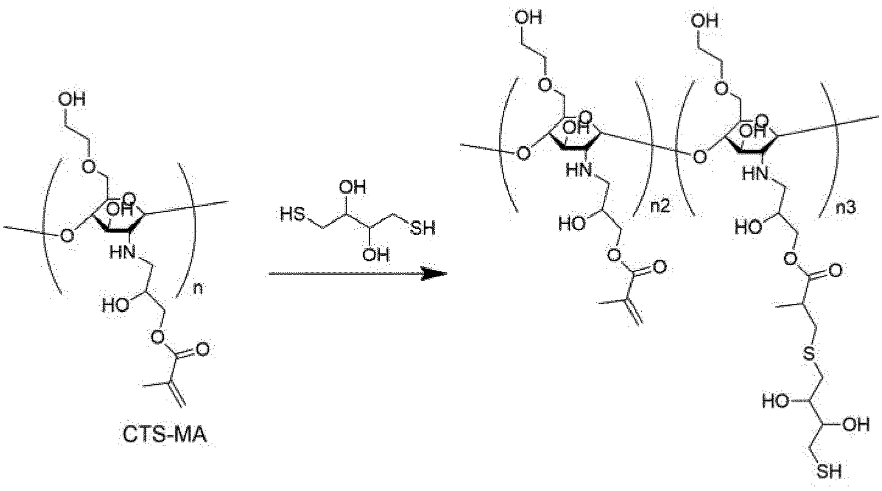

CTS-MA

CTS-MA-SH1

FIG. 10

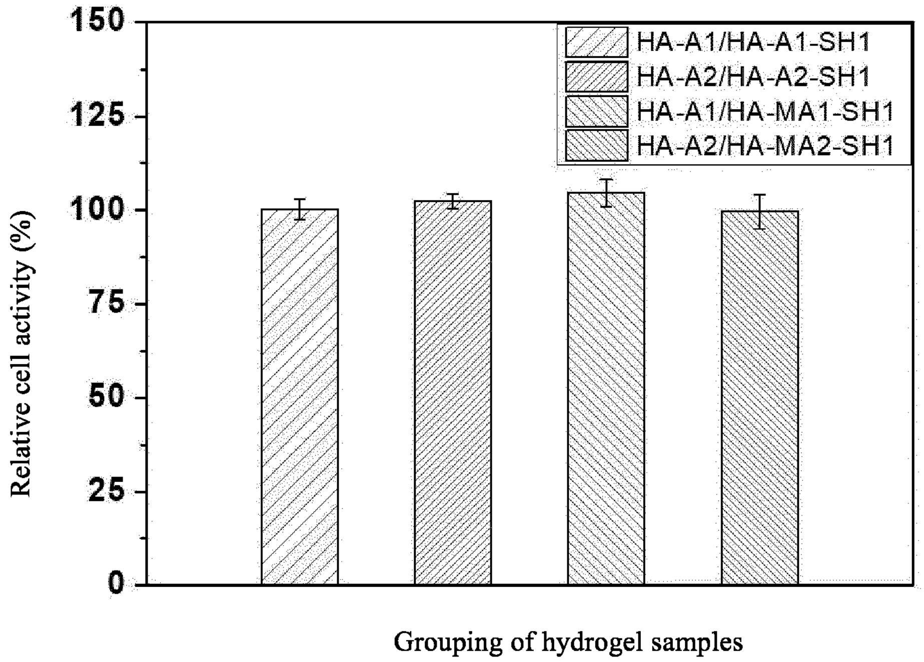
150
125
100
75
50
25
0
Relative cell activity (%)
HA-A1/HA-A1-SH1
HA-A2/HA-A2-SH1
HA-A1/HA-MA1-SH1
HA-A2/HA-MA2-SH1
Grouping of hydrogel samples

FIG. 11

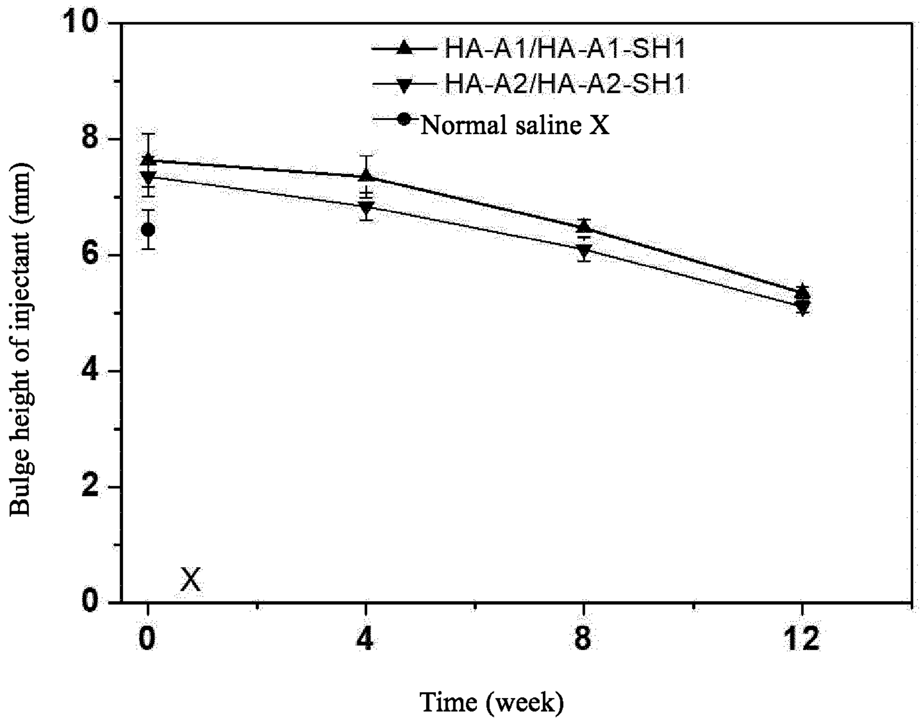
10
8
6
4
2
0
Bulge height of injectant (mm)
HA-A1/HA-A1-SH1
HA-A2/HA-A2-SH1
Normal saline X
X
0
4
8
12
Time (week)

FIG. 12

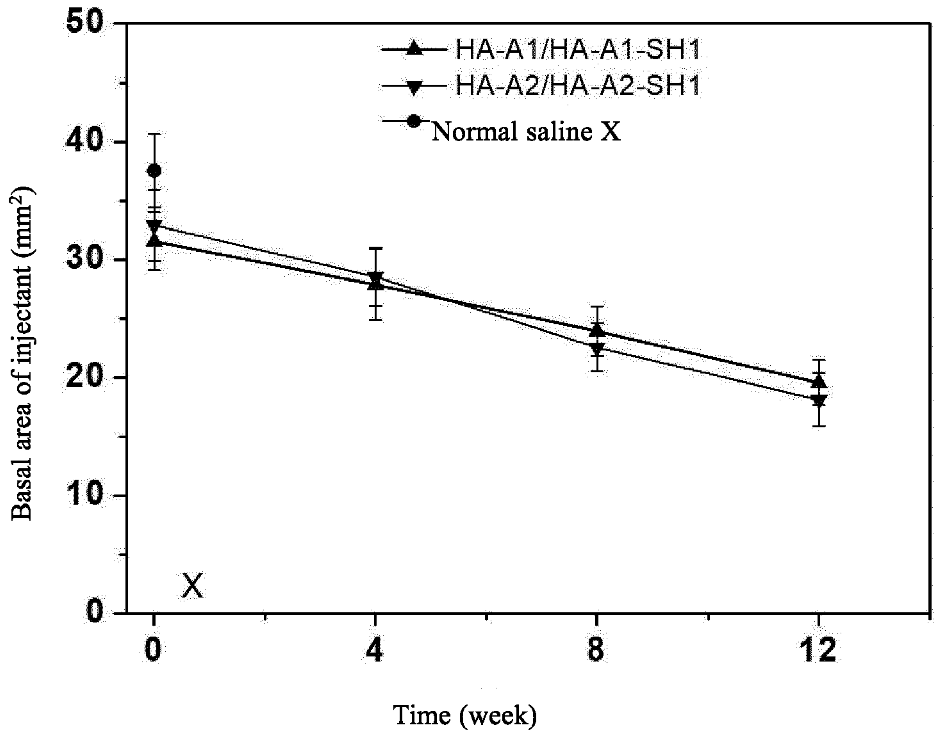
HA-A1/HA-A1-SH1
HA-A2/HA-A2-SH1
Normal saline X
Basal area of injectant (mm²)
Time (week)
50
40
30
20
10
0
X
0
4
8
12

FIG. 13

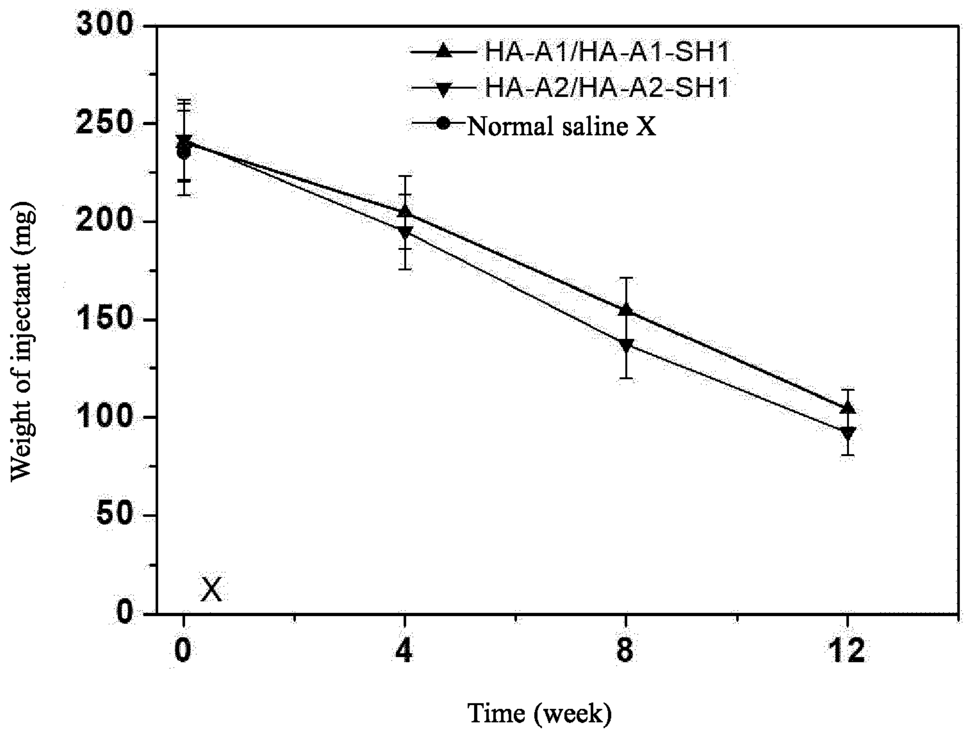
HA-A1/HA-A1-SH1
HA-A2/HA-A2-SH1
Normal saline X
Weight of injectant (mg)
Time (week)
300
250
200
150
100
50
0
X
0
4
8
12

FIG. 14

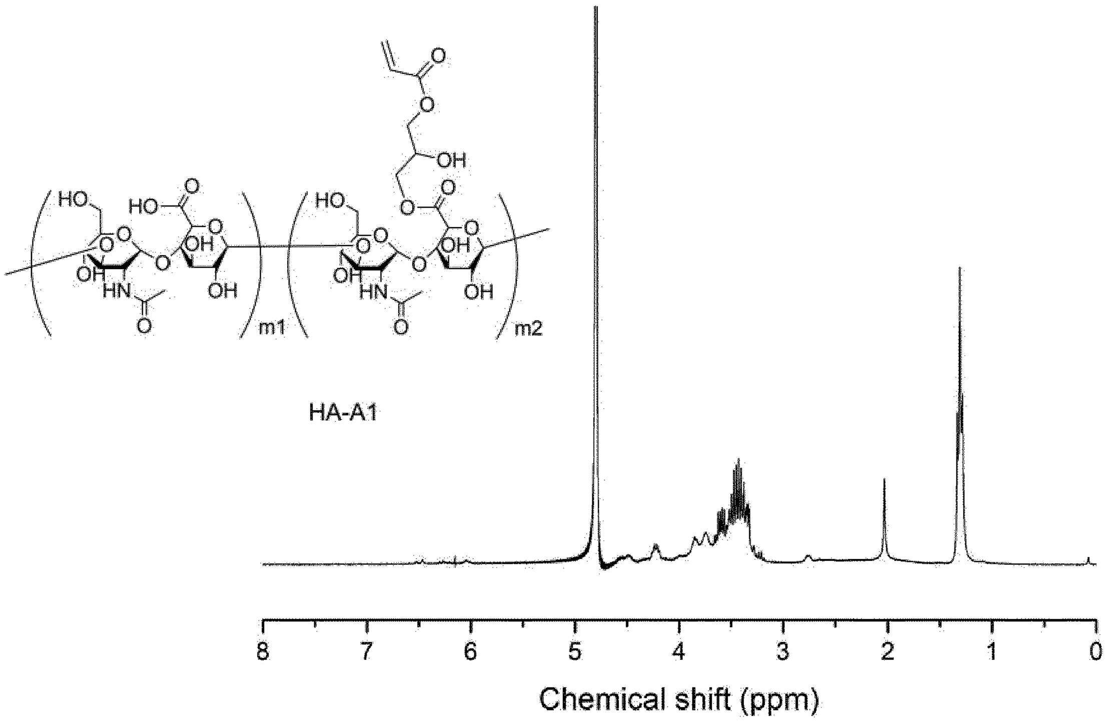
HA-A1
8 7 6 5 4 3 2 1 0
Chemical shift (ppm)

FIG. 15

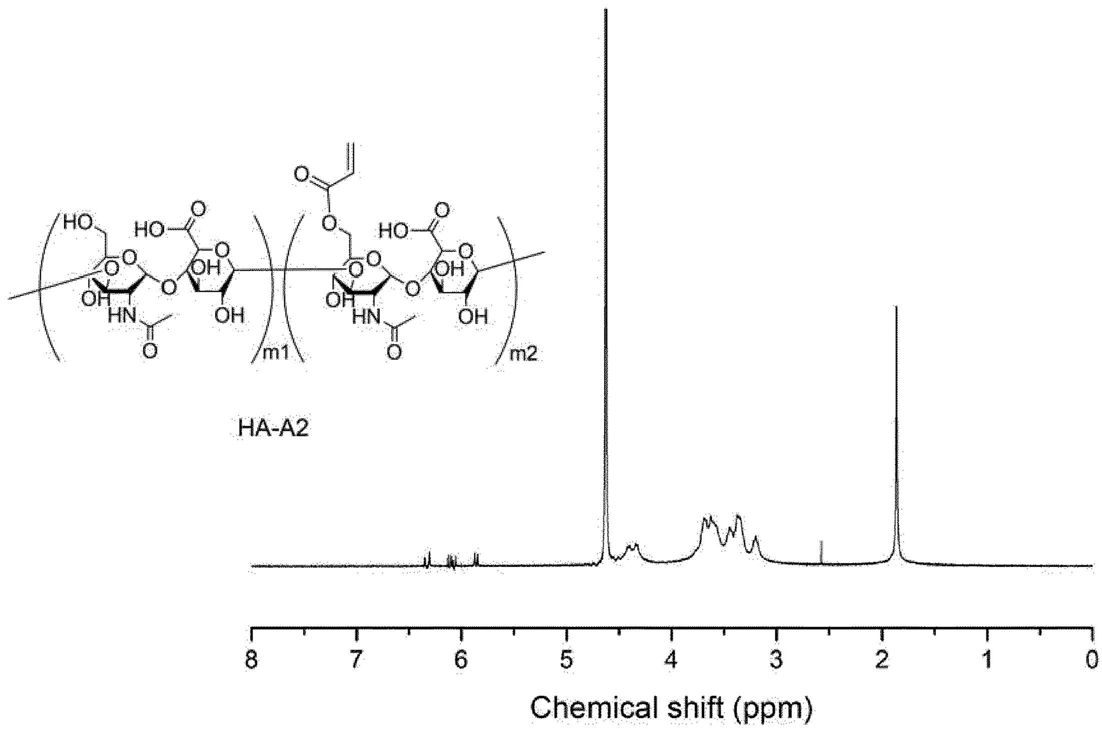
HA-A2
8 7 6 5 4 3 2 1 0
Chemical shift (ppm)

FIG. 16

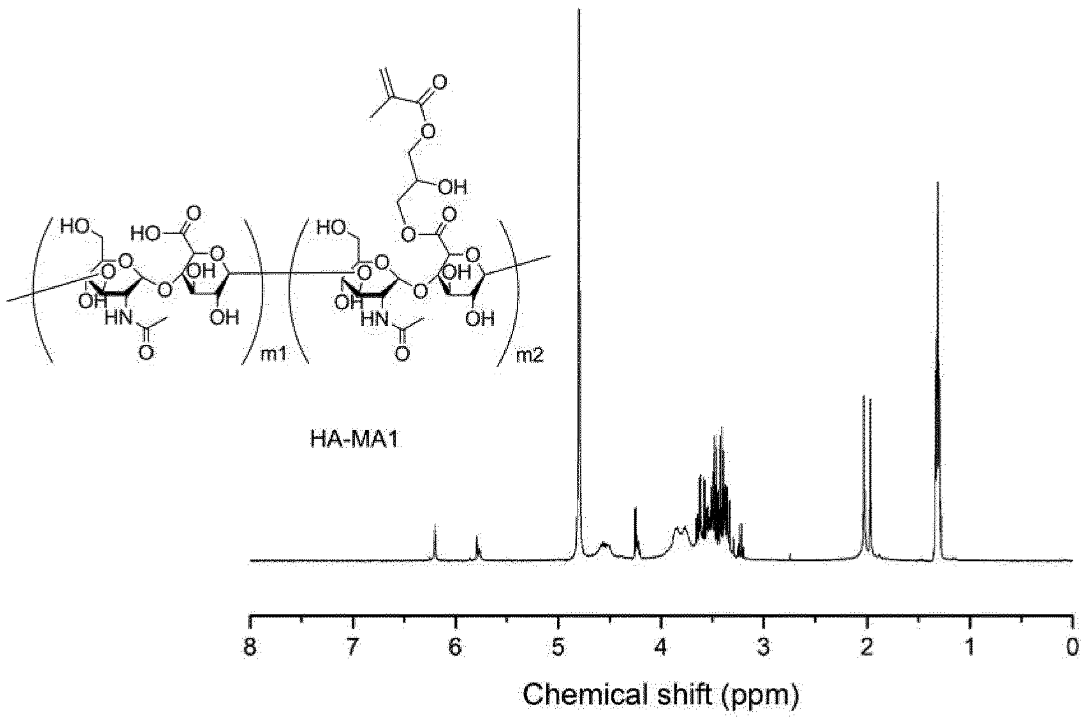
HA-MA1
8 7 6 5 4 3 2 1 0
Chemical shift (ppm)

FIG. 17

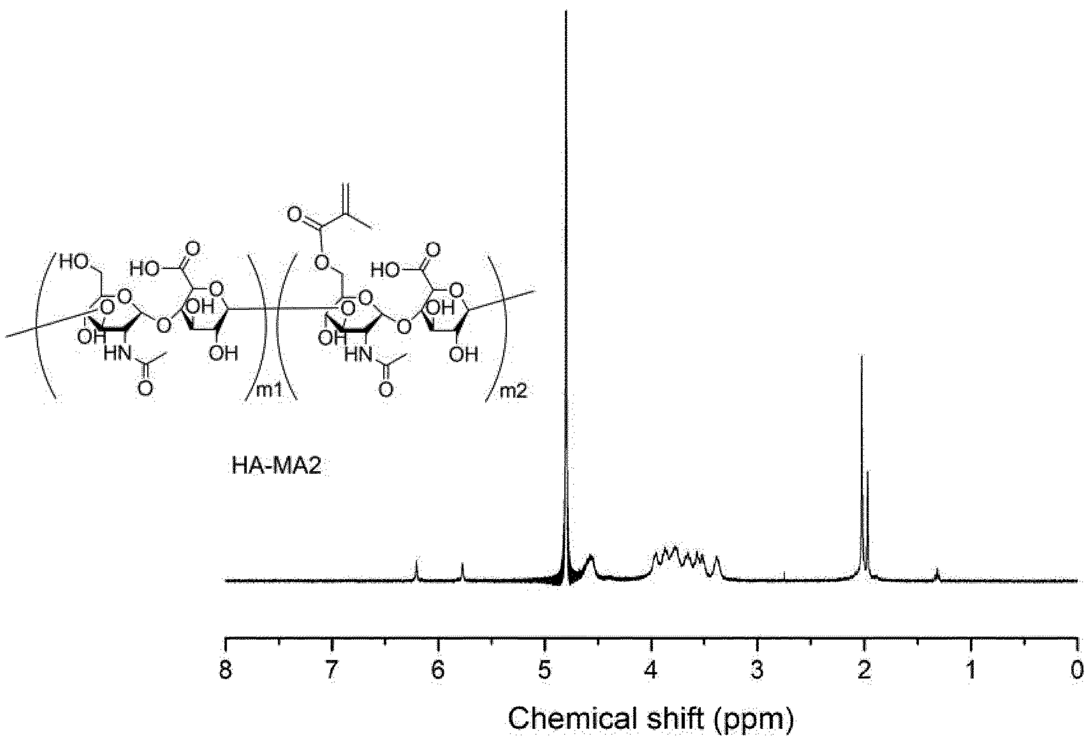
HA-MA2
8 7 6 5 4 3 2 1 0
Chemical shift (ppm)

FIG. 18

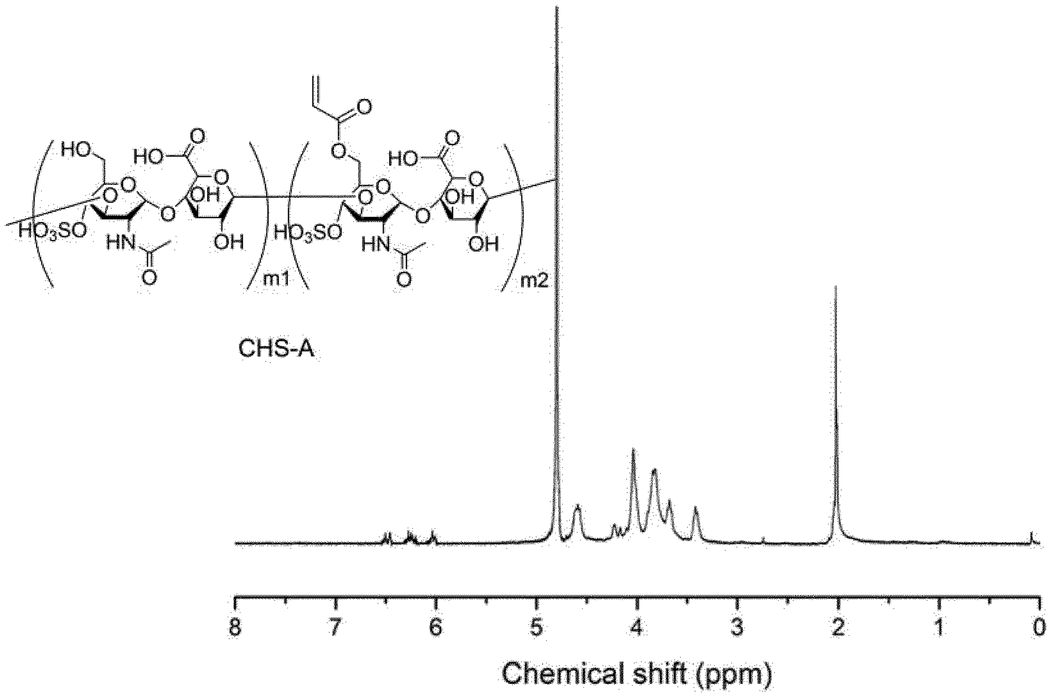
CHS-A
8 7 6 5 4 3 2 1 0
Chemical shift (ppm)

FIG. 19

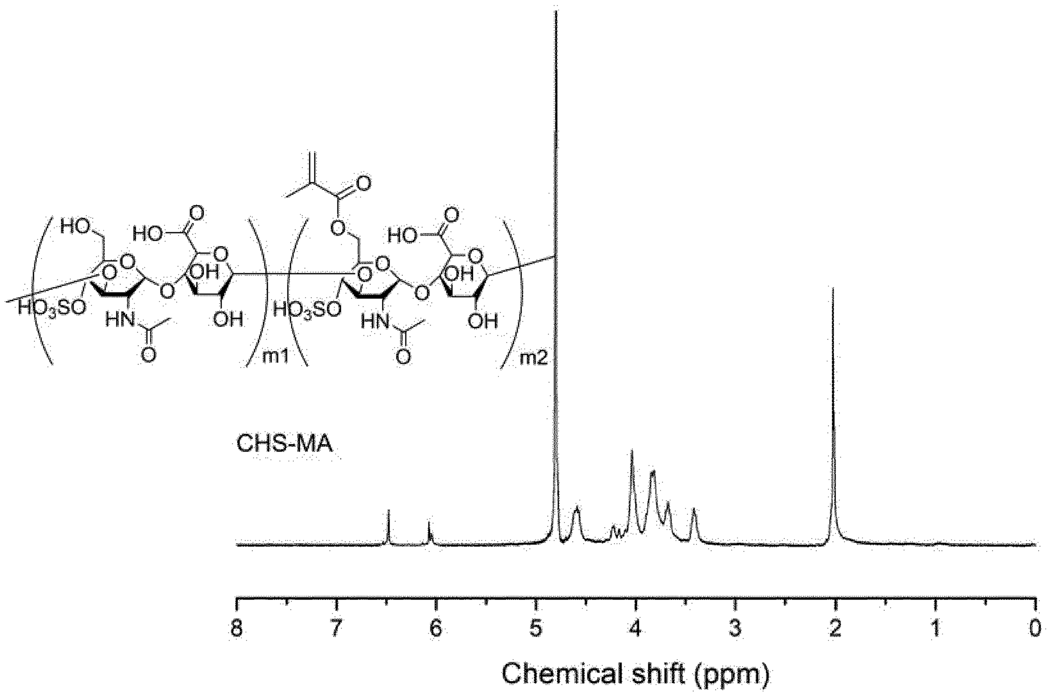
CHS-MA
8 7 6 5 4 3 2 1 0
Chemical shift (ppm)

FIG. 20

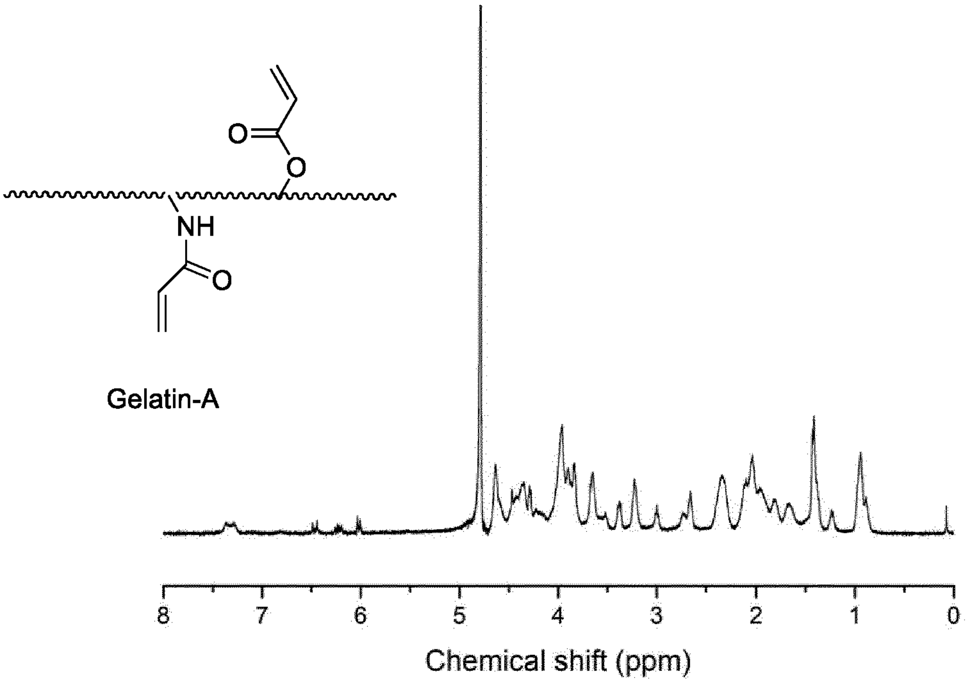
Gelatin-A
8 7 6 5 4 3 2 1 0
Chemical shift (ppm)

FIG. 21

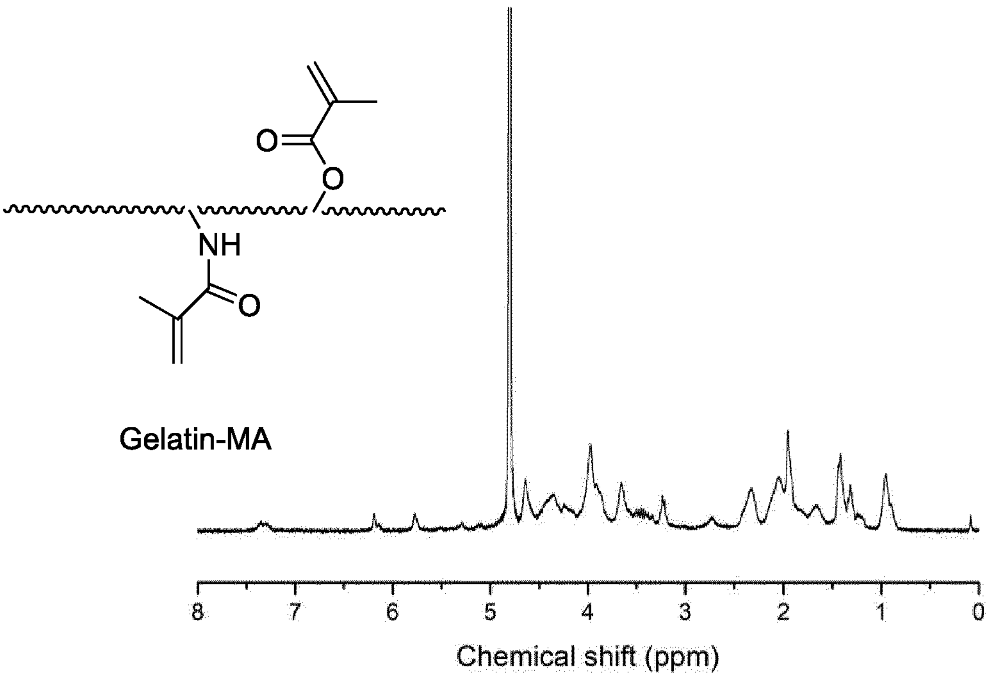
Gelatin-MA
8 7 6 5 4 3 2 1 0
Chemical shift (ppm)

FIG. 22

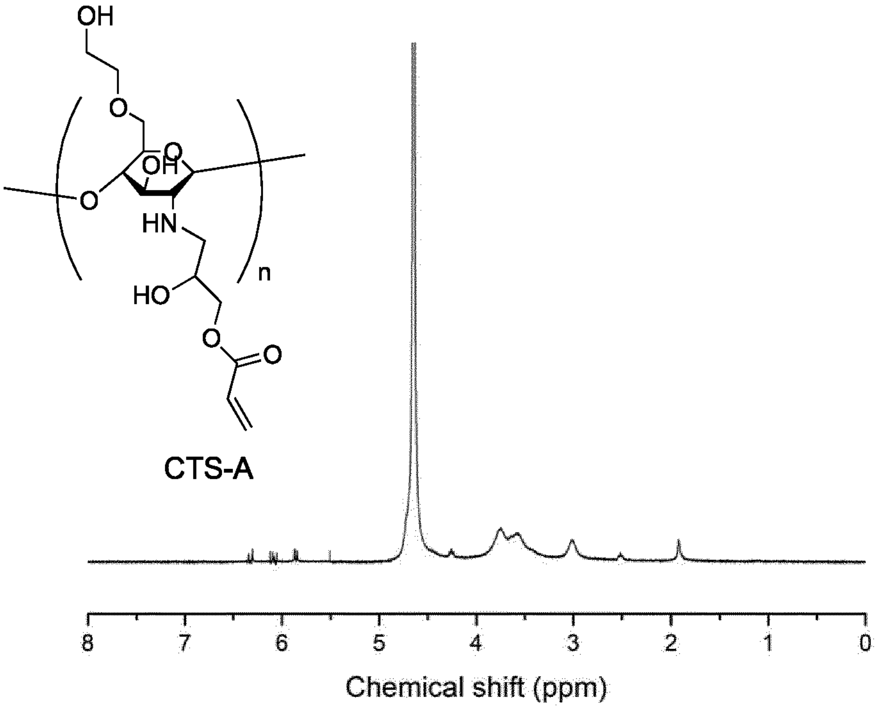
CTS-A
8 7 6 5 4 3 2 1 0
Chemical shift (ppm)

FIG. 23

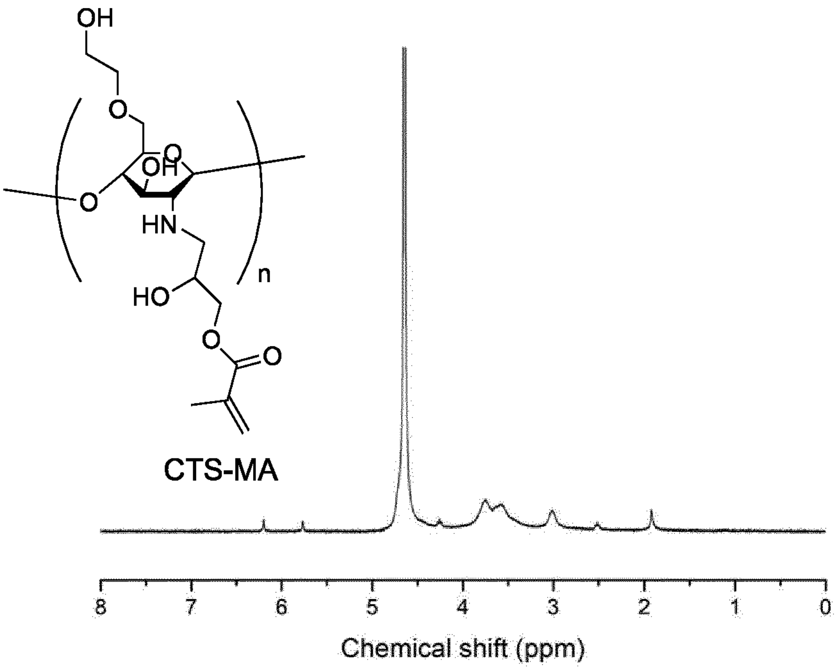
CTS-MA
8 7 6 5 4 3 2 1 0
Chemical shift (ppm)

FIG. 24

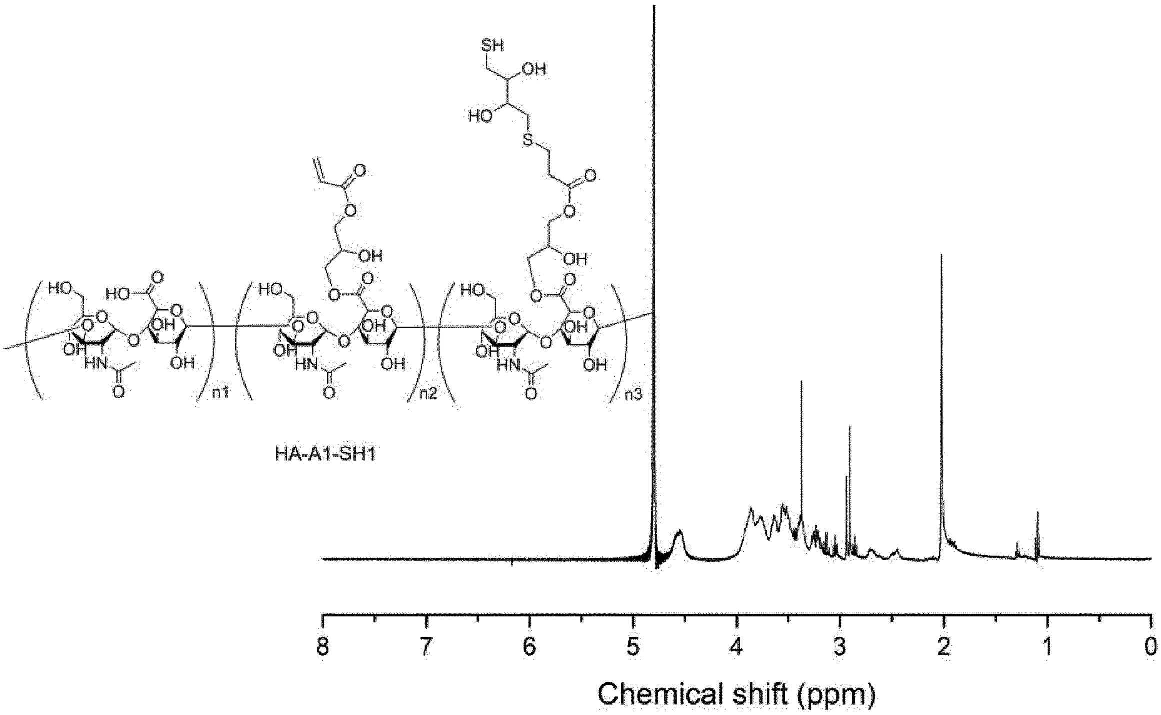


HA-A1-SH1

FIG. 25

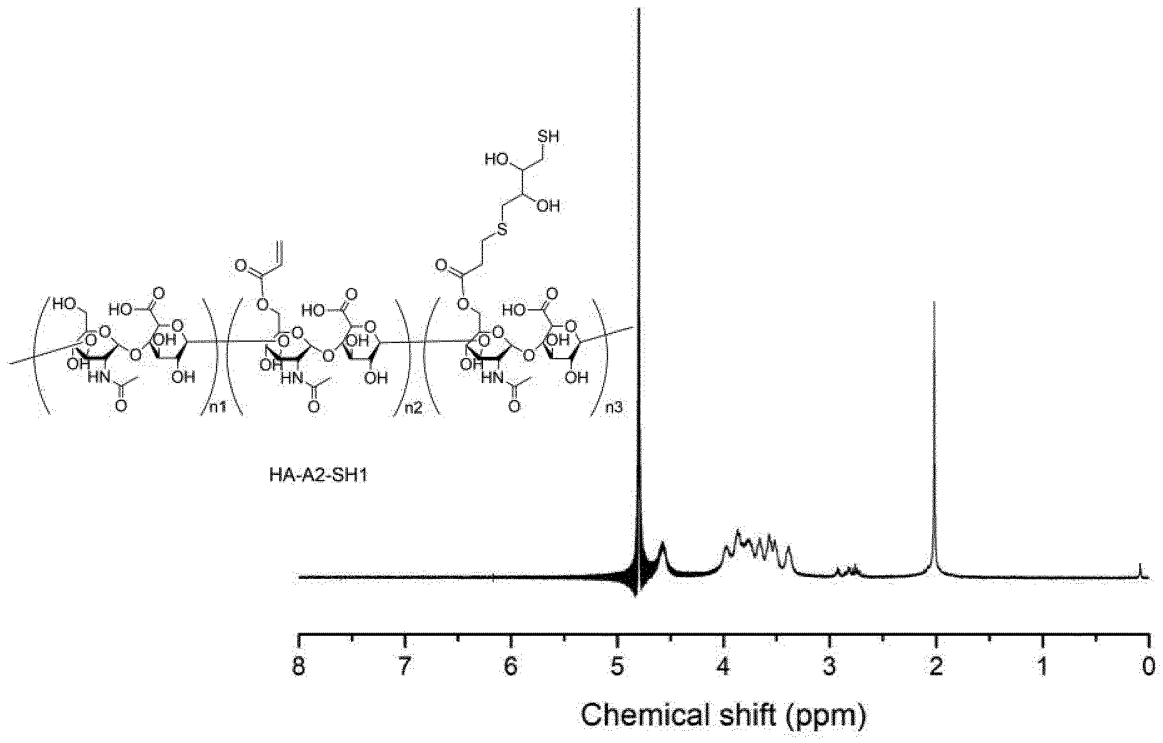


HA-A2-SH1

FIG. 26

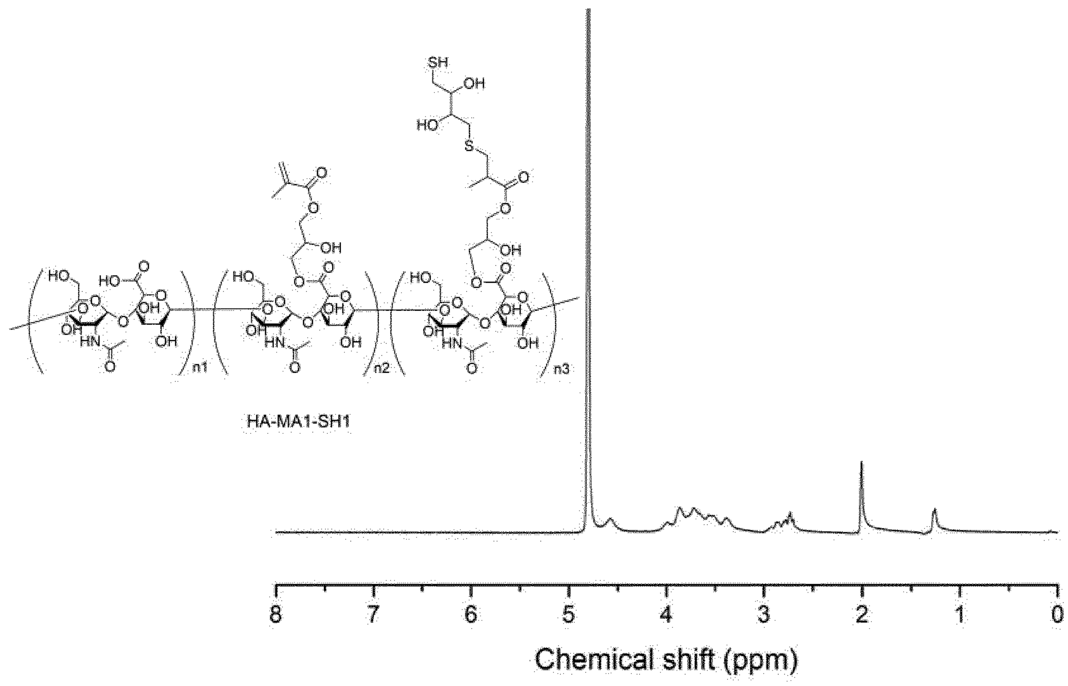
HA-MA1-SH1
8 7 6 5 4 3 2 1 0
Chemical shift (ppm)

FIG. 27

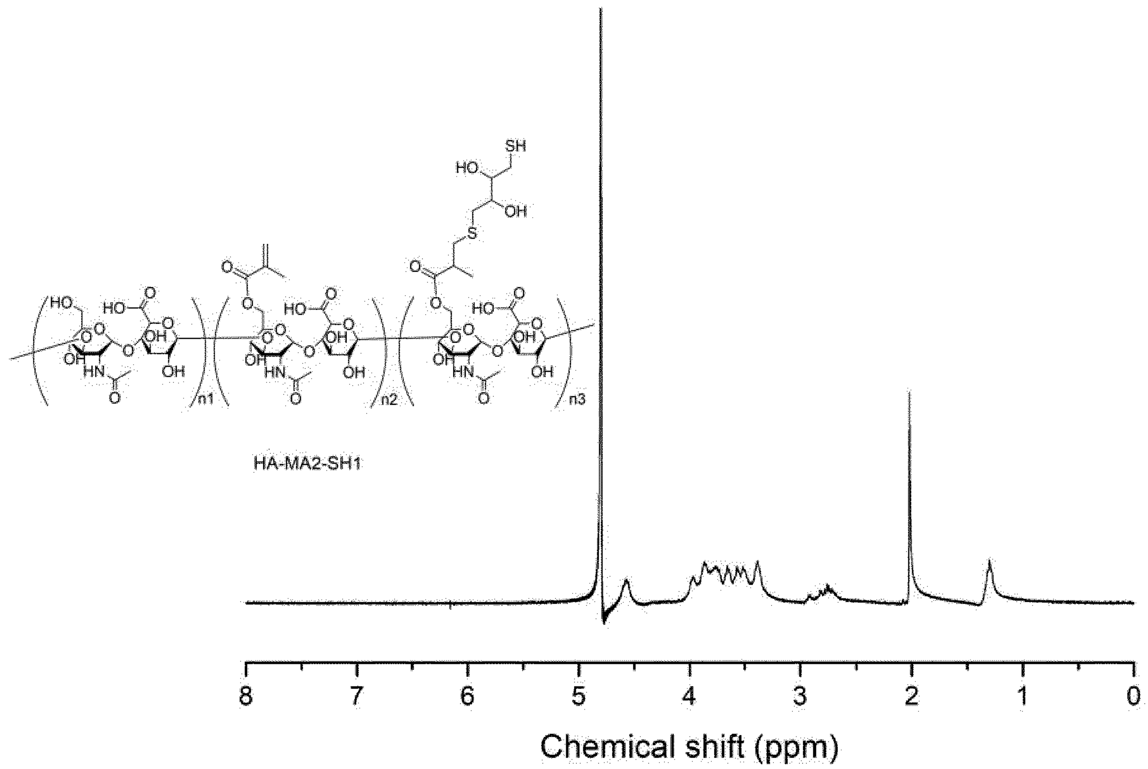
HA-MA2-SH1
8 7 6 5 4 3 2 1 0
Chemical shift (ppm)

FIG. 28

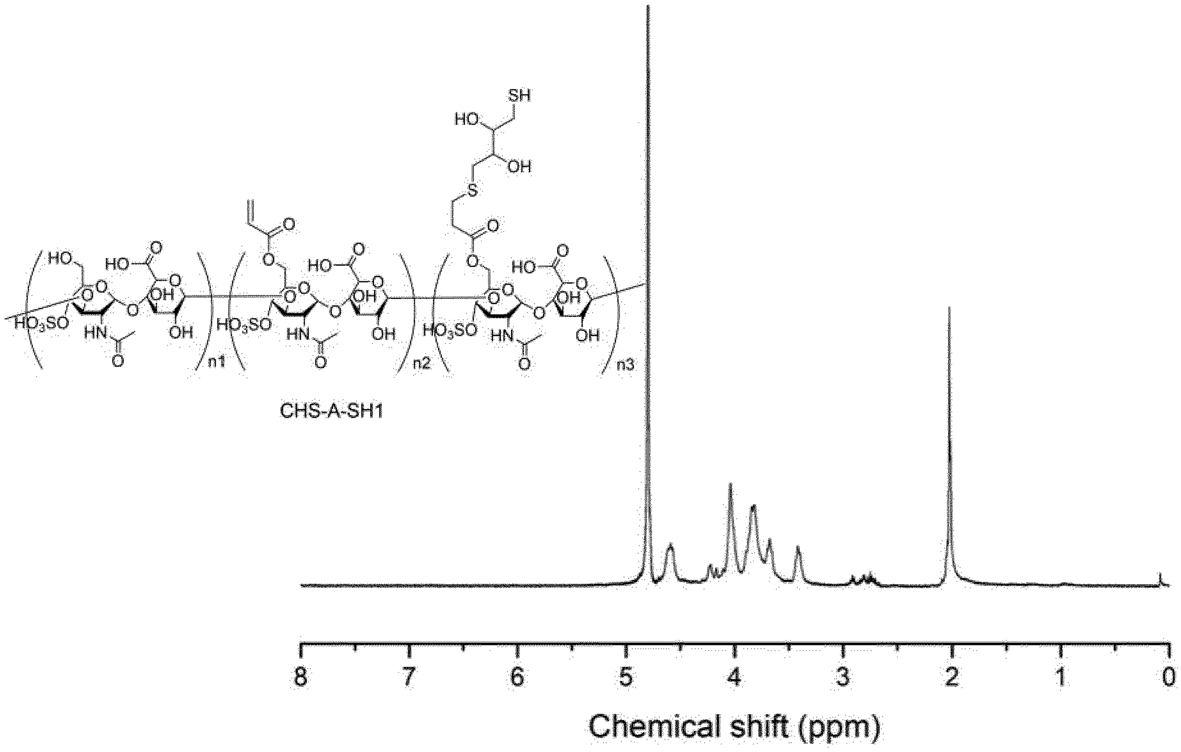


CHS-A-SH1

FIG. 29

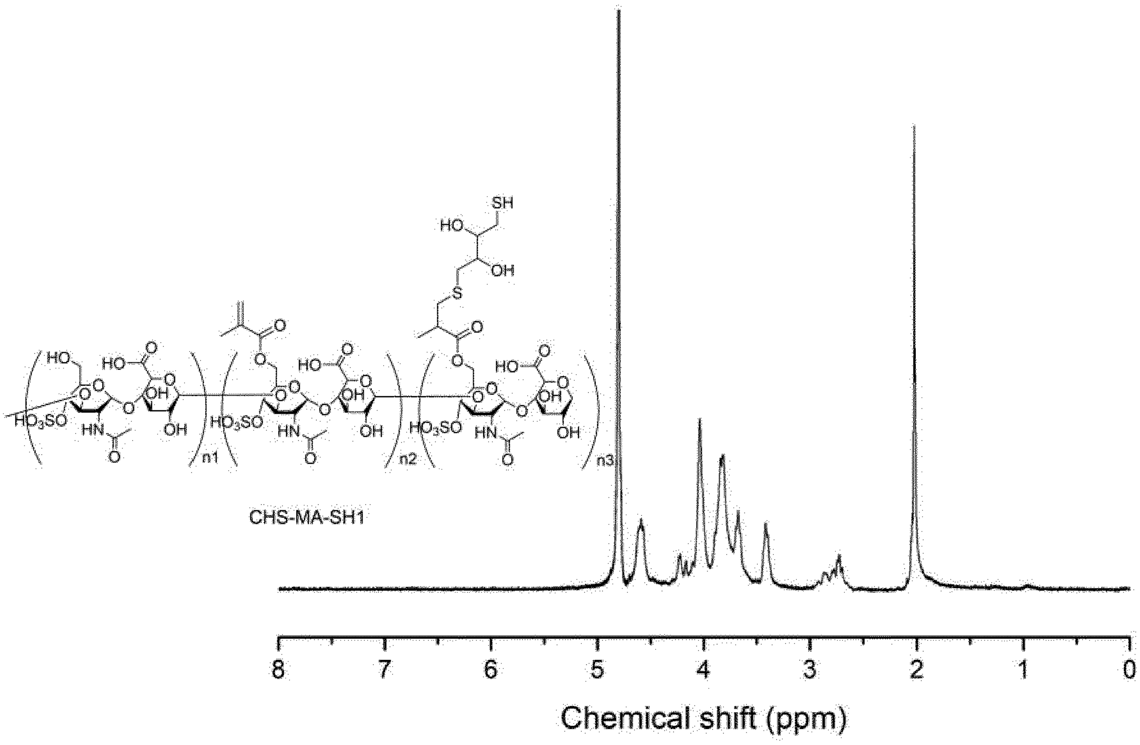


CHS-MA-SH1

FIG. 30

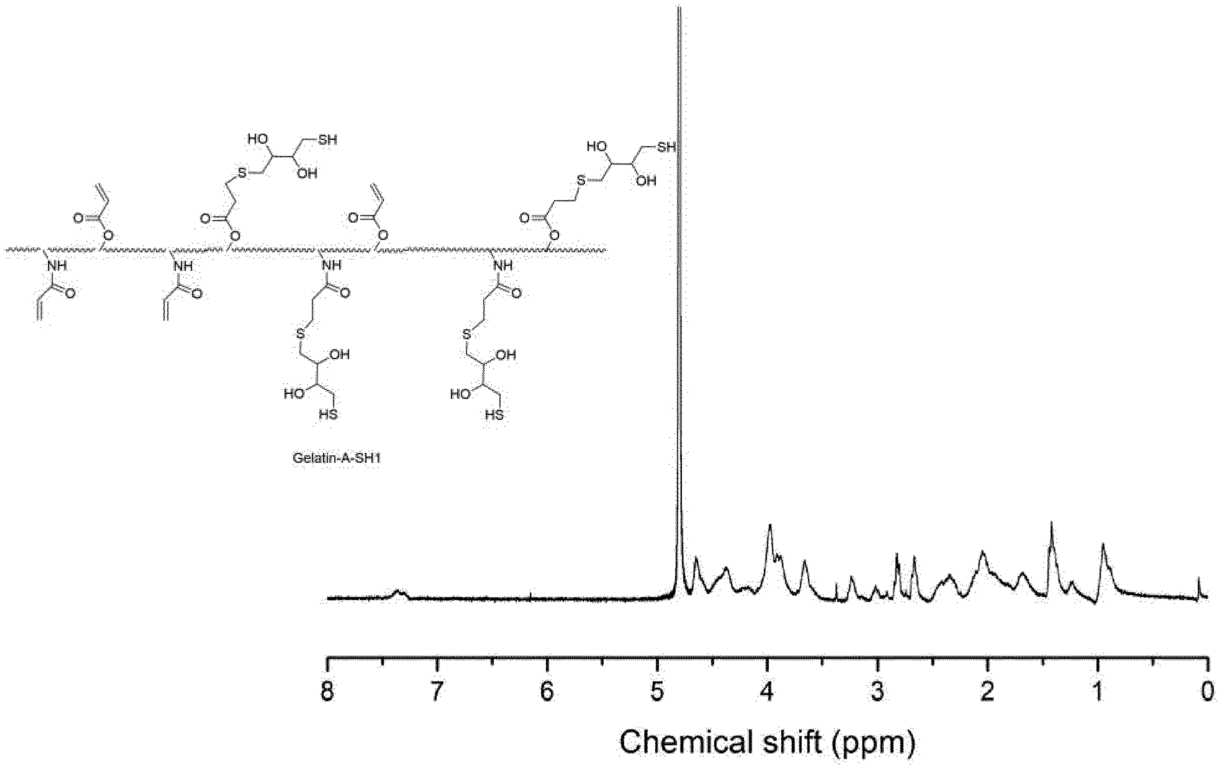


Gelatin-A-SH1

FIG. 31

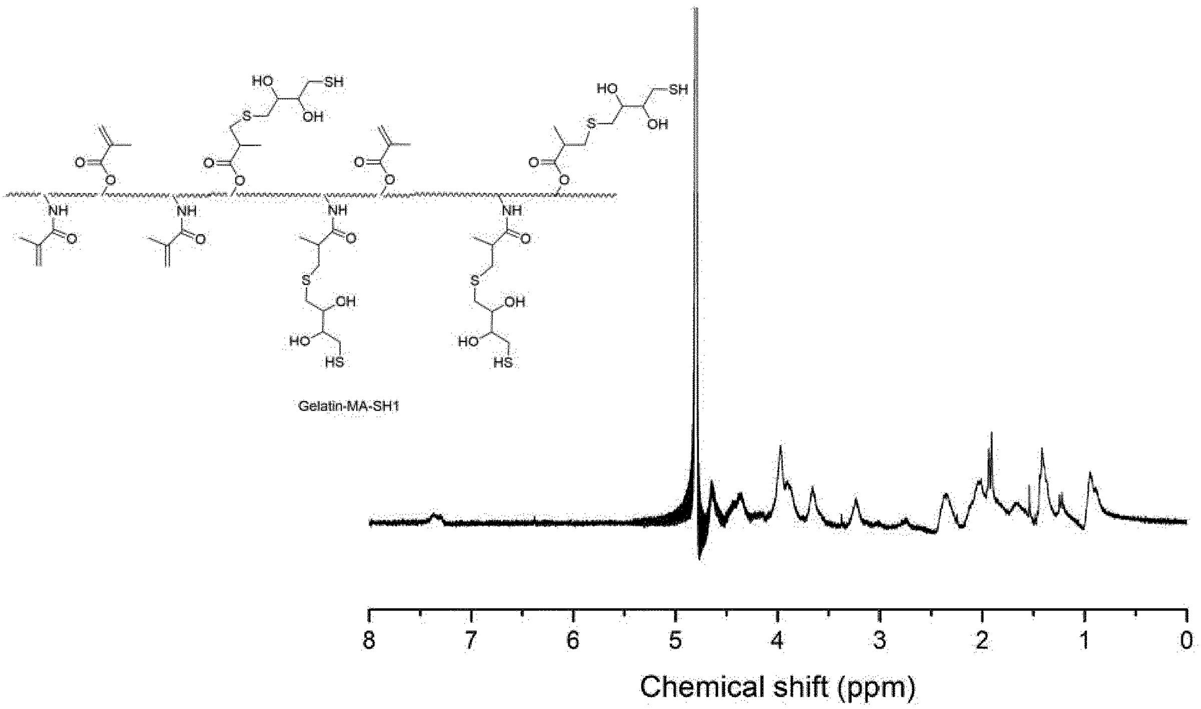


Gelatin-MA-SH1

FIG. 32

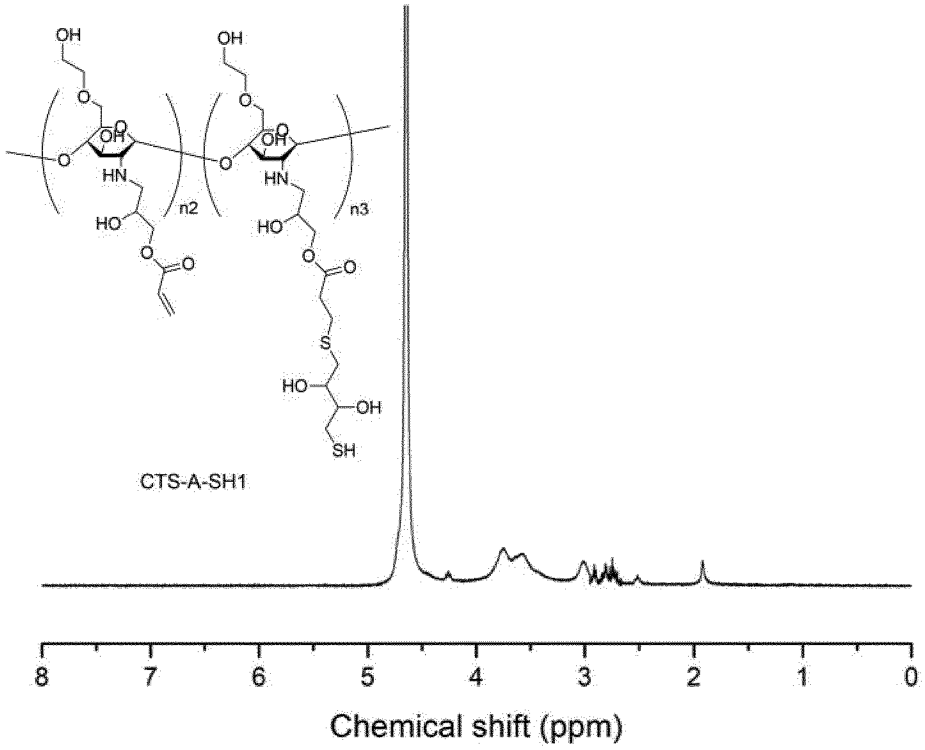
CTS-A-SH1
8 7 6 5 4 3 2 1 0
Chemical shift (ppm)

FIG. 33

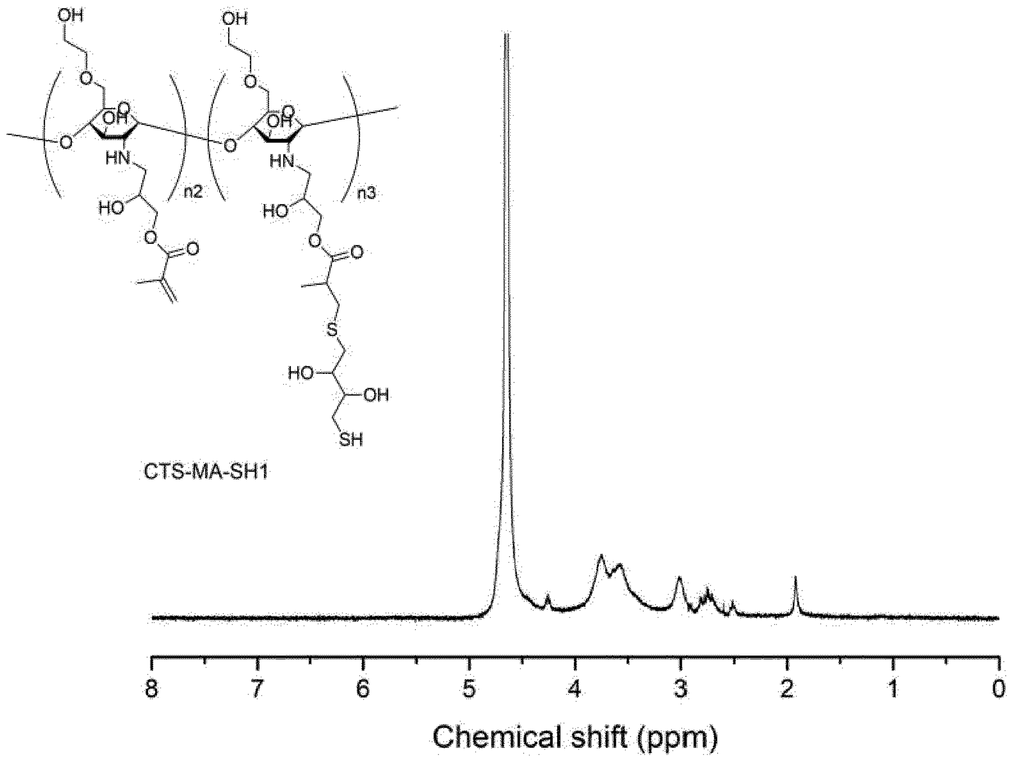
CTS-MA-SH1
8 7 6 5 4 3 2 1 0
Chemical shift (ppm)

FIG. 34

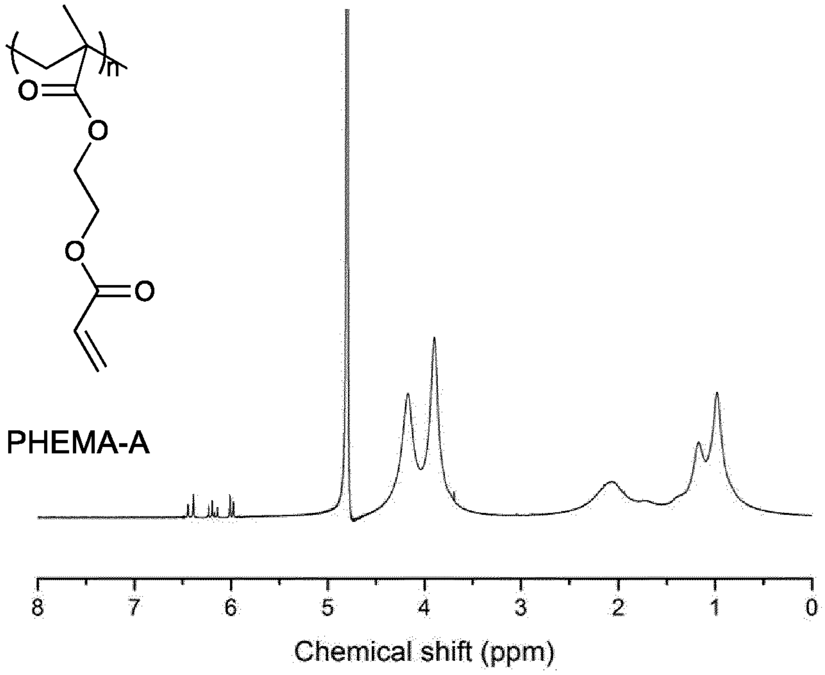
PHEMA-A
8 7 6 5 4 3 2 1 0
Chemical shift (ppm)

FIG. 35

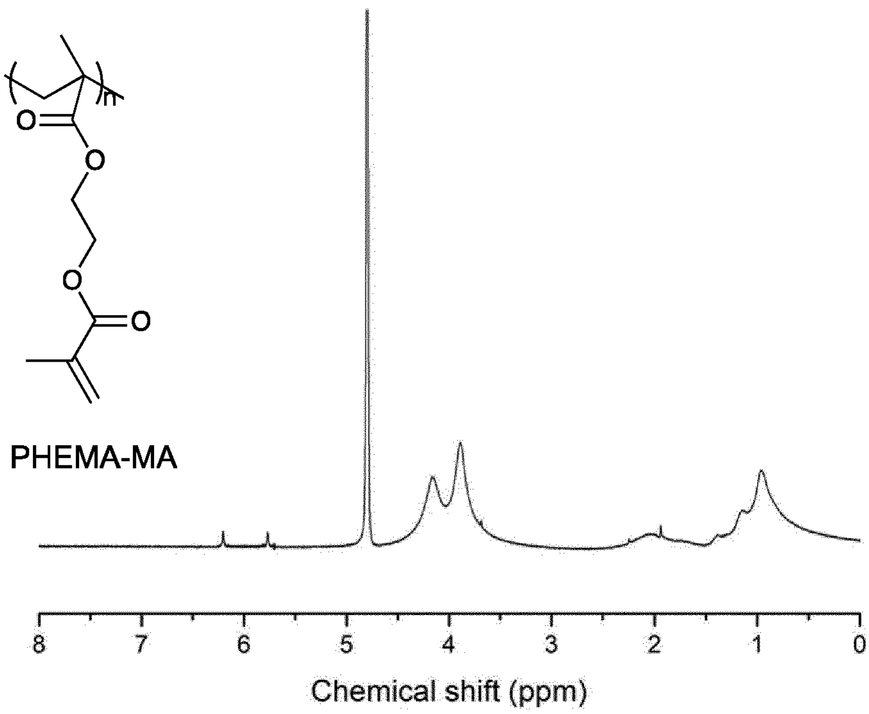
PHEMA-MA
8 7 6 5 4 3 2 1 0
Chemical shift (ppm)

FIG. 36

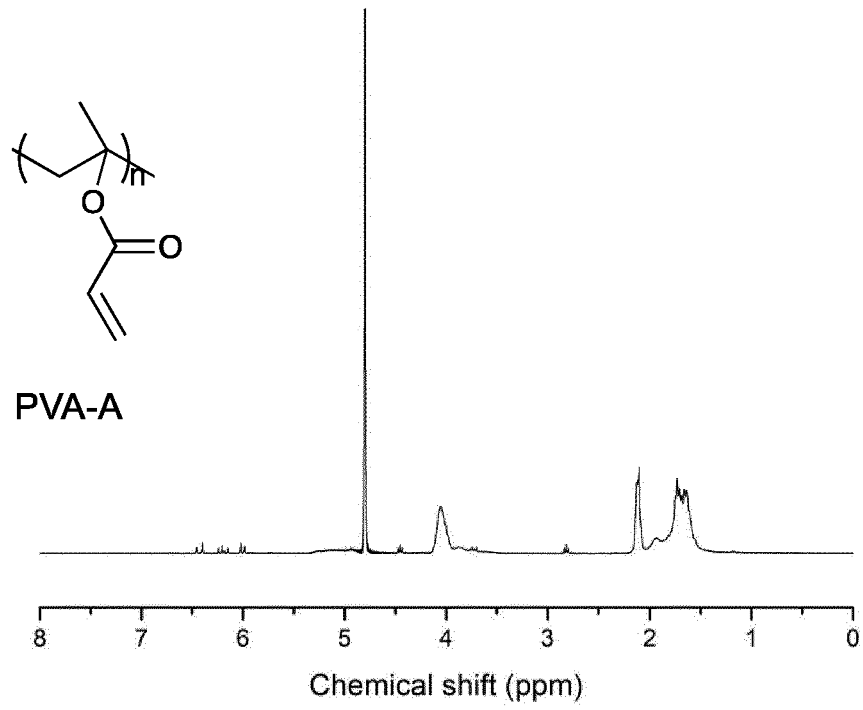
PVA-A
8
7
6
5
4
3
2
1
0
Chemical shift (ppm)

FIG. 37

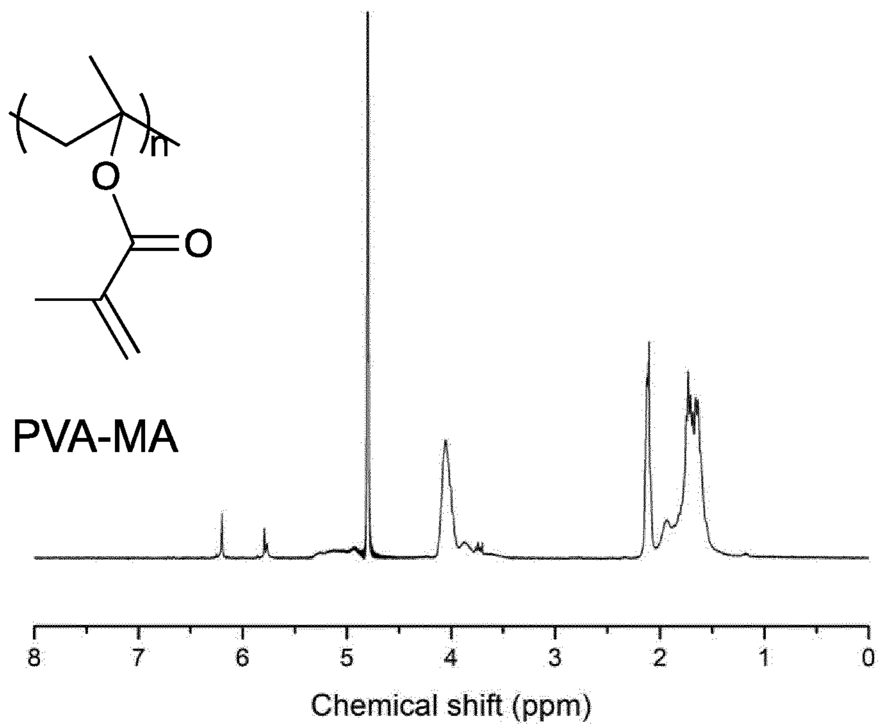
PVA-MA
8
7
6
5
4
3
2
1
0
Chemical shift (ppm)

FIG. 38

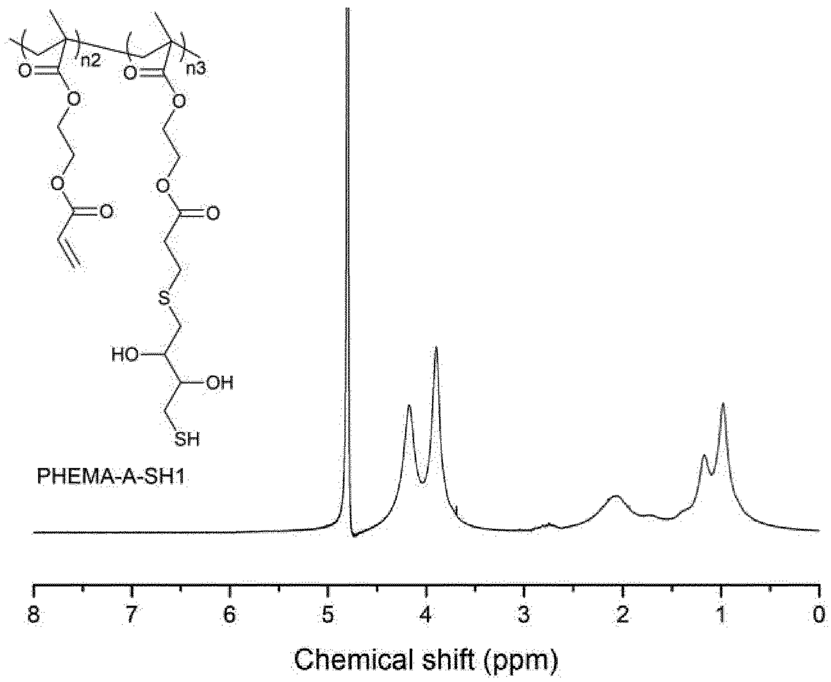
PHEMA-A-SH1
8
7
6
5
4
3
2
1
0
Chemical shift (ppm)

FIG. 39

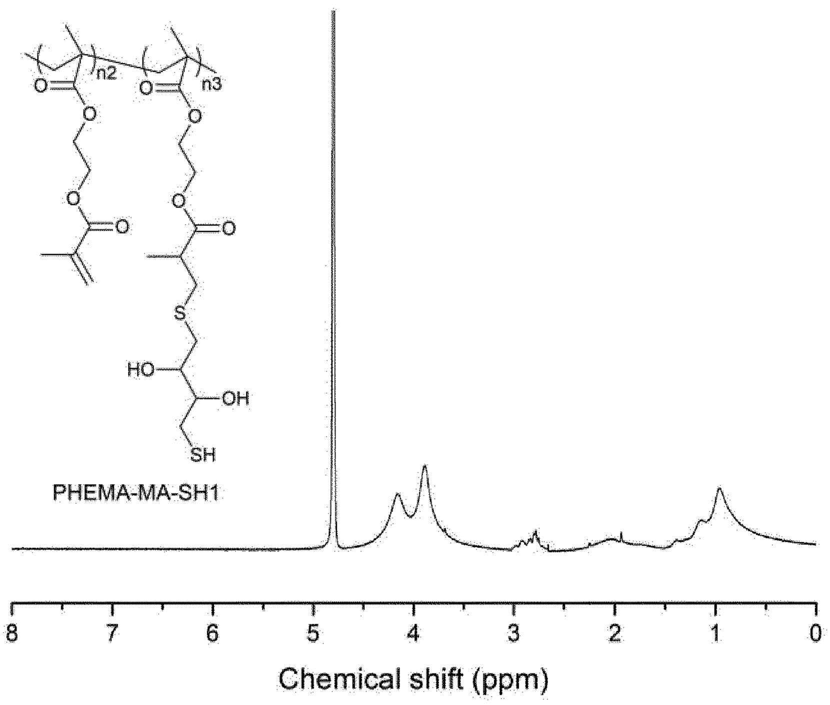
PHEMA-MA-SH1
8
7
6
5
4
3
2
1
0
Chemical shift (ppm)

FIG. 40

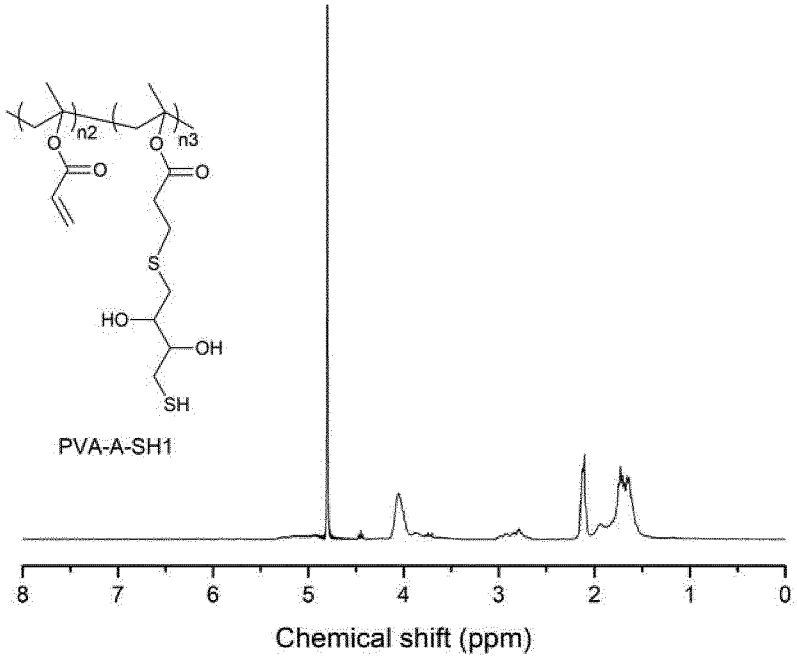
PVA-A-SH1
8
7
6
5
4
3
2
1
0
Chemical shift (ppm)

FIG. 41

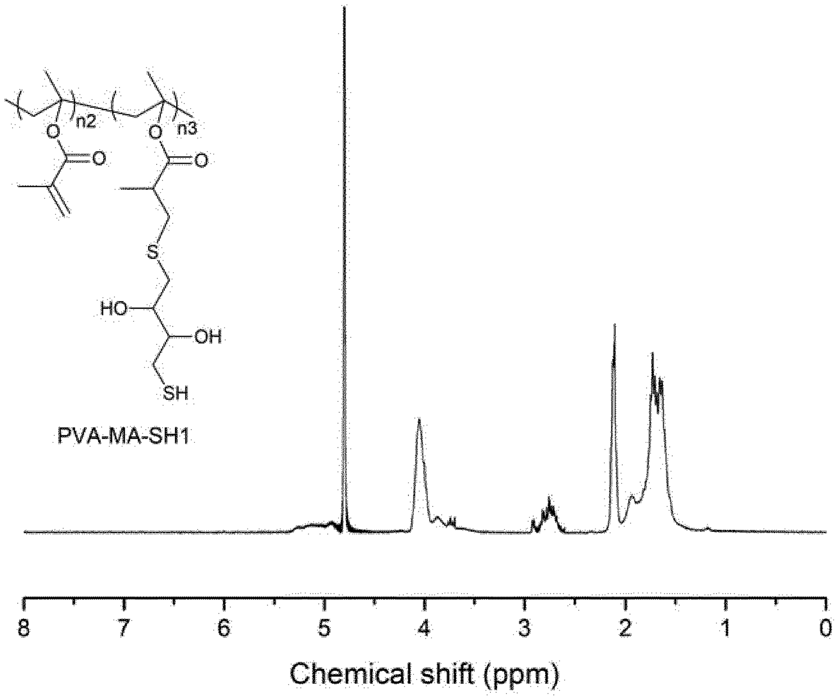
PVA-MA-SH1
8
7
6
5
4
3
2
1
0
Chemical shift (ppm)

FIG. 42

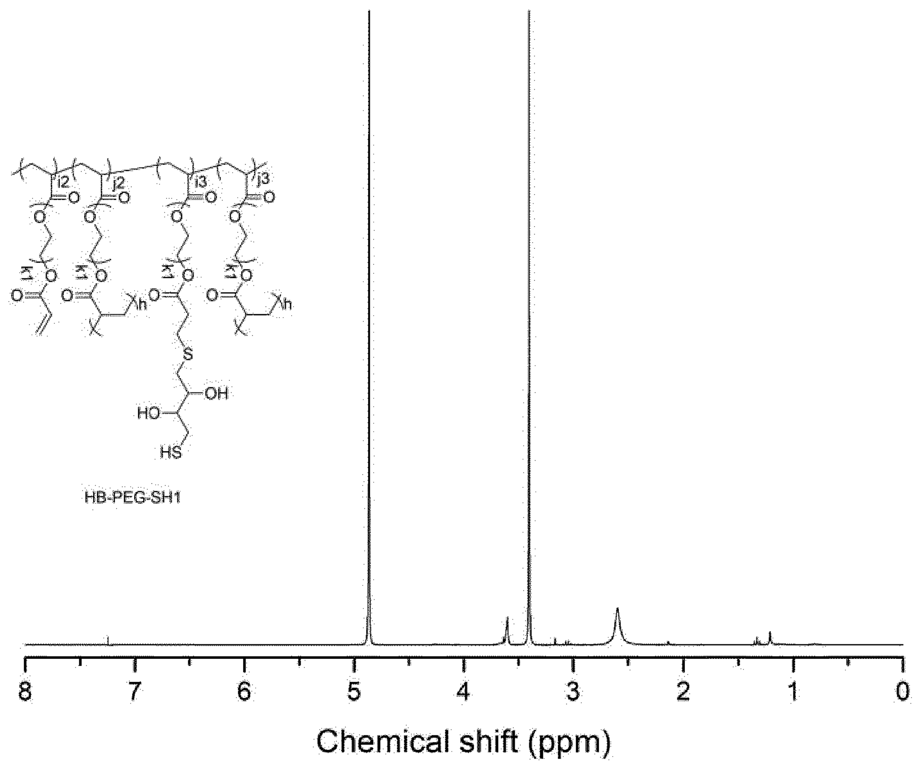

HB-PEG-SH1

FIG. 43

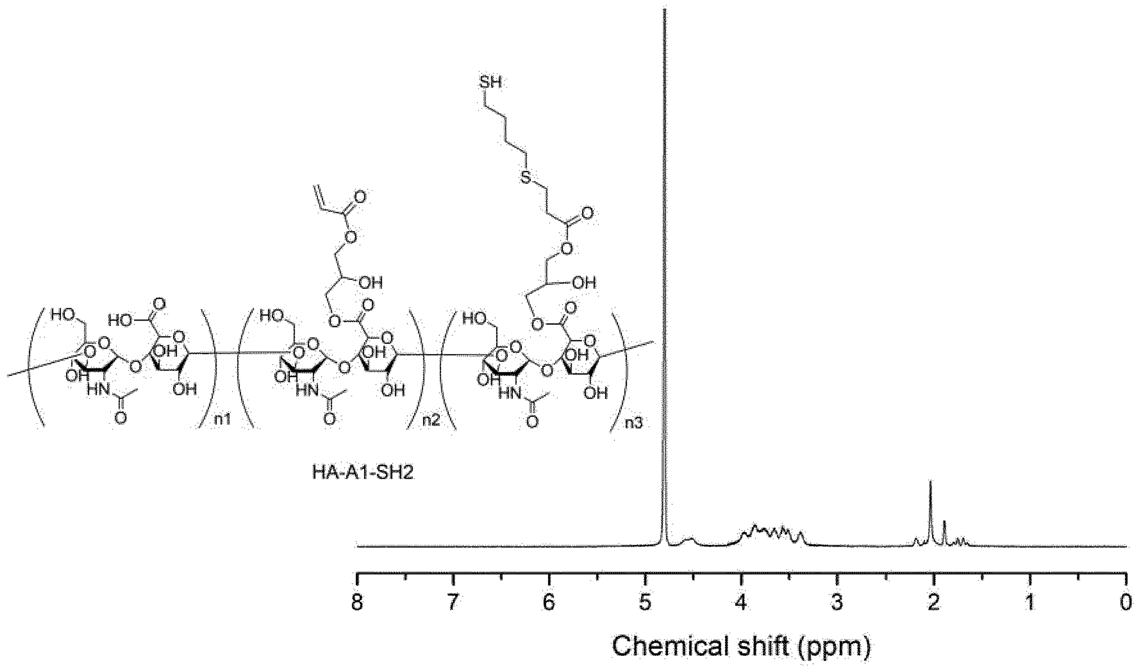

HA-A1-SH2

FIG. 44

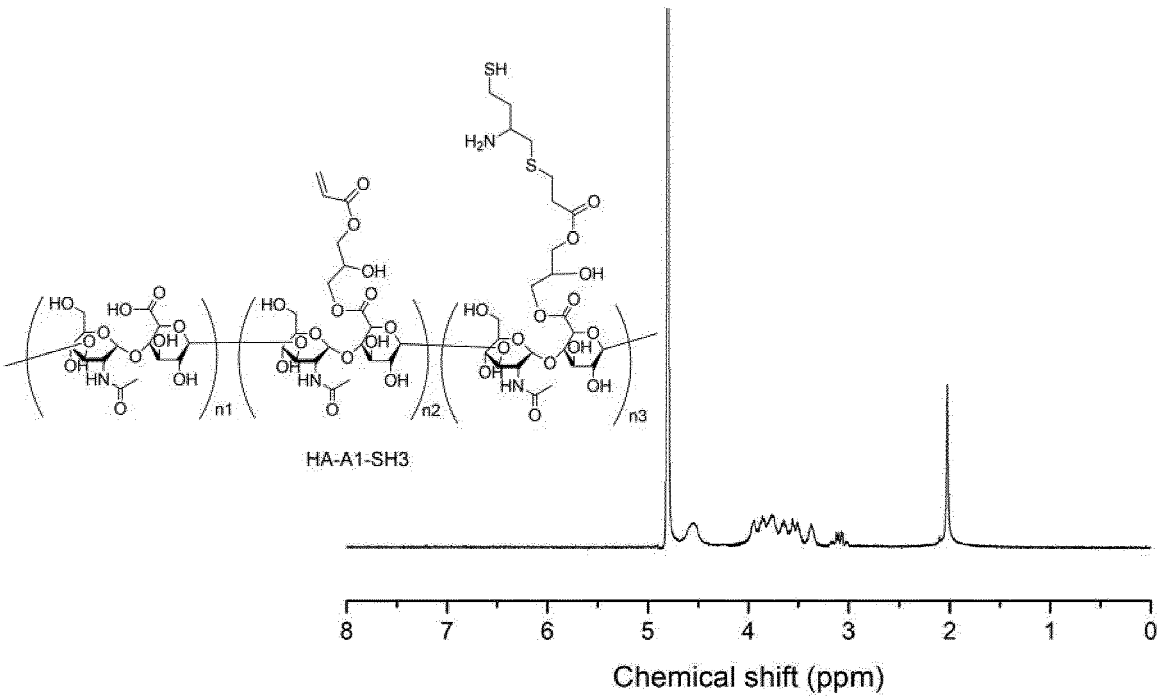


HA-A1-SH3

FIG. 45

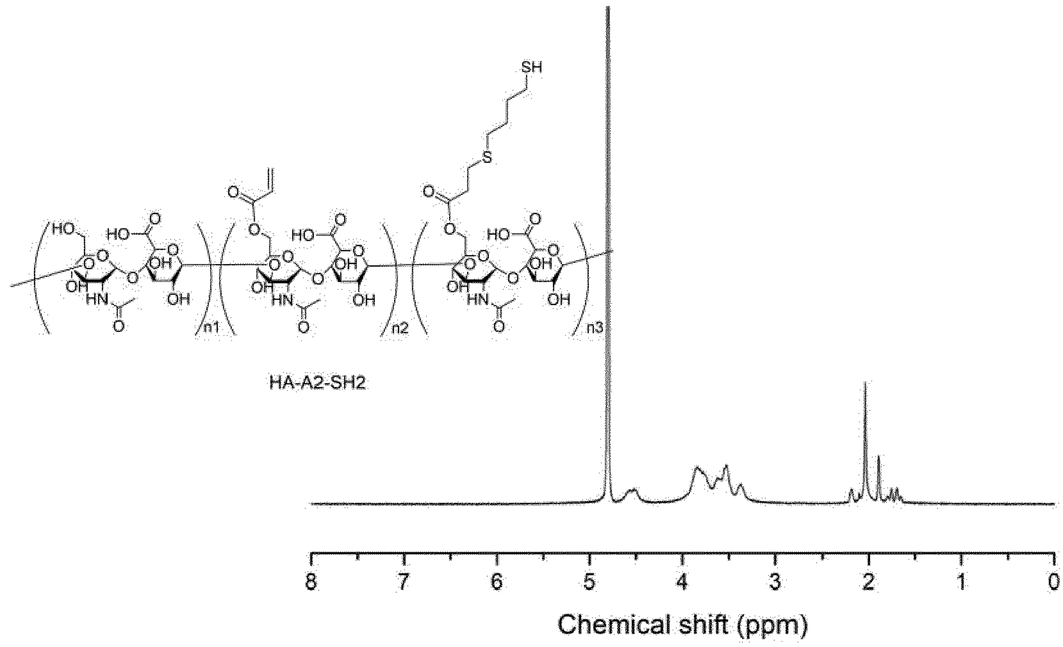


HA-A2-SH2

FIG. 46

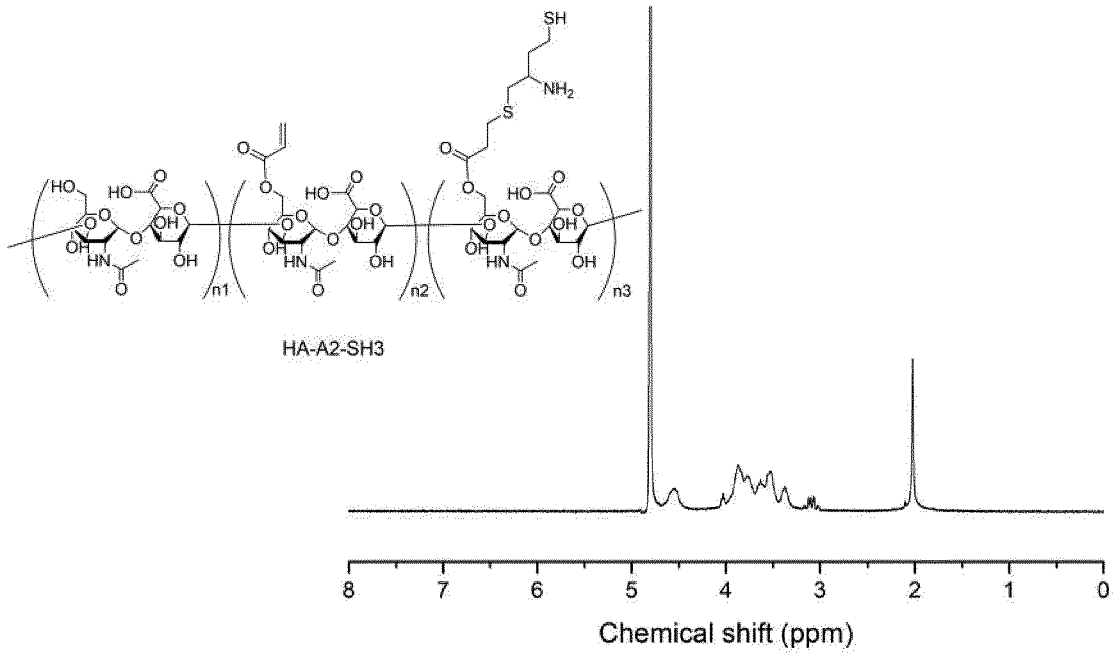


HA-A2-SH3

FIG. 47

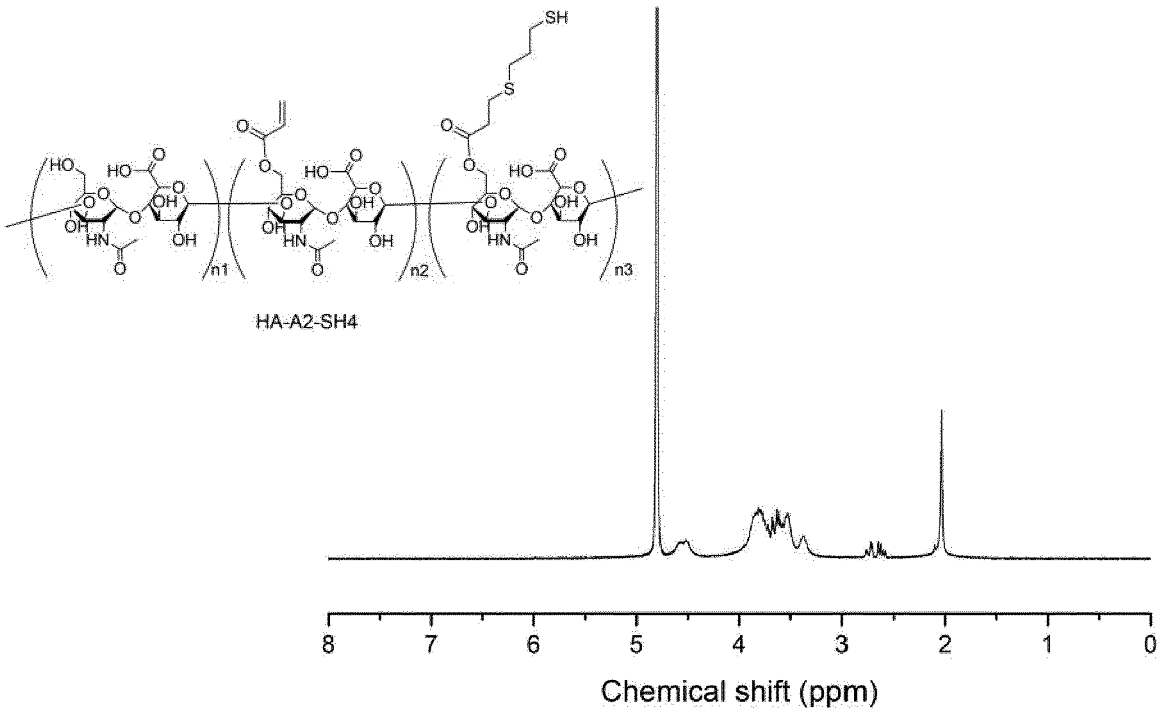


HA-A2-SH4

FIG. 48

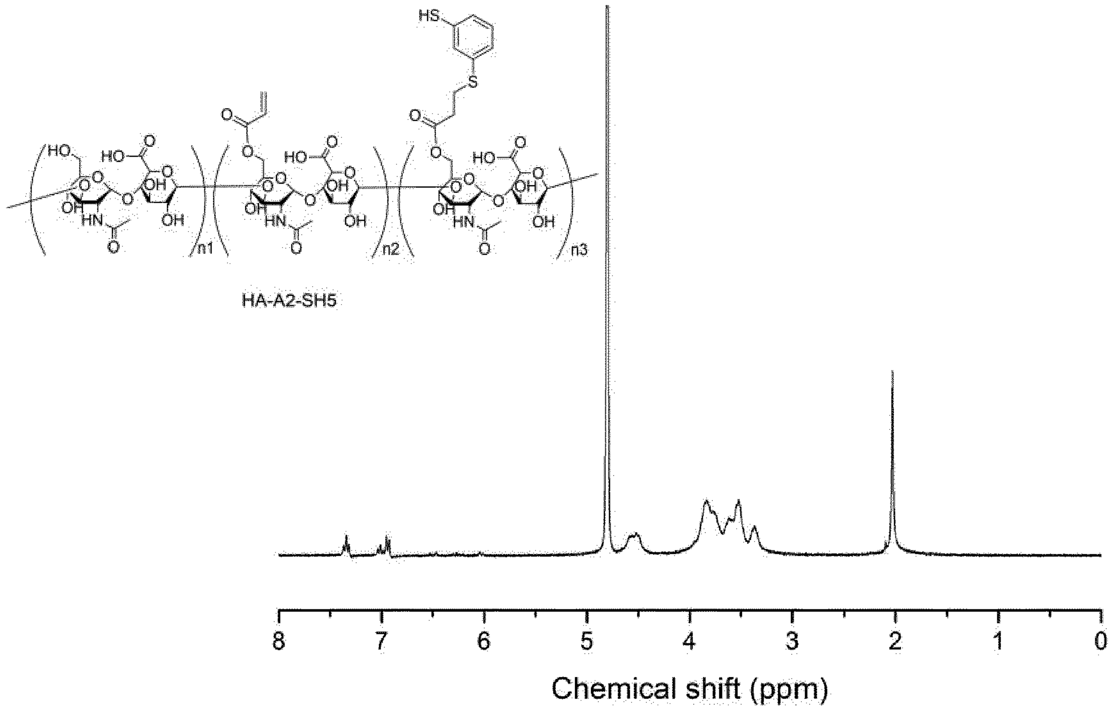
HA-A2-SH5
8 7 6 5 4 3 2 1 0
Chemical shift (ppm)

FIG. 49

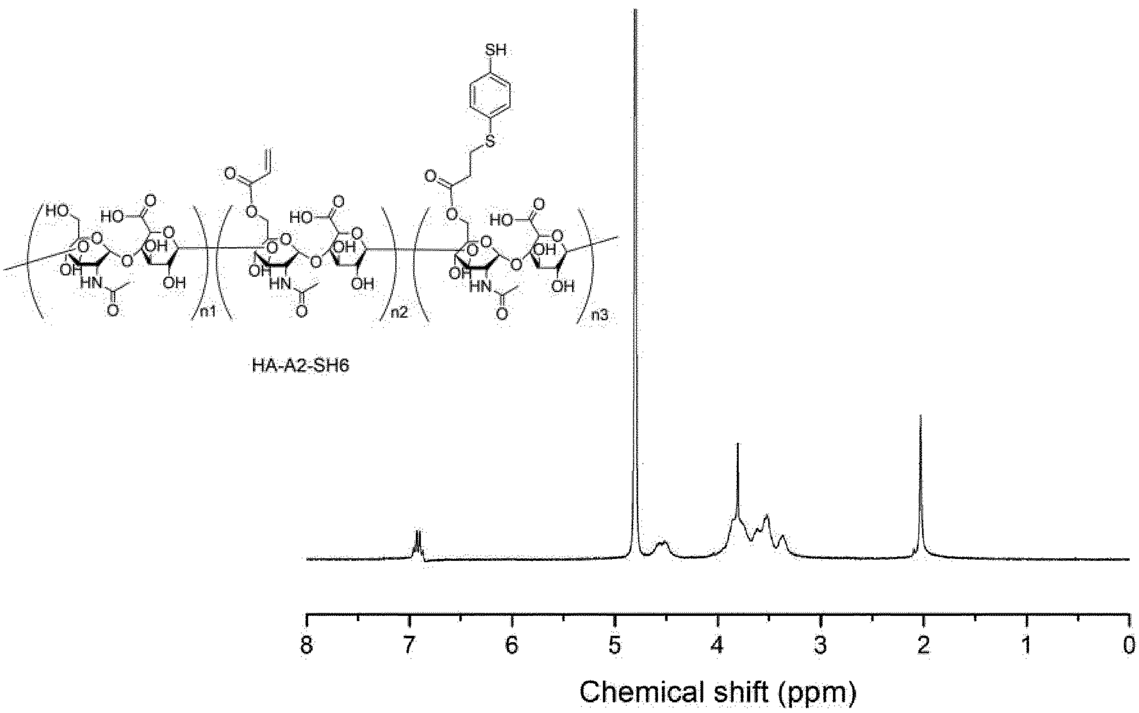
HA-A2-SH6
8 7 6 5 4 3 2 1 0
Chemical shift (ppm)

FIG. 50

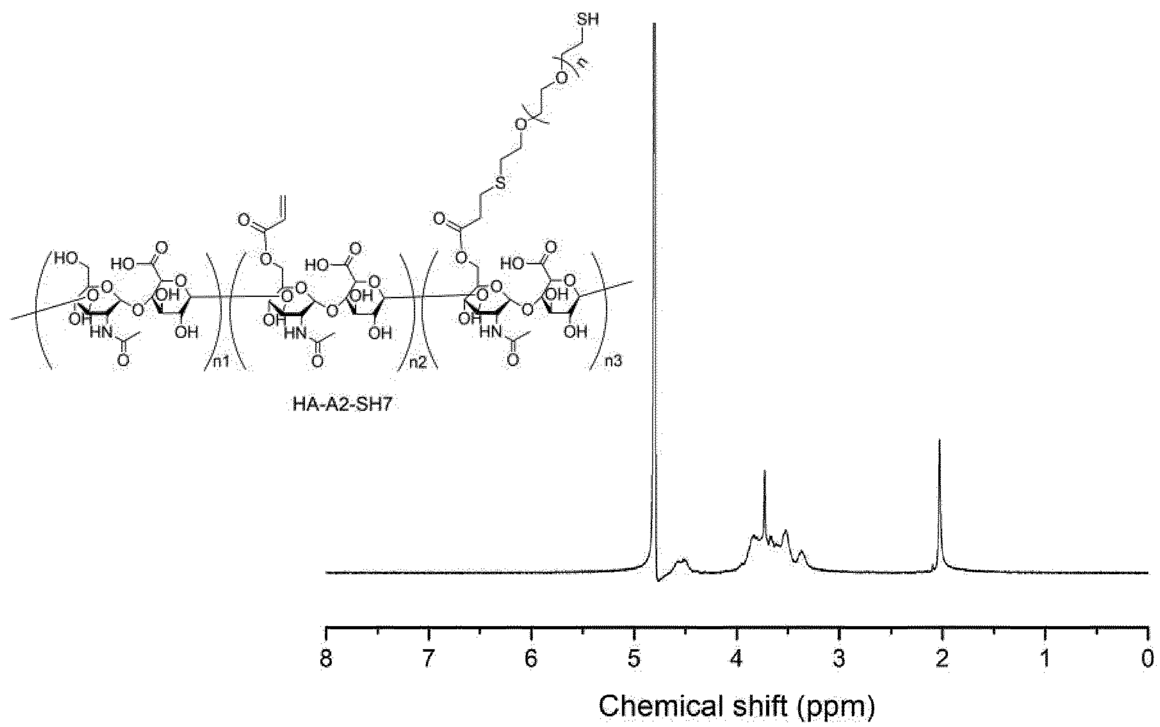


HA-A2-SH7

FIG. 51

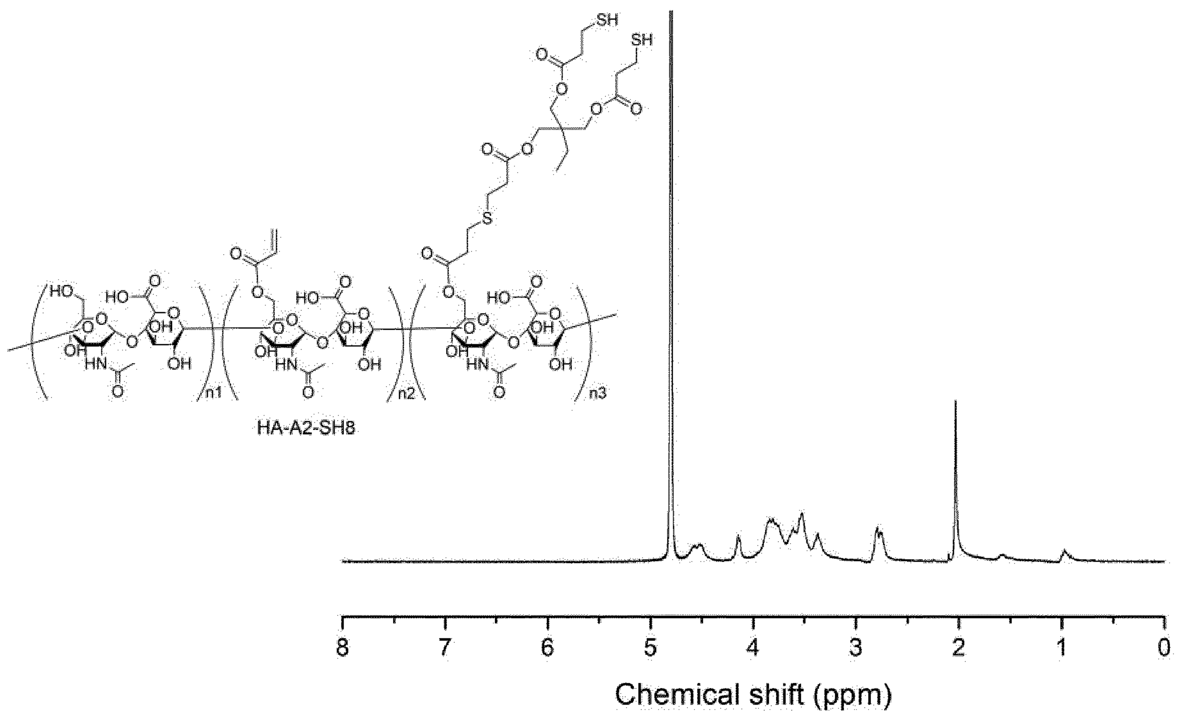


HA-A2-SH8

FIG. 52

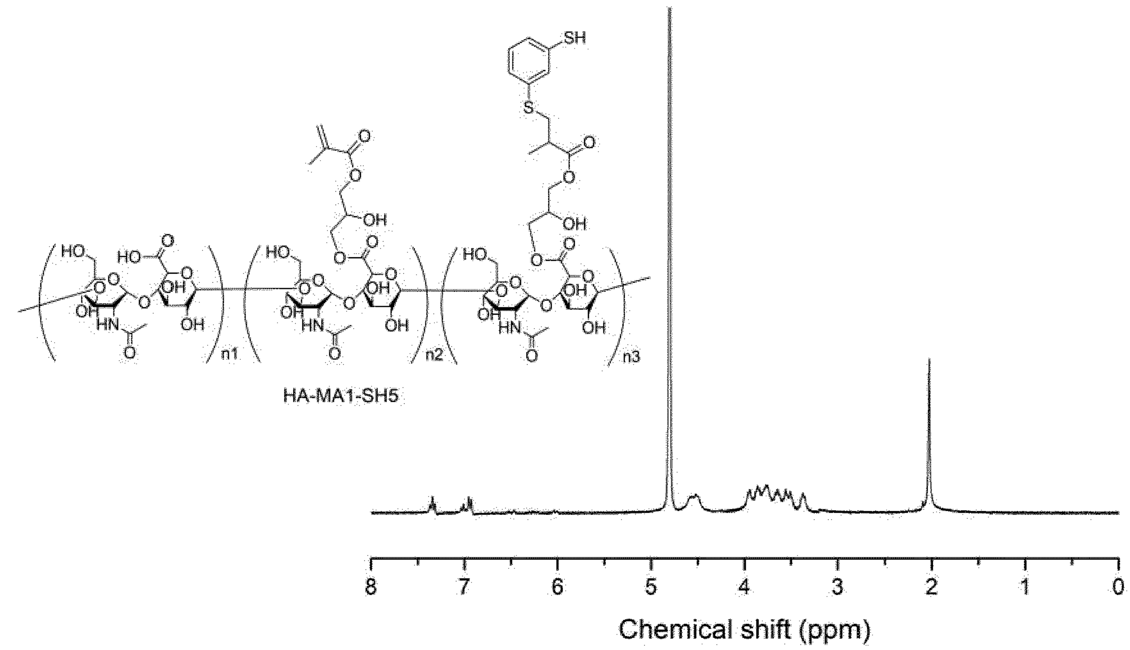
HA-MA1-SH5
8 7 6 5 4 3 2 1 0
Chemical shift (ppm)

FIG. 53

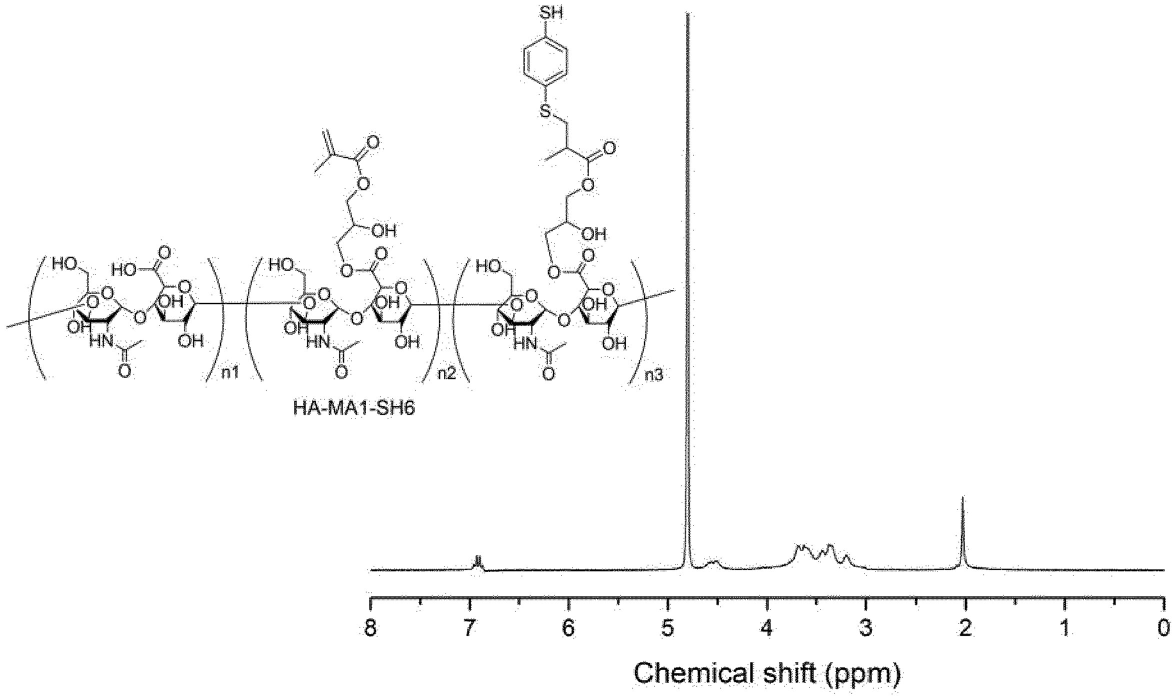
HA-MA1-SH6
8 7 6 5 4 3 2 1 0
Chemical shift (ppm)

FIG. 54

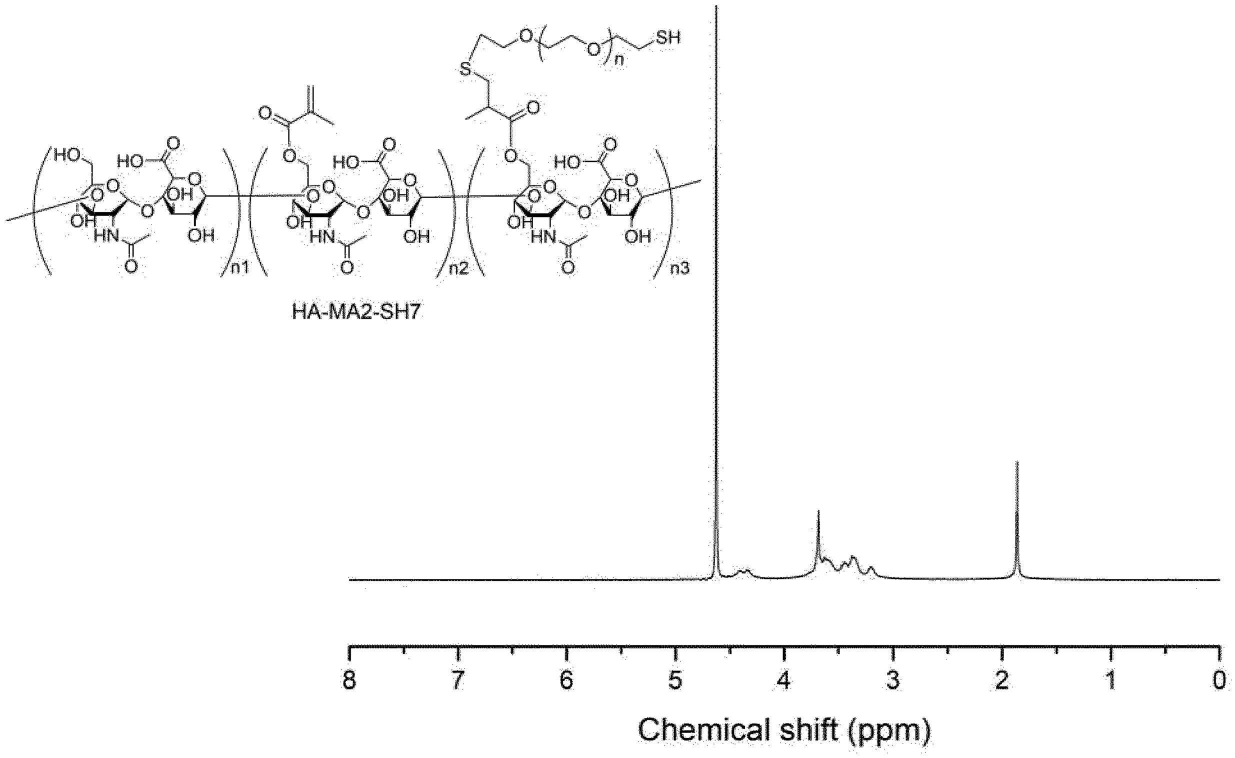


HA-MA2-SH7

FIG. 55

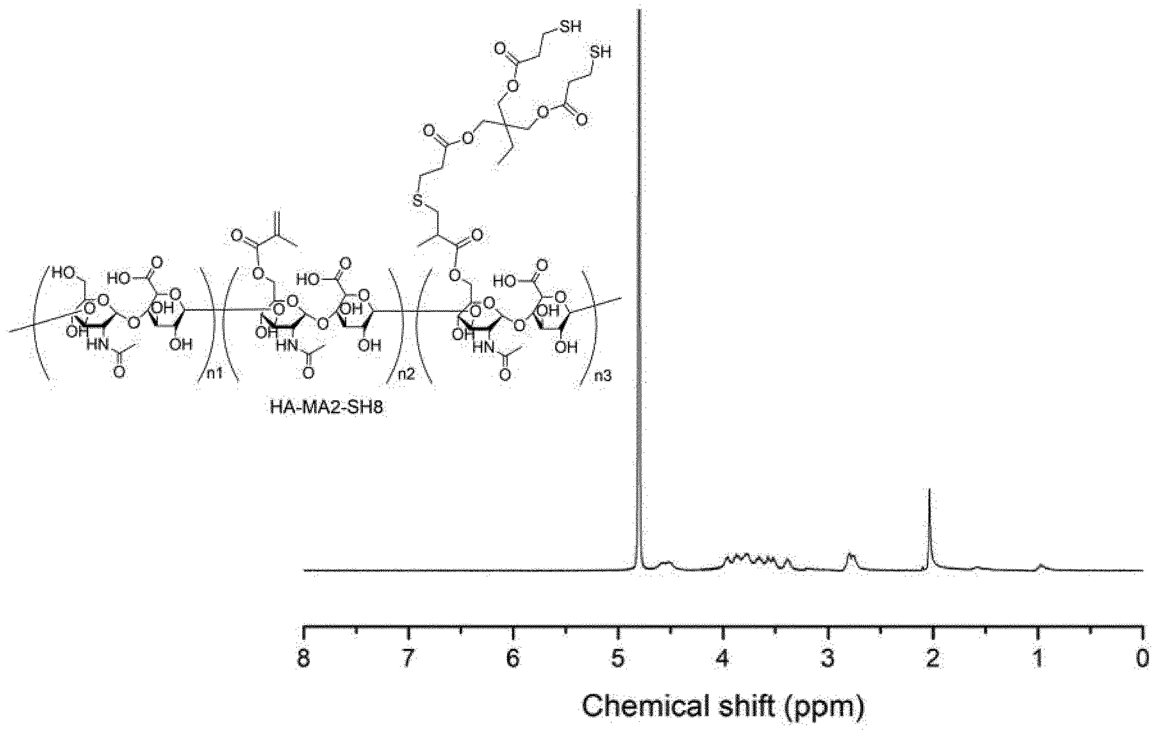


HA-MA2-SH8

FIG. 56

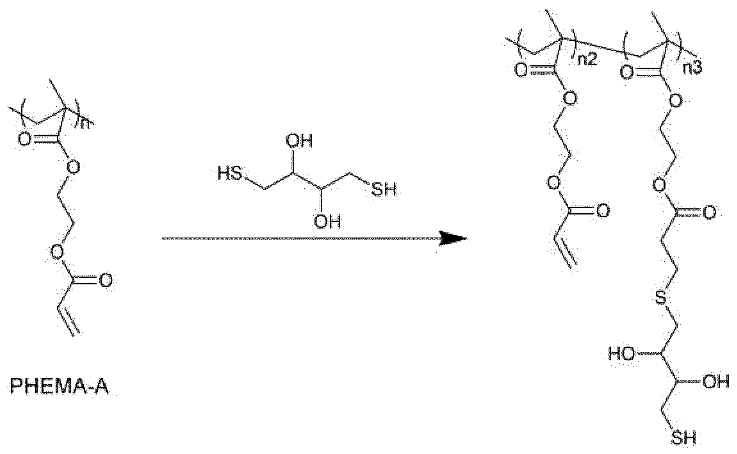

PHEMA-A

PHEMA-A-SH1

FIG. 57

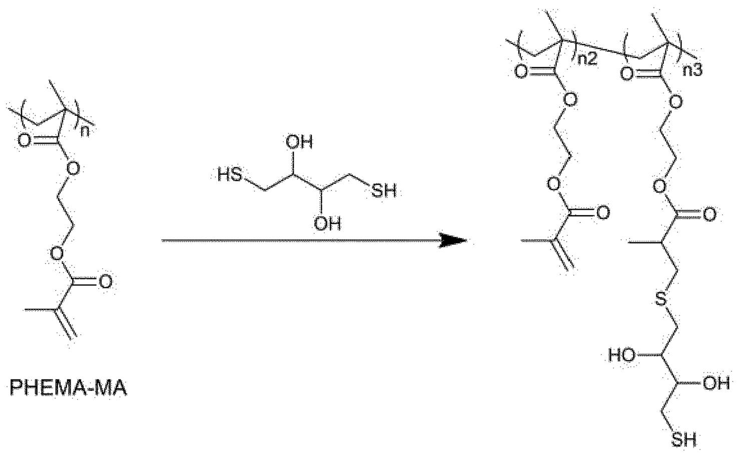

PHEMA-MA

PHEMA-MA-SH1

FIG. 58

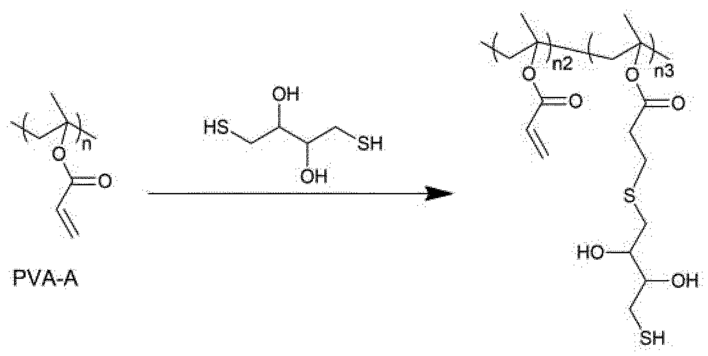

PVA-A

PVA-A-SH1

FIG. 59

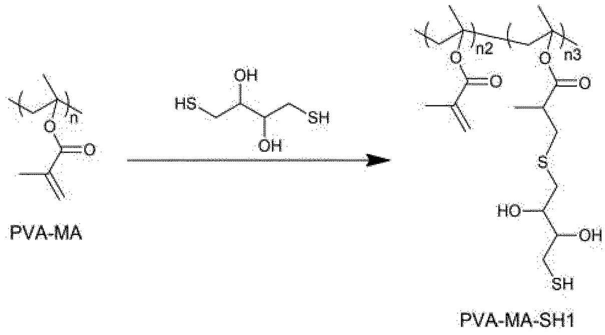


FIG. 60

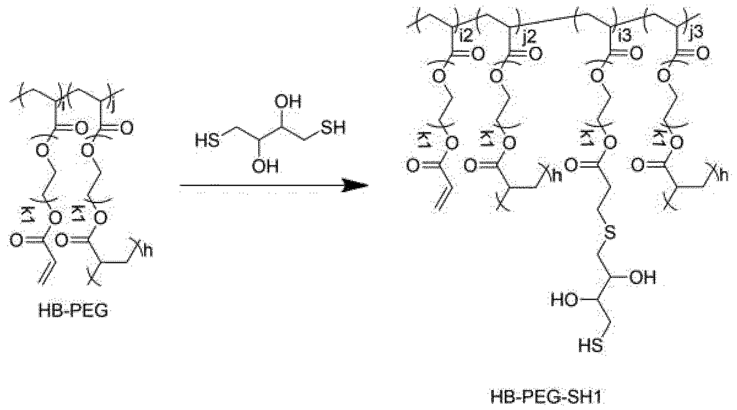


FIG. 61

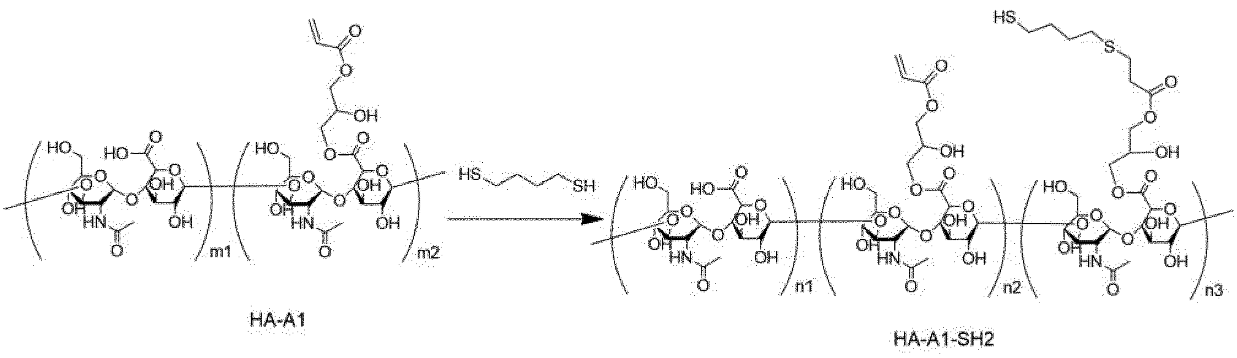


FIG. 62

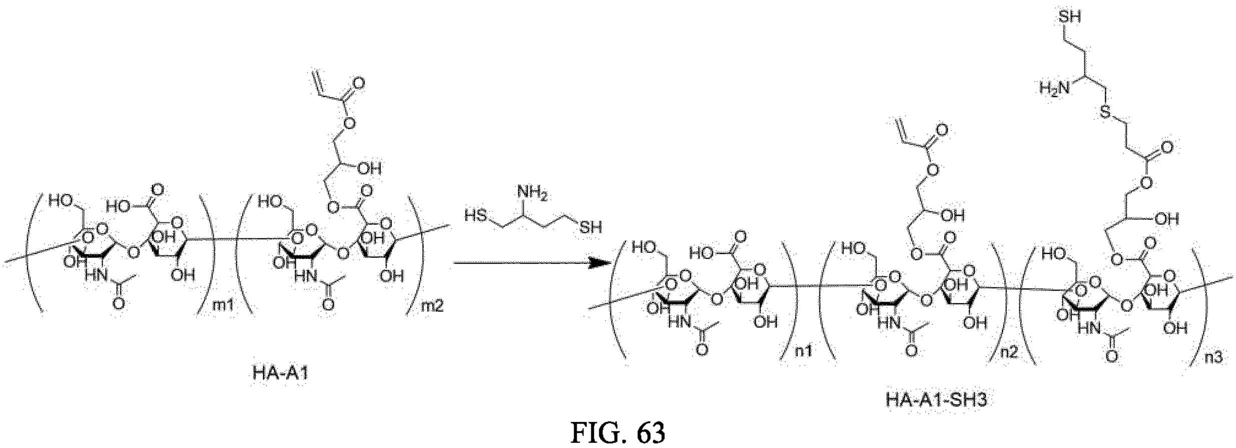


FIG. 63

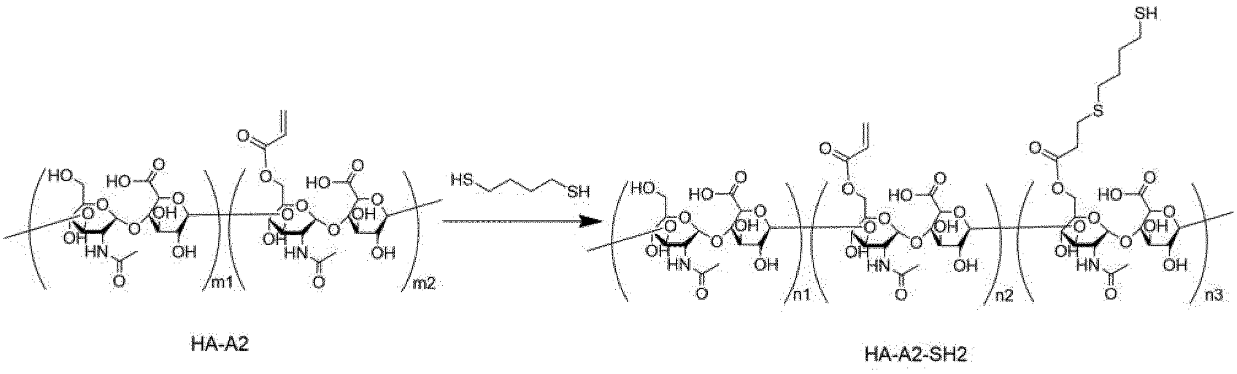

FIG. 64

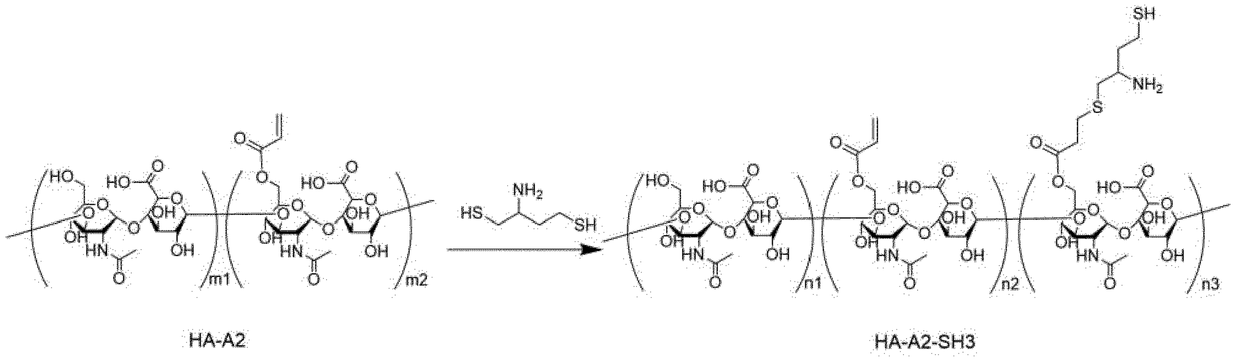

FIG. 65

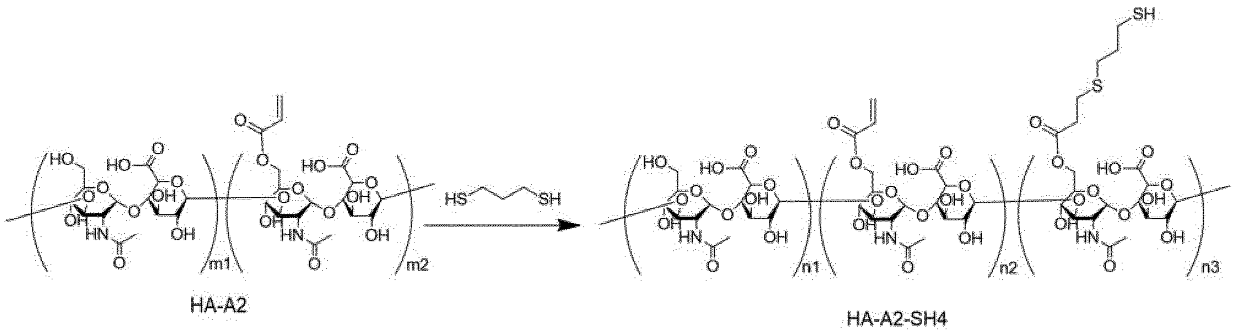

FIG. 66

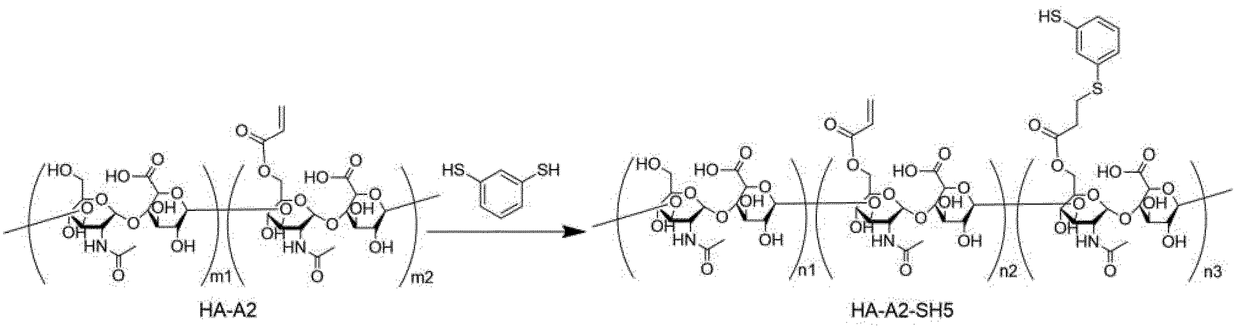

FIG. 67

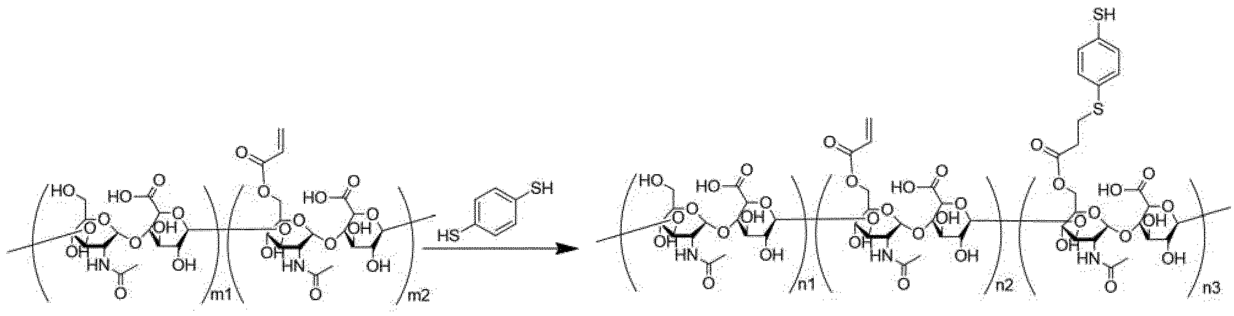

HA-A2 HA-A2-SH6

FIG. 68

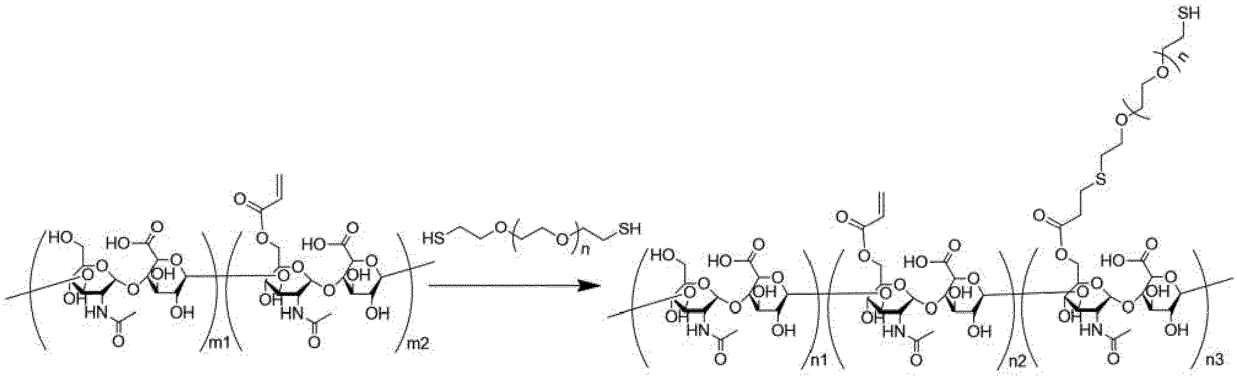

HA-A2 HA-A2-SH7

FIG. 69

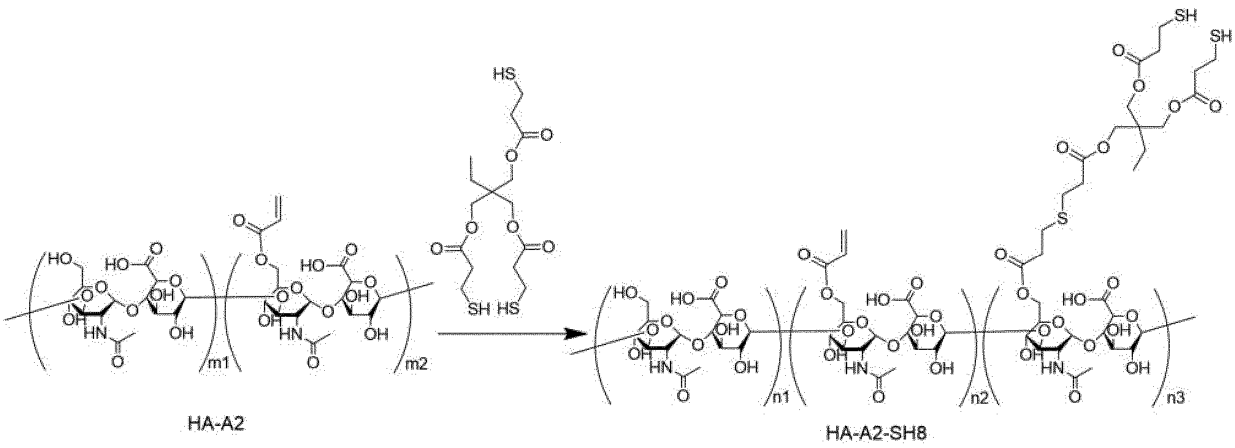

HA-A2 HA-A2-SH8

FIG. 70

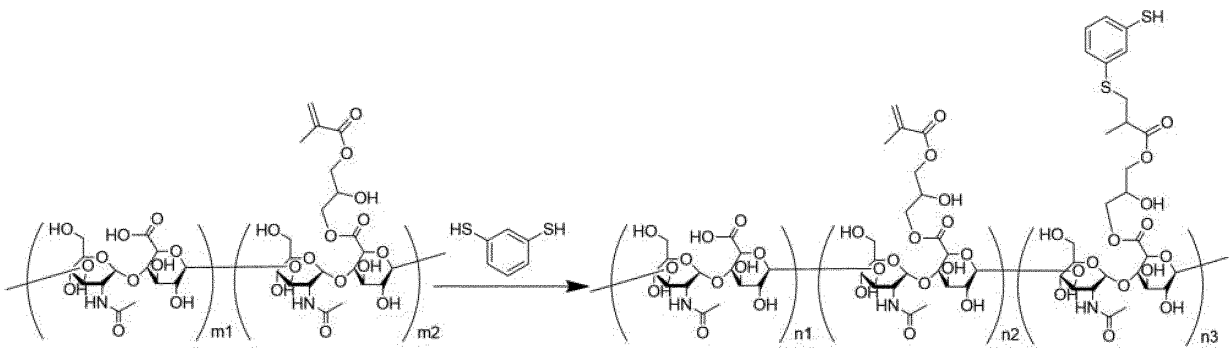

HA-MA1

HA-MA1-SH5

FIG. 71

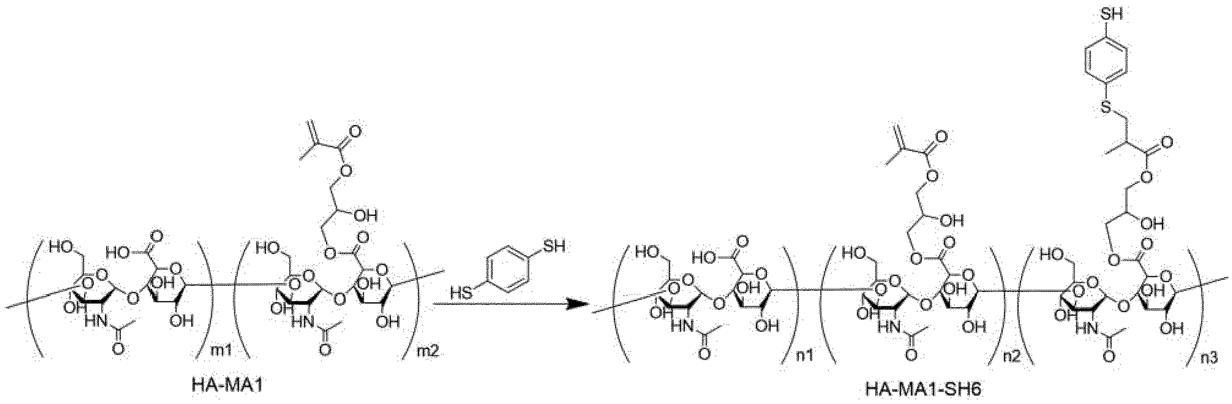

HA-MA1

HA-MA1-SH6

FIG. 72

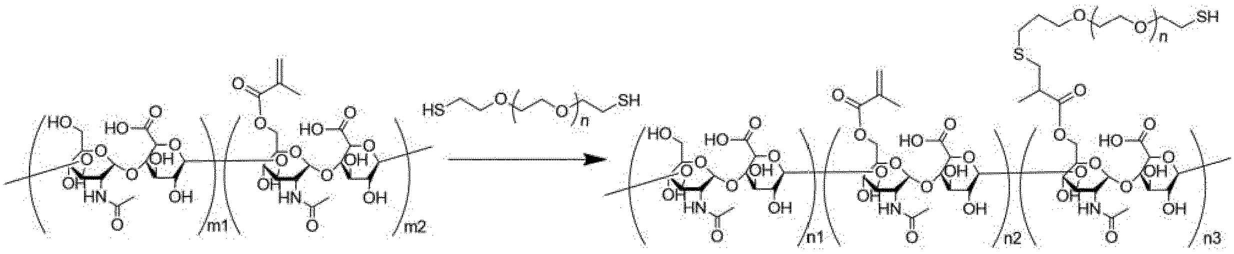

HA-MA2

HA-MA2-SH7

FIG. 73

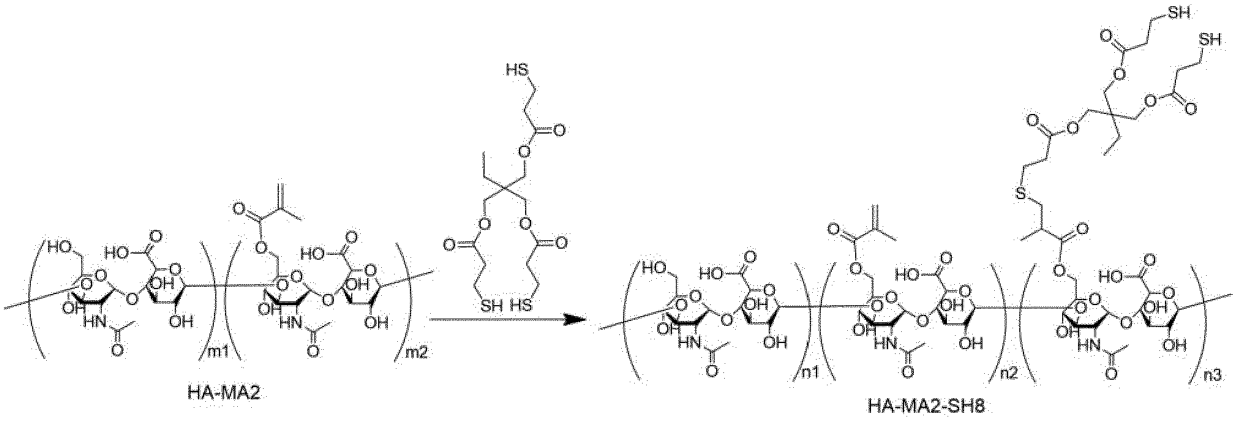

HA-MA2

HA-MA2-SH8

FIG. 74

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201911130069
- US 20170355799 A1

### Non-patent literature cited in the description

- Injectable hyaluronic acid microhydrogels for controlled release formulatioin of erythropoietin. *Journal of Biomadical Materials Research Part A,* 27 October 2006, 916-924